(19) Europäisches Patentamt
European Patent Office
Office européen des brevets

(11) Publication number: 0 042 734 B1

(12) EUROPEAN PATENT SPECIFICATION

(45) Date of publication of patent specification: 20.03.85

(21) Application number: 81302755.4

(22) Date of filing: 18.06.81

(51) Int. Cl.$^4$: C 11 B 9/00, A 23 L 1/226, A 24 B 15/30, A 61 K 7/00, C 07 C 31/125, C 07 C 31/28, C 07 C 47/02, C 07 C 49/203, C 07 C 69/14, C 07 C 69/145, C 07 C 69/24

(54) **Olefin dimers, derivatives thereof and organoleptic uses thereof.**

(30) Priority: 19.06.80 US 160788
04.09.80 US 184132
18.09.80 US 188576
09.10.80 US 195630
09.10.80 US 195672
04.12.80 US 212887
04.12.80 US 212993
05.02.81 US 231771
27.02.81 US 231771
05.02.81 US 231773
12.02.81 US 233861
09.04.81 US 252334

(43) Date of publication of application:
30.12.81 Bulletin 81/52

(45) Publication of the grant of the patent:
20.03.85 Bulletin 85/12

(84) Designated Contracting States:
CH DE FR GB LI NL

(56) References cited:
DE-C- 894 594
FR-A-2 391 986

(73) Proprietor: INTERNATIONAL FLAVORS & FRAGRANCES INC.
521 West 57th Street
New York New York 10019 (US)

(72) Inventor: Boden, Richard M.
150 Park Road
Monmouth Beach New Jersey 07750 (US)
Inventor: Dekker, Lambert
384 W. Shore Drive
Wyckoff New Jersey 07481 (US)
Inventor: Schmitt, Frederick Louis
49 McCampbell Road
Holmdel New Jersey 07735 (US)
Inventor: van Loveren, Augustinus G.
1 Brookside Way
Rye New York 10573 (US)
Inventor: Geiger, John H., Jr.
1187 Idalia Avenue
Lakewood New Jersey 08701 (US)

(74) Representative: Brown, John David et al
FORRESTER & BOEHMERT Widenmayerstrasse 4/I
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 042 734 B1

Courier Press, Leamington Spa, England.

(56) References cited:
GB-A-1 227 244
US-A-3 510 536
US-A-3 655 791
US-A-3 959 396

S.ARCTANDER: "Perfume and flavor chemicals". 1969,vol. I,II, no. 974,1031,1032,1494,1502,2145, Newark, New York USA.

**Description**

This invention relates to a process for augmenting or enhancing the aroma or taste of a consumable material, as well as to compounds having organoleptic utility.

FR—A—2,391,986 discloses the use of 2,3,5,5-tetramethylhexanal for augmenting or enhancing the aromas of perfume compositions, in particular floral perfume compositions, and states that 2,3,5,5-tetramethylhexanal may be prepared by the oxidation, using chromic acid, or the catalytic dehydrogenation of the corresponding alcohol, 2,3,5,5-tetramethylhexanol.

It has been determined that certain diisoamylene compounds or derivatives are capable of imparting or enhancing a variety of fragrances and flavors to various consumable materials. These diisoamylene compounds or derivatives are either in the alternative or in combination:

A. one or more diisoamylenes

B. one or more diisoamylene epoxides

C. one or more acylated diisoamylenes, of which the Ketones defined according to the structure

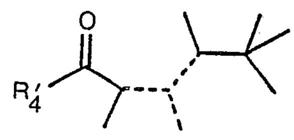

wherein R4′ represents C1—C3 lower alkyl and wherein one of the dashed lines represents a carbon-carbon double bond and the other dashed lines represent carbon-carbon single bonds, do not form a part of the present invention

D. one or more alcohol moiety-containing reduction products of one or more acylated diisoamylenes

E. one or more ketal or thioketal derivatives of one or more acylated diisoamylenes

F. one or more oxo reaction products of one or more diisoamylenes

G. one or more alkyl metallo derivatives of one or more acylated diisoamylenes

H. one or more hydrolysis products of one or more alkyl metallo derivatives of one or more acylated derivatives of one or more diisoamylenes, or

I. compounds defined according to the structure

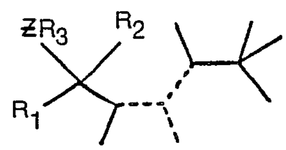

wherein z represents oxygen or sulfur; R1 and R2 represent C1—C4 alkyl; R3 represents hydrogen, C1—C3 lower alkyl or C1—C3 lower acyl; with the proviso that when Z is oxygen, R3 is not hydrogen; wherein one of the dashed lines represents a carbon-carbon double bond and the other dashed lines represent carbon-carbon single bonds.

Specifically, the "consumable materials" which are augmented or enhanced are in the alternative one or more perfume compositions, one or more perfumed articles, one or more colognes, one or more foodstuffs, one or more chewing gums, one or more toothpastes, one or more medicinal products, one or more chewing tobaccos, one or more smoking tobacco compositions, or one or more smoking tobacco articles.

More specifically, the diisoamylenes as set forth above are actually dimers of isoamylene produced according to the reaction:

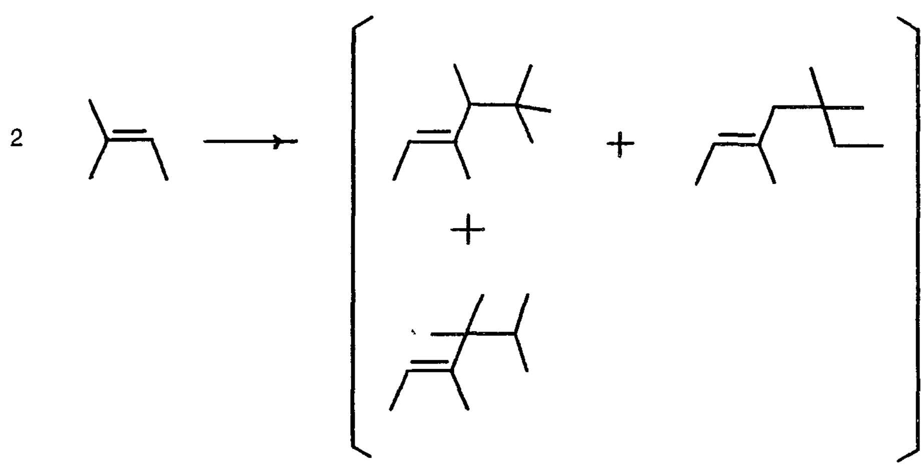

The diisoamylene epoxides mentioned above are epoxides of the foregoing dimers of isoamylene and are produced according to the following reaction sequence:

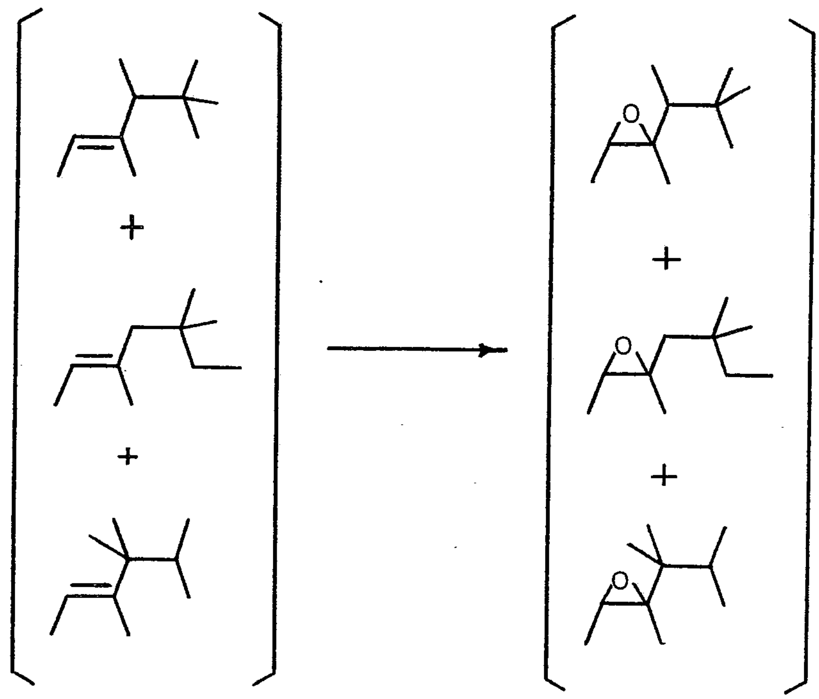

The acylated diisoamylenes are produced according to the reaction:

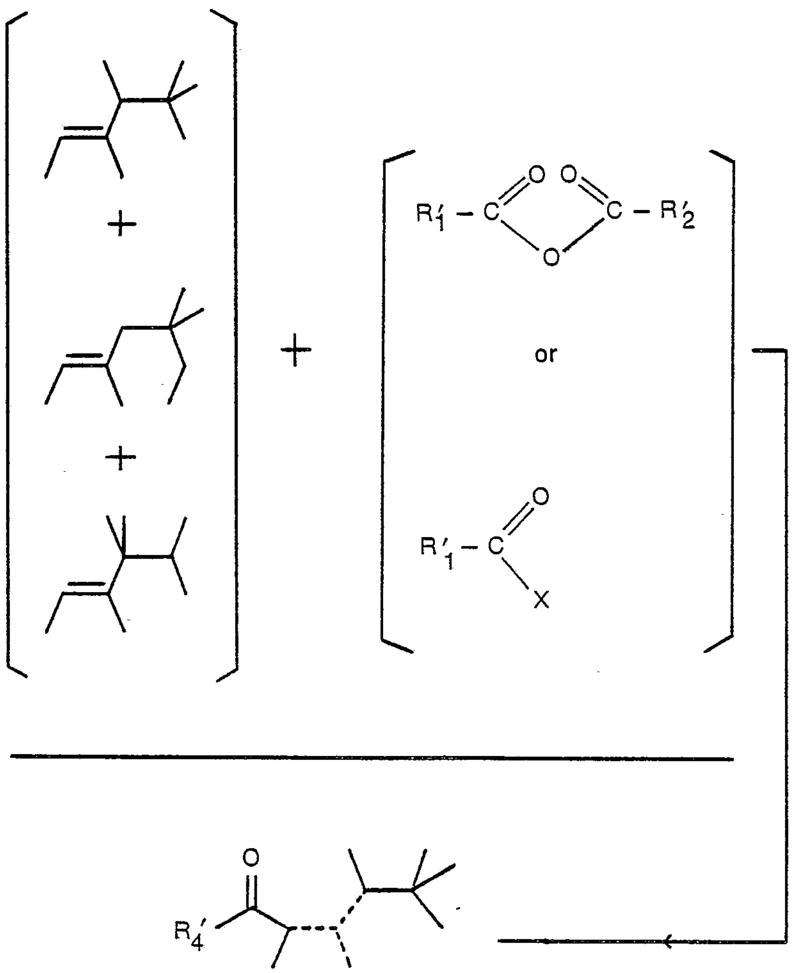

wherein $R_4'$ is $C_1$—$C_4$ alkyl; $R_1'$ and $R_2'$ are the same or different $C_1$—$C_4$ lower alkyl and $R_3'$ is $C_1$—$C_4$ lower alkyl with $R_4'$ being $R_1'$, $R_2'$ or $R_3'$. The ketones in which $R_4'$ is $C_1$—$C_3$ lower alkyl from the subject of, and are claimed in our European Patent Application No. 81301859.5 filed 28.04.81 (published on 17.03.82 under No. 0047572). These acylated diisoamylenes may be further reacted to form other valuable compounds of this invention in the following manner:

Firstly, the ketones may be reacted to form compounds having the generic structure:

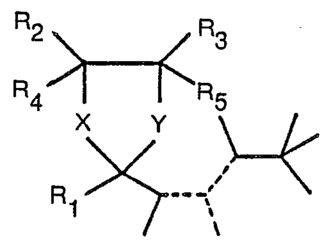

in accordance with the reaction sequence:

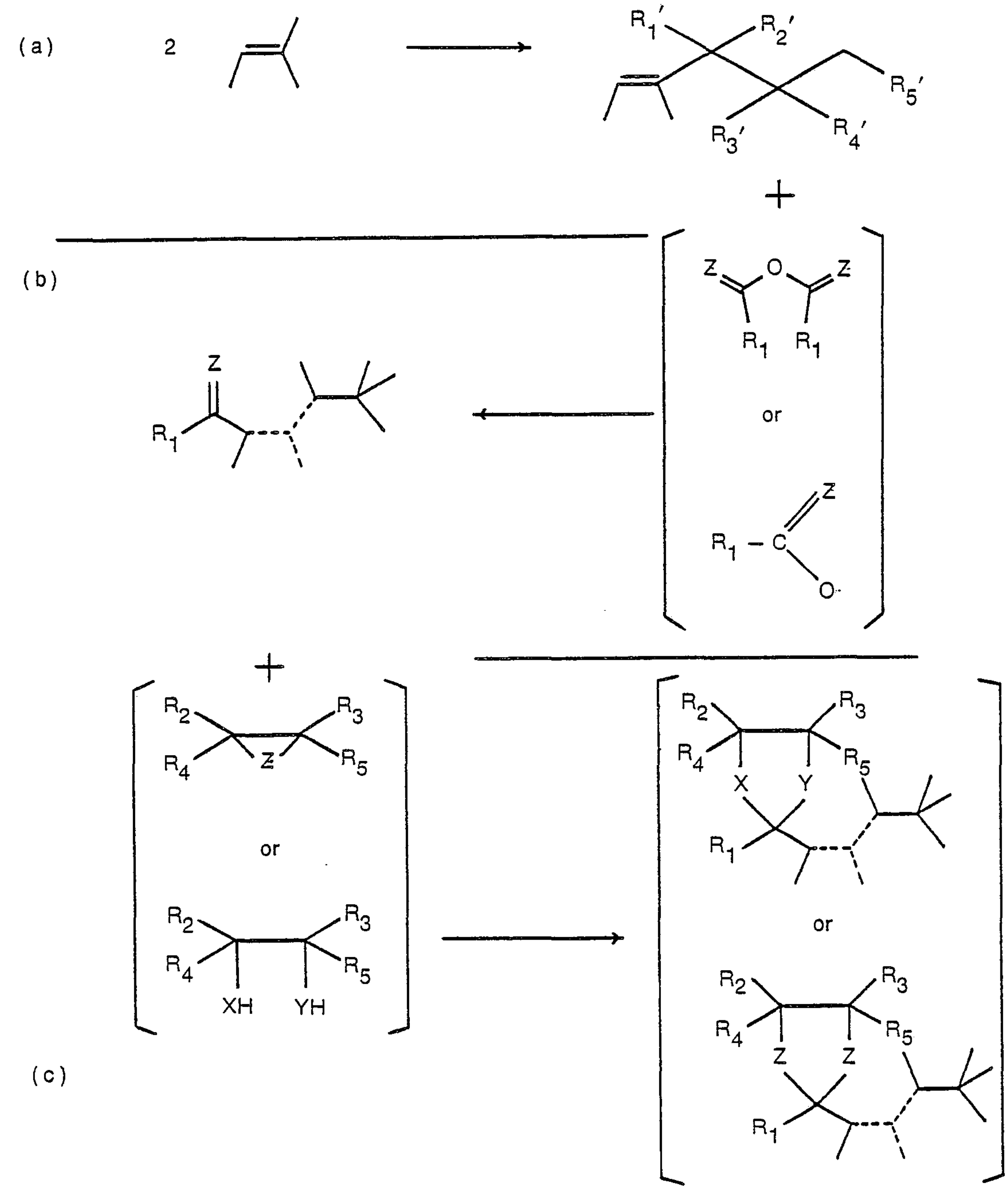

wherein $R_1$ represents $C_1$—$C_3$ lower alkyl; $R_2$, $R_3$, $R_4$ and $R_5$ represent the same or different hydrogen or $C_1$—$C_3$ lower alkyl; X and Y represent the same or different oxygen or sulfur; and one of the dashed lines represents a carbon-carbon double bond and the other dashed lines represent carbon-carbon single bonds and wherein Z represents one of X or Y and wherein $R_1'$, $R_2'$, $R_3'$, $R_4'$ and $R_5'$ represent hydrogen or methyl with the provisos that (i) at least one of $R_1'$ and $R_2'$ represents methyl when $R'_5$ is hydrogen; (ii) at least one of $R_3'$ and $R_4'$ represents methyl; (iii) the sum of the carbon atoms in $R_1'$, $R_2'$, $R_3'$, $R_4'$ and $R_5'$ is three and (iv) $R_1'$ and $R_2'$ represent hydrogen when $R_5'$ is methyl.

Secondly, such ketones may be reacted with an organometallic compound such as a Grignard reagent, and the resultant organometallic compound may be hydrolyzed and, optionally, further reacted. The resulting alcohol or alcohol derivative may be used for its organoleptic properties. Such compounds have the generic structure:

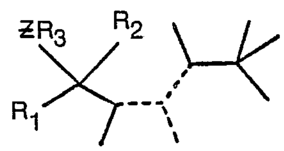

wherein $R_1$, $R_2$, $R_3$ and Z are defined as above, and such compounds may be produced according to the reaction sequence:

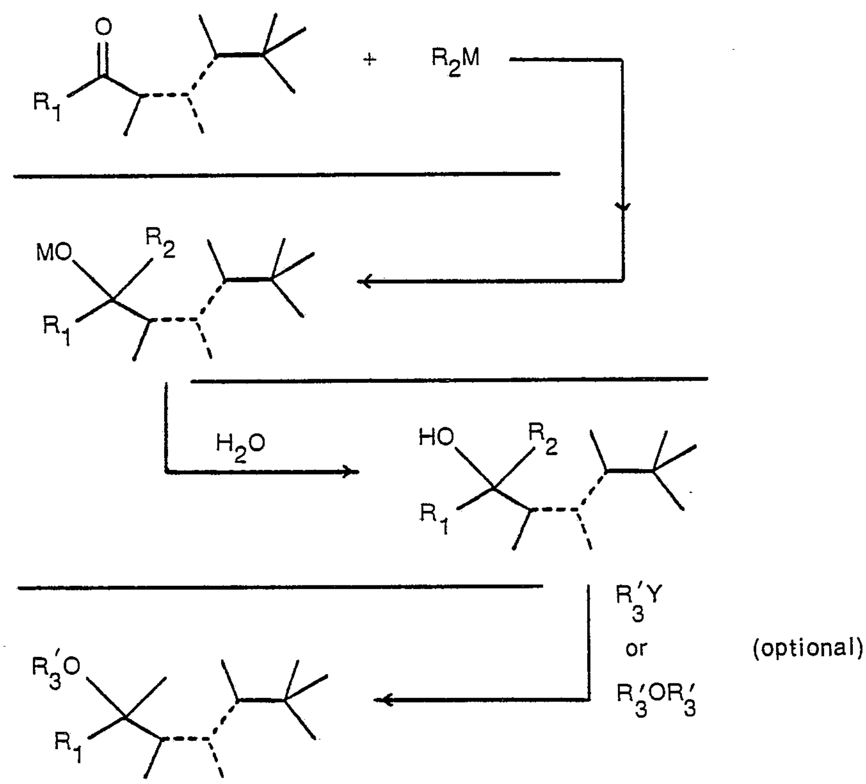


Additionally, the ketones may be directly reduced as by means of hydrogenation or a reducing agent such as an alkali metal borohydride or aluminum isopropylate or lithium aluminum hydride to form the compounds defined according to the structure:

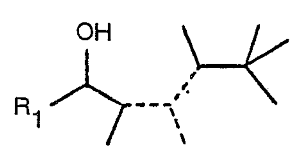

according to the reaction:

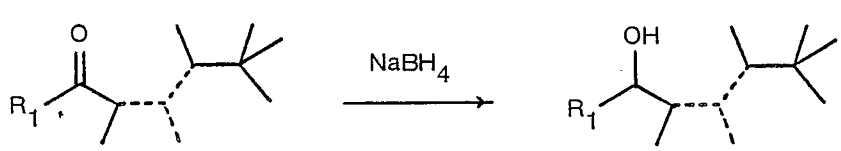

Such ketones can also be reacted with carbon monoxide and hydrogen via a "oxo" reaction to produce primary alcohols and aldehydes according to the reaction:

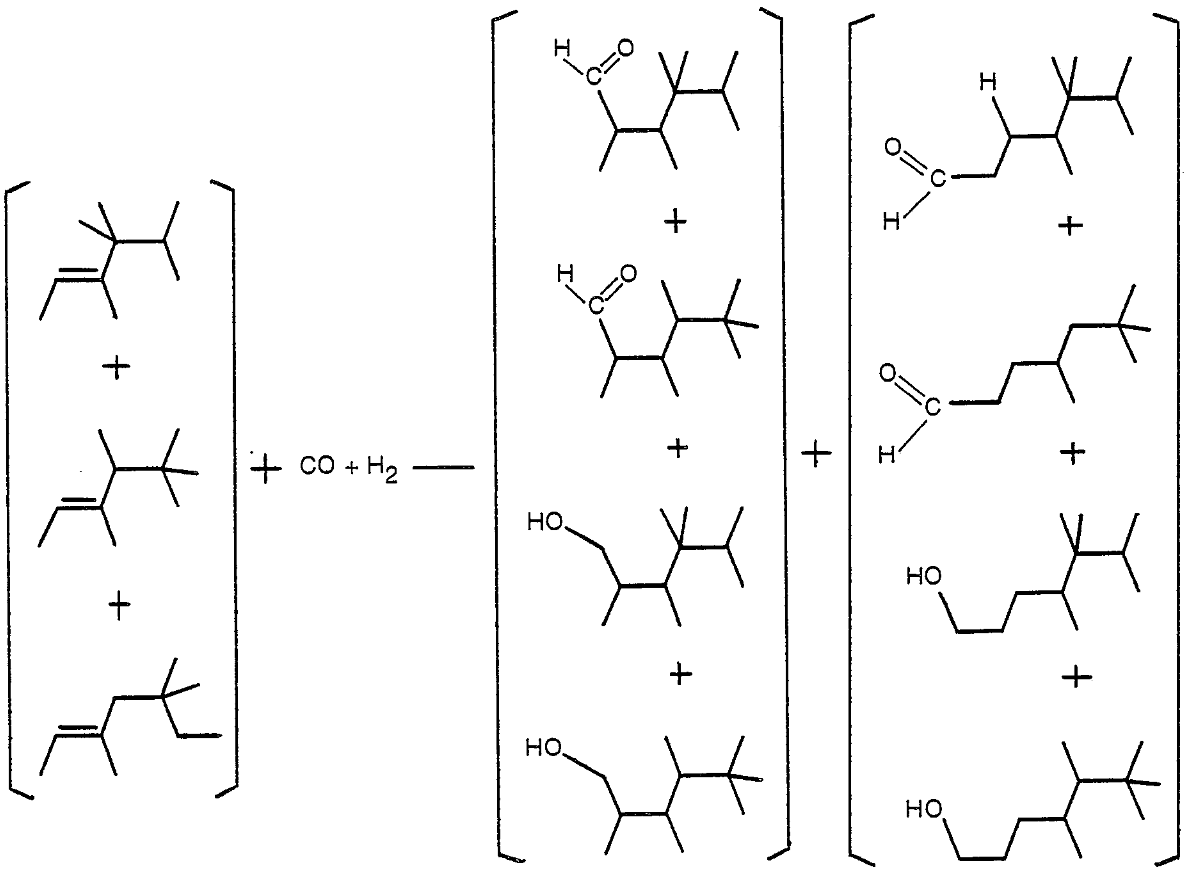


(for example), in order to form compounds having the structure:

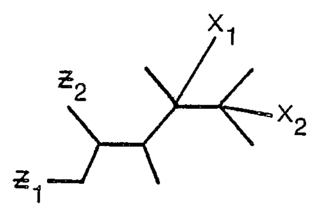

wherein one of $X_1$ or $X_2$ is hydrogen and the other of $X_1$ or $X_2$ is methyl and wherein one of $Z_1$ or $Z_2$ is hydroxymethyl having the structure:

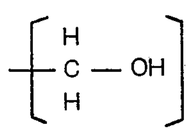

or carboxaldehyde having the structure:

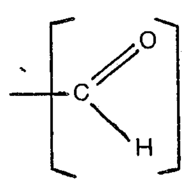

and the other of $Z_1$ or $Z_2$ is hydrogen.

The diisoamylene compounds or derivatives of our invention augment or enhance woody, piney,

herbaceous, eucalyptol, minty, sweet, rich, warm woody, fruity, amber, cognac-like, sandalwood-like, sweet floral, cedar-like, amber-like, sweet, patchouli, camphoraceous, fruity and orris-like aroma characteristics of perfumes, colognes and perfumed articles thereby causing one or more of said diisoamylene compounds or derivatives to be useful particularly in patchouli, pine and sandalwood fragrances.

It has further been discovered that novel solid and liquid foodstuff, chewing gum , medicinal products, toothpastes and chewing tobacco compositions and flavoring compositions therefor having woody, citrus, floral, ionone-like, incense-like, oriental, grapefruit-like, piney, fruity, rosin-like, sweet, minty, herbaceous, bread-like, caraway-like, dill, nutty, earthy, woody-balsamic, walnut kernel-like and walnut skin aroma and taste nuances may be provided by the utilization of one or more of the diisoamylene compounds or derivatives defined according to our invention.

It has further been discovered that novel solid and liquid tobacco and tobacco flavoring compositions having sweet, woody, oriental, camphoraceous, fruity, spicy, vetiver-like and cedar-like aroma and taste nuances both prior to and on smoking in the main stream and in the side stream may be provided by the utilization of one or more of the diisoamylene compounds or derivatives defined according to our invention.

It is also a part of our invention wherein sweet, minty, herbaceous, bread-like, caraway-like and dill-like aroma and taste nuances are provided by the utilization of the unsaturated branched ketones having the structure:

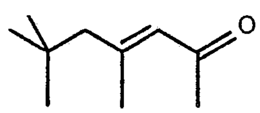

and /or

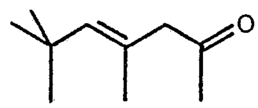

defined according to the genus:

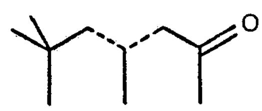

wherein one of the dashed lines represents a carbon-carbon double bond and the other dashed line represents a carbon-carbon single bond.

A first aspect of our invention contemplates augmenting or enhancing fragrances of such consumable materials as perfumes, perfumed articles (e.g., solid or liquid anionic, cationic, nonionic or zwitterionic detergents, cosmetic powders, fabric softener compositions and dryer-added fabric softener articles) and colognes by adding thereto a small, but effective, amount of at least one of the compounds defined according to the generic structure:

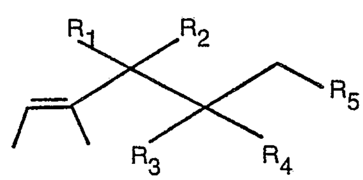

wherein $R_1$, $R_2$, $R_3$, $R_4$ and $R_5$ represent hydrogen or methyl with the provisos that (i) at least one or $R_1$ and $R_2$ represents methyl when $R_5$ is hydrogen; (ii) at least one of $R_3$ and $R_4$ represents methyl; (iii) the sum of the carbon atoms in $R_1$, $R_2$, $R_2$, $R_4$ and $R_5$ is three; and (iv) $R_1$ and $R_2$ represents hydrogen when $R_5$ is methyl.

More specifically, this first aspect of our invention concerns the use and synthesis of the structures of the compounds:

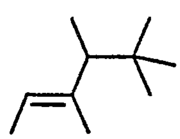

,

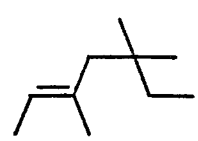

and /or

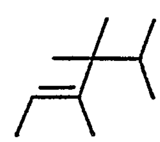

Insofar as this particular aspect of our invention is concerned, these diisoamylene derivatives having the structures:

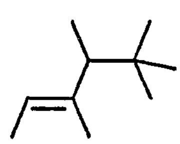

,

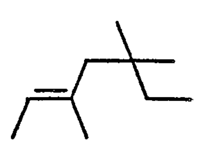

and /or

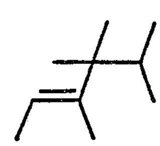

may be prepared by reacting 2-methyl-2-butene in the presence of an acidic catalyst which may be a Lewis acid such as zinc chloride, aluminum chloride, aluminum bromide, diethyl aluminum chloride, diethyl aluminum bromide, ethyl aluminum dichloride, and diethyl aluminum bromide, boron trifluoride, boron trifluoride etherate, or any of the other catalysts enumerated in the following references:

i — Murphy & Lane, Ind. Eng. Chem., Prod. Res. Dev., Vol. 14, No. 3, 1975 p. 167 (Title: Oligomerization of 2-Methyl-2-Butene in Sulfuric and Sulfuric-Phosphoric Acid Mixtures).

ii — Whitmore & Mosher, Vol. 68, J. Am. Chem. Soc., February, 1946, p. 281 (Title : The Depolymerization of 3,4,5,5-Tetramethyl-2-hexene and 3,5,5-Trimethyl-2-heptene in Relation to the Dimerization if Isoamylenes).

iii — Whitmore & Stahly, Vol. 67, J. Am. Chem. Soc., December, 1945, p. 2158 (Title: The Polymerization of Olefins. VIII The Depolymerization of Olefins in Relation to Intramolecular Rearrangements. ii).

iv — U.S. Patent 3,627,700, issued on December 14, 1971, (Zuech).

v — U.S. Patent 3,638,181, issued on November 3, 1970, (Banks).

vi — U.S. Patent 3,461,184, issued on August 12, 1969 (Hay, et al).

vii — Gurwitsch, Chemische Berichte, 1912, Vol. 2, p. 796 (Production of Di-isoamylene From Isoamylene Using Mercury Acetate Catalyst).

Depending upon the conditions of reaction, including temperature, pressure, mole ratio of 2-methyl-2-butene: catalyst concentration of 2-methyl-2-butene in solvent, concentration of catalyst in solvent and time of reaction, the ratio and nature of isomers will vary in an as yet undetermined fashion. In any event, this invention contemplates all isomers of diisoamylene defined according to the structures:

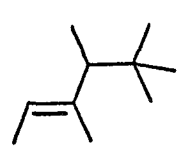
,
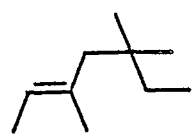
and / or
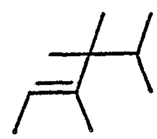

taken alone or in admixture in all proportions, when used in augmenting or enhancing the aroma of perfume compositions, perfumed articles and colognes.

A second aspect of our invention contemplates augmenting or enhancing fragrances of such consumable materials as perfumes, perfumed articles (e.g., solid or liquid anionic, cationic, nonionic or zwitterionic detergents, cosmetic powders, fabric softener compositions and dryer-added fabric softener articles) and colognes by adding thereto a small but effective amount of at least one of the compounds defined according to one of the structures:

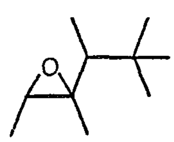
,
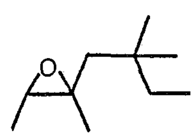
and / or
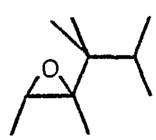

The diisoamylene epoxide compounds of our invention augment or enhance woody, eucalyptol and minty aroma characterists of perfumes, perfumed articles and colognes, thereby causing one or more of said diisoamylene epoxide compounds to be useful particularly in "eucalyptus" type fragrances. Furthermore, the diisoamylene epoxide compounds of our invention have unexpected and unobvious stability, particularly in the presence of strong oxidizing agents such as hypochlorite bleach solutions. Thus, the diisoamylene epoxide compounds of our invention can be used particularly to augment or enhance the aroma of perfumed bleach compositions, particularly perfumed hypochlorite bleach compositions.

Concerning this second aspect of our invention, the diisoamylene epoxide derivatives of our invention having the structures:

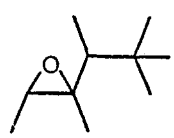
,
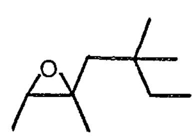
and / or
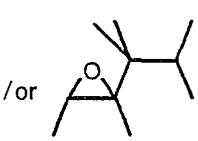

were defined according to the generic structure:

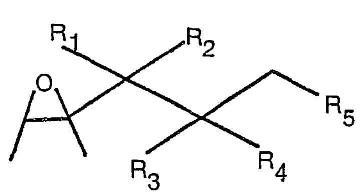

wherein $R_1$, $R_2$, $R_3$, $R_4$ and $R_5$ are the same or different and each represents hydrogen or methyl with the proviso that (i) the sum total of carbon atoms in $R_1$, $R_2$, $R_3$, $R_4$ and $R_5$ is three, and (ii) $R_1$ and $R_2$ represents hydrogen when $R_5$ represents methyl, and (iii) when either $R_1$ or $R_2$ is methyl, $R_5$ is hydrogen, may be prepared by first reacting 2-methyl-2-butene in the presence of an acidic catalyst which may be a Lewis acid such as zinc chloride, aluminum chloride, aluminum bromide, diethyl aluminum chloride, diethyl aluminum bromide, ethyl di-aluminum chloride and ethyl di-aluminum bromide, boron trifluoride, boron trifluoride etherate or any of the other catalysts enumerated in the references cited supra.

Then one or more of the compounds having the structures:

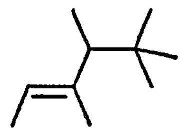 , 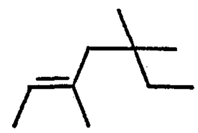 and /or 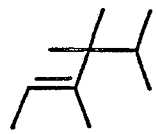

is then epoxidized using a suitable epoxidizing agent according to the conditions as set forth in either of U.S. Patents 3,896,180 issued on July 22, 1975 or U.S. Patent 3,723,478 issued on March 27, 1973.

Insofar as the second reaction to form the compounds having the structures:

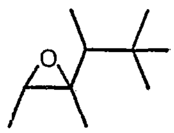 , 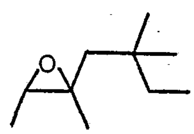 and /or 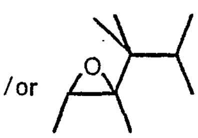

depends upon the conditions of reaction, including temperature, pressure, mole ratio of compounds having the structures:

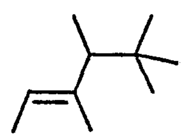 , 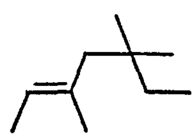 and /or 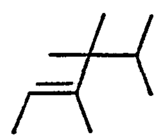

to catalyst, concentration of compounds having the structures:

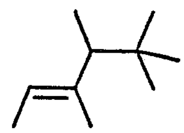 , 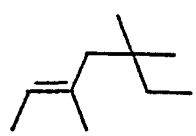 and /or 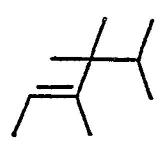

in solvent, concentration of catalyst, e.g., peracetic acid, perbenzoic acid or perphthalic acid in solvent and time of reaction, the ratio and nature of isomers having the structures:

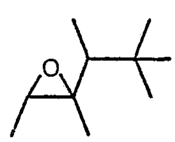 , 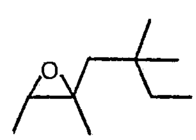 and /or 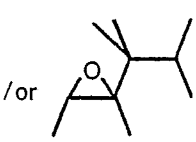

will vary in an as yet undetermined fashion. In any event, this invention contemplates the use of all isomers of the diisoamylene epoxide defined according to the generic structure:

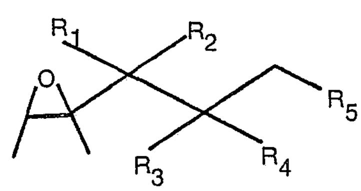

wherein $R_1$, $R_2$, $R_3$, $R_4$ and $R_5$ are the same or different and each represents hydrogen or methyl with the proviso that (i) the sum total of carbon atoms in $R_1$, $R_2$, $R_3$, $R_4$ and $R_5$ is three, and (ii) $R_1$ and $R_2$ represent hydrogen when $R_5$ represents methyl and (iii) when either $R_1$ or $R_2$ is methyl, $R_5$ is hydrogen, taken alone or in admixture in all proportions when used in augmenting or enhancing the aroma of perfume compositions, perfumed articles and colognes.

A third aspect of our invention contemplates augmenting or enhancing fragrances of articles which have been previously subjected to the action of aqueous hypochlorite bleaches by first admixing a potassium, sodium or lithium hypochlorite bleach solution (with which the said articles are to be ultimately

treated) with a small but effective amount of at least one of the compounds defined according to the generic structure:

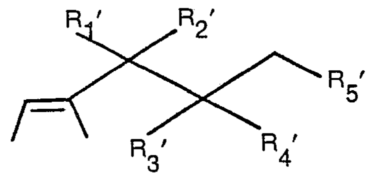

wherein $R_1'$, $R_2'$, $R_3'$, $R_4'$ and $R_5'$ are the same or different and each represents hydrogen or methyl with the provisos that (i) the sum total of the carbon atoms in $R_1'$, $R_2'$, $R_3'$, $R_4'$ and $R_5'$ is three; and (ii) $R_1'$ and $R_2'$ represents hydrogen when $R_5'$ represents methyl; and (iii) when either $R_1'$ or $R_2'$ is methyl, $R_5'$ is hydrogen.

More specifically in this third aspect of our invention, the diisoamylene compounds augment or enhance woody, piney and herbaceous aroma characteristics of articles previously treated with hypochlorite bleaches and also "cover" the aesthetically displeasing "hypochlorite-induced" aroma which is known to accompany such treated articles. Thus, in accordance with our invention, a hypochlorite bleach is admixed with an effective amount of diisoamylene derivative and in addition a stabilizing and emulsifying quantity of at least one compound defined according to the structure:

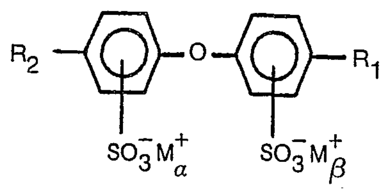

wherein at least one of $R_1$ or $R_2$ is $C_{10}$—$C_{12}$ branched or straight chain alkyl and when only one of $R_1$ or $R_2$ is $C_{10}$—$C_{12}$ straight chain or branched chain alkyl, the other of $R_1$ of $R_2$ is hydrogen and wherein $M_\alpha$ and $M_\beta$ are the same or different and each represent lithium, sodium or potassium.

A fourth aspect of our invention contemplates augmenting or enhancing fragrances of articles which have been previously subjected to the action of aqueous hypochlorite bleaches by first admixing a potassium, sodium or lithium hypochlorite bleach solution (with which the said articles are to be ultimately treated) with a small but effective amount of at least one of the compounds defined according the structure:

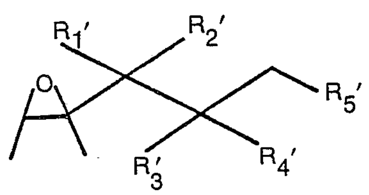

wherein $R_1'$, $R_2'$, $R_3'$, $R_4'$ and $R_5'$ are the same or different and each represents hydrogen or methyl with the provisos that (i) the sum total of the carbon atoms in $R_1'$, $R_2'$, $R_3'$, $R_4'$ and $R_5'$ is three; (ii) $R_1'$ and $R_2'$ represent hydrogen when $R_5'$ represents methyl and (iii) when either $R_1'$ or $R_2'$ is methyl, $R_5'$ is hydrogen.

The diisoamylene epoxide compounds of our invention augment or enhance woody, eucalyptol-like and minty aroma characteristics of articles previously treated with hypochlorite bleaches and also "cover" the aesthetically displeasing "hypochlorite-induced" aroma which is known to accompany such treated articles. Thus, a hypochlorite bleach in accordance with our invention is admixed with an effective amount of diisoamylene epoxide derivative and, in addition, a stabilizing and emulsifying quantity of at least one compound defined according to the structure:

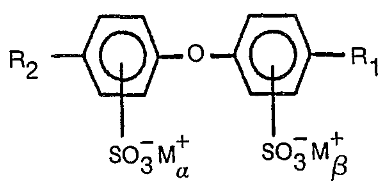

wherein at least one of $R_1$ or $R_2$ is $C_{10}$—$C_{12}$ branched or straight chain alkyl and when only one of $R_1$ or $R_2$ is $C_{10}$—$C_{12}$ straight chain or branched chain alkyl, the other of $R_1$ or $R_2$ is hydrogen and wherein $M_\alpha$ and $M_\beta$ are the same or different and each represent lithium, sodium or potassium.

A fifth aspect of our invention concerns the discovery that novel solid and liquid foodstuff, chewing gum, medicinal products, toothpastes and chewing tobacco compositions, and flavoring compositions therefor having woody, citrus, floral, ionone-like, incense-like, oriental, grapefruit-like, piney, fruity and rosin-like aroma nuances as well as woody, citrus, floral, ionone-like, oriental, grapefruit-like, piney, fruity

and rosin-like taste nuances with bitter undertones; as well as novel tobacco and tobacco flavoring compositions having sweet, woody, oriental, camphoraceous, fruity and spicy aroma and taste nuances prior to smoking, and woody, peppery and oriental-like aroma and taste nuances on smoking, in both the main stream and the side stream; as well as novel perfume compositions, colognes and perfumed articles (e.g., solid or liquid anionic, cationic, nonionic and zwitterionic detergents, cosmetic powders, hair preparations, fabric softener compositions and dryer-added fabric softener articles) having an intense and long-lasting sweet, rich, warm woody, fruity, amber, rum/cognac-like, sandalwood, sweet floral and vetiver-like aroma nuances with patchouli topnotes as well as minty undertones, may be provided by the utilization of one or more branched acyclic, unsaturated ketone derivatives, defined according to the generic structure:

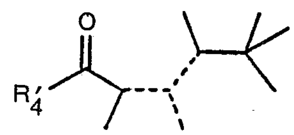

wherein $R_4'$ represents $C_1$—$C_4$ lower alkyl; wherein in each molecule, one of the dashed lines represents a carbon-carbon double bond and the other dashed lines represent carbon-carbon single bonds. Organoleptic uses of the said ketones wherein $R_4'$ is $C_1$—$C_3$ lower alkyl form the subject of, and are claimed in, our European Patent Application No. 81301859.5 filed 28.04.81 (published on 17.03.82 under No. 0047572).

Unless otherwise specified, representation herein of carbon-carbon double bonds are intended to indicate a "cis" isomer, a "trans" isomer, or a mixture of "cis" and "trans" isomers with respect to that carbon-carbon double bond in the event that the carbon-carbon double bond is susceptible of such "cis-trans" isomerism.

The novel branched, unsaturated ketones of our invention may be prepared by first preparing diisoamylene as by dimerizing isoamylene according to the reaction:

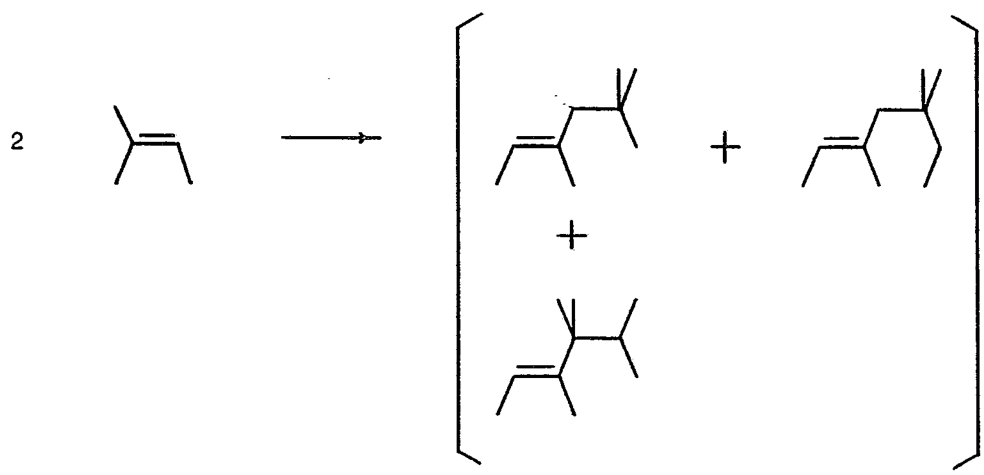

The resulting diisoamylene material thus produced, is used "as is" in the subsequent acylation reaction. Thus, for example, the subsequent acylation reaction may be carried out on the mixture of diisoamylene molecules according to the reaction:

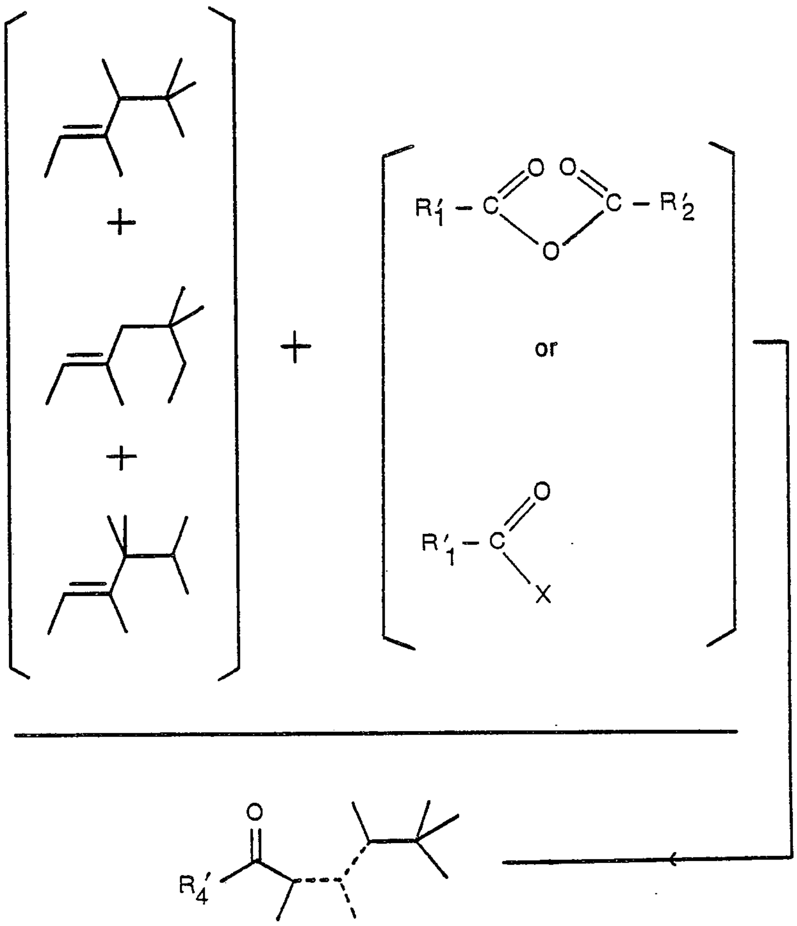


wherein the acylation is carried out using an organic acid anhydride or a mixed anhydride, or an acyl halide. In the foregoing reaction, $R_1'$, $R_2'$ and $R_3'$ represent the same or different $C_1$—$C_4$ alkyl, e.g., methyl, ethyl, n-propyl, i-propyl or i-butyl; $R_4'$ is $R_1'$, $R_2'$ or $R_3'$ and X represents chloro or bromo; and one of the dashed lines in the reaction product represents a carbon-carbon double bond, and each of the other dashed lines represent carbon-carbon single bonds (the reaction product in which $R_4'$ is methyl, ethyl, n-propyl or i-propyl is claimed in our European Patent Application No. 81301859.5 filed 28.04.81, published on 17.03.82 under No. 0047572, and does not *per se* form a part of the present invention). This reaction is carried out in the presence of an acid catalyst which may be either a Lewis acid or a mineral acid. When using Lewis acids such as boron trifluoride etherate, zinc chloride, aluminum chloride, zinc bromide, diethyl aluminum chloride, ethyl aluminum dichloride, stannic chloride or zinc bromide, the temperature of reaction may vary between 0°C and 80°C, with a preferred reaction temperature of between 10°C and 50°C. When using a mineral acid such as methane sulfonic acid, sulfuric acid, phosphoric acid, polyphosphoric acid or a mixture of methane sulfonic acid and phosphorous pendoxide, the temperature of reaction may vary between 25°C and 150°C with a preferred reaction temperature of between 40°C and 110°C.

The mole ratio of dimer of isoamylene:acylating agent (e.g., acyl anhydride) may vary between 1:1.1 and 2:1.1, with a preferable mole ratio of diisoamylene dimer:acylating agent being about 1:0.7. Various acyl anhydrides or acyl halides may be used, for example:

Acetic anhydride
Propionic anhydride
n-Butyric anhydride
i-Butyric anhydride
Acetic propionic anhydride
Acetic n-butyric anhydride
Acetic i-butyric anhydride
Propionic i-butyric anhydride
Propionic n-butyric anhydride
Acetyl chloride
Acetyl bromide
Isovaleryl chloride
Isovaleryl bromide
Propionyl chloride

The concentration of catalysts in the reaction mass may vary from 2.5 weight percent to 150 weight percent with a preferred concentration (Lewis acid or mineral acid) being between 5 and 10 percent by weight of the reaction mass.

Although an inert solvent may be used in the reaction mass (e.g., benzene, toluene, xylene, dichloromethane or 1,2-dichlorobenzene), it is preferred that no solvents be used, and that the reaction be carried out in the absence of solvent.

Although pressures greater than or less than atmospheric pressure may be used, no specific advantages are seen in using higher or lower pressures insofar as conversion, yield or time of reaction is concerned.

Accordingly, it is most preferred to use atmospheric pressure as a reaction condition.

The following table sets forth a specific reaction product contemplated within the scope of our invention, and its individual organoleptic properties; that is, insofar as smoking tobacco aroma and taste are concerned, food flavor is concerned, and perfume aroma is concerned.

TABLE A

| Structure of Reaction Product | Fragrance Properties | Foodstuff Flavor Properties | Smoking Tobacco Flavor & Aroma Properties |
|---|---|---|---|
| 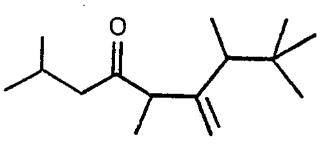 | A full-bodied long-lasting powerful, woody aroma | Guava-like aroma and taste | A tropical oriental aroma and taste both prior to and on smoking in the main stream and the side stream |

A sixth aspect of our invention involves the discovery that novel solid and liquid foodstuff, chewing gum, medicinal product and toothpaste compositions and flavoring compositions therefor having sweet, spicy, minty, herbaceous, bread-like, caraway-like and dill-like aroma and taste nuances may be provided by the utilization of one or both of the unsaturated branched ketones having the structures:

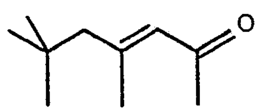 and /or 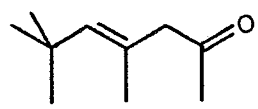

or defined according to the genus:

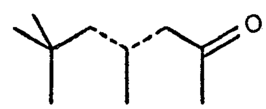

wherein one of the dashed lines represents a carbon-carbon double bond and the other dashed line represents a carbon-carbon single bond. These Ketones are added to said consumable material in the amount of from 0.02 ppm to about 50 ppm based on the total weight of said material.

The compounds having the structures:

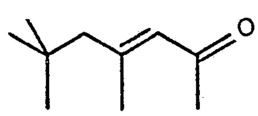 and /or 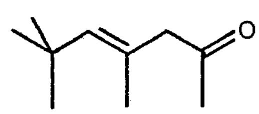

may be obtained by first dimerizing isobutylene according to the teachings of U.S. Patent 2,315,046 or according to the teachings of Japanese Kokai 79,157,510 published on December 12, 1979 (abstracted in Chem. Abstracts Volume 93:25885a) wherein the dimerization of isobutylene is indicated to take place in the presence of alkylaluminum chloride catalysts such as ethyl aluminum dichloride.

The resulting mixture of dimers of isobutylene may be separated or may be used as is in the next reaction wherein the dimer or mixture of dimers is acylated with either acetic anhydride or acetyl chloride or acetyl bromide in the presence of a catalyst such as boron trifluoride, boron trifluoride etherate, stannic chloride, ethyl aluminum dichloride or diethyl aluminum chloride according to, for example, the reaction:

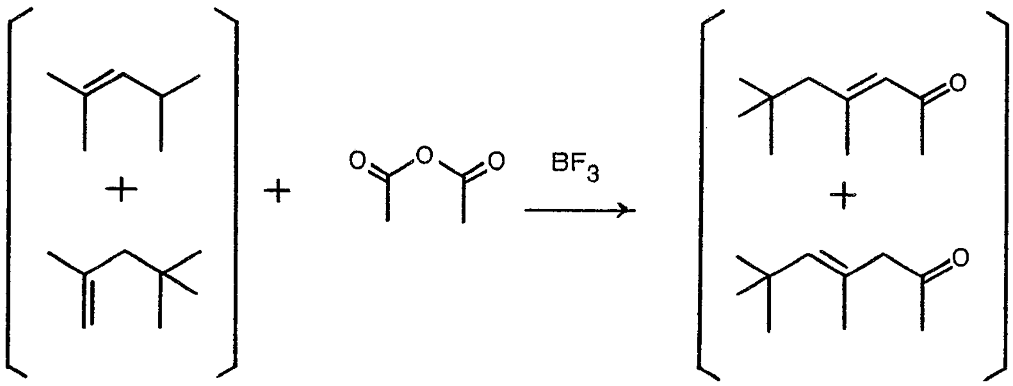


The conditions of the acylation are also taught in U.S. Patent 2,315,046 as well as in U.S. Patent 2,246,032.

A seventh aspect of our invention contemplates augmenting or enhancing fragrances of such consumable materials as perfumes, perfumed articles (e.g., solid or liquid anionic, cationic, nonionic or zwitterionic detergents, cosmetic powders, fabric softener compositions, dryer-added fabric softener articles, hair conditioners, and bleaching compositions including hypochlorite bleaches) and colognes by adding thereto a small but effective amount of at least one of the compounds defined according to the generic structure:

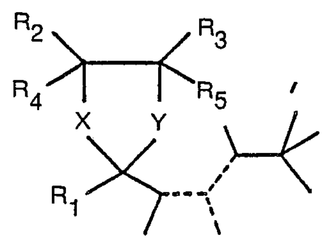

wherein $R_1$ represents $C_1$—$C_3$ lower alkyl; $R_2$, $R_3$, $R_4$ and $R_5$ represent the same or different hydrogen or $C_1$—$C_3$ lower alkyl; X and Y represent the same or different oxygen or sulfur; and one of the dashed lines represents a carbon-carbon double bond and the other dashed lines represent carbon-carbon single bonds.

Furthermore, this seventh aspect of our invention also contemplates augmenting or enhancing the aroma or taste of such consumable materials as foodstuffs, chewing gums, medicinal products, toothpastes and chewing tobaccos by adding thereto a small but effective amount of at least one of the compounds defined according to the generic structure:

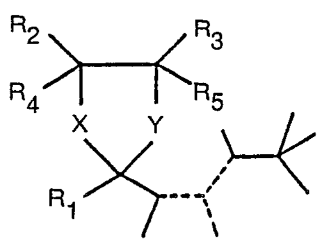

wherein $R_1$ represents $C_1$—$C_3$ lower alkyl; $R_2$, $R_3$, $R_4$ and $R_5$ represent the same or different hydrogen or $C_1$—$C_3$ lower alkyl; X and Y represent the same or different oxygen or sulfur; and one of the dashed lines represents a carbon-carbon double bond and the other dashed lines represent carbon-carbon single bonds.

Furthermore, this seventh aspect of our invention contemplates augmenting or enhancing the aroma or taste of smoking tobacco or smoking tobacco articles in both the main stream and the side stream both prior to and on smoking by adding to smoking tobacco or part of a smoking tobacco article such as a wrapper or filter a small but effective amount of at least one of the compounds defined according to the generic structure:

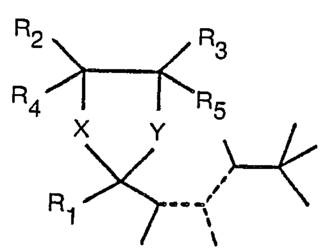

wherein $R_1$ represents $C_1$—$C_3$ lower alkyl; $R_2$, $R_3$, $R_4$ and $R_5$ represent the same or different hydrogen or $C_1$—$C_3$ lower alkyl; X and Y represent the same or different oxygen or sulfur; and one of the dashed lines represents a carbon-carbon double bond and the other dashed lines represent carbon-carbon single bonds.

The dioxolane, dithiolane and oxathiolane compounds of our invention augment or enhance pleasant cedar, amber-like, woody, sweet and patchouli-like aroma characteristics of perfumes, perfumed articles and colognes thereby causing one or more of said dioxolane, dithiolane or oxathiolane compounds to be useful particularly in patchouli-like fragrances. The great stability of such compounds particularly the dioxolane-type compounds causes them to be useful particularly in patchouli-fragranced hypochlorite type bleach compositions such as, for example CLOROX®.

The dithiolane, dioxolane and oxathiolane compounds of our invention also augment or enhance the sweet, woody, vetiver-like, and cedar-like aroma and taste nuances of black tobacco and cigar tobacco both prior to and on smoking in both the main stream and the side stream.

The dioxolane, dithiolane and oxathiolane compounds of our invention also augment or enhance the aroma or taste of foodstuffs, chewing gums, medicinal products, toothpastes and chewing tobaccos by imparting or enhancing the nutty, earthy, woody-balsamic, walnut kernel-like and walnut skin flavor and aroma nuances thereof.

The dithiolanes, dioxolanes and oxathiolane compounds of our invention defined according to the generic structure:

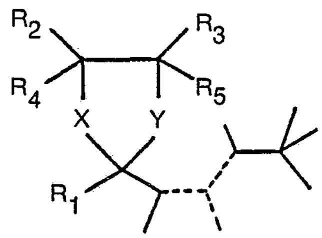

wherein $R_1$ represents $C_1$—$C_3$ lower alkyl; $R_2$, $R_3$, $R_4$ and $R_5$ represent the same or different hydrogen or $C_1$—$C_3$ lower alkyl; X and Y represent the same or different oxygen or sulfur; and one of the dashed lines represents a carbon-carbon double bond and the other dashed lines represent carbon-carbon single bonds, may be prepared by first preparing diisoamylene by means of dimerization of isoamylene having the structure:

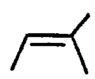

whereby the compounds having the structures:

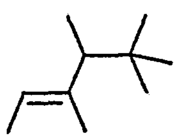 , 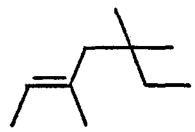 and 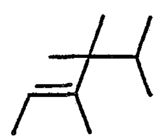

are formed. These compounds can also be defined according to the structure:

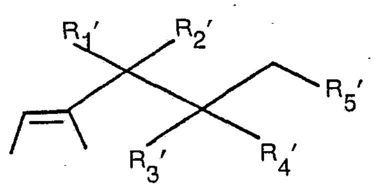

wherein $R_1'$, $R_2'$, $R_3'$, $R_4'$ and $R_5'$ represent hydrogen or methyl with the provisos that (1) at least one of $R_1'$ and $R_2'$ represents methyl when $R'_5$ is hydrogen, (ii) at least one of $R_3'$ and $R_4'$ represents methyl; (iii) the sum of the carbon atoms in $R_1'$, $R_2'$, $R_3'$, $R_4'$ and $R_5'$ is three and (iv) $R_1'$ and $R_2'$ represent hydrogen when $R_5'$ is methyl. These diisoamylene compounds may be prepared according to the procedures cited supra.

The resulting compounds are then acylated or thioacylated with an acylating agent such as an acylhalide or an acylanhydride or a thioacylhalide or a thioacylanhydride having the generic structure:

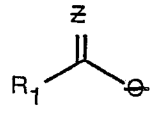 or 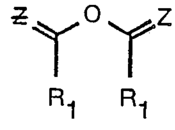

wherein Z represents oxygen or sulfur; wherein $R_1$ represents $C_1$—$C_3$ lower alkyl and wherein -θ- represents halogen which may be chloro or bromo, whereby a compound having the structure:

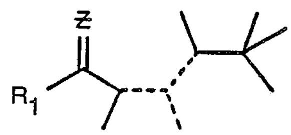

is formed wherein Z is one of oxygen or sulfur and one of the dashed lines is a carbon-carbon double bond and the other dashed lines in each of the molecules formed are carbon-carbon single bonds. Such compounds can be further defined according to one of the structures:

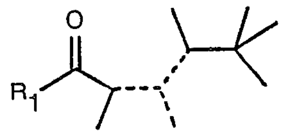

or

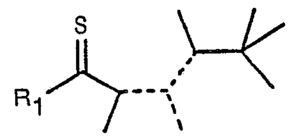

wherein $R_1$ is $C_1$—$C_3$ lower alkyl and one of the dashed lines in each of the molecules represents a carbon-carbon double bond and the other dashed lines in each of the molecules represent carbon-carbon single bonds.

These thiones or ketones, as the case may be, are then further reacted with either a thiirane, epoxide, glycol, dithiol or thiol-alcohol having one of the structures:

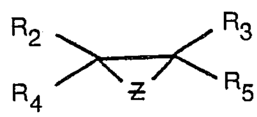

or

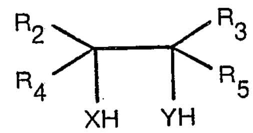

thereby forming a compound having the generic structure:

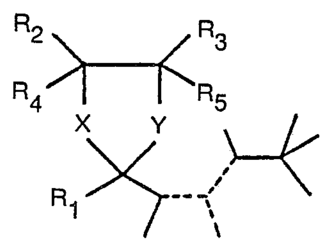

wherein $R_1$ represents $C_1$—$C_3$ lower alkyl; $R_2$, $R_3$, $R_4$ and $R_5$ represent the same or different hydrogen or $C_1$—$C_3$ lower alkyl; X and Y represent the same or different oxygen or sulfur; and one of the dashed lines represents a carbon-carbon double bond and the other dashed lines represent carbon-carbon single bonds.

The foregoing sequence of reactions is illustrated as follows:

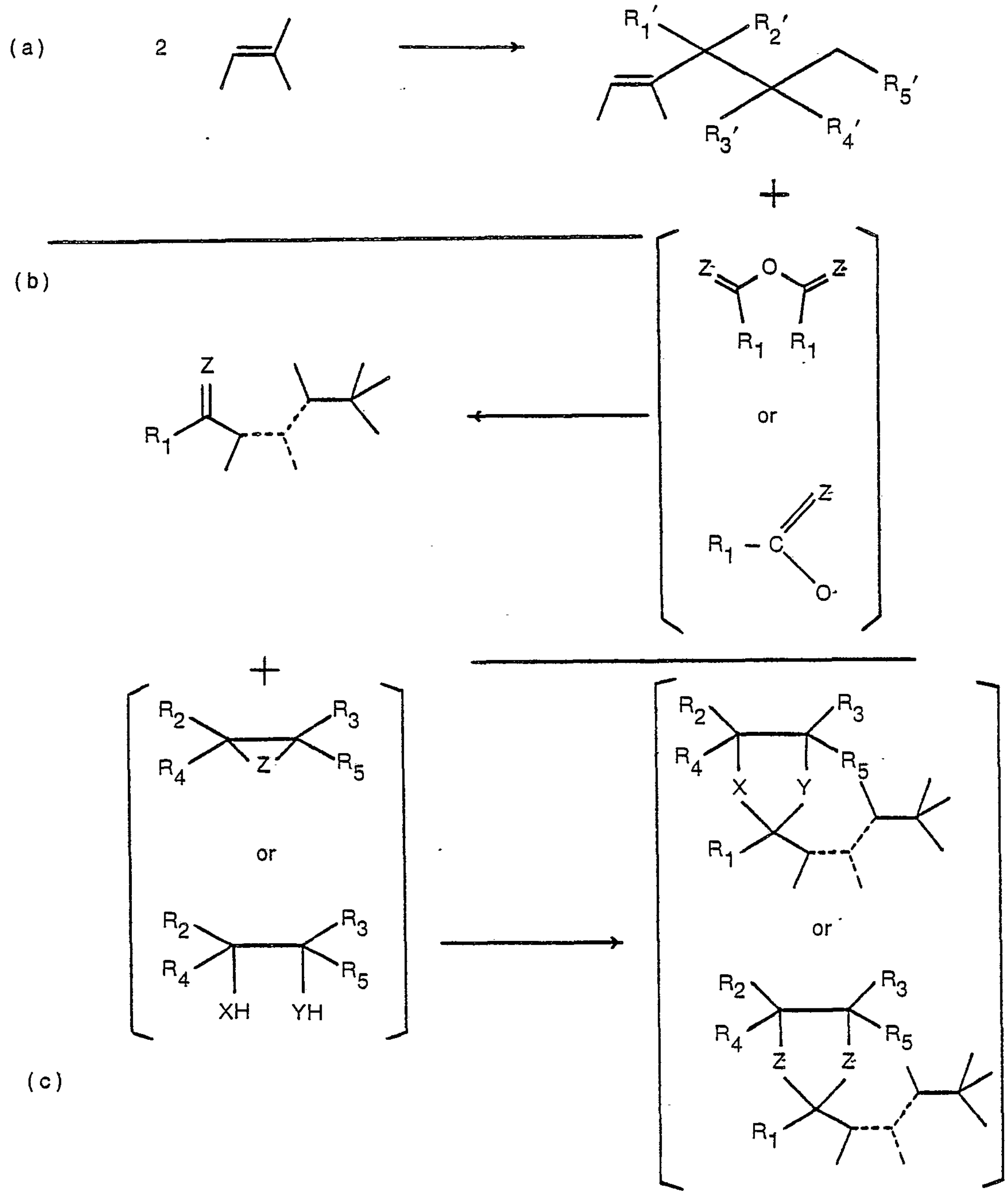

wherein $R_1$ represents $C_1$—$C_3$ lower alkyl; $R_2$, $R_3$, $R_4$ and $R_5$ represent the same or different hydrogen or $C_1$—$C_3$ lower alkyl; X and Y represent the same or different oxygen or sulfur; and one of the dashed lines represents a carbon-carbon double bond and the other dashed lines represent carbon-carbon single bonds and wherein Z represents one of X or Y and wherein $R_1'$, $R_2'$, $R_3'$, $R_4'$ and $R_5'$ represent hydrogen or methyl with the provisos that (i) at least one of $R_1'$ and $R_2'$ represents methyl when $R_5'$ is hydrogen; (ii) at least one of $R_3'$ and $R_4'$ represents methyl; (iii) the sum of the carbon atoms in $R_1'$, $R_2'$, $R_3'$ $R_4'$ and $R_5'$ is three and (iv) $R_1'$ and $R_2'$ represent hydrogen when $R_5'$ is methyl.

The compounds produced according to our invention may be in the form of mixtures of isomers; that is, mixtures of "cis" and "trans" isomers as well as mixtures of "stereoisomers". Thus, the compound having the structure:

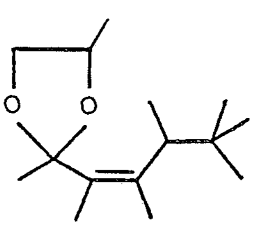

for example, may have the methyl group on the dioxolane ring either in "cis" or "trans" configuration about the said dioxolane ring as follows:

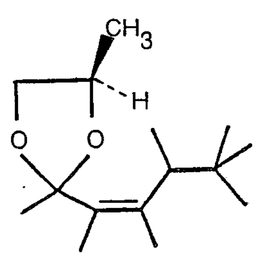

or

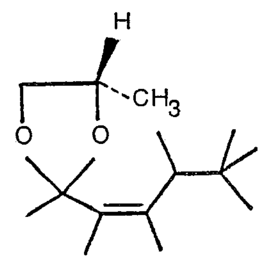

Furthermore, the double bond in the side chain of the dioxolane ring may be in either "cis" or "trans" configuration with respect to the methyl moieties bonded to the carbon atoms forming the carbon-carbon double bond as follows:

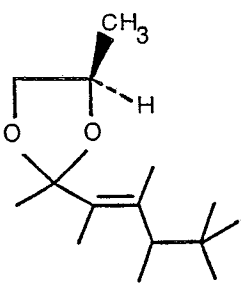

and

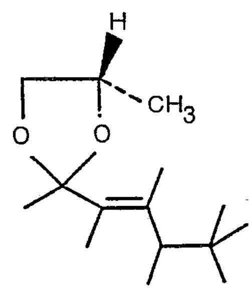

Furthermore, a "stereoisomer" of such a compound may be shown as follows:

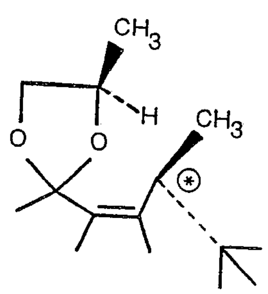

wherein the symbol:

"(*)"

represents the location of an asymmetric carbon atom.

Furthermore, the foregoing reaction sequence may be more specifically illustrated by the following reaction wherein the thiirane or epoxide compound may be in either "cis" or "trans" configuration and wherein $R_4$ and $R_5$ represent hydrogen:

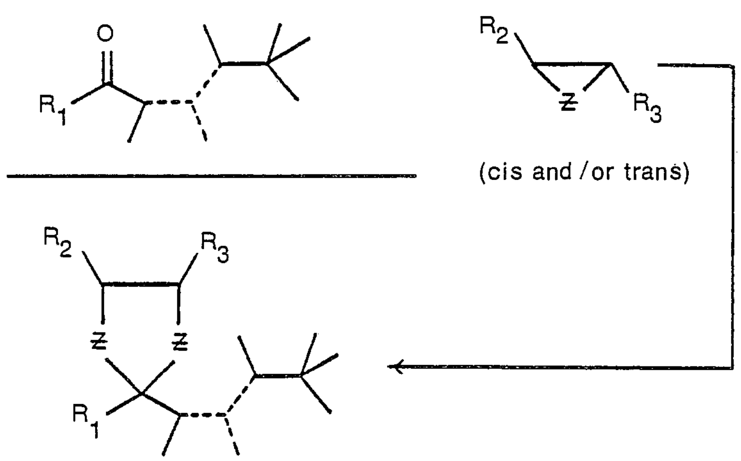

The reaction products of our invention which are oxathianes may also be prepared by first reacting the diisoamylene acylation derivatives with phosphorous pentasulfide to form a thioketone and then reacting the resulting thioketone with vicinal epoxides according to the reaction scheme:

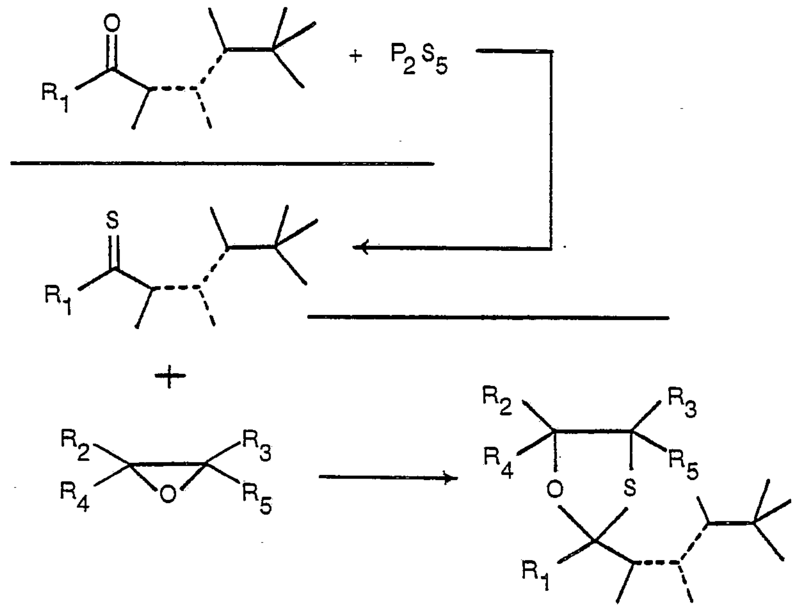

wherein $R_1$ represents $C_1$—$C_3$ lower alkyl; $R_2$, $R_3$, $R_4$ and $R_5$ represent the same or different hydrogen or $C_1$—$C_3$ lower alkyl; X and Y represent the same or different oxygen or sulfur; and one of the dashed lines represents a carbon-carbon double bond and the other dashed lines represent carbon-carbon single bonds.

When carrying out the reaction scheme:

(a)

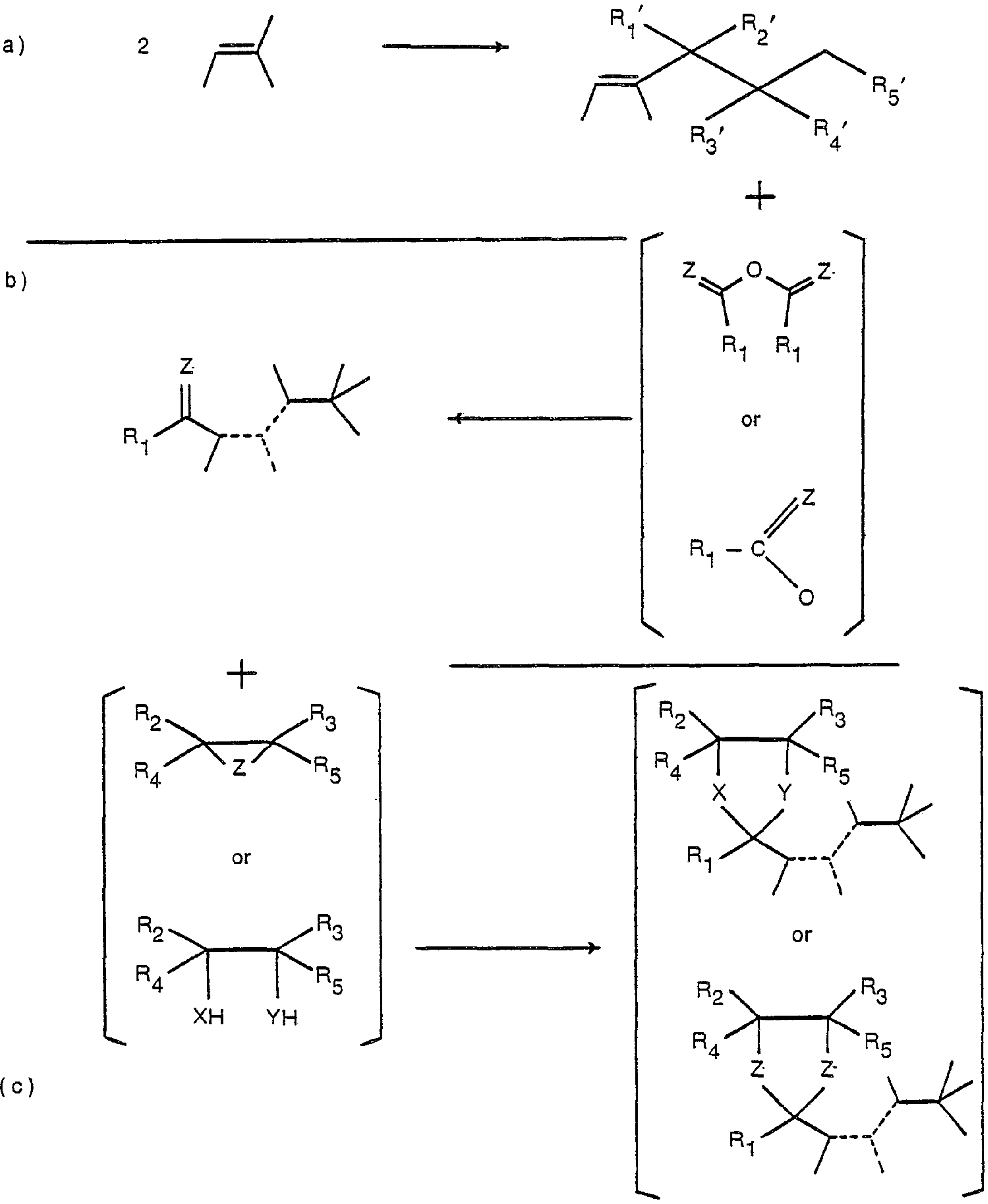

(b)

or

or

or

(c)

the diisoamylene material which is first produced may either be separated into constituent isomers or may be used "as is" in the subsequent acylation reaction or thioacylation reaction. Thus, for example, the subsequent acylation reaction may be carried out on the mixture of diisoamylene molecules according to the reaction:

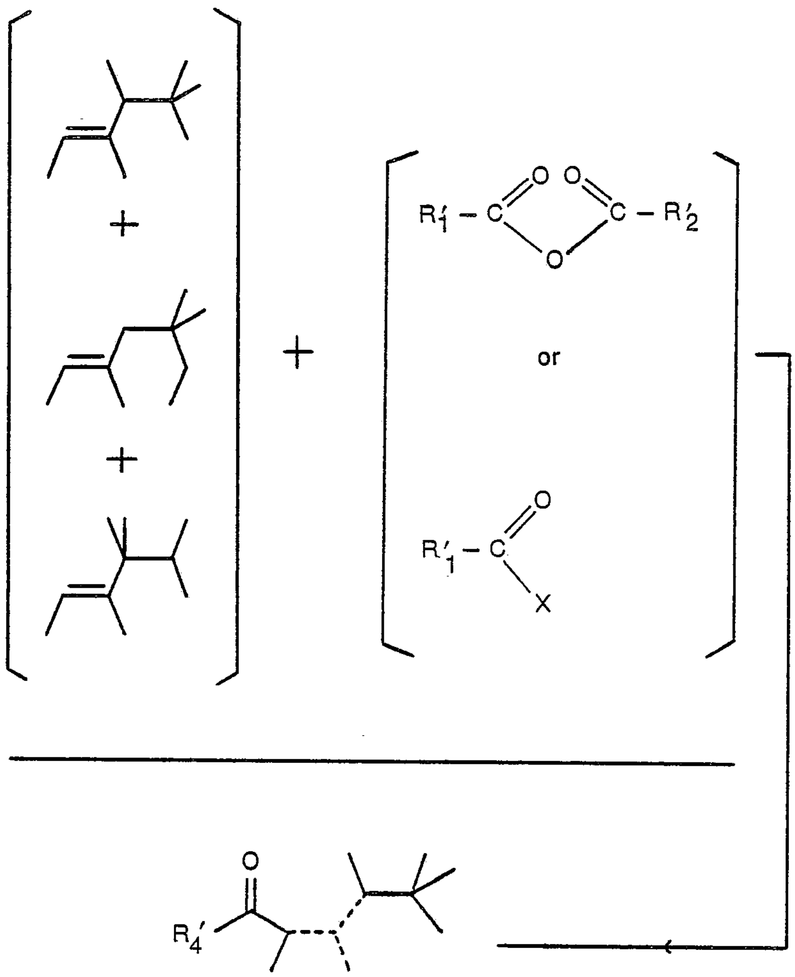


wherein the acylation is carried out using an organic acid anhydride or a mixed anhydride or an acyl halide or a thioacyl halide or an organic thioacid anhydride.

The reaction whereby the ketone is reacted with the epoxide or thiirane to form the oxathiolane, dithiolane or dioxolane according to the sequence:

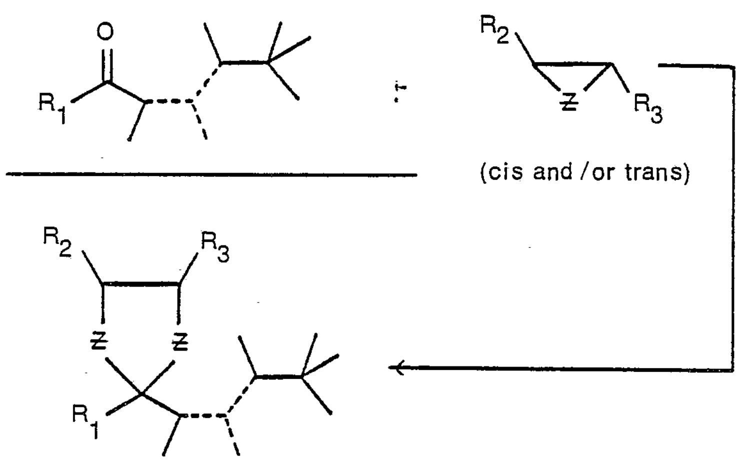


or the reaction of the ketone or thioketone with the diol, dithiol or thiol alcohol according to the reaction sequence, for example:

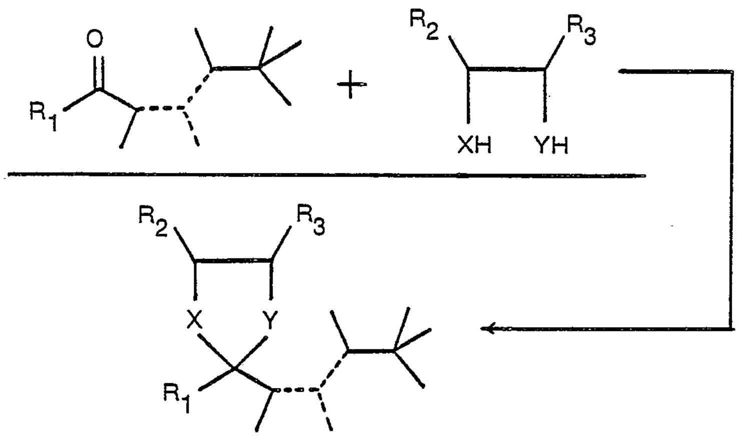

is carried out in the presence of an acid catalyst which, is this case, is a Lewis acid such as boron trifluoride, boron trifluoride etherate, zinc chloride, ethyl aluminum dichloride, stannic chloride of zinc bromide. The temperature of reaction may vary between 0 and 85°C with a preferred reaction temperature of between 10°C and 50°C.

The mole ratio of acylated or thioacylated diisoamylene having the structure:

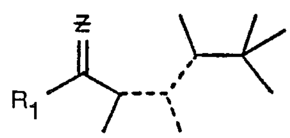

to epoxide or thiirane having the structure:

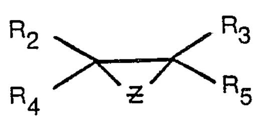

or glycol, dithiol or hydroxythiol having the structure:

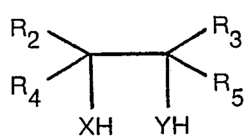

may vary from 1:1 of acylated or thioacylated diisoamylene: epoxide, thiirane, glycol, dithiol or thioalcohol up to 1:2.

The following table sets forth reactants and dioxolane, oxathiolane, and dithiolane reaction products contemplated by our invention:

TABLE B

| Thiirane, Epoxide, Glycol, Thioalcohol or Dithiol Reactant | Acylated or Thioacylated Diisoamylene Reactant | Dioxolane, Dithiolane or Oxathiolane Reaction Product |
|---|---|---|
| 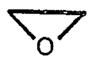 | 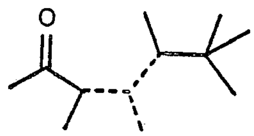 | 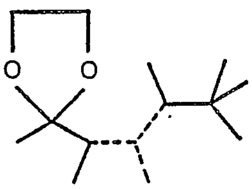 |
| 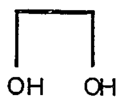 | 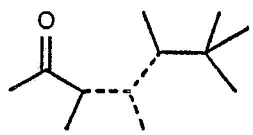 | 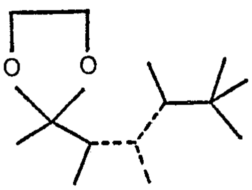 |
| 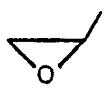 | 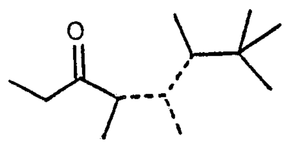 | 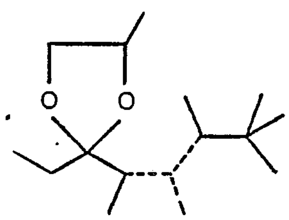 |
| 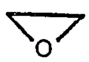 | 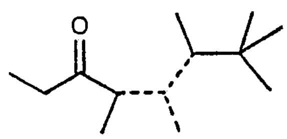 | 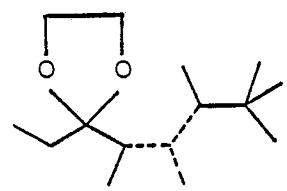 |
| 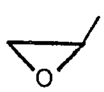 | 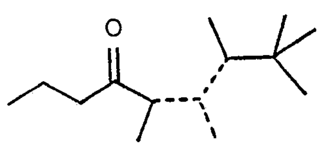 | 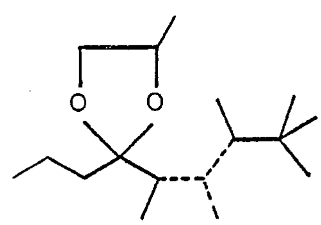 |
| 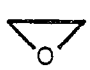 | 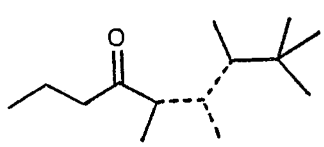 | 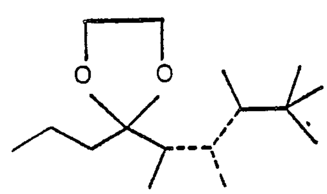 |

TABLE B (continued)

| Thiirane, Epoxide, Glycol, Thioalcohol or Dithiol Reactant | Acylated or Thioacylated Diisoamylene Reactant | Dioxolane, Dithiolane or Oxathiolane Reaction Product |
|---|---|---|
| 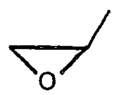 | 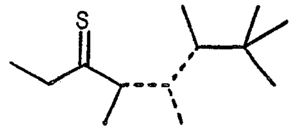 | 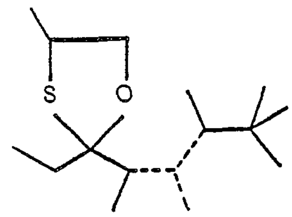 |
| 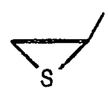 | 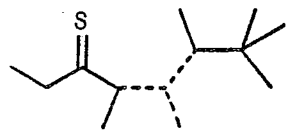 | 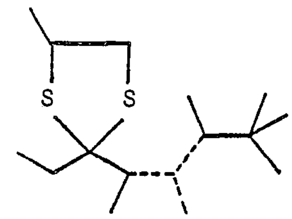 |
| 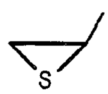 | 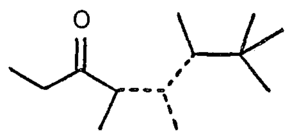 | 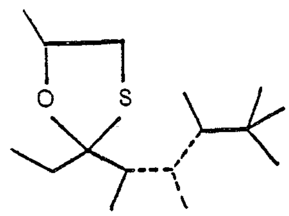 |
| 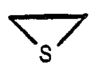 | 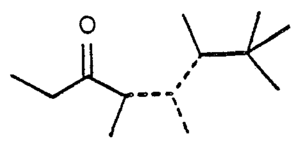 | 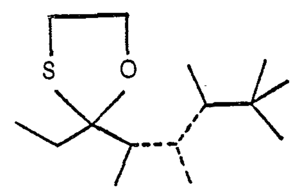 |
| 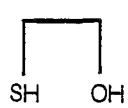 | 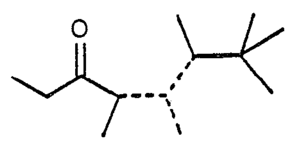 | 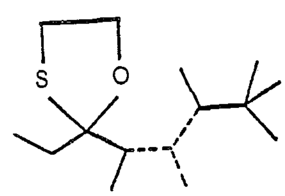 |
| 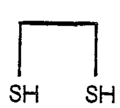 | 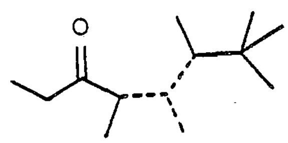 | 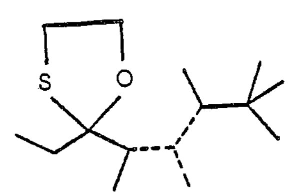 |

TABLE B (continued)

| Thiirane, Epoxide, Glycol, Thioalcohol or Dithiol Reactant | Acylated or Thioacylated Diisoamylene Reactant | Dioxolane, Dithiolane or Oxathiolane Reaction Product |
|---|---|---|
| 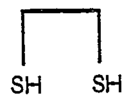 | 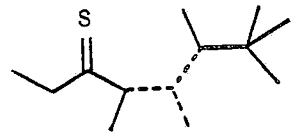 | 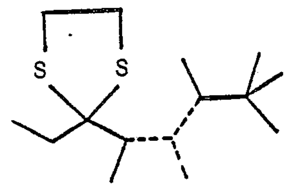 |
| 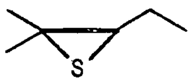 | 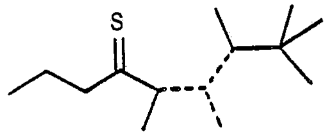 | 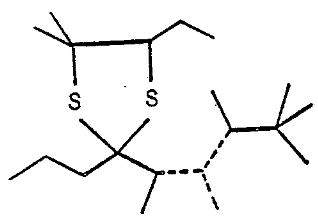 |
| 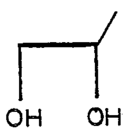 | 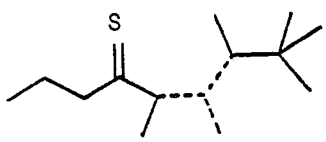 | 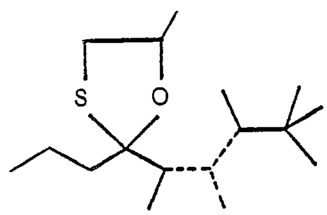 |
| 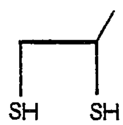 | 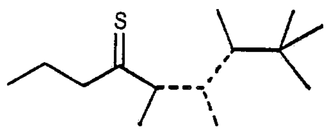 | 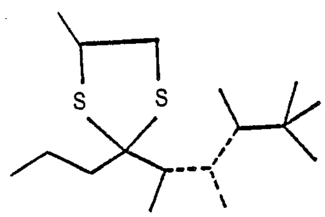 |
| 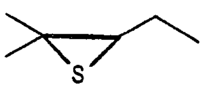 | 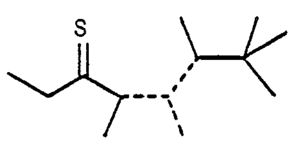 | 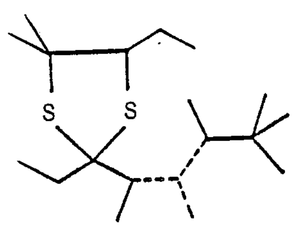 |
| 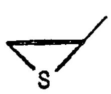 | 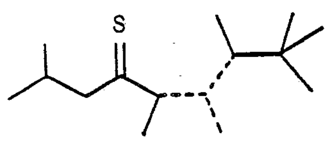 | 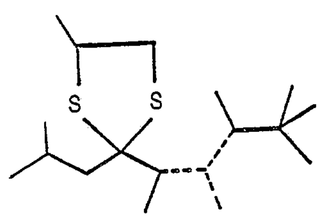 |

TABLE B (continued)

| Thiirane, Epoxide, Glycol, Thioalcohol or Dithiol Reactant | Acylated or Thioacylated Diisoamylene Reactant | Dioxolane, Dithiolane or Oxathiolane Reaction Product |
|---|---|---|
| 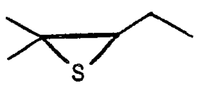 | 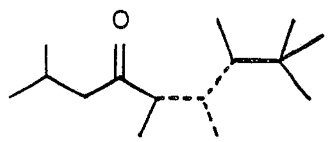 | 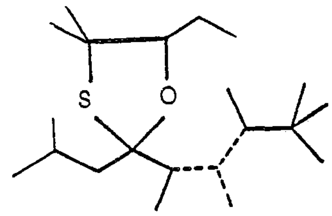 |
| 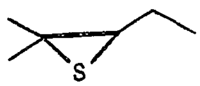 | 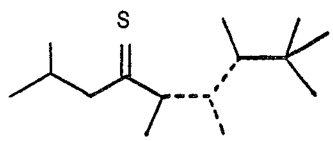 | 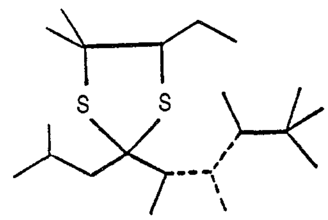 |
| 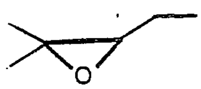 | 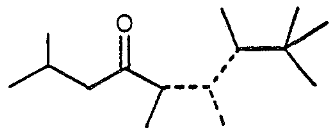 | 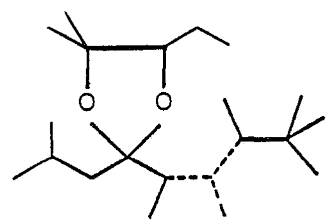 |
| 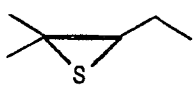 | 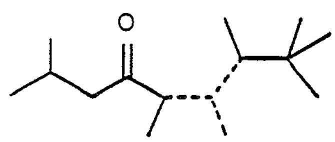 | 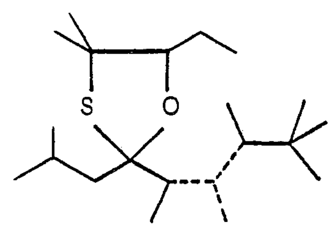 |
| 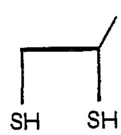 | 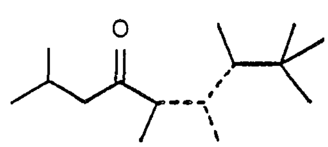 | 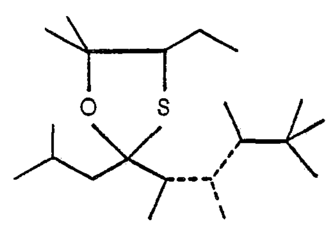 |
| 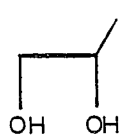 | 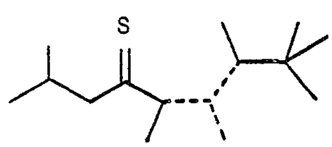 | 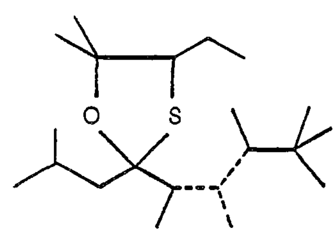 |

When a gaseous reactant is used such as thiirane having the structure:

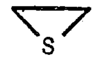

or ethylene oxide having the structure:

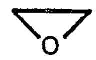

the conditions of the reaction involve pressures substantially greater than atmospheric so that the reactant is constant contact at whatever temperature the reaction is carried out with the other reactant. Thus, when ethylene oxide having the structure:

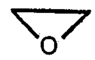

is reacted with the ketone mixture having the structure:

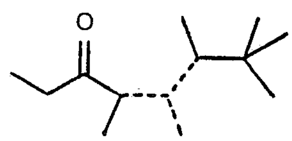

in order to yield the reaction product mixture having the structure:

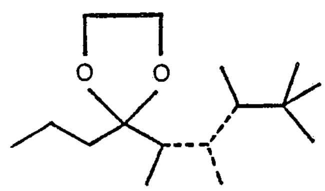

the reaction is preferably carried out at pressures of from about 2 up to about 10 bar and temperatures of from about 50°C up to about 100°C in the presence of a Lewis acid such as stannic chloride or boron trifluoride etherate.

An eighth aspect of our invention concerns the determination that certain branched chain olefinic tertiary alcohols and derivatives thereof are capable of imparting a variety of flavors and fragrances to various consumable materials. Briefly, our invention contemplates branched chain unsaturated tertiary alcohols, thio alcohols, esters, ethers, thio esters, and thio ethers defined according to the generic structure:

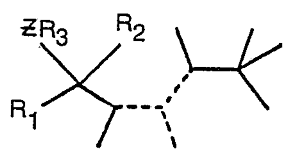

wherein Z represents oxygen or sulfur; wherein $R_1$ represents $C_1$—$C_4$ alkyl; wherein $R_2$ represents $C_1$—$C_4$ alkyl; wherein $R_3$ represents hydrogen, MgX, Li, $C_1$—$C_3$ lower alkyl and $C_1$—$C_3$ lower acyl; wherein one of the dashed lines represents a carbon-carbon double bond and each of the other of the dashed lines represents a carbon-carbon single bond and wherein X represents chloro, bromo or iodo. Thus, the compounds contemplated within the scope of our invention are either oxygenated compounds defined according to the structure:

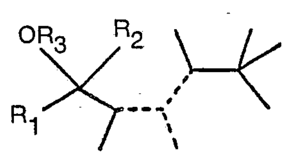

or sulfur containing compounds defined according to the structure:

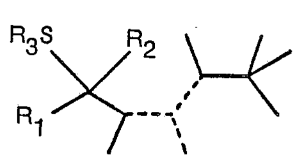

wherein $R_1$, $R_2$, $R_3$ and the dashed lines are defined as above.

The branched chain olefinic alcohols, thiols, esters and ethers, thio esters and thio ethers of our invention are either usable in admixture with one another, or the isomers are usable in admixture with one another such as mixtures of compounds defined according to the structure:

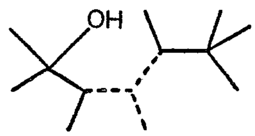

wherein one of the dashed lines of each of the molecules of the mixture represents a carbon-carbon double bond and each of the other of the dashed lines of each of the molecules of the mixture represents a carbon-carbon single bond or they may be used as individual compounds which are, for example, defined according to structures such as:

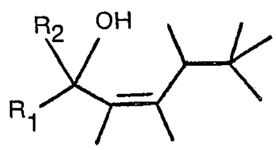 ; 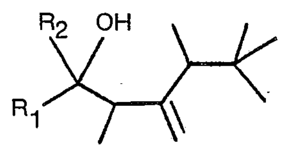 ; 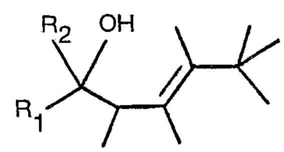 ;

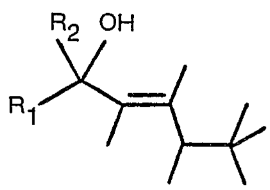 and 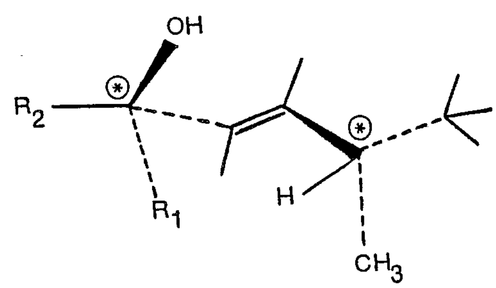

wherein the compound having the structure:

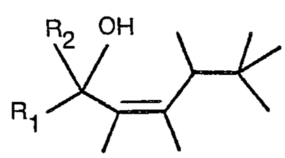

differs from the compound having the structure:

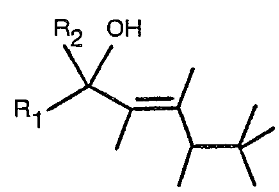

and that one is "cis" with respect to the methyl groups on the carbon atoms which make up the carbon-carbon double bond and wherein the structure:

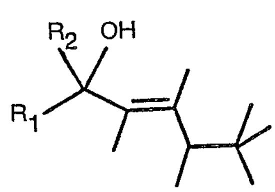

represents a " trans" isomer with respect to the methyl moieties bonded to the carbon atoms making up the carbon-carbon double bond and wherein the structure:

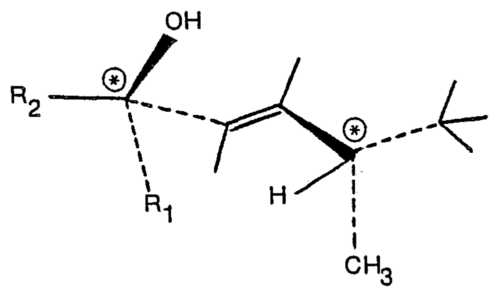

represents a stereo isomeric configuration wherein the carbon atoms having the "*" are asymmetric carbon atoms in the molecule and wherein the compound is a "trans" isomer with respect to the methyl moieties bonded to the carbon atoms which make up the carbon-carbon double bond.

The branched chain olefinic alcohols, thiols, esters, ethers, thio esters and thio ethers of our invention are obtained by means of reaction of ketones, produced as indicated supra, with alkyl lithium compounds or alkyl magnesium halide compounds to form organometallic intermediates defined according to the structure:

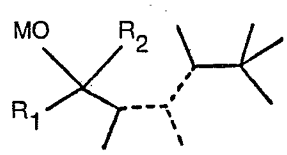

wherein M represents MgX or Li; wherein $R_1$ and $R_2$ each represent $C_1$—$C_4$ lower alkyl; wherein one of the dashed lines represents a carbon-carbon double bond and each of the other of the dashed lines represents a carbon-carbon single bond and wherein X represents chloro, bromo or iodo. This organometallic intermediate may be isolated or retained in situ in the reaction medium and then hydrolized to form the alcohols of our invention defined according to the structure:

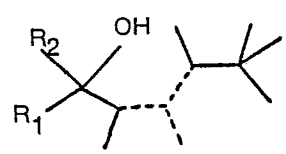

These alcohols may then be reacted with $P_2S_5$ to form the thiols of our invention according to the reaction:

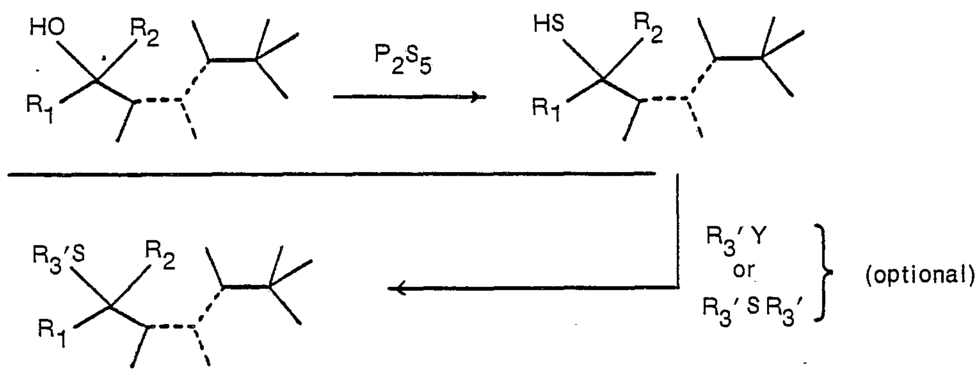


and these thiols may either be used as is or may optionally be reacted with $R_3'Y$ or $R_3'SR_3'$ to form the thio ethers or thio esters of our invention according to the reaction sequence:

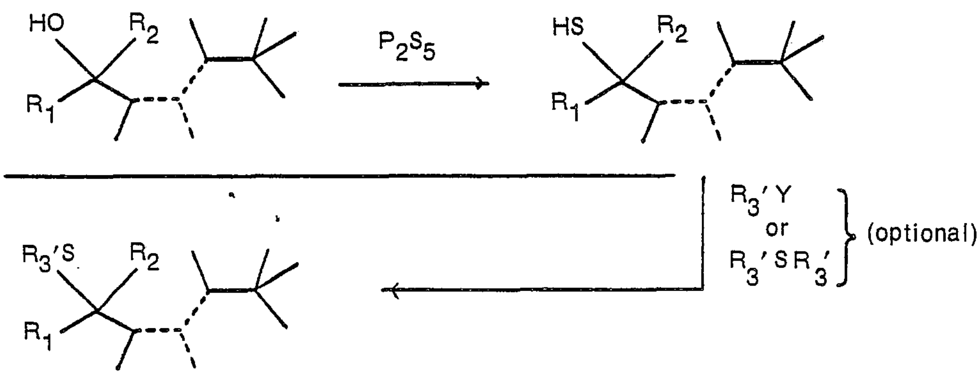


wherein $R_3'$ represents $C_1$—$C_3$ lower alkyl or $C_1$—$C_3$ acyl. Optionally, the alcohols of our invention having the structures:

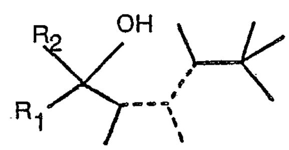

may be reacted with $R_3'Y$ or $R_3'OR_3'$ to form the esters or ethers of our invention and the reaction forming the alcohols, esters and ethers of our invention is illustrated by the reaction

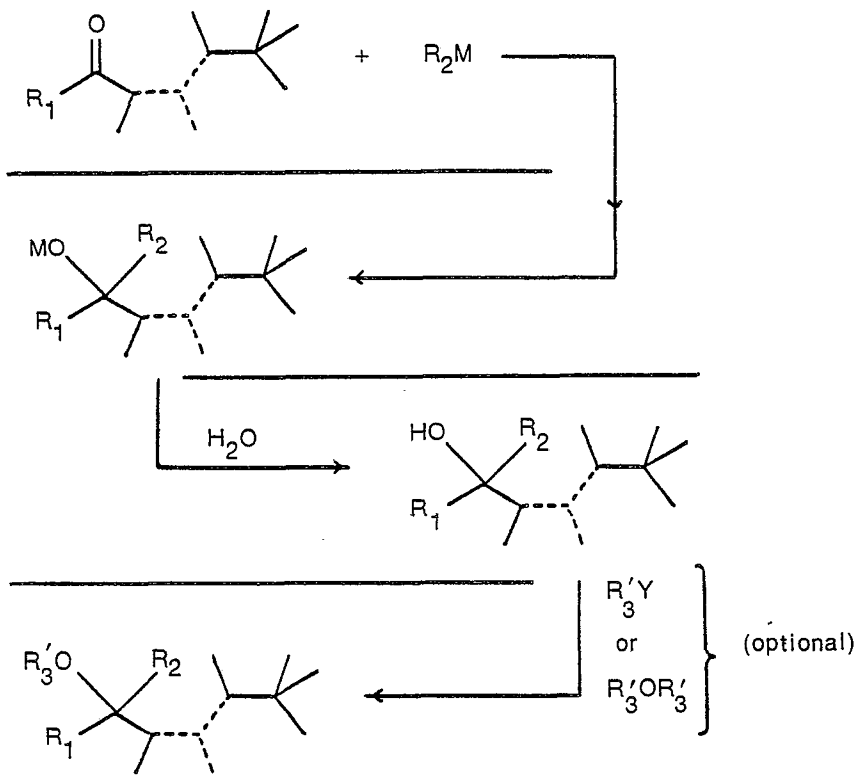


The foregoing reaction also shows the formation of the organometallic compounds contemplated by our invention. In the foregoing reaction sequences, $R_3'$ represents $C_1$—$C_3$ alkyl or acyl and Y represents chloro, bromo or iodo.

The reaction temperatures for reacting the alkyl lithium derivative or the Grignard reagent with the ketone having the structure:

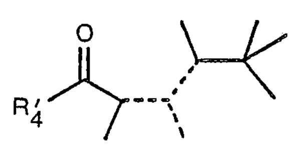

wherein $R_4'$ represents $C_1$—$C_4$ alkyl, may vary from 25°C up to 50°C. The reaction is carried out in an inert solvent such as tetrahydrofuran or diethylether and may be carried out at atmospheric pressure, superatmospheric pressures or sub-atmospheric pressures. The preferred mole ratio of ketone having the structure:

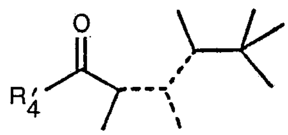

to organometallic compound, that is, alkyl lithium or alkyl magnesium halide is preferably 1:1 and if any material is used in excess, it is the ketone having the structure:

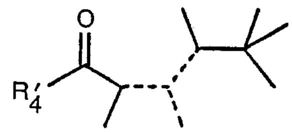

The hydrolysis of the organometallic compound thus formed, having the structure:

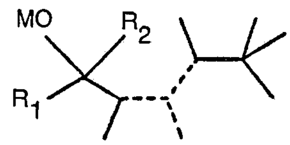

is carried out in dilute aqueous mineral acid such as aqueous sulfuric acid at concentrations of 0.05 molar up to about 1 molar, or aqueous hydrochloric acid or aqueous phosphoric acid. The temperature of hydrolysis may vary from 0°C up to about 50°C with ambient temperatures being most convenient and preferred

The reaction of the phosphoric pentasulfide with the alcohol having the structure:

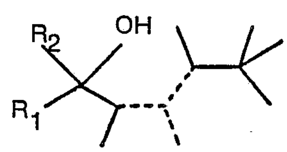

may be carried out at temperatures of from 0°C up to 50°C, again with ambient temperatures being preferred. This reaction also takes place in the presence of solvent which is inert to the reaction ingredients, such as benzene, toluene or xylene.

The reaction to form the ethers, esters, thio ethers and thio esters by reacting the corresponding alcohol having the structure:

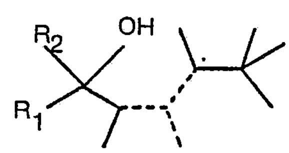

or the corresponding thiol having the structure:

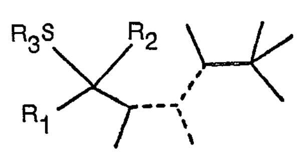

wherein $R_3$ is hydrogen, may be carried out with standard etherifying or esterification reagents such as acetyl chloride, acetyl bromide, n-propionyl chloride, n-propionyl bromide, acetic anhydride, acetic-propionic anhydride, and n-butyric anhydride in the presence of an acid catalyst such as sulfuric acid (in the case of the esterification) and methyl iodide, ethyl iodide, and propyl iodide (in the case of the etherification) at temperatures of between 0°C and 50°C also in the presence of a solvent. In producing the ethers, it is preferable to first react the alcohol or thiol with sodium hydride thereby forming the sodium salt and then reacting the resulting sodium salt with methyl iodide, ethyl iodide or the like via a "Williamson"

synthesis. In the alternative, the ethers may be formed by reaction with alcohol dialkyl sulfates such as dimethyl sulfate or diethyl sulfate.

Examples of branched chain unsaturated tertiary alcohols and their perfumery properties according to our invention are as follows:

TABLE C

| Structure | Organoleptic Properties (in Perfumery and in Perfumed Articles) |
|---|---|
| 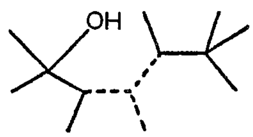 Mixture prepared according to Example XIII wherein in each of the molecules of the mixture one of the dashed lines represents a carbon-carbon double bond and each of the other of the dashed lines represents a carbon-carbon single bond. | An amber, woody, camphoraceous aroma with patchouli topnotes. |
| Structure | Organoleptic Properties (in Perfumery and in Perfumed Articles) |
| 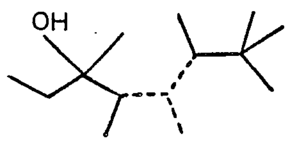 Prepared according to Example XIV, a mixture wherein in each of the molecules of the mixture one of the dashed lines represents carbon-carbon double bond and each of the other of the dashed lines represents a carbon-carbon single bond. | An amber, woody, fruity aroma. |
| 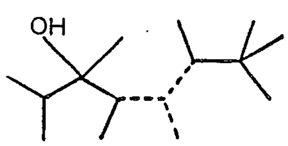 A mixture produced according to Example XV, wherein in each of the molecules of the mixture one of the dashed lines is a carbon-carbon double bond and each of the other of the dashed lines represents a carbon-carbon single bond. | An orris-like aroma. |

TABLE C (continued)

| Structure | Organoleptic Properties (in Perfumery and in Perfumed Articles) |
| --- | --- |
| 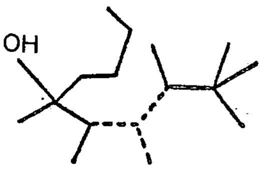 A mixture produced according to Example XVI, wherein in each of the molecules of the mixture one of the dashed lines is a carbon-carbon double bond and each of the other of the dashed lines represents a carbon-carbon single bond. | A floral, woody aroma. |

The individual branched chain tertiary alcohols, ethers, esters, thiols, thio ethers and thio esters of our invention can be obtained in purer form or in substantially pure form by conventional purification techniques. Thus, the products can be purified and/or acylated by distillation, extraction, crystallization, preparative chromatographic techniques and the like. It has been found desirable to purify the branched chain unsaturated tertiary alcohols, ethers, esters, thiols, thio ethers and thio esters of our invention by fractional distillation under vacuum.

A ninth aspect of our invention concerns the determination that certain branched chain olefinic secondary alcohols are capable of imparting a variety of flavors and fragrances to various consumable materials. Briefly, our invention contemplates branched chain unsaturated secondary alcohols defined according to the generic structure:

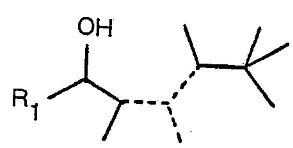

wherein $R_1$ represents methyl or isopropyl and wherein one of the dashed lines represents a carbon-carbon double bond and the other dashed lines represent carbon-carbon single bonds.

With respect to this ninth aspect of our invention, the branched chain olefinic secondary alcohols of our invention are either usable in admixture with one another, or the isomers are usable in admixture with one another such as mixtures of compounds defined according to the structure:

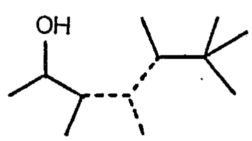

wherein one of the dashed lines in each of the molecules of the mixture represents a carbon-carbon double bond and the other dashed lines of each of the molecules of the mixture represent carbon-carbon single bonds or they may be used as individual compounds which are, for example, defined according to structures such as:

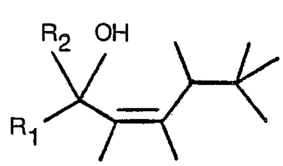 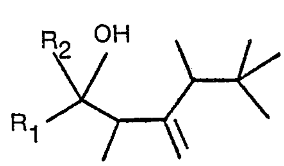

(wherein $R_1$ is methyl or isopropyl and $R_2$ is hydrogen)

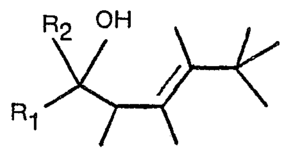

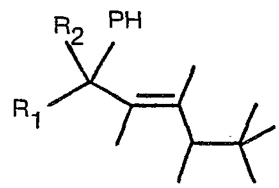

and

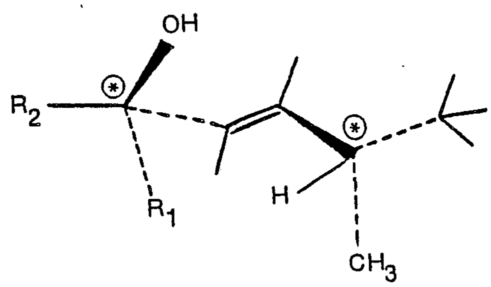

wherein the compound having the structure:

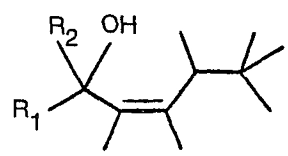

differs from the compound having the structure:

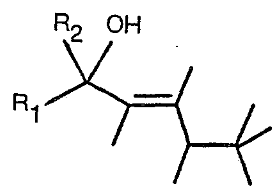

and that one is "cis" with respect to the methyl groups on the carbon atoms which make up the carbon-carbon double bond and wherein the structure:

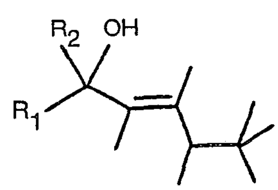

represents a "trans" isomer with respect to the methyl moieties bonded to the carbon atoms making up the carbon-carbon double bond and wherein the structure:

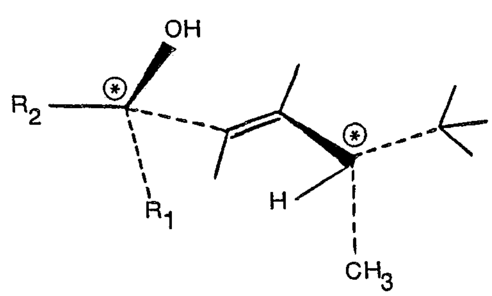

represents a stereo isomeric configuration wherein the carbon atoms having the "*" are asymmetric

carbon atoms in the molecule and wherein the compound is a "trans" isomer with respect to the methyl moieties bonded to the carbon atoms which make up the carbon-carbon double bond.

The branched chain olefinic secondary alcohols of our invention are obtained by means of reaction of the ketones indicated to be produced supra with a reducing agent such as:

(a) one or more alkali metal borohydrides, e.g., sodium borohydride, lithium borohydride and potassium borohydride;

(b) hydrogen, using a catalyst such as 5% palladium on carbon, 5% palladium on calcium carbonate or palladium on barium sulfate (e.g., "Lindlar Catalyst");

(c) lithium aluminum hydride; or

(d) aluminum alkoxides, such as aluminum isopropoxide and aluminum secondary epoxide according to the reaction:

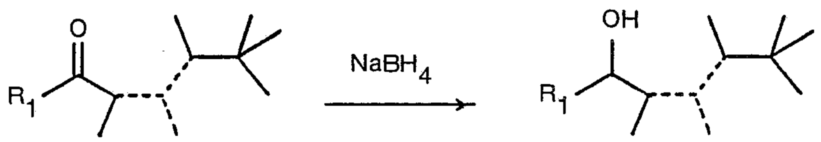


and

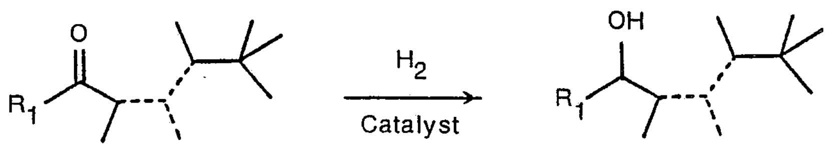


or in general

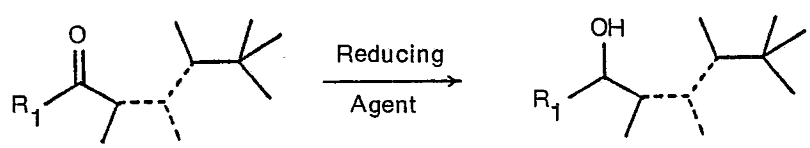


When carrying out the reaction for reacting the ketone having the structure:

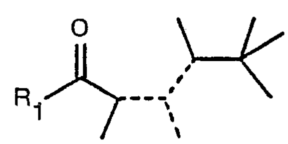

with an alkali metal borohydride such as sodium borohydride the reaction is carried out in the presence of a protic solvent which reacts relatively slowly or not at all with the alkali metal borohydride when compared to the reaction of the alkali metal borohydride with the ketone having the structure:

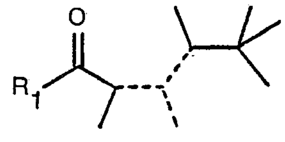

Specific workable solvents which must "solvate" the carbonyl moiety in order to enable the reaction to proceed at a reasonable rate are isopropyl alcohol, n-propanol, n-butanol, isobutyl alcohol and p-butyl alcohol.

The temperature of reaction is necessarily a function of:

(i) the yield desired

(ii) the time of reaction

(iii) the nature of the solvent used

(iv) the pressure of the vapor over the reaction mass

(v) the concentration of the reactant, the alkali metal borohydride and the ketone having the structure:

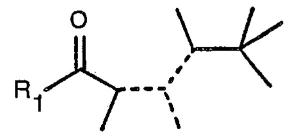

in the solvent

(vi) the desired rate of reaction, and

(vii) the ratio of alkali metal borohydride:ketone having the structure:

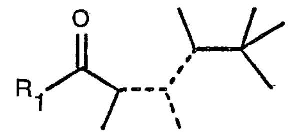

It is preferred to carry out the reaction at reflux conditions at atmospheric pressure. Thus, when using isopropyl alcohol as a solvent where the mole ratio of alkali metal borohydride:ketone having the structure:

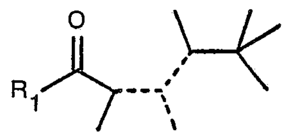

is 1:2, the temperature of reaction is about 73°C and the time of reaction is 3 hours. In the case of using an alkali metal borohydride, the alcohol acts as a "solvent" and not as a "reactant".

On the other hand, when using the aluminum alkoxide such as aluminum secondary butoxide and aluminum isopropoxide, the solvent must be a source of hydrogen which is the actual reducing agent in the reaction. Thus, it is necessary that the "solvent" be a "reactable solvent" such as isopropyl alcohol and not merely a solvating solvent.

The mole ratio of alkali metal borohydride:ketone having the structure:

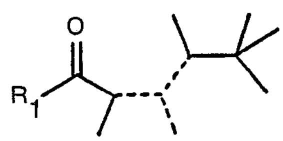

is preferably 1:2, which means that the equivalent ratio regarding hydrogen:ketone is 2:1; that is, the alkali metal borohydride is in 100% excess since theoretically only one mole of the alkali metal borohydride is needed to react with 4 moles of ketone, since one mole of alkali metal borohydride provides 4 atoms of hydrogen. Interestingly and surprisingly, in this reaction and in all of the above reactions the double bond does get reduced during the reaction.

Insofar as the hydrogenation reaction is concerned with the ketone having the structure:

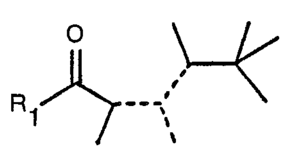

as the starting material or one of the ketones defined according to the structure:

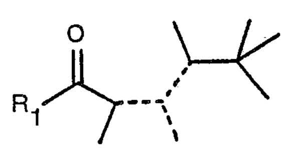

as being a starting material, the ketone is reacted with hydrogen in the presence of a Raney nickel catalyst

or a palladium on carbon catalyst or a "Lindlar" catalyst (palladium on calcium carbonate) or palladium on barium sulfate. The percentage of palladium in the palladium on carbon catalyst or in the palladium on calcium carbonate catalyst or in the palladium on barium sulfate catalyst varies from about 2% up to about 7% with a percentage of palladium in the palladium on carbon catalyst or in the palladium on calcium carbonate catalyst or in the palladium on barium sulfate catalyst being preferred to be 5%. The temperature of reaction for the hydrogenation may vary from about 10°C up to about 100°C with a preferred reaction temperature of 25°C—35°C. Since the reaction is exothermic it is usually necessary to provide external cooling to the reaction mass during the course of the reaction. The pressure of hydrogen over the reaction mass may vary from about 5 psig ($3.4 \times 10^4$ N/m$^2$) up to about 100 psig ($6.9 \times 10^5$ N/m$^2$) with the most preferred pressure being 20 psig ($1.4 \times 10^5$ N/m$^2$). Pressures greater than 150 psig ($10^6$ N/m$^2$) will give rise to amounts of fully saturated alcohol. The hydrogenation reaction may be carried out in the presence of or in the absence of a solvent. When a solvent is used, it is required that it be an inert (nonreactive) solvent such as isopropyl alcohol, hexane or ethanol. If a solvent is used, it is preferred that the mole ratio of solvent:ketone having the structure:

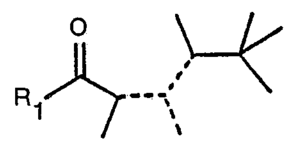

be approximately 1:1. Where a palladium containing catalyst is used, the percentage of catalyst in the reaction mass may vary from 0.125% up to about 2.0% with a percentage of catalyst of about 0.25% being preferred. Where a Raney nickel catalyst is used, the percentage of catalyst in the reaction mass may vary from about 3% up to about 10% with a percentage of catalyst of about 5% being preferred.

If the reaction is carried out in the presence of the alkali metal borohydride, the reaction mass is neutralized using weak acid and the reaction product is then further washed with water and, if necessary, sodium carbonate. In any event, the reaction mass is ultimately distilled fractionally to yield the desired saturated alcohol product having the generic structure:

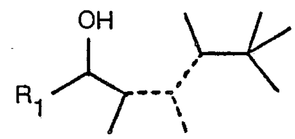

wherein $R_1$ is methyl or isopropyl and one of the dashed lines represents a carbon-carbon double bond and the other dashed lines represent carbon-carbon single bonds.

Examples of branched chain unsaturated alcohols of our invention and their perfumery and tobacco flavor properties according to our invention are as follows:

TABLE D

| | Organoleptic Properties | |
|---|---|---|
| Identification of Secondary Alcohol | Perfumery Properties | Tobacco Flavor Properties |
| Mixture of compounds defined according to the structure: 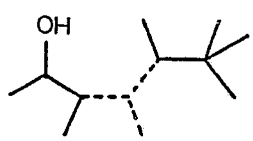 wherein in each component of the mixture, one of the dashed lines represents a carbon-carbon double bond and the other dashed lines represent carbon-carbon single bonds produced according to the process of Example XIX infra. | A woody, amber, fruity aroma with strong vetiver nuances on dry-out after six hours. | An intense woody, oriental-like and minty aroma and taste both prior to and on smoking in the main stream and the side stream. |

TABLE D (continued)

| Identification of Secondary Alcohol | Organoleptic Properties | |
|---|---|---|
| | Perfumery Properties | Tobacco Flavor Properties |
| Mixture of compounds defined according to the structure: 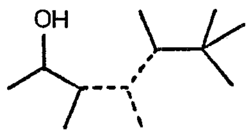 wherein in each of the molecules of the mixture, one of the dashed lines represents a carbon-carbon double bond the other dashed lines represent carbon-carbon single bonds, prepared according to the process of Example XX infra. | An intense woody aroma. | An oriental woody aroma and taste both prior to and on smoking in the main stream and in the side stream. |

The individual branched chain secondary alcohols or our invention can be obtained in purer form or in substantially pure form by conventional purification techniques. Thus, the products can be purified by distillation, extraction, crystallization, preparative chromatographic techniques (including high pressure liquid chromatography) and the like. It has been found desirable to purify the branched chain unsaturated secondary alcohols of our invention by fractional distillation under vacuum.

A tenth aspect of our invention involves the determination that $C_{11}$ branched chain aldehydes and alcohols defined according to the structure:

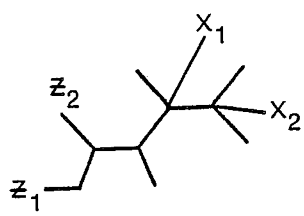

wherein one of $X_1$ or $X_2$ is hydrogen and the other of $X_1$ or $X_2$ is methyl and wherein one of $Z_1$ or $Z_2$ is hydroxy-methyl having the structure:

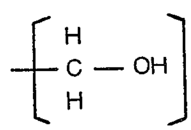

or carboxaldehyde having the structure:

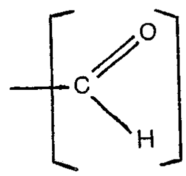

and the other of $Z_1$ or $Z_2$ is hydrogen produced according to the reactions:

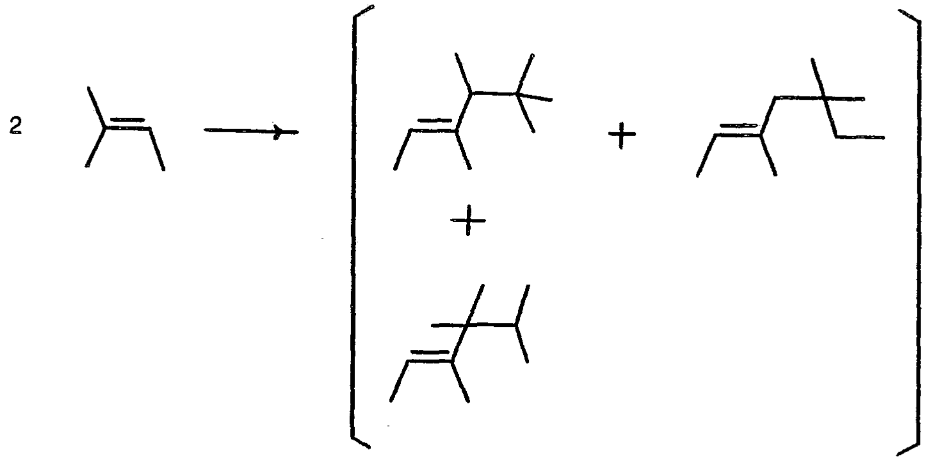

followed by, for example, the reaction:

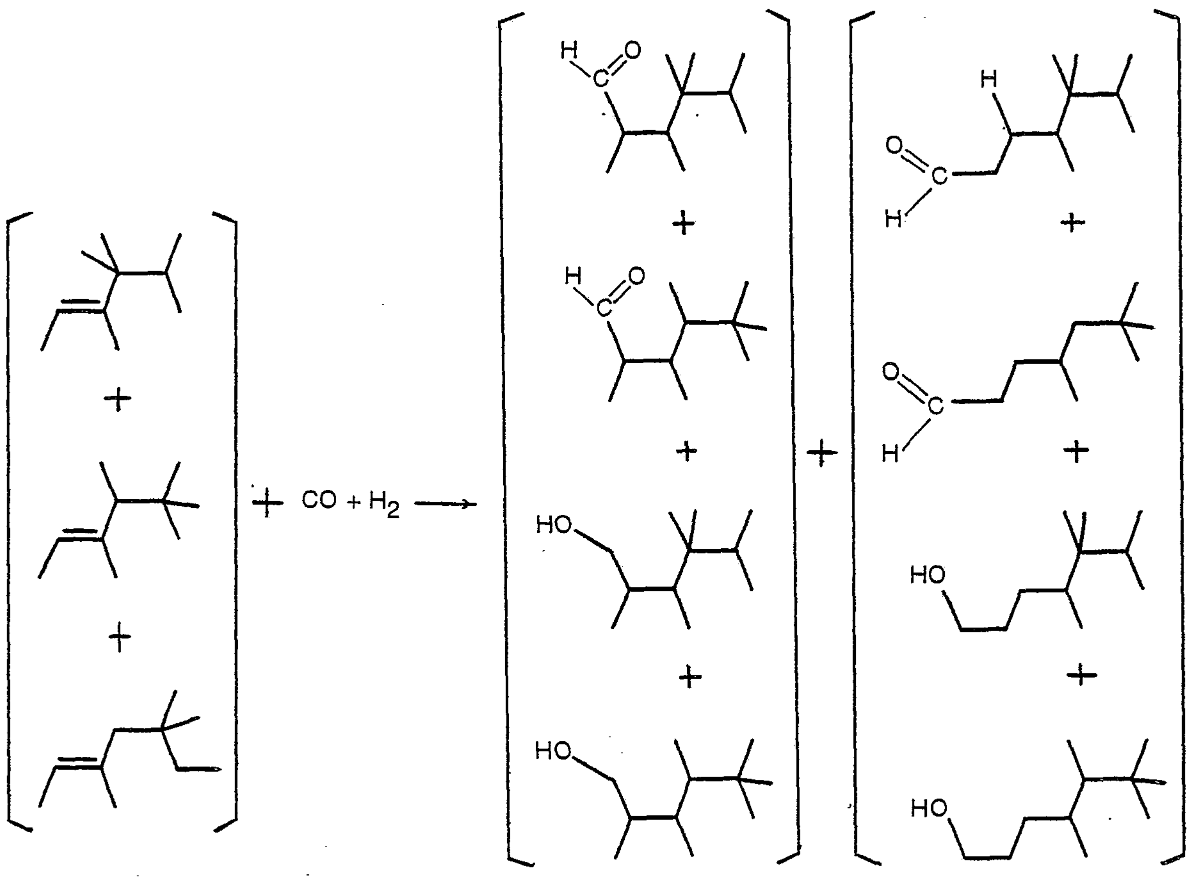


are capable of imparting or augmenting or enhancing a variety of fragrances in or to consumable materials.

Briefly this tenth aspect of our invention comtemplates augmenting or enhancing fragrances of such consumable materials as perfumes, perfumed articles, (e.g. solid or liquid anionic, cationic, nonionic or zwitterionic detergents, cosmetic powders, fabric softener compositions, dryer-added fabric softener articles and perfumed hypochlorite bleaches) and colognes by adding thereto a small but effective amount of at least one of the compounds defined according to the generic structure:

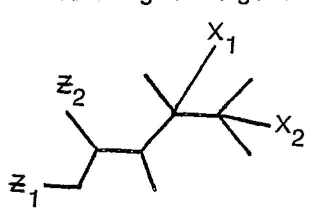

wherein one of $X_1$ or $X_2$ is hydrogen and the other of $X_1$ or $X_2$ is methyl and one of $Z_1$ or $Z_2$ is hydrogen and the other of $Z_1$ or $Z_2$ is hydroxymethyl having the structure:

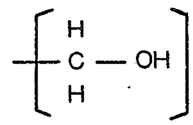

or carboxaldehyde having the structure:

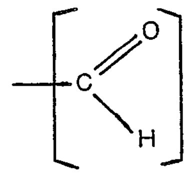

More specifically, the structures of the compounds useful in practising our invention are as follows:

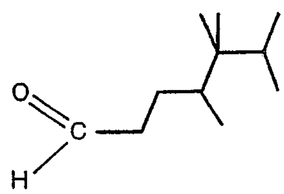 ; 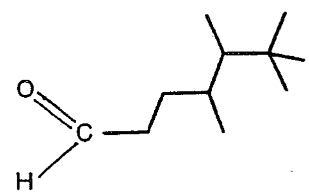 ;

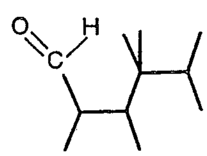 ; 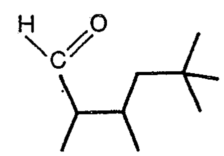 ;

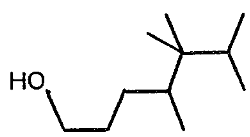 ; 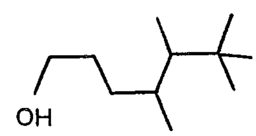 ;

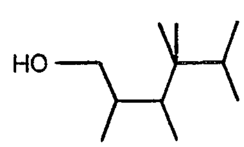 and 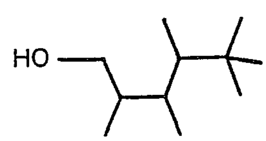

The $C_{11}$ branched chain aldehydes and alcohols of our invention augment or enhance green, citrus, melony, woody, peanut oil-like and vetiver-like aroma characteristics. The perfumes, perfumed articles and colognes thereby causing one or more of said $C_{11}$ branched chain aldehyde and alcohol compounds to be useful particularly in citrus and vetiver-type fragrances.

The $C_{11}$ branched chain alcohol and aldehyde derivatives of our invention defined according to the structure:

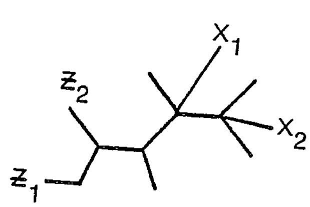

wherein one of $X_1$ or $X_2$ is hydrogen and the other of $X_1$ and $X_2$ is methyl and one of $Z_1$ or $Z_2$ is hydrogen and the other of $Z_1$ or $Z_2$ is hydroxymethyl having the structure:

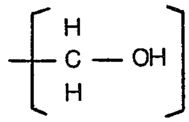

or carboxaldehyde having the structure:

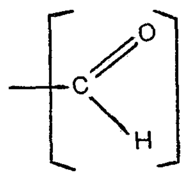

may be prepared by first (i) reacting 2-methyl-2-butene in the presence of an acidic catalyst which may be a Lewis acid such as zinc chloride, aluminum chloride, aluminum bromide, diethyl aluminum chloride, diethyl aluminum bromide, ethyl aluminum dichloride, and diethyl aluminum bromide, boron trifluoride, boron trifluoride etherate, or any of the other catalysts as set forth, supra and then (ii) reacting the resulting mixture or components thereof with a mixture of carbon monoxide and hydrogen using a particular range of temperatures and partial pressures of hydrogen and carbon monoxide over one of several alternative "oxo" type reaction catalysts over a period of residence times depending upon the several variables involved in this reaction.

As to the "oxo" reaction (ii) of the diisoamylene derivatives with carbon monoxide and hydrogen carried out, for example, according to the reaction sequence:

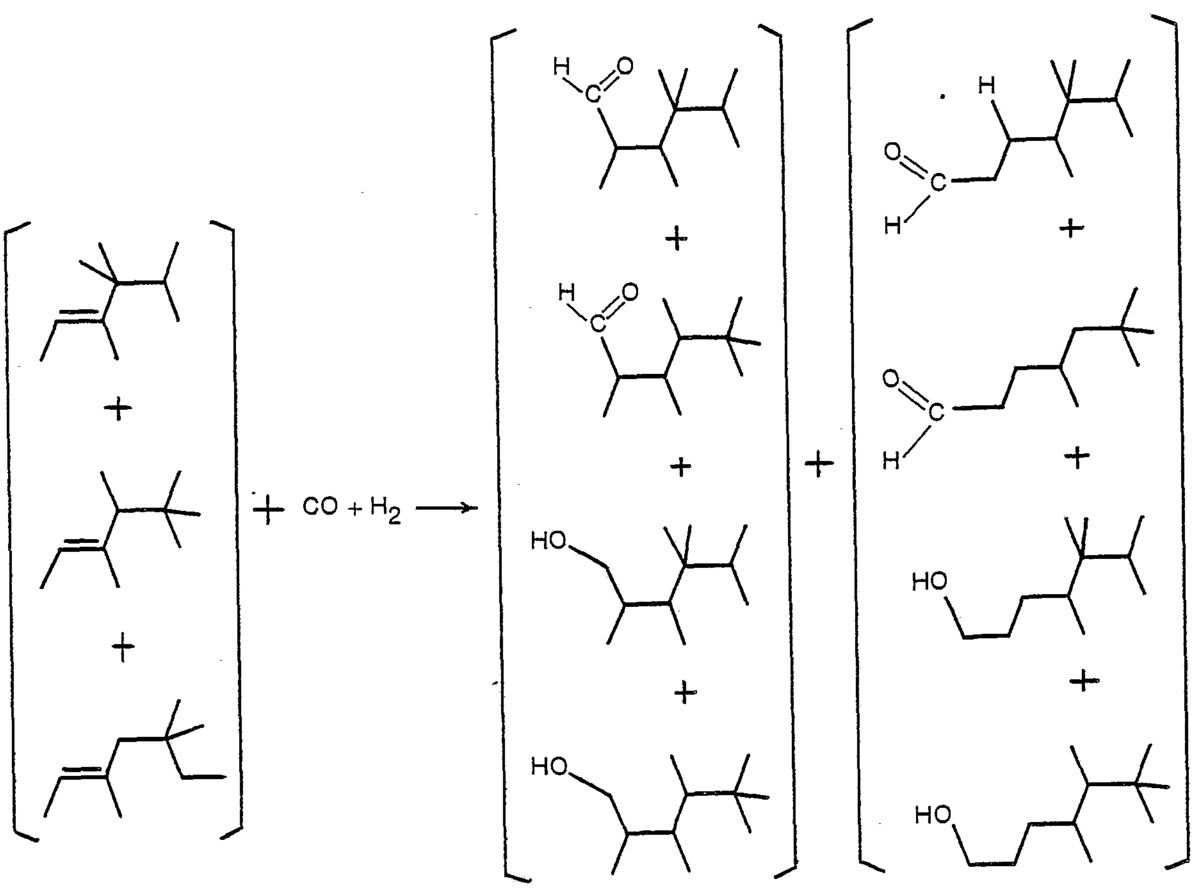

the reaction is carried out at temperatures of between 150°C and 300°C at pressures of between 50 and 250 atmospheres (between $5 \times 10^6$ and $25 \times 10^6$ N/m²); with the ratio of partial pressure of carbon monoxide:hydrogen being from 0.1:1 up to 1:0.1. Any oxo type reaction catalyst but, most preferably, the catalyst to yield the best perfume mixtures are as follows:

Dicobalt octacarbonyl
Cobalt octanoate
Palladium chloride
Rhodium trichloride
Iron pentacarbonyl
Nickel tetracarbonyl
Polymer-bonded rhodium catalyst (e.g., rhodium bonded on a polystyrene substrate)
tris-triphenyl phosphine rhodium-1-chloride.

Depending upon the conditions of reaction including, temperature, partial pressures of diisoamylene, carbon monoxide and hydrogen, mole ratio of diisoamylene:catalyst, concentration of diisoamylene in solvent, concentration of catalyst in solvent and time of reaction, the ratio and nature of the isomers of aldehydes, the isomers of the alcohols and the ratio of the aldehydes to the alcohols will vary in an as yet undetermined fashion.

In any event, this invention contemplates all isomers of $C_{11}$ aldehydes and alcohols defined according to the structures:

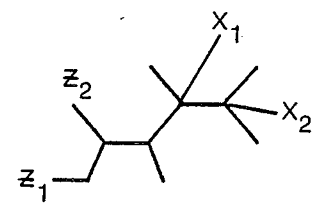

wherein one of $X_1$ or $X_2$ is hydrogen and the other of $X_1$ or $X_2$ is methyl; and wherein one of $Z_1$ or $Z_2$ is hydrogen and the other of $Z_1$ or $Z_2$ is hydroxymethyl having the structure:

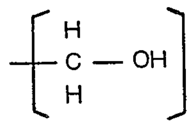

or carboxaldehyde having the structure:

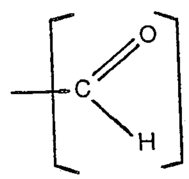

As olfactory agents, the $C_{11}$ branched chain alcohols and aldehydes taken alone or in admixture of our invention, can be formulated into, or used as components of, a "perfume composition" or can be used as components of a "perfumed article", or the perfume composition may be added to perfumed articles.

It will be appreciated from the present disclosure that the diisoamylene compounds and derivatives or diisobutylene derivatives according to the present invention can be used to alter, vary, fortify, modify, enhance or otherwise improve the flavor of a wide variety of materials which are ingested, consumed or otherwise organoleptically sensed.

The term "alter" in its various forms will be understood herein to mean the supplying or imparting of a flavor character or note to an otherwise bland, relatively tasteless substance, or augmenting an existing flavor characteristic where the natural flavor is deficient in some regard or supplementing the existing flavor impression to modify the organoleptic character.

Such compounds are accordingly useful in flavoring compositions. A flavoring composition is taken to mean one which contributes a part of the overall flavor impression by supplementing or fortifying a natural or artificial flavor in a material or one which supplies substantially all the flavor and/or aroma character to a consumable article.

The term "enhance" is intended herein to mean the intensification of a particular aroma or taste nuance without changing the quality or nature of said nuance and without adding an additional aroma or taste nuance to the consumable material, the organoleptic properties of which are enhanced.

The term "foodstuff" as used herein includes both solid and liquid ingestible materials for man or animals which materials do, but need not, have nutritional value. Thus, foodstuffs include meats, gravies, soups, convenience foods, malt, alcoholic and other beverages, milk and dairy products, seafoods including fish, crustaceans, molluscs and the like, candies, vegetables, cereals, soft drinks, snacks, dog and cat foods, other veterinary products and the like.

As used herein, the term "medicinal product" includes both solids and liquids which are ingestible non-toxic materials which have medicinal value such as cough syrups, cough drops, asprin and chewable medicinal tablets.

The term "chewing gum" is intended to mean a composition which comprises a substantially water-insoluble, chewable plastic gum base such as chicle, or substitutes therefor, including jelutong, guttakay, rubber or certain comestible natural or synthetic resins or waxes. Incorporated with the gum base in admixture therewith may be plasticizers or softening agents, e.g., glycerine, and a flavoring composition which incorporates one of the diisoamylene compounds or derivatives or diisobutylene derivatives of our invention, and in addition, sweetening agents which may be sugars, including sucrose or dextrose and/or artificial sweeteners such as cyclamates or saccharin. Other optional ingredients may also be present.

Substances suitable for use herein as co-ingredients or flavoring adjuvants are well known in the art for such use, being extensively described in the relevant literture. It is a requirement that any such material be "ingestibly" acceptable and thus non-toxic and otherwise non-deleterious particularly from an organoleptic standpoint whereby the ultimate flavor and/or aroma of the consumable material used is not caused to have unacceptable aroma and tastes nuances. Such materials may, in general, be characterized as flavoring adjuvants or vehicles comprising broadly, stabilizers, thickeners, surface active agents, conditioners, other flavorants and flavor intensifiers.

Stabilizer compounds include preservatives, e.g. sodium chloride, antioxidants, e.g., calcium and sodium ascorbate, ascorbic acid, butylated hydroxyanisole (mixture of 2- and 3-tertiary-butyl-4-hydroxyanisole), butylated hydroxytoluene (2,6-di-tertiary-butyl-4-methylphenol), propyl gallate and the like and sequestrants, e.g., citric acid.

Thickener compounds include carriers, binders, protective colloids, suspending agents, emulsifiers and the like, e.g., agar agar, carrageenan, cellulose and cellulose derivatives such as carboxymethyl cellulose and methyl cellulose; natural and synthetic gums such as gum arabic, gum tragacanth gelatin, proteinaceous materials; lipids; carbohydrates; starches, pectins and emulsifiers, e.g., mono and diglycerides of fatty acids, skim milk powder, hexoses, pentoses, disaccharides, e.g., sucrose, corn syrup and the like.

Surface active agents include emulsifying agents, e.g., fatty acids such as capric acid, caprylic acid, palmitic acid, myristic acid and the like, mono and diglycerides of fatty acids, lecithin, defoaming and flavor-dispersing agents such as sorbitan mono-stearate, potassium stearate, hydrogenated tallow alcohol and the like.

Conditioners include compounds such as bleaching and maturing agents, e.g., benzoyl peroxide, calcium peroxide, hydrogen peroxide and the like; starch modifiers such as peracetic acid, sodium chlorite, sodium hypochlorite, propylene oxide, succinic anhydride and the like, buffers and neutralizing agents, e.g., sodium acetate, ammonium bicarbonate, ammonium phosphate, citric acid, lactic acid, vinegar and the like; colorants, e.g., carminic acid, cochineal, tumeric and curcuma and the like; firming agents such as aluminum sodium sulfate, calcium chloride and calcium gluconate; texturizers, anti-caking agents, e.g., aluminum calcium sulfate and tribasic calcium phosphate; enzymes; yeast foods, e.g., calcium lactate and calcium sulfate; nutrient supplements, e.g., iron salts such as ferric phosphate, ferrous glyconate and the like, riboflavin, vitamins, zinc sources such as zinc chloride, zinc sulfate and the like.

Other flavorants and flavor intensifiers include aldehydes, esters, natural oils, alcohols, sulfides, ketones, lactones, carboxylic acids and hydrocarbons such as heliotropin, terpineol-4, benzaldehyde, anisaldehyde, phenyl acetaldehyde, benzyl formate, benzyl acetate, cis-3-hexenyl benzoate, methyl hexanoate, hexanal, eucalyptol, eugenol, acetaldehyde, ethyl acetate, ethyl butyrate, turpentine gum oil, limonene, gum camphor, isobornyl acetate, borneol, cinnamic aldehyde, cuminic aldehyde, furfural, methyl cinnamate, cassia oil, vanillin, maltol, pharahydroxybenzyl acetone, dimethyl sulfide, alpha-ionone, acetic acid, isobutyl acetate, acetone butyric acid, formic acid, valeric acid, amyl acetate, amyl butyrate, anethol, benzyl salicylate, diacetyl, di-methyl, anthranilate, ethyl methylphenylglycidate, ethyl succinate, ethyl valerate, geraniol, cis-3-hexen-1-ol, 2-hexenyl acetate, 2-hexenyl butyrate, 4-(p-hydroxyphenyl)-2-butanone, beta-ionone, isobutyl cinnamate, jasmine, lemon essential oil, methyl butyrate, methyl caproate, methyl disulfide, methyl p-naphthyl ketone, orris butter, rose absolute, terpenyl acetate, gamma-undecalactone, vanilla and alcohol.

The specific flavoring adjuvant selected for use may be either solid or liquid depending upon the desired physical form of the ultimate product, i.e., foodstuff, whether simulated or natural, should in any event, (i) be organoleptically compatible with the diisoamylene compounds and derivatives or diisobutylene derivatives of our invention by not covering or spoiling the organoleptic properties (aroma and/or taste) thereof: (ii) be nonreactive with the diisoamylene compounds or derivatives or diisobutyl derivatives of our invention and (iii) be capable of providing an environment in which the diisoamylene compounds or derivatives or diisobutylene derivatives can be dispersed or admixed to provide a homogeneous medium.

In addition, selection of one or more flavoring adjuvants as well as the quantities thereof will depend upon the precise organoleptic character desired in the finished product. Thus, in the case of flavoring compositions, ingredient selection will vary in accordance with the foodstuff, chewing gum, medicinal product or toothpaste to which the flavor and/or aroma are to be imparted, modified, altered or enhanced. In contradistinction, in the preparation of solid products, e.g., simulated foodstuffs, ingredients capable of providing normally solid compositions should be selected such as various cellulose derivatives.

As will be appreciated by those skilled in the art, the amount of diisoamylene compounds or derivatives or diisobutylene derivatives employed in a particular instance can vary over a relatively wide range, depending upon the desired organoleptic effects to be achieved. Thus, correspondingly greater amounts would be necessary in those instances wherein the ultimate food composition to be flavored is relatively bland to the taste, whereas relatively minor quantities may suffice for purposes of enhancing the composition merely deficient in natural flavor or aroma. The primary requirement is that the amount selected must be effective, i.e., sufficient to alter, modify or enhance the organoleptic characteristics of the parent composition, whether foodstuff per se, chewing gum per se, medicinal product per se, toothpaste per se, or flavoring composition.

The use of insufficient quantities of diisoamylene compounds or derivatives or diisobutylene derivatives (e.g., less than 0.05 ppm or less than 0.02 ppm for the diisobutylene derivatives) will substantially vitiate any possibility of obtaining the desired results, while excess quantities (e.g., greater than 100 ppm or greater than 50 ppm for the diisobutylene derivatives) prove needlessly costly and, in extreme cases, may disrupt the flavor/aroma balance thus proving self-defeating. According, the terminology "effective amount" and "sufficient amount" is to be accorded a significance in the context of the present invention consistent with the obtention of desired flavoring effects.

Thus and with respect to ultimate food compositions, chewing gum compositions, medicinal product compositions and toothpaste compositions, it is found that quantities of diisoamylene compounds and derivatives and diisobutylene derivatives ranging from a small but effective amount, e.g., 0.05 ppm up to about 100 ppm or 0.02 ppm up to about 50 ppm for the diisobutylene derivatives, based on the total composition are suitable. Concentrations in excess of the maximum quantity stated are not normally recommended, since they fail to provide commensurate enhancement of organoleptic properties. In those instances wherein the diisoamylene compounds, derivatives and diisobutylene derivatives are added to the foodstuff as an integral component of the flavoring composition, it is, of course, essential that the total quantity of flavoring composition employed be sufficient to yield an effective diisoamylene compound, derivative or diisobutylene derivative concentration in the foodstuff product.

Food flavoring compositions prepared in accordance with the present invention preferably contain the diisoamylene compound, derivatives and diisobutylene derivatives in concentrations ranging from about 0.1% up to about 15% by weight based on the total weight of the said flavoring composition.

The compositions described herein can be prepared according to conventional techniques well known as typified by cake betters and fruit drinks and can be formulated by merely admixing the involved ingredients within the proportions stated in a suitable blender to obtain the desired consistency, homogeneity of dispersion, etc. Alternatively, flavoring compositions in the form of particulate solids can be conveniently prepared by mixing the diisoamylene compounds, diisoamylene derivatives and diisobutylene derivatives with, for example, gum arabic, gum tragacanth, carrageenan and the like, and thereafter spray-drying the resultant mixture whereby to obtain the particulate solid product. Pre-prepared flavor mixes in powder form, e.g., a fruit-flavored powder mix, are obtained by mixing the dried solid components, e.g., starch, sugar and the like and the diisoamylene compounds, diisoamylene derivatives and diisobutylene derivatives in a dry blender until the requisite degree of uniformity is achieved.

It is presently preferred to combine with the diisoamylene compounds, diisoamylene derivatives and diisobutylene derivatives of our invention, the following adjuvants:

heliotropin; terpineol-4; benzaldehyde; anisaldehyde; phenyl acetaldehyde; benzyl formate; benzyl acetate; cis-3-hexenyl benzoate; methyl hexanoate; hexanal; eucalyptol; eugenol; acetaldehyde; ethyl acetate; ethyl butyrate; turpentine gum oil; limonene; gum camphor; isobornyl acetate; borneol; cinnamic aldehyde; cuminic aldehyde; furfural; methyl cinnamate; cassia oil; vanillin; maltol; parahydroxybenzylacetone; dimethyl sulfide; alpha-ionone; acetic acid; isobutyl acetate; acetone; butyric acid; formic acid; valeric acid; amyl acetate; amyl butyrate; anethol, benzyl salicylate; diacetyl; dimethyl anthranilate; ethyl methylphenylglycidate; ethyl succinate; ethyl valerate; geraniol; cis-3-hexen-1-ol; 2-hexenyl acetate; 2-hexenyl butyrate; hexyl butyrate; 4-(p-hydroxyphenyl)-2-butanone; beta-ionone; isobutyl cinnamate; jasmine; lemon essential oil; methyl butyrate; methyl capronate; methyl disulfide; methyl p-naphthyl ketone; orris butter; rose absolute; terpenyl acetate; gamma-undecalactone; vanilla and ethyl alcohol.

An additional aspect of our invention provides an organoleptically improved smoking tobacco product and additives therefor, as well as methods of making the same which overcome problems heretofore encountered in which specific desired sweet, woody, oriental, camphoraceous, fruity, spicy, vetiver-like and cedar-like characteristics of natural "Turkish-like" tobacco (prior to smoking and on smoking in the main stream and in the side stream) are created or enhanced or modified or augmented and may be readily controlled and maintained at the desired uniform level regardless of variations in the tobacco components of the blend.

This invention further provides improved tobacco additives and methods whereby various desirable natural aromatic tobacco flavoring characteristics with sweet, woody, oriental, camphoraceous, fruity, spicy, vetiver-like and cedar-like taste and aroma nuances may be imparted to smoking tobacco products and may be readily varied and controlled to produce the desired uniform flavoring characteristics.

In carrying out this aspect of our invention, we add to smoking tobacco materials or a suitable substitute therefor e.g., dried lettuce leaves, an aroma and flavor additive containing as an active ingredient one or more diisoamylene compounds and diisoamylene derivatives of our invention.

In addition to the diisoamylene compounds and diisoamylene derivatives of our invention, other flavoring and aroma additives may be added to the smoking tobacco material or substitute therefor either separately or in mixture with the diisoamylene compounds and diisoamylene derivatives as follows:

I. Synthetic materials

beta-ethyl-cinnamaldehyde; eugenol; dipentene; damascenone; maltol; ethyl maltol, delta undecalactone; delta decalactone; benzaldehyde; amyl acetate; ethyl butyrate; ethyl valerate; ethyl acetate; 2-hexenol-1; 2-methyl-5-isopropyl-1,3-nonadiene-8-one; 2,6-dimethyl-2,6-undecadiene-10-one; 2-methyl-5-isopropylacetophenone; 2-hydroxy-2,5,5,8a-tetramethyl-1-(2-hydroxyethyl)-decahydronaphthalene; dodecahydro-3a,6,6,9a-tetramethyl-naphtho-(2,1 furan); 4-hydroxyhexanoic acid, gamma lactone; and polyisoprenoid hydrocarbons defined in Example V of U.S. Patent No. 3,589,372 issued on June 29, 1971

II. Natural oils

celery seed oil; coffee extract; bergamot oil; cocoa extract; nutmeg oil; and origanum oil.

An aroma and flavoring concentrate containing one or more diisoamylene compounds and diisoamylene derivatives of our invention and, if desired, one or more of the above indicated additional flavoring additives may be added to the smoking tobacco material, to the filter or to the leaf or paper wrapper. The smoking tobacco material may be shredded, cured, cased and blended tobacco material or tobacco substitutes (e.g., lettuce leaves) or mixtures thereof. The proportions of flavoring additives may be varied in accordance with the taste but insofar as enhancement or the imparting of natural and/or sweet notes and/or woody notes and/or oriental-like notes and/or camphoraceous notes and/or fruity notes and/or spicy notes and/or vetiver like notes and/or cedar-like notes and/or peppery notes, we have found that satisfactory results are obtained when the proportion by weight of the sum total of diisoamylene compounds or diisoamylene derivatives to smoking tobacco material is between 50 ppm and 1,500 ppm (0.015%—0.15%). We have further found that satisfactory results are obtained if the proportion by weight of the sum total of diisoamylene compounds and diisoamylene derivatives used to flavoring material is between 1,500 and 15,000 ppm (0.15%—1.5%).

Any convenient method for incorporating the diisoamylene compounds and diisoamylene derivative(s) into the tobacco product may be employed. Thus, the diisoamylene compounds and diisoamylene derivatives taken alone or along with other flavoring additives may be dissolved in a suitable solvent such as ethanol, diethyl ether and/or volatile organic solvents and the resulting solution may either be spread on the cured, cased and blended tobacco material or the tobacco material may be dipped into such solution. Under certain circumstances, a solution of the diisoamylene compounds and diisoamylene derivatives taken alone or taken further together with other flavoring additives as set forth above, may be applied by means of a suitable applicator such as a brush or roller on the paper or leaf wrapper for the smoking product, or it may be applied to the filter by either spraying or dipping, or coating.

Furthermore, it will be apparent that only a portion of the tobacco or substitute therefor need to be treated and the thus treated tobacco may be blended with other tobaccos before the ultimate tobacco product is formed. In such cases, the tobacco treated may have the diisoamylene compounds or diisoamylene derivatives or both in excess of the amounts or concentrations above indicated so that when blended with other tobaccos, the final product will have the percentage within the indicated range.

While our invention is particularly useful in the manufacture of smoking tobacco, such as cigarette tobacco, cigar tobacco and pipe tobacco, other tobacco products, formed from the sheeted tobacco dust or fines may also be used. Likewise, the diisoamylene compounds and diisoamylene derivatives of our invention can be incorporated with materials such as filter tip materials (e.g., cellulose acetate filters wherein sweet, woody, oriental, spicy, fruity, patchouli, vetiver and cedar-like effects are desired), seam paste, packaging materials and the like which are used along with tobacco to form a product adapted for smoking. Furthermore, the diisoamylene compounds and diisoamylene derivatives of our invention can be added to certain tobacco substitutes of natural or synthetic origin (e.g., dried lettuce leaves) and, accordingly, by the term "tobacco" as used throughout this specification is meant any composition intended for human consumption by smoking or otherwise, whether composed of tobacco, of tobacco plant parts, or substitute materials or both.

The diisoamylene compounds and diisoamylene derivatives of our invention and one or more auxiliary perfume ingredients including, for example, hydrocarbons, alcohols, ketones, aldehydes, nitriles, esters, lactones, synthetic essential oils and/or natural essential oils other than those materials which make up the subject: "diisoamylene compounds and derivatives" of our invention may be admixed so that the combined odors of the individual components produce a pleasant and desired fragrance, particularly and preferably in the woody, patchouli-like, amber, vetiver, piney, minty, herbaceous, eucalyptol, sandalwood-like, orris-like and/or cedarwood fragrances. Such perfume compositions usually contain (a) the main note

or the "bouquet" or foundation stone of the composition; (b) modifiers which round off and accompany the main note; (c) fixatives which include odorous substances which lend a particular note to the perfume throughout all stages of evaporation and substances which retard evaporation; and (d) topnotes which are usually low-boiling fresh-smelling materials.

In perfume compositions, it is the individual components which contribute to their particular olfactory characteristics, however, the overall sensory effect of the perfume composition will be at least the sum total of the effects of each of the ingredients. Thus, one or more of the diisoamylene compounds or diisoamylene derivatives of our inventon can be used to alter, modify or enhance the aroma characteristics of a perfume composition, for example, by utilizing or moderating the olfactory reaction contributed by another ingredient in the composition.

The amount of diisoamylene compound or diisoamylene derivative(s) of our invention which will be effective in perfume compositions as well as in the perfumed articles and colognes depends on many factors, including the other ingredients, their amounts and the effects which are desired. It has been found that perfume compositions containing as little as 0.01% of the diisoamylene compound and/or diisoamylene derivative(s) or even less (e.g., 0.005%) can be used to impart pleasant woody, piney, herbaceous, eucalyptol, minty, sweet, rich, warm woody, fruity, amber, rum/cognac-like, sandalwood-like, sweet floral, vetiver-like, cedar-like, amber-like, sweet, patchouli, camphoraceous, fruity and orris-like aromas to soaps, cosmetics, detergents (including solid or liquid anionic, cationic, nonionic or zwitterionic detergents) or other products. The amount employed can range up to 70% of the fragrance components and will depend on considerations of cost, nature of the end product, the effect on the finished product and the particular fragrance sought.

The diisoamylene compounds and the diisoamylene derivatives of our invention are useful (taken alone or together with other ingredients in perfume compositions) in detergents and soaps, space odorants and deodorants, perfumes, colognes, toilet water, bath preparations, such as lacquers, brilliantines, pomades and shampoos; cosmetic preparations such as creams, deodorants, hand lotions and sun screens; powders such as talcs, dusting powders, face powders and the like. As little as 0.05% of the diisoamylene compounds and/or diisoamylene derivative(s) will suffice to impart pleasant woody, piney, herbaceous, eucalyptol, minty, sweet, rich, warm woody, fruity, amber, rum/cognac-like, sandalwood-like, sweet floral, vetiver-like, cedar-like, amber-like, patchouli-like, camphoraceous, fruity and orris-like aromas to perfumed articles which are desired to have woody, piney, vetiver-like, amber-like, cedar-like, patchouli-like and sandalwood-like aromas. Generally no more than 3% of the diisoamylene compound and/or diisoamylene derivative(s) based on the ultimate end product (e.g., the perfumed article) is required.

In addition, the perfume composition or fragrance composition of our invention can contain a vehicle or carrier for the diisoamylene compound and/or diisoamylene derivative(s). The vehicle can be a liquid such as a non-toxic alcohol, a non-toxic glycol or the like. The carrier can also be an absorbent solid, such as a gum (e.g., gum arabic), or components for encapsulating the composition (such as gelatin) as by coacervation.

Another aspect of this invention consists of an aqueous solution of at least one alkali metal hypochlorite containing a stable perfume oil having as its main ingredient one of the above named diisoamylene compounds or diisoamylene derivatives and a surface active agent either consisting (i) solely of a $C_1$—$C_{12}$ alkyl diphenyl oxide dialkali metal sulfonate having the structure:

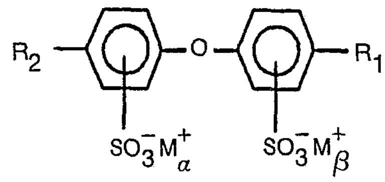

wherein at least one of $R_1$ or $R_2$ is straight chain or branched chain $C_1$—$C_{12}$ alkyl and when only one of $R_1$ or $R_2$ is straight chain or branched chain $C_{10}$—$C_{12}$ alkyl, the other of $R_1$ or $R_2$ is hydrogen and wherein $M_\alpha$ and $M_\beta$ are the same or different and each is an alkali metal such as sodium, potassium or lithium; for example the compounds defined according to the structure:

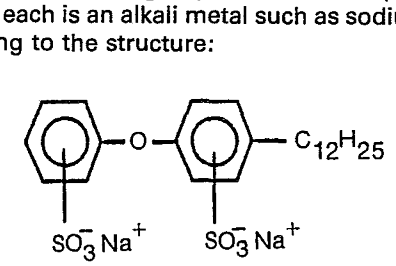

(wherein $C_{12}H_{25}$ represents several $C_{12}$ branched chain moieties) or the compounds defined according to the structure:

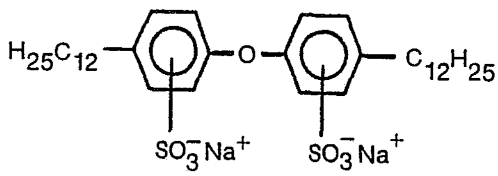

(wherein $C_{12}H_{25}$ represents several $C_{12}$ branched chain moieties) or the compounds defined according to the structure:

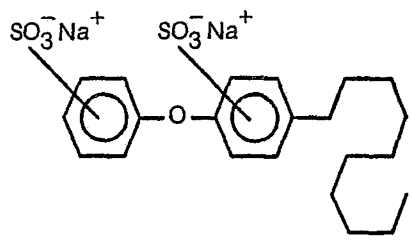

or the compounds defined according to the structure:

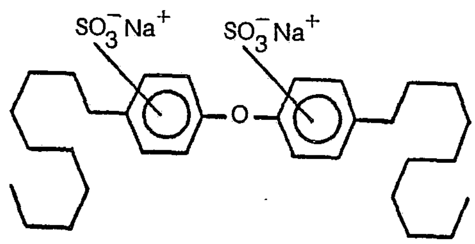

or mixtures of one or more of the foregoing compounds, or (ii) a mixture of at least one compound defined according to the generic structure:

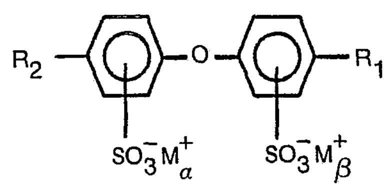

(wherein $R_1$, $R_2$, $M_\alpha$ and $M_\beta$ are defined supra) and an amine oxide composition consisting essentially of one or more morpholine and/or dimethyl $C_{11}$—$C_{13}$ straight chain alkyl amide oxide having the generic structure:

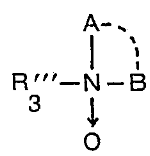

in an amount greater than 55% of said amine oxide composition wherein $R_3'''$ is straight chain alkyl having from 11 up to 13 carbon atoms and A and B are each separately methyl, or taken together, complete a morpholine ring, this solution having a pH in the range of 11—14.0.

The chain lengths of the $R_3'''$ moiety (or moieties) of the predominating alkyl dimethyl amine oxides of the amine oxide composition aids in providing for an aqueous hypochlorite bleach or sterilizing solution which can be perfumed in the woody, piney, vetiver-like, patchouli-like, orris-like, sandalwood-like and herbaceous aroma formulations required for our invention, but the compound having the structure:

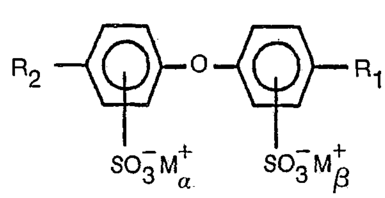

or mixture of such compounds taken alone or taken further together with the alkyl dimethyl amide oxide composition will aid even further in providing such a perfume hypochlorite bleach formulation and the compounds defined according to the structure:

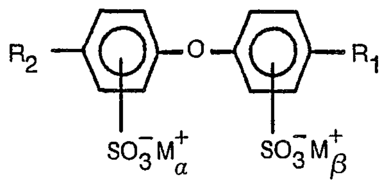

as stated above may be used alone.

The concentration of the compound of matter consisting essentially of the compounds having the structure:

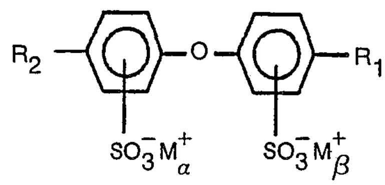

(wherein $R_1$, $R_2$, $M_\alpha$ and $M_\beta$ are defined supra) taken alone or taken in admixture with the amine oxide composition required to create the transparent liquid phase or gel phase solution of this invention is from 0.10% up to 2.0% based on the total weight of solution. Concentrations of diphenyl oxide derivatives less then 0.10% or mixtures of diphenyl oxide derivatives and amine oxides of less than 0.10% will not give rise to the desired single liquid or gel phase system containing the desired special perfume oil consisting essentially of one or more diisoamylene isomers having woody, piney, herbaceous, eucalyptol-like, minty, sweet, fruity, amber, rum/cognac-like, sandalwood-like, sweet floral, vetiver-like, cedar-like, and orris-like aroma profiles required for this invention. From a commercial standpoint, the concentration of $C_{10}$—$C_{12}$ straight chain or branched chain alkyl substituted diphenyl oxide dialkali metal sulfonate (hereinafter referred to as "diphenyl oxide derivative") taken alone or taken in conjunction with amine oxide greater than 2.0% based on the total weight of hypochlorite solution are not needed and give rise to unnecessary costs.

The pH range of the aqueous alkali metal hypochlorite solution containing the diphenyl oxide derivative composition defined according to the structure:

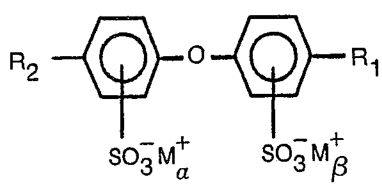

taken alone or in conjunction with the amine oxide composition having the structure:

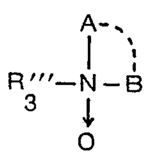

and one of the requisite perfume formulations consisting essentially of one or more of the diisoamylene derivatives of our invention which are stable to aqueous hypochlorite and which are capable of yielding and imparting the desired and required overall herbaceous, piney, woody, eucalyptol-like, minty, sweet, rich, fruity, amber, rum/cognac-like, sandalwood-like, vetiver-like, cedar-like, camphoraceous, fruity and orris-like fragrance impression, is critical; that is, a pH of from 11 up to 14.0 is required for the composition of our invention, with a preferred pH range being between 12.0 and 13.1. The requisite pH range is achieved by adding an aqueous solution of alkali metal hydroxide (e.g., from 1 molar up to 12.5 molar) to the alkali metal hypochlorite solution which has or will have added to it the $C_{10}$—$C_{12}$ straight or branched chain alkyl substituted diphenyl oxide dialkali metal sulfonate-diisoamylene perfume oil premix or the $C_{10}$—$C_{12}$ straight or branched chain alkyl substituted diphenyl oxide dialkali metal sulfonate-amine oxide-diisoamylene or diisoamylene derivative-containing perfume oil premix.

The percentage of diisoamylene-containing perfume oil or diisoamylene derivative-containing perfume oil or diisoamylene per se or diisoamylene derivative per se having the properties which yield

herbaceous, woody, piney, eucalyptol-like, minty, sweet, warm woody, fruity, amber, rum/cognac-like, sandalwood, cedar, patchouli-like, camphoraceous, fruity and orris-like aromas is in the range of from 0.01% up to 0.8%, preferably from 0.02% up to 0.2% based on the total final weight of alkali metal hypochlorite solution. Lower concentrations of such perfume oils will not be adequate to give rise to the desired substantial diminution or elimination of the characteristic disagreeable hypochlorite aroma (which exists on, for example, laundry and/or the hands of the individual user which have been in direct contact with the hypochlorite bleach or sterilizing solution subsequent to the use of aqueous hypochlorite solutions as a general domestic bleach or sterilizer). Quantities of perfume oil greater than 0.8% have been found to be uneconomical and unnecessary for the practice of our invention although, in the case of using the diisoamylene or the diisoamylene derivatives of our invention as aromatizing agents (without any additional adjuvants), the very low cost of the diisoamylene or diisoamylene derivatives and the very high stability of the diisoamylene or the diisoamylene derivatives of our invention give rise to a very commercial economical use even at very high levels, that is higher than 0.8%.

Several processes may be used in order to produce a hypochlorite bleaching or sterilizing solution whereby the desired woody, piney, herbaceous, eucalyptol, minty, sweet, rich, warm woody, fruity, amber, rum/cognac-like, sandalwood, sweet floral, vetiver-like, cedar, camphoraceous and orris-like aroma profiles are imparted to the articles treated with said hypochlorite solutions. Thus, for example, the diisoamylene or diisoamylene derivative-containing perfume oil may be premixed with the diphenyl oxide derivative or the diphenyl oxide derivative-amide oxide solubilizer-stabilizer having the structures:

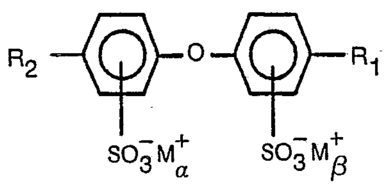

and

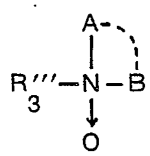

and the resulting perfume oil-diphenyl oxide derivative or perfume oil-diphenyl oxide derivative-amine oxide premix is then mixed with the hypochlorite bleaching or sterilizing solution with stirring. Immediately after such addition, an aqueous alkali metal hydroxide solution is added to the mixture to bring the pH to the range of 11—14.0. A pH of less than 11 is not desired since it is difficult to achieve a single phase stable system at such low pH's. A pH higher than 14.0 will also create a system which (1) is unnecessarily corrosive; (2) will narrow the range of perfume oils usable in conjunction with the diisoamylene or diisoamylene derivative in the system and (3) will limit the particular ingredients usable in such perfume oils in conjunction with the diisoamylenes or diisoamylene derivatives. On the other hand, if the diisoamylene or diisoamylene derivatives are used alone, a pH of 14.0 is acceptable.

The aqueous alkali metal hydroxide can be added to the aqueous alkali metal hypochlorite solution before adding the diphenyl oxide derivative (taken alone or in conjunction with the amine oxide) or the diisoamylene or diisoamylene-containing perfume oil or diisoamylene derivative or diisoamylene derivative-containing perfume oil. Indeed, the ingredients: the diisoamylene or diisoamylene derivative or diisoamylene-containing perfume oil or diisoamylene derivative-containing perfume oil; the alkali metal hydroxide and the diphenyl oxide derivative or the diphenyl oxide derivative-amine oxide composition, having the structures, respectively:

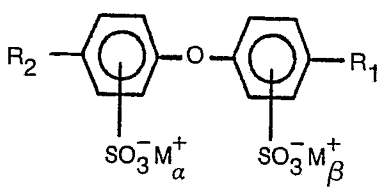

and

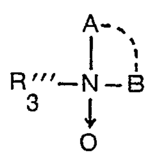

may be added or admixed in any order which is convenient to the formulator. One desirable process involves first forming the diphenyl oxide derivative or diphenyl oxide derivative-amine oxide composition-diisoamylene, diisoamylene derivative, diisoamylene-containing perfume oil "premix" or diisoamylene derivative-containing perfume oil "premix", mixing the premix with the alkali metal hypochlorite solution and finally adjusting the pH of the solution with alkali metal hydroxide to bring the pH to within the range of 11—14.0. A second, more preferable, process involves first adjusting the pH of the aqueous alkali metal hypochlorite solution to 11—14.0 and then admixing the solution with the aforedescribed "premix".

The alkali metal hypochlorites preferred in the practice of our invention are: sodium hypochlorite, potassium hypochlorite and lithium hypochlorite or mixtures of same. The alkali metal hydroxides preferred in the practice of this invention are: lithium hydroxide, potassium hydroxide and sodium hydroxide or, if desired, mixtures of such hydroxides.

The temperature at which the composition of our invention remains both substantially stable and commercially useful for the purposes set forth herein, that is, remains as a clear single aqueous or gel phase and retains (1) the desired properties inherent in the known bleaching and sterilizing uses of aqueous alkali metal hypochlorite liquid or gel solutions, and (2) the properties imparted thereto as a result of the diisoamylene or diisoamylene derivative or diisoamylene-containing perfume oils which impart to articles previously subjected to the aqueous metal hypochlorite gel or liquid solutions a herbaceous and/or piney and/or woody and/or eucalyptol-like and/or minty and/or warm woody and/or fruity and/or amber and/or rum/cognac-like and/or sandalwood and/or cedar and/or patchouli-like and/or camphoraceous and/or orris-like aroma profile varies from approximately 20°F (−7°C) up to approximately 120°F (49°C). At temperatures below 20°F (−7°C) a two-phase system usually occurs and at temperatures higher than 120°F (49°C) the bleaching or sterilizing efficiency of the compositions of our invention is diminished at an excessive rate.

When it is desired to (1) initially form the $C_{10}$—$C_{12}$ straight chain or branched chain diphenyl oxide alkali metal sulfonate or diphenyl oxide derivative-amine oxide-diisoamylene or diisoamylene derivative or diisoamylene-containing perfume oil premix (which is in the gel phase or the liquid phase); (2) then combine the resulting premix with an aqueous alkali metal hypochlorite solution and then (3) adjust the pH of the resulting solution to the range of 11—14.0, then the following temperature of mixing ranges are considered to be within the scope of this invention:

(a) Formation of the diphenyl oxide derivative or diphenyl oxide-amine oxide-diisoamylene or diisoamylene-containing perfume oil premix or diisoamylene derivative or diisoamylene derivative-containing perfume oil premix −20°F−150°F (−7°C−66°C).

(b) Mixing of the premix with aqueous alkali metal hydrochlorite solution −20°F−120°F (−7°C−49°C).

(c) Adjustment of pH of solution to the range of 11—14.0 using aqueous alkali metal hydroxide solution −20°F−120°F (−7°C−49°C).

In any event, whenever a mixing unit operation involves the aqueous alkali metal hypochlorite solution, the temperature of mixing is limited to the range of 20°F−120°F (−7°C−49°C). Where the mixing unit operation involves the mixing of a diisoamylene, diisoamylene derivative, diisoamylene-containing perfume oil or diisoamylene derivative-containing perfume oil, the upper bound of the temperature range is limited by the stability of the least stable ingredient in the diisoamylene or diisoamylene derivative composition of matter or diisoamylene or diisoamylene derivative-containing perfume oil usable in the practice of our invention; and the lower bound of said temperature range is limited by the least temperature where a single liquid phase or gel phase including the perfume oil will exist. Where a unit mixing operation of the process of our invention involves the mixing of one or more diphenyl oxide derivatives having the generic structure:

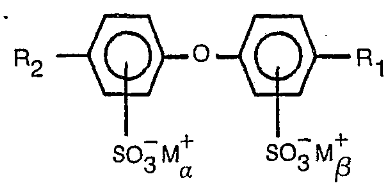

taken alone or taken together with one or more amine oxides having the generic structure:

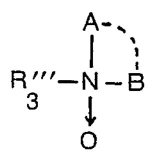

with other materials, the upper bound of the temperature range is the decomposition point of any one of the diphenyl oxide derivatives or amine oxide components and the lower bound is the least temperature where a single liquid phase or gel phase, including the diphenyl oxide derivatives or diphenyl oxide-amine oxide mixture will exist.

Preferred diphenyl oxide derivative compositions from a practical standpoint useful in the practice of our invention are compounds having the structure:

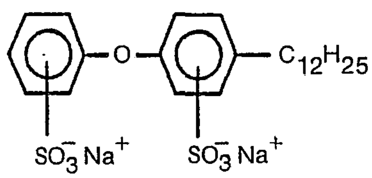

wherein the $C_{12}H_{25}$ moiety represents one or a series of different branched chains; compounds defined according to the structure:

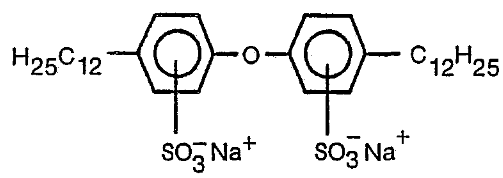

wherein the $C_{12}H_{25}$ moiety represents one or a series of different branched chains; compounds defined according to the structure:

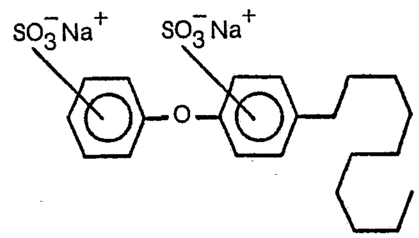

and compounds defined according to the structure:

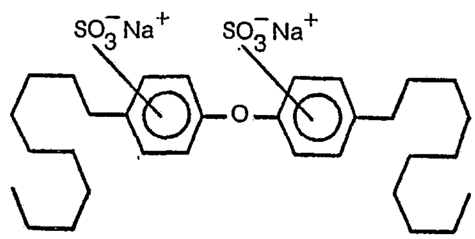

otherwise known as DOWFAX® 2A1 in the case where $R_1$ or $R_2$ represents branched $C_{12}H_{25}$ alkyl chains and the other of $R_1$ or $R_2$ represents hydrogen, or DOWFAX® 3B2 in the case where $R_1$ or $R_2$ represents a straight $C_{10}$ alkyl chain and the other of $R_1$ or $R_2$ represents hydrogen (DOWFAX® being a registered trademark of the Dow Chemical Company of Midland, Michigan).

When used in conjunction with the diphenyl oxide derivatives, preferred amine oxide compositions, from a practical standpoint, useful in the practice of our invention are the commercially available (1) dimethyl "cocoamine" oxide (a mixture which is dominated by di-methyl-$C_{12}$—$C_{16}$ straight chain alkyl amine oxides; more particularly a mixture containing approximately 70% $C_{12}$ straight chain alkyl amine oxides, approximately 25% of straight chain $C_{14}$ alkyl amine oxides and approximately 4% straight chain $C_{16}$ alkyl amine oxides) and (2) N-cocomorpholine oxide, a mixture dominated by straight chain $C_{12}$—$C_{16}$ alkyl morpholine oxides (specifically containing approximately 70% straight chain $C_{12}$ alkyl morpholine oxide, approximately 25% straight chain $C_{14}$ alkyl morpholine oxide, and approximately 4% straight chain $C_{16}$ alkyl morpholine oxide). Commercial examples of such amine oxide compositions are: AROMOX® DMC-W and AROMOX® DMMC-W which are 30% aqueous dimethyl cocoamine oxide solutions and AROMOX® NCMDW which is a 40% aqueous N-cocomorpholine oxide solution, each of which is produced by the Armac Division of AKZO of Chicago, Illinois. These materials are described in Brochure 68011, published by Armour Industrial Chemicals, P.O.B. 1805, Chicago, Illinois 60690. Other preferred amine oxides are n-undecyl dimethyl amine oxide and n-tridecyl dimethyl amine oxide.

The percentage of hypochlorite ion in the compositions of our invention may vary from about 1% up to about 20% for the desired effects to be produced using the diphenyl oxide derivative or diphenyl oxide derivative-amine oxide-diisoamylene or diisoamylene derivative or diisoamylene-containing perfume oil or diisoamylene derivative-containing perfume oil compositions covered by our invention. The usual percent of alkali metal hypochlorite in solution is about 5%, the percentage of sodium hypochlorite in such materials as CLOROX®, the registered trademark of Clorox Corporation.

As stated above, the diisoamylene, diisoamylene derivative, diisoamylene derivative-containing perfume oil or diisoamylene-containing perfume oil used in conjunction with the aqueous alkali metal hypochlorite solution must have such properties as to be able (1) to impart to the resulting "aqueous alkali metal hypochlorite" liquid or gel solution either a woody or piney or herbaceous or eucalyptol-like or minty or sweet or rich or warm woody or fruity or amber or rum/cognac-like or sandalwood or sweet floral or vetiver-like or cedar or patchouli-like or camphoraceous or orris-like aroma or combination thereof; (2) to effect a substantial diminution or elimination of the disagreeable "hypochlorite" aroma which is imparted

to surfaces (e.g. bleached laundry or the hands of the user which are in direct contact with the hypochlorite solution) on which known aqueous alkali metal hypochlorite solutions have been used; and (3) to impart to the surfaces with which such aqueous alkali metal hypochlorite solutions are in contact, a pleasant long-lasting stable herbaceous, piney, woody, eucalyptol-like, minty, sweet, rich, warm woody, fruity, amber, rum/cognac-like, sandalwood, cedar, patchouli-like, camphoraceous and/or orris-like aroma. Examples of ingredients usable and suitable for the aforementioned purposes, that is, usable in conjunction with the diisoamylenes or diisoamylene derivatives and usable in conjunction with the hypochlorites and diphenyl oxide derivatives of our invention are as follows:

a. Material useful for aiding in imparting a "woody" aroma:

1. Cedryl alkyl ethers covered by U.S. Patent No. 3,373,208 such as cedryl methyl ether;

2. Isochroman musks covered by U.S. Patent Nos. 3,360,530 and 3,591,528 such as 6-oxo-1,1,3,3,8-pentamethyl-2,3,5,6,7,8-hexahydro-1H-benz(f)indene;

3. Polycyclic ethers covered by U.S. Patent No. 3,281,432, such as octahydro-1,3a,6-trimethyl-1H-1,6a,ethanopentaleno-(1,2-C)furan;

4. Polycyclic ketones such as hexahydro-1,1,5,5-tetramethyl-2H-2,4a-methanonaphthalen-8-(5H)one.

b. Materials useful for aiding in imparting "herbaceous" aroma:

1. Indane musks covered by U.S. Patent No. 2,889,367 such as 4-acetyl-1,1-dimethyl-6-t-butyl indane;

2. Naphthyl ethers such as β-naphthyl methyl ether and β-naphthyl ethyl ether;

3. Cyclohexyl ethyl ether;

4. Aryl alkanones as described in Canadian Patent No. 780,489 such as 2,5-dimethyl-5-phenylhex-anone-3.

c. Materials useful for imparting a "piney" aroma:

1. "Pinoacetaldehyde" prepared according to U.S. Patent No. 3,636,113;

2. Polycyclic alkanone derivatives prepared according to U.S. Patent No. 4,076,853.

It will be understood that a number of materials which impart such aromas as set forth above will not be useful for use in our invention because they are interalia easily oxidized by the alkali metal hypochlorite in the system. Examples are 1,5,9-trimethyl-12-acetyl-cyclododecatriene-1,5,8 and 1,5,9-trimethyl-12-cyclododecadiene-1,8 covered by British Patent No. 1,204,409.

A basic feature of our invention concerns the fact that the only detergent group needed or desirable in the composition of our invention is the class of diphenyl oxides defined according to the structure:

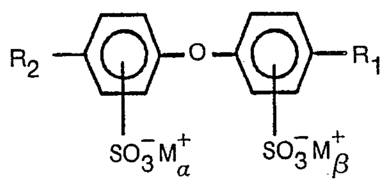

wherein $R_1$, $R_2$, $M_\alpha$ and $M_\beta$ are define supra, taken alone or in conjunction with the class of morpholine and/or dimethyl $C_{11}$—$C_{13}$ straight chain alkyl amine oxides defined according to the structure:

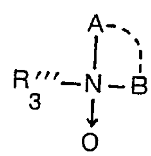

More specifically, such detergents as sodium decyl ether sulfate, sodium myristyl ether sulfate, sodium lauryl ether sulfate and lithium lauryl ether sulfate are neither desired nor are they required. Furthermore, the well known hydrotropes employed in prior art compositions such as the well known family of clarifying agents comprising the alkali metal or alkali earth metal salts of mono- and polyalkylated benzene or naphthalene sulfonates such as sodium xylene or magnesium toluene sulfonate are again neither desired nor are they required in the compositions intended to be encompassed by the instant invention.

Another basic feature of our invention concerns the fact that when it is desired to have a gel phase composition, thickener agents may be employed in conjunction with the system of hypochlorite bleach-diisoamylene or diisoamylene derivative or diisoamylene-containing perfume or diisoamylene derivative-containing perfume-diphenyl oxide derivative or diphenyl oxide derivative-amine oxide derivative, having the structure:

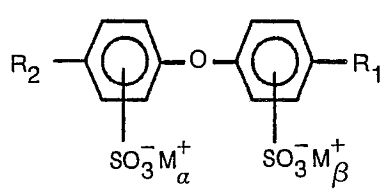

and having the structure:

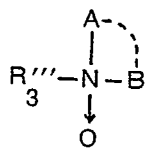

of our invention.

Furthermore, a gel may be formed by adding to the "premix" a thickener agent where the premix contains the diisoamylene, diisoamylene derivative, diisoamylene-containing perfume oil or diisoamylene derivative-containing perfume oil as well as the diphenyl oxide derivative or diphenyl oxide derivative-amine oxide derivative.

Thus, sodium palmitate, sodium stearate, sodium laurate, potassium palmitate, potassium stearate, potassium laurate, lithium palmitate, lithium stearate and/or lithium laurate or combinations of the foregoing may be added to the compositions of matter of our invention to provide a thickened gel-type hypochlorite bleach which is in addition to being in a semi-solid state, unobviously, advantageously and unexpectedly stable over long periods of time. Percentages of thickening agents such as sodium palmitate, sodium stearate, sodium laurate, potassium palmitate, potassium stearate, potassium laurate, lithium palmitate, lithium stearate or lithium laurate or combinations of these which may be used in the thickened compositions of our invention are from 1% by weight up to 12% by weight of the thickener based on overall weight of hypochlorite bleach-diphenyl oxide derivative or diphenyl oxide derivative-amine oxide-diisoamylene, diisoamylene derivative, diisoamylene-containing perfume or diisoamylene derivative-containing perfume composition of our invention. By the same token, the percentages of thickening agents as stated above, which may be used in thickened premix compositions of our invention are from about 5% by weight up to about 60% by weight of thickener based on overall weight of premix which is diphenyl oxide derivative or diphenyl oxide derivative-amine oxide-diisoamylene, diisoamylene derivative, diisoamylene-containing perfume composition or diisoamylene derivative-containing perfume composition of our inventoin.

The following examples are given to illustrate embodiments of the invention as it is now preferred to practice it. It will be understood that these examples are illustrative and the invention is not to be considered restricted thereto except as indicated in the appended claims.

## Example I

*Preparation of Diisoamylene derivatives*

Reaction:

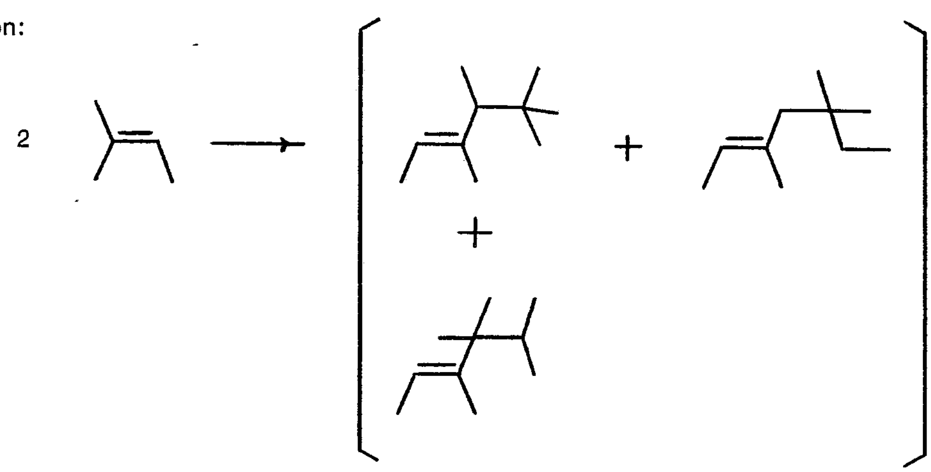

Diisoamylene is prepared according to one of the procedures set forth in the following references:

i — Murphy & Lane, Ind. Eng. Chem., Prod. Res. Dev., Vol. 14, No. 3, 1975 p. 167 (Title: Oligomerization of 2-Methyl-2-Butene in Sulfuric and Sulfuric-Phosphoric Acid Mixtures).

ii — Whitmore & Mosher, Vol. 68, J. Am. Chem. Soc., February, 1946, p. 281 (Title: The Depolymerization of 3,4,5,5-Tetramethyl-2-hexene and 3,5,5-Trimethyl-2-heptene in Relation to the Dimerization of Isoamylenes).

iii — Whitmore & Stahly, Vol. 67, J. Am. Chem. Soc., December, 1945, p. 2158 (Title: The Polymerization of Olefins. VIII The Depolymerization of Olefins in Relation to Intramolecular Rearrangements. ii).

iv — U.S. Patent 3,627,700, issued on December 14, 1971, (Zuech).

v — U.S. Patent 3,538,181, issued on November 3, 1970, (Banks).

vi — U.S. Patent 3,461,184, issued on August 12, 1969 (Hay, et al).

vii — Gurwitsch, Chemische Berichte, 1912, Vol. 2, p. 796 (Production of Di-isoamylene From Isoamylene Using Mercury Acetate Catalyst).

As an illustration, and not by way of limitation, the following Example sets forth the preparation of diisoamylenes useful in producing the fragrances of our invention.

Over a period of ten hours, 2-methyl-2-butene is pumped through a 5′ × 5/8 (0.625 inch) (1.52 m × 16 mm) tube packed with 15.0 g of polystyrene sulfonic acid catalyst, at a temperature of 100°C and at a pressure of 400 psig ($2.8 \times 10^6$ N/m$^2$).

The resulting material was distilled in a fractionation column in order to separate the diisoamylene from the higher molecular weight polymers, which are formed during the reaction as by-products.

Figure 1A represents the GLC profile for the reaction product of example I using a 70% sulfuric acid catalyst at 35°C.

Figure 1B represents the GLC profile for the reaction product of Example I using an Amberlyst® 15 acetic ion exchange resin catalyst at a temperature of 150°C.

Figure 1C represents the GLC profile for the reaction product of Example I, using an Amberlyst® 15 catalyst at 100°C.

Figure 1D represents the GLC profile for the reaction product of Example I, using a sulfuric acid catalyst and an alpha-methylsthyrene diluent at 35°C according to the conditions of United Kingdom Patent Specification 796,130 (crude reaction product).

Figure 1E represents the GLC profile for the reaction product of Example I, using a sulfuric acid catalyst, at 35°C and an alpha-methylstyrene diluent according to the conditions of United Kingdom Patent Specification 796,130 (distilled reaction product).

Figure 2A represents the NMR spectrum for Peak 1 of the GLC profile of Figure 1E. Peak 1 has been determined by analysis to be the compound having the structure:

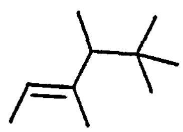

Figure 2B represents the infra-red spectrum for Peak 1 of the GLC profile of Figure 1E.

Figure 3A represents the NMR spectrum for Peak 2 of the GLC profile of Figure 1E. Peak 2 contains compounds having the structures:

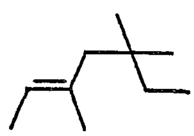

and

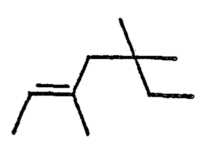

Figure 3B represents the infra-red spectrum for Peak 2 of the GLC profile of Figure 1E.

Figure 4 represents the NMR spectrum for Peak 2 of the GLC profile of Figure 1B.

Example II

*Preparation of diisoamylene epoxide derivatives*

Reaction:

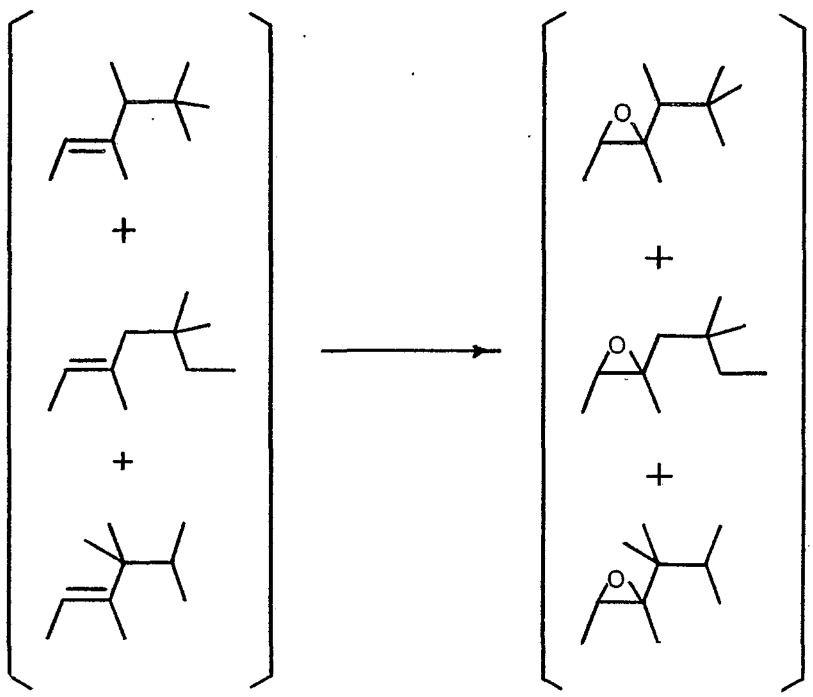

Into a 12 liter reaction flask, equipped with stirrer, reflux condenser, thermometer, addition funnel and cooling bath is placed 5,478 ml of diisoamylene containing the compounds having the structures:

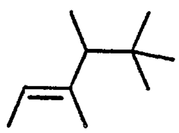 , 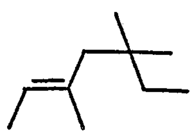 and 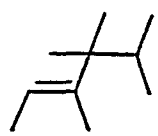

produced according to Example I. To the diisoamylene material is added 120 grams of sodium carbonate. Over a period of 2.5 hours 5,130 grams (4,520 ml or 27 moles) of 40% peracetic acid is added to the reaction mass while maintaining the temperature at 20°C—26°C. 200 grams additional sodium carbonate is then added to the reaction mass. The reaction mass is then cooled to room temperature and transferred to a 5 gallon ($1.9 \times 10^{-2}$ $m^3$) separatory funnel. The reaction mass is then washed as follows:

a. 1 liter water

b. three 1.5 liter portions of 12.5% sodium hydroxide solution (to eliminate peroxides)

c. three 1.5 liter portions of saturated sodium chloride solution.

The reaction mass is then distilled on an 18" (0.46 m) Goodloe column, yielding the following fractions:

| Fraction Number | Vapor Temp. (°C) | Liquid Temp. (°C) | Pressure mm/Hg | N/m$^2$ ×10$^2$ | Weight of Fraction (g) |
|---|---|---|---|---|---|
| 1 | 79/85 | 88/88 | 40/40 | (53) | 180 |
| 2 | 85 | 88 | 40 | ( „ ) | 153 |
| 3 | 85 | 88 | 40 | ( „ ) | 203 |
| 4 | 83/85 | 88/88 | 40 | ( „ ) | 210 |
| 5 | 86 | 88 | 40 | ( „ ) | 176 |
| 6 | 86 | 88 | 40 | ( „ ) | 196 |
| 7 | 86 | 90 | 40 | ( „ ) | 209 |
| 8 | 85/86 | 89/91 | 40 | ( „ ) | 192 |
| 9 | 86 | 98 | 40 | ( „ ) | 138 |
| 10 | 86 | 160 | 40 | ( „ ) | 73 |
| 11 | 84 | 220 | 40 | ( „ ) | 18 |

NMR, IR and mass spectral analysis yield the information that the reaction product contains three compounds having the structures:

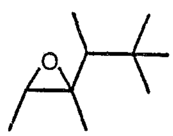 , 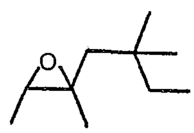 and 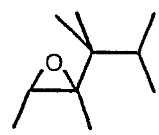

Figure 5 is the GLC profile of the reaction product of Example II containing the compounds having the structures:

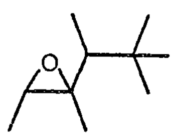 , 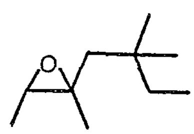 and 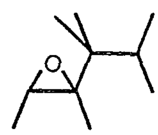

Figure 6 is the NMR spectrum for the reaction product of Example II containing the compounds having the structures:

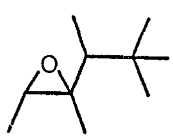 , 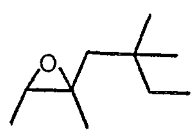 and 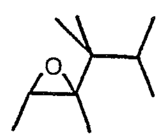

Figure 7 is the infra-red spectrum for the reaction product of Example II containing the compounds having the structures:

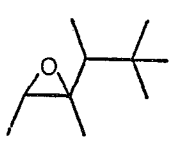 , 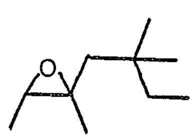 and 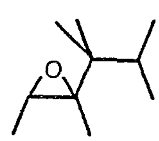

Example III

*Preparation of acetyl derivative of diisoamylene*

Reaction:

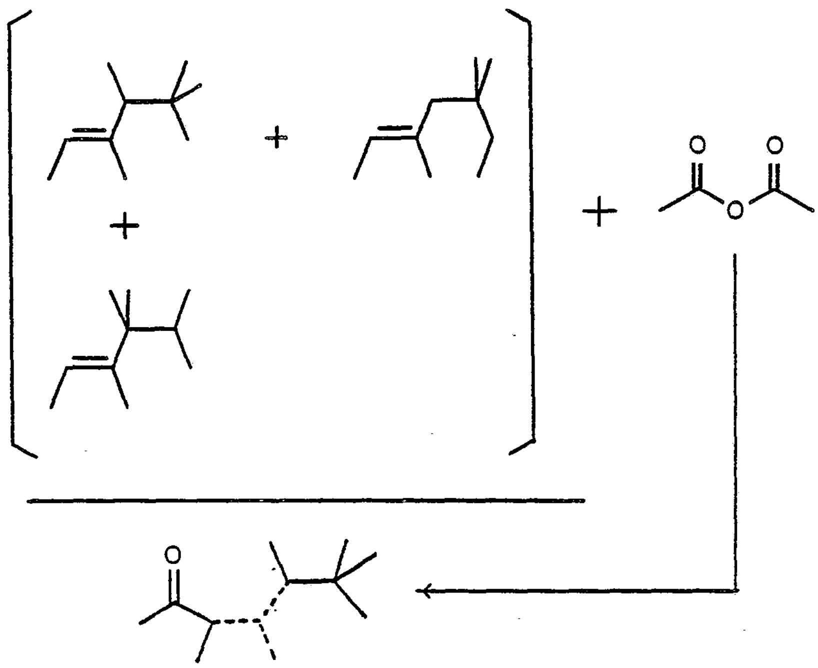

wherein in each of the structures containing dashed lines, these structures represent mixtures of molecules wherein in each of the molecules, one of the dashed lines represents a carbon-carbon double bond and the other dashed lines represent carbon-carbon single bonds.

Into a 2 liter reaction flask equipped with stirrer, thermometer, reflux condenser and heating mantle, is placed 1000 grams of acetic anhydride and 80 grams of boron trifluoride diethyl etherate. The resulting mixture is heated to 80°C and, over a period of 40 minutes, 690 grams of diisoamylene prepared according to the illustration in Example I, supra is added. The reaction mass is maintained at 82—85°C for a period of 5.5 hours, whereupon it is cooled to room temperature. The reaction mass is then added to one liter of water and the resulting mixture is stirred thereby yielding two phases; an organic phase and an aqueous phase. The organic phase is separated from the aqueous phase and neutralized with two liters of 12.5% sodium hydroxide followed by one liter of saturated sodium chloride solution. The resulting organic phase is then dried over anhydrous sodium sulfate and distilled in a one plate distillation column, yielding the following fractions:

| Fraction Number | Vapor Temp. (°C) | Liquid Temp. (°C) | Pressure mm/Hg | (N/m$^2$ × $10^{-2}$) | Weight of Fraction (g.) |
|---|---|---|---|---|---|
| 1 | 33/68 | 62/77 | 8/8 | (11) | 161 |
| 2 | 69 | 79 | 4 | (5) | 100 |
| 3 | 72 | 86 | 3.0 | (4.0) | 191 |
| 4 | 88 | 134 | 3.0 | ( „ ) | 189 |

The resulting material is then distilled on a multi-plate fractionation column, yielding the following fractions at the following reflux ratios:

| Fraction Number | Vapor Temp. (°C) | Liquid Temp. (°C) | Pressure mm/Hg. | (N/m$^2$ × $10^{-2}$) | Reflux Ratio R/D | Weight of Fraction (g.) |
|---|---|---|---|---|---|---|
| 1 | 30/65 | 62/83 | 5/5 | ( 7 ) | 9:1 | 30.8 |
| 2 | 68 | 84 | 5 | ( „ ) | 9:1 | 52.8 |
| 3 | 68 | 85 | 5 | ( „ ) | 9:1 | 34 |
| 4 | 69 | 87 | 5 | ( „ ) | 9:1 | 43 |
| 5 | 69 | 87 | 5 | ( „ ) | 9:1 | 34 |
| 6 | 71 | 88 | 4 | ( 5 ) | 4:1 | 41 |
| 7 | 70 | 88 | 5 | ( 7 ) | 4:1 | 36.5 |
| 8 | 71 | 91 | 5 | ( „ ) | 4:1 | 42 |
| 9 | 73 | 95 | 3 | ( 4 ) | 4:1 | 42.5 |
| 10 | 80 | 106 | 3 | ( „ ) | 4:1 | 39 |
| 11 | 80 | 142 | 3 | ( „ ) | 4:1 | 50.8 |
| 12 | 80 | 220 | 3 | ( „ ) | 4:1 | 24 |

GLC, NMR, IR and mass spectral analyses yield the information that the resulting material is a mixture of cis and trans isomers having a generic structure:

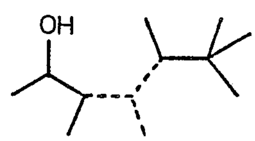

wherein in each of the molecules, one of the dashed lines is a carbon-carbon double bond and the other dashed lines are carbon-carbon single bonds and, primarily, this mixture contains the molecular species (cis and trans isomers) as follows:

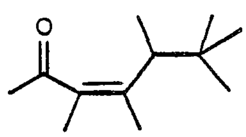 ; 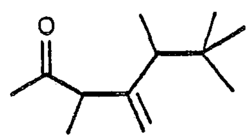 and 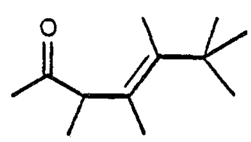

Figure 8 sets forth the GLC profile for the reaction product of this example containing compounds defined according to the structure:

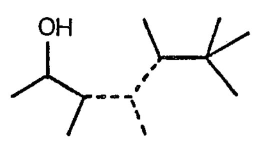

wherein in each molecule of the mixture, one of the dashed lines is a carbon-carbon double bond and the other dashed lines are carbon-carbon single bonds.

Figure 9A represents the infra-red spectrum of Peak 3 of the GLC profile of Figure 8.
Figure 9B represents the infra-red spectrum of Peak 4 of the GLC profile of Figure 8.
Figure 9C represents the infra-red spectrum for Peak 5 of the GLC profile of Figure 8.
Figure 9D represents the infra-red spectrum for Peak 6 of the GLC profile of Figure 8.
Figure 9E represents the infra-red spectrum for Peak 7 of the GLC profile of Figure 8
Figure 9F represents the infra-red spectrum for Peak 8 of the GLC profile of Figure 8.
Figure 9G represents the infra-red spectrum for Peak 9 of the GLC profile of Figure 8.
Figure 9H represents the infra-red spectrum for Peak 10 of the GLC profile of Figure 8.

Figure 9J represents the NMR spectrum for the mixture of compounds having the structures:

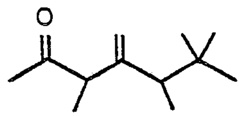

and

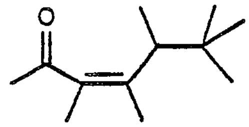

produced according to this example.

Figure 9K represents the NMR spectrum for the compound having the structure:

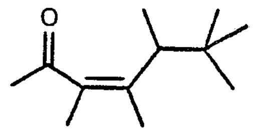

produced according to this example.

Figure 9L represents the NMR spectrum for the compound containing the structure:

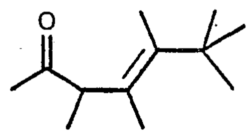

produced according to this example.

Example IV

*Preparation of propionyl derivative of diisoamylenes*

Reaction:

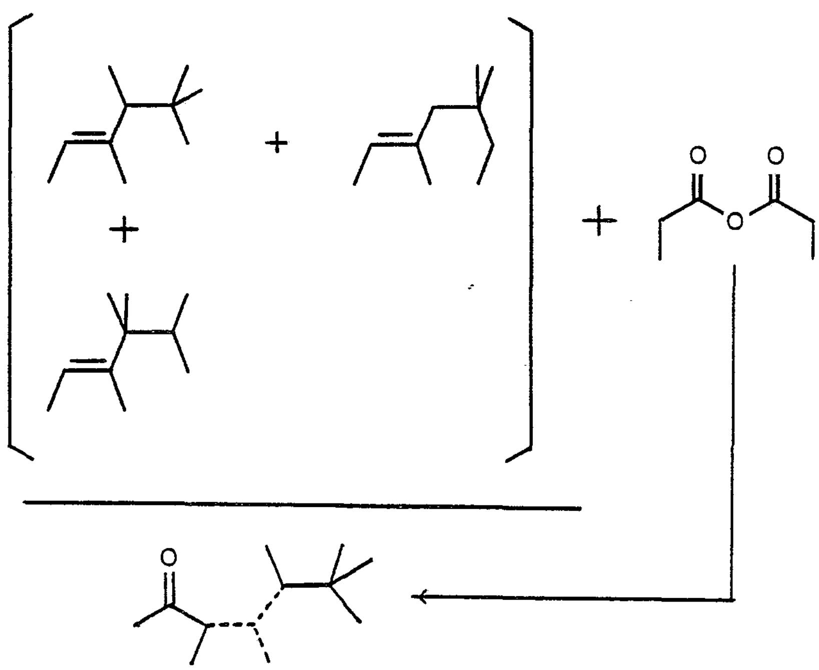

wherein in each of the structures containing dashed lines, these structures represent mixtures of molecules wherein in each of the molecules, one of the dashed lines represents a carbon-carbon double bond and the other dashed lines represent carbon-carbon single bonds.

Into a 5 liter reaction flask equipped with reflux condenser, addition funnel, thermometer, "Thermowatch", heating mantle and nitrogen purge accessory is placed 1000 grams (7.45 moles) of propionic anhydride, 94% and 91.4 ml (0.745 moles) of boron trifluoride etherate. The resulting mixture is heated to 65°C. Over a twenty-five minute period, 1,501 ml (7.45 moles) of the diisoamylene prepared according to the illustration of Example I is added while maintaining the reaction mass at 65—70°C. The reaction mass is then stirred for a period of thirty minutes at 65°C whereupon it is cooled and poured into a 3 liter separatory funnel. 75 ml water is then added, followed by 75 ml 50% aqueous sodium hydroxide and

another 25 ml water. The reaction mass is then poured into a 4 liter beaker and cooled to room temperature using a dry ice-isopropyl alcohol bath. The reaction mass is then added to a 5 liter separatory funnel and the lower aqueous layer is removed. The upper organic phase is washed with 500 cc of saturated sodium sodium chloride. The organic phase is then washed with 500 cc 5% sodium hydroxide followed by 500 cc saturated sodium chloride, followed by 500 cc saturated sodium chloride, followed by 500 cc of 5% sodium hydroxide. The pH of the oil is now in a range of 6—7. The oil is then again washed with 500 cc saturated sodium chloride.

The aqueous phase is extracted with 400 ml diethyl ether. The resulting material is then distilled on a two inch (50 mm) splash column yielding the following fractions:

| Fraction Number | Vapor Temp. (°C) | Liquid Temp. (°C) | Pressure mm/Hg. | ($N/m^2$ $\times 10^{-2}$) | Weight of Fraction (g.) |
|---|---|---|---|---|---|
| 1 | 25/75 | 60/85 | 50/50 | (67) | 144 |
| 2 | 74 | 87 | 38 | (51) | 184 |
| 3 | 34 | 40 | 4 | ( 5 ) | 186 |
| 4 | 55 | 78 | 3 | ( 4 ) | 212 |
| 5 | 87 | 94 | 3 | ( „ ) | 181 |
| 6 | 95 | 114 | 3 | ( „ ) | 210 |
| 7 | 170 | 155 | 3 | ( „ ) | 80 |
| 8 | 160 | 225 | 3 | ( „ ) | 42 |

Fractions 5, 6 and 7 are then bulked for redistillation and the bulked material is distilled on a one inch (25 mm) Goodloe Silver Mirror column, yielding the following fractions:

| Fraction Number | Vapor Temp. (°C) | Liquid Temp. (°C) | Pressure mm/Hg. | ($N/m^2$ $\times 10^{-2}$) | Reflux Ratio R/D | Weight of Fraction (g.) |
|---|---|---|---|---|---|---|
| 1 | 17/60 | 81/90 | 3/1.4 | (4/1.9) | 1:4 | 42 |
| 2 | 58 | 89 | 1.0 | (1.3) | 4:1 | 48 |
| 3 | 63 | 93 | 1.0 | ( „ ) | 4:1 | 37 |
| 4 | 68 | 94 | 1.0 | ( „ ) | 4:1 | 48 |
| 5 | 70 | 94 | 1.0 | ( „ ) | 4:1 | 43 |
| 6 | 72 | 95 | 1.8 | (2.4) | 2:1 | 39 |
| 7 | 72 | 94 | 1.7 | (2.3) | 2:1 | 87 |
| 8 | 74 | 108 | 1.6 | (2.1) | 2:1 | 48 |
| 9 | 82 | 135 | 1.6 | ( „ ) | 2:1 | 48 |
| 10 | 110 | 220 | 1.0 | (1.3) | 2:1 | 37 |

Fractions 2—10 are then bulked and redistilled on a 1 foot (305 mm) Goodloe Silver Mirror column, again yielding the following fractions:

| Fraction Number | Vapor Temp. (°C) | Liquid Temp. (°C) | Pressure mm/Hg. | (N/m² × $10^{-2}$) | Reflux Ratio | Weight of Fraction (g.) |
|---|---|---|---|---|---|---|
| 1 | 52/58 | 83/85 | 1.4/1.2 | (1.9/1.6) | 4:1 | 46 |
| 2 | 59 | 86 | 1.1 | (1.5) | 4:1 | 50 |
| 3 | 61 | 89 | 1.1 | ( „ ) | 4:1 | 53 |
| 4 | 61 | 89 | .9 | (1.2) | 4:1 | 57 |
| 5 | 61 | 91 | .8 | (1.1) | 4:1 | 44 |
| 6 | 61 | 91 | .8 | ( „ ) | 4:1 | 41 |
| 7 | 65 | 101 | .8 | ( „ ) | 4:1 | 42 |
| 8 | 68 | 115 | .8 | ( „ ) | 4:1 | 49 |
| 9 | 74 | 135 | .8 | ( „ ) | 4:1 | 17 |
| 10 | 88 | 230 | .8 | ( „ ) | 4:1 | 17 |

The resulting material is analyzed using GLC, IR, mass spectral and NMR analyses, yielding information that the resulting material is a mixture of compounds defined according to the generic structure:

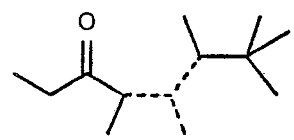

wherein in each molecule of the mixture, one of the dashed lines is a carbon-carbon double bond and the other dashed lines are carbon-carbon single bonds.

Figure 10 represents the GLC profile for the reaction product of this example containing a mixture of compounds each of which is defined according to the generic structure:

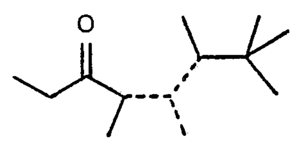

wherein in each molecule, one of the dashed lines is a carbon-carbon double bond and the other dashed lines are carbon-carbon single bonds.

Figure 11 represents the infra-red spectrum for the product produced according to this example containing the compounds having the structures:

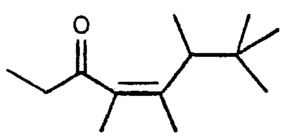 ; 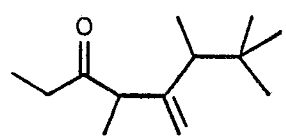 and 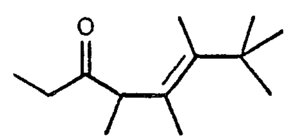

Figure 12 represents the mass spectrum for the reaction product of this example, containing the compounds having the structures:

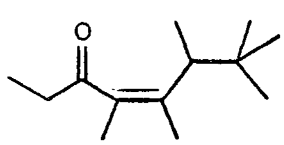 ; 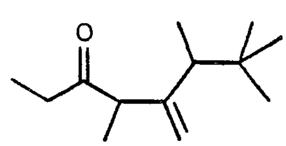 and 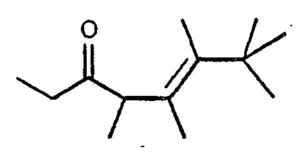

Example V

*Preparation of n-butyryl derivative of diisoamylene*

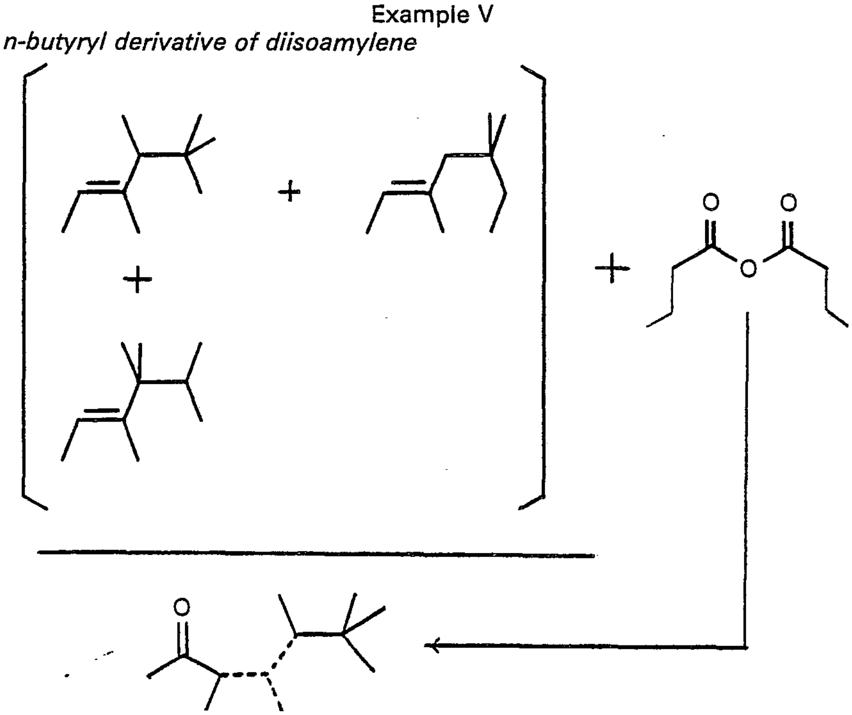

wherein in each of the structures containing dashed lines, these structures represent mixtures of molecules wherein in each of the molecules, one of the dashed lines represents a carbon-carbon double bond and the other dashed lines represent carbon-carbon single bonds.

Into a 5 liter reaction flask equipped with electric stirrer, heating mantle, thermometer, 24/40 "Y" joint, addition funnel and reflux condenser is added 960 grams of n-butyric anhydride, followed by 105 ml boron trifluoride. The resulting mixture is heated to 65°C and a Thermowatch is attached (reaction must not exceed a pot temperature of 65°C).

The reaction mass is heated to 65°C and dropwise addition of 1,725 ml of diisoamylene, prepared according to the illustration of Example I is added over a period of 3.5 hours while maintaining the reaction mass at a temperature of 65°C.

At the end of the addition, the reaction mass is cooled to 38°C and then transferred to a 5 liter separatory funnel. 75 ml of 50% aqueous sodium hydroxide and 100 ml water are then added to the reaction mass. The reaction mass now exists in two phases; an aqueous phase and an organic phase. The organic phase is washed with one liter of saturated sodium chloride solution thereby creating a pH of 4—5. The reaction mass is then washed with 1 liter of 12.5% sodium hydroxide, stirred for fifteen minutes, and then separated. The resulting organic phase is then dried over anhydrous magnesium sulfate and distilled on a 1 inch (25 mm) Stone column yielding the following fractions:

| Fraction Number | Vapor Temp. (°C) | Liquid Temp. (°C) | Pressure mm/Hg. | ($N/m^2 = 10^{-2}$) |
|---|---|---|---|---|
| 1 | 40/46 | 63/65 | 30/20 | (40/27) |
| 2 | 66 | 77 | 40 | (53) |
| 3 | 66 | 77 | 35 | (47) |
| 4 | 66 | 87 | 33 | (44) |
| 5 | 69 | 90 | 20 | (27) |
| 6 | 64 | 100 | 15 | (20) |
| 7 | 95 | 110 | 2 | ( 3 ) |
| 8 | 97 | 110 | 2 | ( „ ) |
| 9 | 125 | 160 | 2 | ( „ ) |

The resulting fractions 7, 8 and 9 are bulked and redistilled on a 2 foot (610 mm) stainless steel column yielding the following fractions:

| Fraction Number | Vapor Temp. (°C) | Liquid Temp. (°C) | Pressure mm/Hg. | ($N/m^2$ $\times 10^{-2}$) | Reflux Ratio | Weight of Fraction (g.) |
|---|---|---|---|---|---|---|
| 1 | 47/74 | | 1.8 | (2.4) | 4:1 | 53 |
| 2 | 74 | 105 | 1.4 | (1.9) | 4:1 | 85 |
| 3 | 74 | 107 | 1.4 | ( „ ) | 4:1 | 96 |
| 4 | 74 | 107 | 1.4 | ( „ ) | 4:1 | 89 |
| 5 | 70 | 105 | 1.0 | (1.3) | 4:1 | 66 |
| 6 | 75 | 110 | 1.0 | ( „ ) | 4:1 | 44 |
| 7 | 84 | 165 | 1.0 | ( „ ) | 4:1 | 66 |
| 8 | 80 | 220 | 1.0 | ( „ ) | 4:1 | 12 |

Figure 13 represents the GLC profile for the reaction product of this example containing compounds defined according to the generic structure:

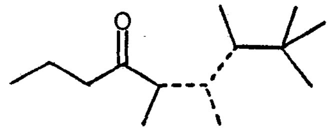

wherein in each of the molecules of the mixture, one of the dashed lines represents a carbon-carbon double bond and the other dashed lines represent carbon-carbon single bonds.

Figure 14 represents the infra-red spectrum for the reaction product of this example containing the compounds having the structures:

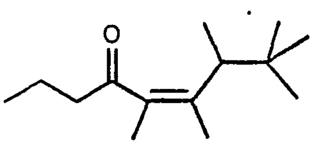

,

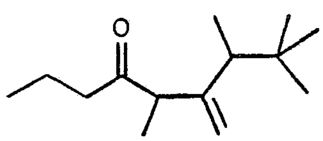

and

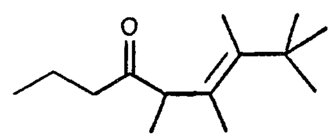

Figure 15 represents the mass spectrum for the reaction product of this example containing the compound having the structures:

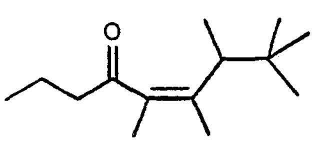

,

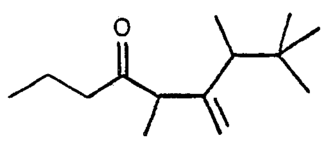

and

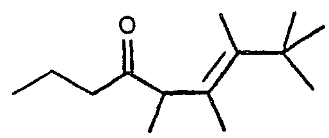

Example VI

*Preparation of isobutyryl derivative of diisoamylene*

Reaction:

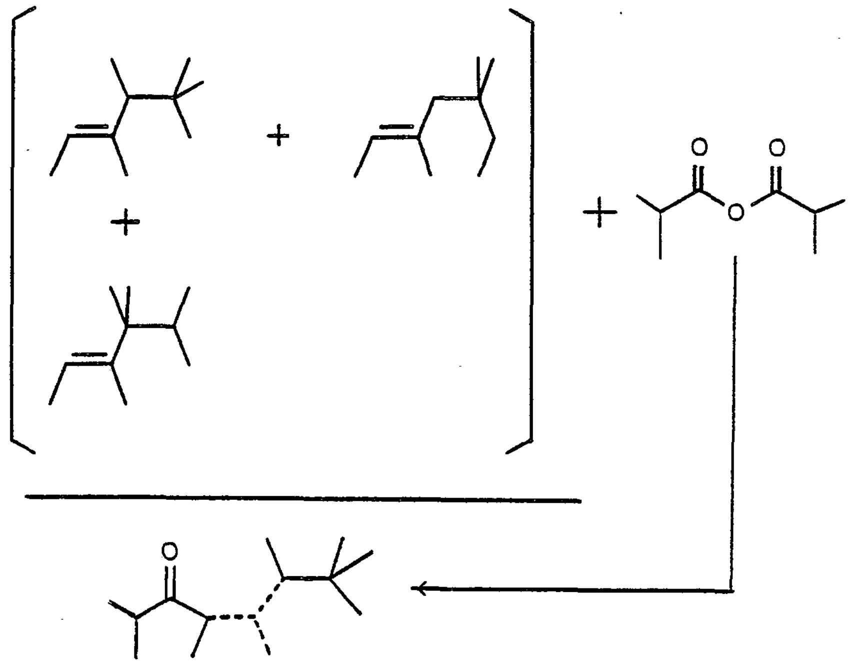

wherein in each of the structures containing dashed lines, these structures represent mixtures of molecules wherein in each of the molecules, one of the dashed lines represents a carbon-carbon double bond and the other dashed lines represent carbon-carbon single bonds.

Into a 5 liter reaction flask equipped with reflux condenser, addition funnel, thermometer, Thermowatch, heating mantle and nitrogen purge accessory is placed 1361 grams (8.6 moles) of isobutyric anhydride. 105 ml (0.86 moles) of boron trifluoride etherate is then added to the isobutyric anhydride. The resulting mixture is then heated to 65°C. Over a period of 4 hours, 1725 grams (8.6 moles) of diisoamylene prepared according to the illustration of Example I is added to the reaction mass, while maintaining the reaction mass at a temperature of 83—85°C.

The reaction mass is then cooled to room temperature and is added to a 5 liter separatory funnel. 75 ml of 50% sodium hydroxide (aqueous) and 100 ml water is then added to the reaction mass thus yielding two phases, an aqueous phase and an organic phase. The lower aqueous phase is removed and the organic phase is washed as follows:

A — 1 liter saturated sodium chloride
B — 1 liter 5% aqueous sodium hydroxide
C — 1 liter saturated sodium chloride
D — 1 liter 12.5% sodium hydroxide
E — 1 liter 12.5% sodium hydroxide

The reaction mass is then distilled on a two inch (50 mm) splash column packed with stones yielding the following fractions:

| Fraction Number | Vapor Temp. (°C) | Liquid Temp. (°C) | Pressure mm/Hg. | ($N/m^2$ × $10^{-2}$) | Weight of Fraction (g.) |
|---|---|---|---|---|---|
| 1 | 29/54 | 54/68 | 29/24 | (39/32) | Starting material |
| 2 | 51 | 68 | 14 | (19) | " |
| 3 | 90 | 68 | 11 | (15) | " |
| 4 | 64 | 98 | 11 | ( " ) | " |
| 5 | 92/94 | 102/108 | 7/5 | (9/7) | 378 |
| 6 | 135 | 165 | 5 | ( 7 ) | 257 |

Fractions 5 and 6 of the resulting distillate are then bulked and redistilled yielding the following fractions:

| Fraction Number | Vapor Temp. (°C) | Liquid Temp. (°C) | Pressure mm/Hg. | ($N/m^2$ × $10^{-2}$) | Reflux Ratio | Weight of Fraction (g.) |
|---|---|---|---|---|---|---|
| 1 | 15/45 | 88/92 | 3/2.5 | (4/3.3) | 4:1 | 21 |
| 2 | 60 | 99 | 2.4 | (3.2) | 4:1 | 13 |
| 3 | 67 | 98 | 2.4 | ( " ) | 4:1 | 35 |
| 4 | 69 | 97 | 2.2 | (2.9) | 4:1 | 49 |
| 5 | 70 | 99 | 2.2 | ( " ) | 4:1 | 59 |
| 6 | 70 | 101 | 2.2 | ( " ) | 4:1 | 50 |
| 7 | 70 | 101 | 2.0 | (2.7) | 4:1 | 37 |
| 8 | 84 | 112 | 1.7 | (2.3) | 4:1 | 33 |
| 9 | 84 | 112 | 1.7 | ( " ) | 4:1 | 63 |
| 10 | 78 | 119 | 1.8 | (2.4) | 4:1 | 37 |
| 11 | 84 | 122 | 1.7 | (2.3) | 4:1 | 51 |
| 12 | 92 | 121 | 1.7 | ( " ) | 4:1 | 43 |
| 13 | 101 | 156 | 1.6 | (2.1) | 4:1 | 27 |
| 14 | 121 | 178 | 1.6 | ( " ) | 4:1 | 85 |
| 15 | 110 | 220 | 1.6 | ( " ) | 4:1 | 33 |

Fractions 3—9 of this distillation are then rebulked and redistilled on a 12 inch (305 mm) Goodloe Silver Mirror column yielding the following fractions:

| Fraction Number | Vapor Temp. (°C) | Liquid Temp. (°C) | Pressure mm/Hg. | (N/m² × 10⁻²) | Reflux Ratio | Weight of Fraction (g.) |
|---|---|---|---|---|---|---|
| 1 | 47/60 | 84/92 | 1.6/1.2 | (2.1/1.6) | 4:1 | |
| 2 | 67 | 93 | 1.2 | (1.6) | 4:1 | 50 |
| 3 | 67 | 94 | 1.2 | ( „ ) | 4:1 | 50 |
| 4 | 67 | 95 | 1.2 | ( „ ) | 4:1 | 52 |
| 5 | 67 | 95 | 1.2 | ( „ ) | 4:1 | 50 |
| 6 | 67 | 98 | 1.2 | ( „ ) | 4:1 | 57 |
| 7 | 67 | 101 | 1.2 | ( „ ) | 4:1 | 57 |
| 8 | 72 | 212 | 1.2 | ( „ ) | 4:1 | 42 |

The resulting reaction product is analyzed by means of GLC, NMR, IR and mass spectral analyses and this confirms that the reaction product is a mixture of compounds defined according to the generic structure:

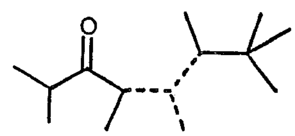

wherein in each of the molecules, one of the dashed lines is a carbon-carbon double bond and the other two of the dashed lines represent carbon-carbon single bonds. The major components of this mixture are compounds having the structures:

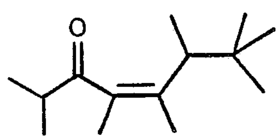 , 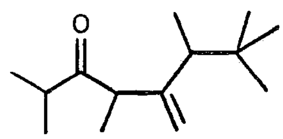 and 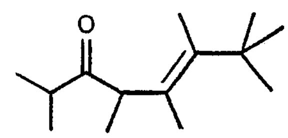 .

Figure 16 represents the GLC profile for the reaction product of this example containing a mixture of compounds, each of which is defined according to the generic structure:

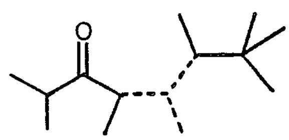

wherein in each of the molecules, one of the dashed lines represents a carbon-carbon double bond and the other dashed lines represent carbon-carbon single bonds.

Figure 17 represents the infra-red spectrum for the reaction product of this example containing the compounds having the structures:

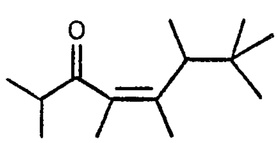 , 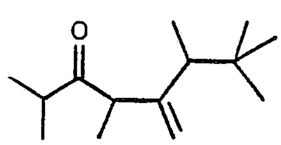 and 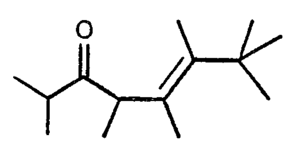

Figure 18 represents the mass spectrum for the reaction product of this example containing the compounds having the structures:

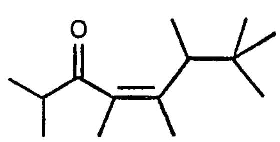 , 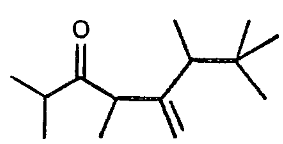 and 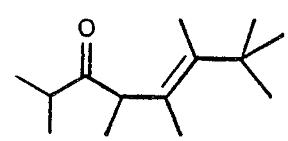

Example VII

*Preparation of acetyl derivative of diisoamylene*

Reaction:

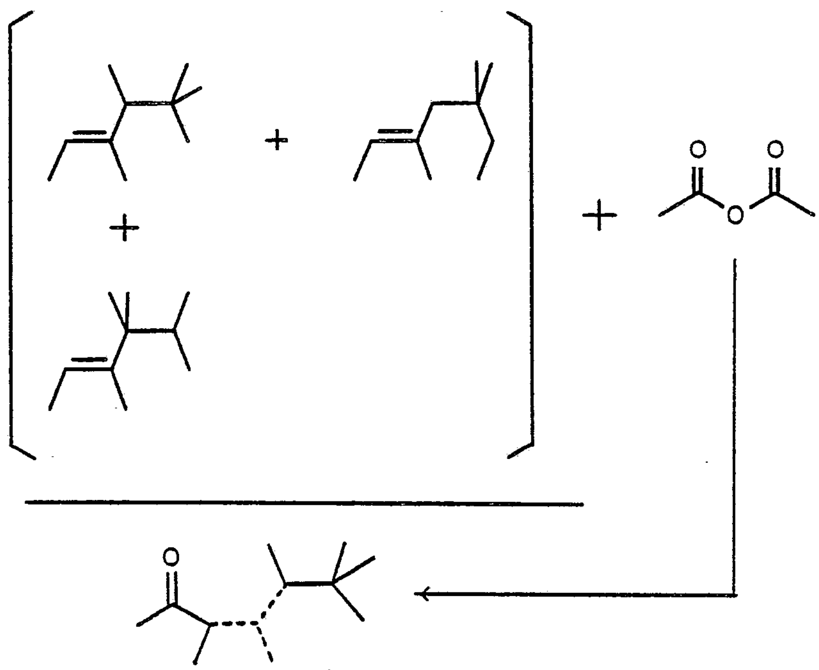

Example VII(A)

Into a 5 liter reaction flask equipped with electric stirrer, thermometer, addition funnel, 24/42 y-tube, condenser, heating mantle and nitrogen purge accessories are added 41 ml of 70% methane sulfonic acid followed by 30 grams of phosphorous pentoxide. The resulting mixture exotherms to 60°C.

Over a period of 7 minutes, 235 ml acetic anhydride is added to the reaction mass while maintaining same at a temperature of 65°C. Over a period of 30 minutes while maintaining the reaction temperature at 80°C, 516 ml of diisoamylene prepared according to the illustration of Example I is added dropwise to the reaction mass. At the end of the addition of the diisoamylene, GLc analysis indicates 42% product.

The reaction mass is added to a 5 gallon ($1.9 \times 10^{-2}$ $m^3$) open head separatory flask containing 1 liter of water.

The resulting mixture is washed with 1 liter of 12% sodium hydroxide followed by 1 liter of saturated sodium chloride solution. 100 ml toluene is added to help separation. GLC, NMR, IR and mass spectral analyses yield the information that the resulting organic phase is a mixture of compounds defined according to the generic structure:

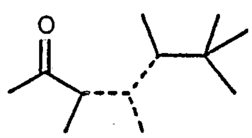

wherein in each of the molecules one of the dashed lines is a carbon-carbon double bond and the other two of the dashed lines represent carbon-carbon single bonds.

The resulting reaction product is then dried over anhydrous magnesium sulfate and distilled on a 3 inch (76 mm) Stone column yielding the following fractions:

| Fraction Number | Vapor Temp. (°C) | Liquid Temp. (°C) | Pressure mm/Hg. | ($N/m^2 \times 10^{-2}$) |
|---|---|---|---|---|
| 1 | 65/65 | 103/92 | 113/35 | (151/47) |
| 2 | 60 | 80 | 1 | ( 1 ) |
| 3 | 52 | 89 | 1 | ( „ ) |
| 4 | 61 | 134 | 1 | ( „ ) |
| 5 | 73 | 140 | 1 | ( „ ) |

Fractions 2, 3 and 4 are bulked and evaluated for their organoleptic properties.

Figure 19 represents the GLC profile for the reaction product of this example containing structures defined according to the genus having the structure:

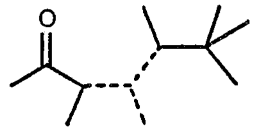

wherein in each of the molecules of the mixture, one of the dashed lines represents a carbon-carbon double bond and the other dashed lines represent carbon-carbon single bonds.

Example VII(B)

To a 500 ml reaction flask equipped with reflux condenser, addition funnel, thermometer, Thermowatch, heating mantle cooling bath and nitrogen purge accessories, is added 406 ml of acetic anhydride and 30 ml boron trifluoride etherate. The reaction mass is heated to 60°C and while maintaining the reaction mass at 60°C over a period of 30 minutes, diisoamylene prepared according to the illustration of Example I is added. The resulting reaction mass is then heated, with stirring at 60°C for a period of 12 hours. At the end of the 12 hour period, the reaction mass is distilled yielding the following fractions:

| Fraction Number | Vapor Temp. (°C) | Liquid Temp. (°C) | Pressure mm/Hg. | ($N/m^2$ $\times 10^{-2}$) | Weight of Fraction (g.) |
|---|---|---|---|---|---|
| 1 | 50/58 | 60/70 | 2.5 | (3.3) | 330 |
| 2 | 67 | 87 | 1.4 | (1.9) | 329 |
| 3 | 71 | 88 | 3.0 | (4.0) | 65 |
| 4 | 90 | 115 | 3.0 | ( „ ) | 195 |

The resulting mass, by GLC, IR, NMR and mass spectral analyses consists of compounds defined according to the generic structure:

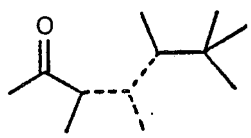

wherein in each of the molecules one of the dashed lines is a carbon-carbon double bond and the other two of the dashed lines represent carbon-carbon single bonds.

Figure 20 sets forth the GLC profile for the reaction product of this example.

Example VIII

*Preparation of propionyl derivative of diisoamylene*

Reaction:

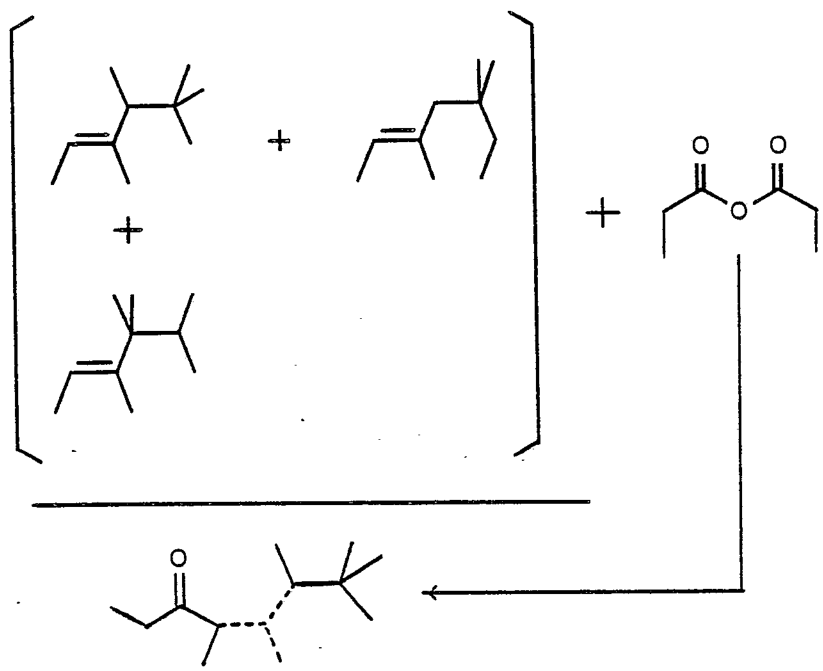

Into 5000 ml reaction flask equipped with reflux condenser, addition funnel, thermometer, Thermowatch, heating mantle, cooling bath and nitrogen purge accessory, is added 415 ml propionic anhydride, 11 grams of methane sulfonic acid and 35 ml of boron trifluoride etherate. The reaction mass is heated to 60°C and over a period of 30 minutes, 1,850 ml of diisoamylene prepared according to the illustration of Example I is added. The reaction mass is then stirred at 60°C for a period of 12 hours. At the end of the 12 hour period, the reaction mass is distilled on a Goodloe fractionation column to yield a mixture of compounds having the generic structure:

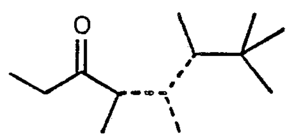

wherein in each of the molecules therein, one of the dashed lines represents a carbon-carbon double bond and each of the other of the dashed lines represents a carbon-carbon single bond. The reaction structures are confirmed by GLC, NMR, IR and mass spectral analyses.

Example IX(A)

*Preparation of isobutyryl derivative of diisoamylene*

Reaction:

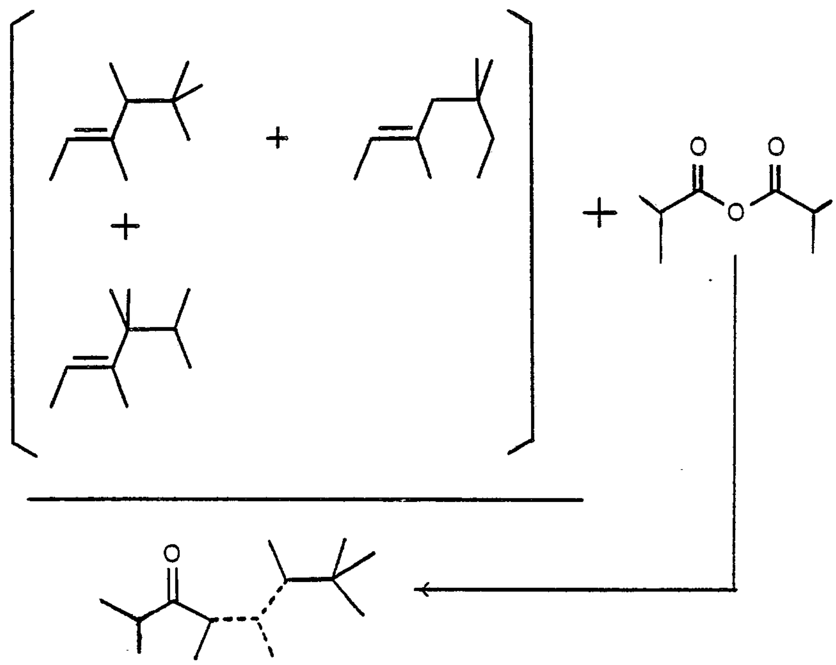

wherein in each of the structures containing dashed lines, these structures represent mixtures of molecules wherein in each of the molecules, one of the dashed lines represents a carbon-carbon double bond and the other dashed lines represent carbon-carbon single bonds.

Into a 5000 ml reaction flask equipped with reflux condenser, addition funnel, thermometer, Thermowatch, heating mantle, cooling bath and nitrogen gas purge accessory, is added 953 ml (6.0 moles) of isobutyric anhydride; 183 grams of polyphosphoric acid and 135 ml 70% methane sulfonic acid. The reaction mass exotherms to 65°C.

Over a period of 20 minutes, while maintaining the reaction mass at 65°C, 1725 grams (8.6 moles) of diisoamylene prepared according to the illustration of Example I is added to the reaction mass. The reaction mass is then heated to 85°C and maintained at that temperature for a period of 10 hours. At the end of the 10 hour period, the reaction mass is cooled and 100 grams of sodium acetate and 1 liter of water are added thereto. The resulting mixture is added to a 5 liter separatory funnel and organic layer is then washed as follows:

A — 1 liter 12.5% sodium hydroxide
B — 2 liters 12.5% sodium hydroxide
C — 1 liter of saturated sodium chloride.

The reaction mass is then distilled on a 1 foot (305 mm) Goodloe column yielding the following fractions:

| Fraction Number | Vapor Temp. (°C) | Liquid Temp. (°C) | Pressure mm/Hg. | ($N/m^2$ $\times 10^{-2}$) | Reflux Ratio | Weight of Fraction (g.) |
|---|---|---|---|---|---|---|
| 1 | 35/50 | 88/93 | 1.8/0.08 | (2.4/0.11) | 4:1 | 41 |
| 2 | 63 | 100 | 0.8 | (1.1) | 4:1 | 48 |
| 3 | 63 | 105 | 0.6 | (0.8) | 4:1 | 73 |
| 4 | 66 | 114 | 0.6 | ( „ ) | 4:1 | 44 |
| 5 | 100 | 145 | 0.6 | ( „ ) | 4:1 | 42 |
| 6 | 101 | 225 | 0.6 | ( „ ) | 4:1 | 29 |

GLC, NMR, IR and mass spectral analyses confirm the information that the resulting product is a mixture of compounds defined according to the generic structure:

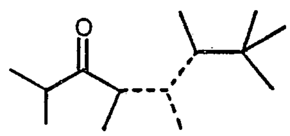

wherein in each molecule of the mixture, one of the dashed lines is a carbon-carbon double bond and the other two of the dashed lines represent carbon-carbon single bonds.

Figure 21 sets forth the GLC profile for the reaction product of this example. (Conditions: SF 96 column, six foot × 1/4 inch (1.83 m × 6 mm); operated at 180°C isothermal).

Example IX(B)

Into a 5000 ml reaction flask equipped with reflux condenser, addition funnel, thermometer, Thermowatch, heating mantle, cooling bath and nitrogen gas purge accessory, is added 953 grams (6.0 moles) of isobutyric anhydride and 105 ml (0.86 moles) of boron trifluoride eterate. The reaction mass is heated to 65°C over a period of 30 minnutes 1725 ml (8.6 moles) of diisoamylene prepared according to the illustration of Example I is added. The reaction mass is then heated to 63—65°C and maintained with stirring at that temperature for a period of 12 hours.

The reaction mass is then cooled to room temperature and 82 grams of sodium acetate are added. The reaction mass is then poured into a 5 liter separatory funnel and washed as follows:

A — 1 liter water
B — 1 liter 12.5% aqueous sodium hydroxide
C — 1 liter 12.5% aqueous sodium hydroxide
D — 1 liter 12.5% aqueous sodium hydroxide
E — 1 liter saturated sodium chloride

The organic layer is then dried over anhydrous sodium sulfate and distilled on a 12 inch (305 mm) Goodloe column yielding the following fractions:

| Fraction Number | Vapor Temp. (°C) | Liquid Temp. (°C) | Pressure mm/Hg. | ($N/m^2$ × $10^{-2}$) | Reflux Ratio | Weight of Fraction (g.) |
|---|---|---|---|---|---|---|
| 1 | 55/67 | 85/92 | 2.4/1.5 | (3.2/2.0) | 4:1 | 50 |
| 2 | 72 | 95 | 1.5 | (2.0) | 4:1 | 72 |
| 3 | 73 | 98 | 1.5 | ( „ ) | 4:1 | 83 |
| 4 | 75 | 104 | 1.4 | (1.9) | 4:1 | 69 |
| 5 | 80 | 112 | 1.4 | ( „ ) | 4:1 | 69 |
| 6 | 80 | 112 | 1.4 | ( „ ) | 4:1 | 12 |
| 7 | 108 | 140 | 1.4 | ( „ ) | 2:3 | 69 |
| 8 | 116 | 180 | 1.4 | ( „ ) | 2:3 | 61 |
| 9 | 110 | 225 | 1.4 | ( „ ) | 2:3 | 9 |

GLC, NMR, IR and mass spectral analyses confirm that the resulting product is a mixture of compounds defined according to the generic structure:

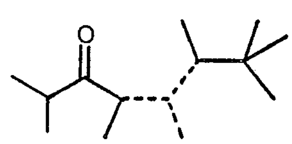

wherein in each of the molecules in the mixture one of the dashed lines represents a carbon-carbon double bond and the other two of the dashed lines represent carbon-carbon single bonds.

Example X

*Preparation of acetyl diisobutylene reaction product*

Reaction:

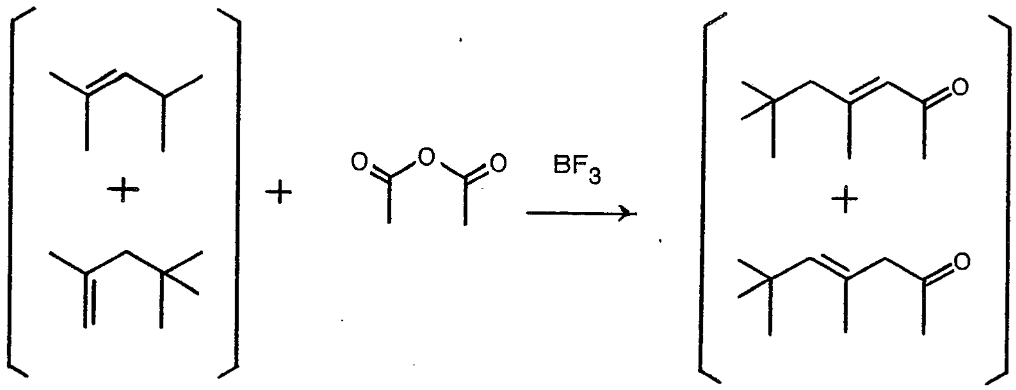


Into a five liter, three-neck reaction vessel equipped with stirrer, thermometer, reflux condenser, heating mantle and addition funnel is placed 1,300 grams of acetic anhydride and 195 grams of boron trifluoride etherate. The resulting mixture is heated to a temperature of 80—85°C. At this point, 690 grams of diisobutylene being a mixture of compounds having the structures:

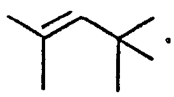
and
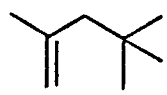

is added dropwise while maintaining the reaction temperature at 80—82°C, over a period of one hour. At the end of the one hour period, the reaction mass is continued to be stirred and maintained at 83°C. At the end of 3.5 hours, the reaction mass is cooled and poured into one liter of water. The reaction mass is then washed as follows:

A—two one-liter water portions

B—Two one-liter 10% aqueous sodium hydroxide portions

C—Two one-liter saturated sodium chloride portions.

The reaction mass is now at a pH of 7. The reaction mass is dried and stripped of solvent using a crude oil weighing 620 grams. The reaction mass is then distilled on a 1′ (305 mm) Goodloe column at reflux ratios of between 9:1 and 4:1 yielding the following fractions:

| Fraction Number | Vapor Temp. (°C) | Liquid Temp. (°C) | Pressure mm/Hg | ($N/m^2$ $\times 10^{-2}$) | Reflux Ratio | Weight of Fraction (g.) |
|---|---|---|---|---|---|---|
| 1 | 43/50 | | 3.0/30 | (4.0/40) | 9:1 | 39 |
| 2 | 50 | 59 | 2.0 | (2.7) | 9:1 | 40 |
| 3 | 50 | 60 | 2.0 | ( „ ) | 9:1 | 43 |
| 4 | 51 | 60 | 2.0 | ( „ ) | 9:1 | 35 |
| 5 | 52 | 61 | 2.0 | ( „ ) | 9:1 | 42 |
| 6 | 52 | 63 | 2.0 | ( „ ) | 9:1 | 43 |
| 7 | 53 | 63 | 2.0 | ( „ ) | 4:1 | 43 |
| 8 | 53 | 63 | 2.0 | ( „ ) | 4:1 | 44 |
| 9 | 53 | 65 | 2.0 | ( „ ) | 9:1 | 41 |
| 10 | 53 | 81 | 2.0 | ( „ ) | 9:1 | 39 |
| 11 | 53 | 223 | 2.0 | ( „ ) | 9:1 | 19 |

The reaction product is determined by GLC, NMR and mass spectral analyses to contain compounds having the structures:

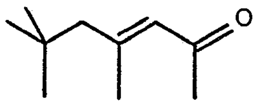 and 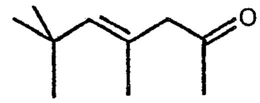

Figure 22 is the GLC profile for distillation fraction 2 of the reaction product of this example containing the compounds having the structures:

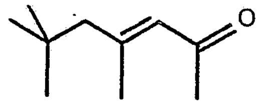 and 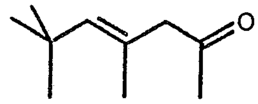

Figure 23 is the GLC profile for distillation fraction 9 of the reaction product of this example containing the compounds having the structures:

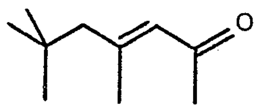 and 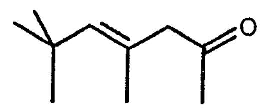

Figure 24 is the NMR spectrum for peak 1 of the GLC profile of Figure 23 containing the compounds having the structures:

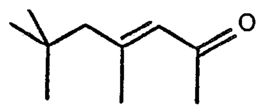 and 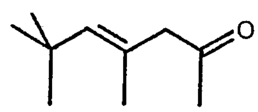

Figure 25 is the NMR spectrum for peak 2 of the GLC profile of Figure 23 containing the compounds having the structures:

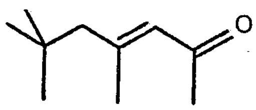 and 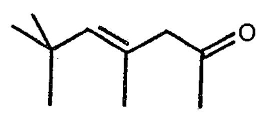

Figure 26 is the infra-red spectrum for peak 1 of the GLC profile of Figure 23 containing the compounds having the structures:

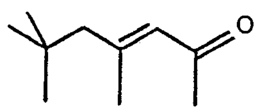 and 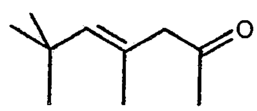

Figure 27 is the infra-red spectrum for peak 2 of the GLC profile of Figure 23 containing the compounds having the structures:

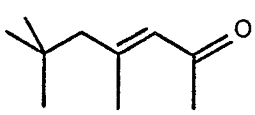 and 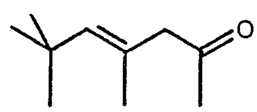

Example XI

*Preparation of propylene ketal of acetal diisoamylene*

Example XI(A)

Reaction:

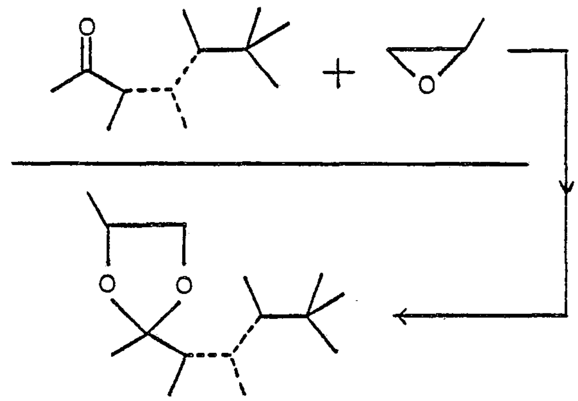

Into a 2000 ml reaction flask equipped with stirrer, heating mantle, addition funnel, reflux condenser, thermometer, cooling bath and nitrogen blanket supply apparatus, is added 365 grams (2.0 moles) of acetyl diisoamylene prepared according to Example III; 300 ml of anhydrous toluene and 24 ml boron trifluoride etherate (0.2 moles). The resulting mixture is heated to 50°C and while maintaining the reaction mass at 50°C, over a period of 1 hour, 250 ml (3.6 moles) of propylene oxide is added while maintaining the reaction temperature at 79—89°C.

The reaction mass is then cooled and stirred for another 12 hours while maintaining the temperature at 30°C.

One liter of 5% aqueous sodium hydroxide is then added to the reaction mass. The reaction mass is transferred to a separatory funnel and the organic layer is washed with two 1-liter saturated sodium chloride portions. The reaction mass is then distilled on a 2″ (50 mm) splash column yielding the following fractions:

| Fraction Number | Vapor Temp. (°C) | Liquid Temp. (°C) | Pressure mm/Hg | ($N/m^2$ $\times 10^{-2}$) | Weight of Fraction (g.) |
|---|---|---|---|---|---|
| 1 | 33/79 | 78/92 | 4.5/2.8 | (6.0/3.7) | |
| 2 | 78 | 89 | 2.0 | (2.7) | |
| 3 | 70 | 88 | 1.6 | (2.1) | |
| 4 | 70 | 88 | 1.6 | ( ,, ) | |
| 5 | 75 | 100 | 1.6 | ( ,, ) | 87 |
| 6 | 71 | 117 | 1.6 | ( ,, ) | 43 |
| 7 | 85 | 137 | 1.6 | ( ,, ) | 27 |
| 8 | 103 | 163 | 1.1 | (1.5) | 19 |
| 9 | 155 | 205 | 1.1 | ( ,, ) | |
| 10 | 185 | 230 | 1.1 | ( ,, ) | |

GLC, NMR, IR and mass spectral analyses yield the information that the resulting product is a mixture of compounds having the structures:

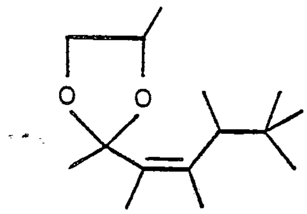

;

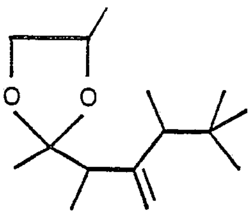

and

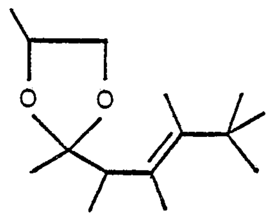

Figure 28 sets forth the GLC profile for the reaction product produced according to this example containing the compounds defined according to the generic structure:

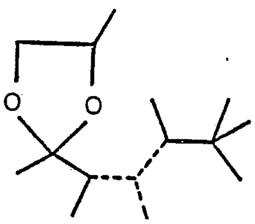

wherein one of the dashed lines in each of the molecules represents a carbon-carbon double bond and the other two of the dashed lines in each of the molecules represent carbon-carbon single bonds.

Figure 29 is the NMR spectrum for the mixture of compounds produced according to this example containing the compounds defined according to the generic structure:

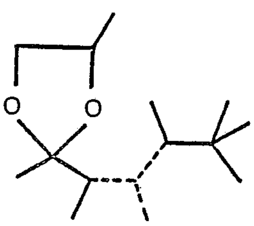

Figure 30 is the infra-red spectrum for the product produced according to this example containing the compounds defined according to the generic structure:

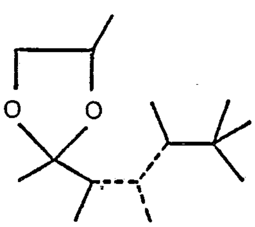

Example XI(B)

Reaction:

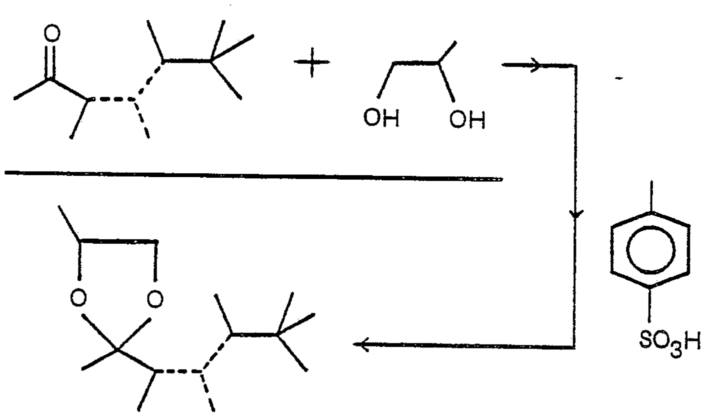

Into a 2000 ml reaction flask equipped with stirrer, heating mantle, addition funnel, reflux condenser, thermometer, cooling bath and nitrogen blanket supply apparatus is added 595 grams (3.0 moles) of acetyl diisoamylene prepared according to Example I; 265 ml (3.5 moles) of propylene glycol; 500 ml of anhydrous toluene and 3.0 grams of partoluene sulfonic acid.

The resulting mixture is heated to reflux in order to strip off as azeotrope the water of reaction. The refluxing is continued for a period of one hour at 125°C while collecting water of reaction in a Bidwell trap. After a period of 14 hours, 53 ml water is collected and the reaction mass is cooled to room temperature.

The reaction mass is transferred to a separatory funnel and is washed with one 500 ml volume of 10% aqueous sodium hydroxide; and three 500 ml volumes of saturated sodium chloride solution. The solvent is then stripped off on a rotary evaporator and the stripped crude is transferred to a distillation flask whereupon the product is first distilled to yield the following fractions:

| Fraction Number | Vapor Temp. (°C) | Liquid Temp. (°C) | Pressure mm/Hg | ($N/m^2$ $\times 10^{-2}$) |
|---|---|---|---|---|
| 1 | 35/75 | 90/87 | 3.8/2.5 | (5.1/3.3) |
| 2 | 77 | 87 | ’.5 | (4.7) |
| 3 | 79 | 90 | 3.5 | ( „ ) |
| 4 | 81 | 94 | 3.5 | ( „ ) |
| 5 | 85 | 98 | 3.5 | ( „ ) |
| 6 | 88 | 100 | 3.5 | ( „ ) |
| 7 | 90 | 105 | 3.5 | ( „ ) |
| 8 | 94 | 200 | 3.5 | ( „ ) |

on a 2″ (50 mm) splash column. Fractions 5, 6, 7 and 8 are then redistilled on the same 2″ (50 ml) splash column to yield the following fractions:

| Vapor Temp. (°C) | Liquid Temp. (°C) | Pressure mm/Hg | ($N/m^2$ $\times 10^{-2}$) | Weight of Fraction (g.) |
|---|---|---|---|---|
| 78/70 | 83/85 | 3/3 | (4) | 22 |
| 82 | 88 | 3 | ( „ ) | 22 |
| 85 | 90 | 3 | ( „ ) | 26 |
| 87 | 91 | 3 | ( „ ) | 24 |
| 87 | 91 | 3 | ( „ ) | 42 |
| 87 | 91 | 3 | ( „ ) | 51 |
| 94 | 98 | 3 | ( „ ) | 46 |
| 98 | 102 | 3 | ( „ ) | 50 |
| 98 | 102 | 3 | ( „ ) | 40 |
| 95 | 120 | 3 | ( „ ) | 16 |
| 210 | 240 | 3 | ( „ ) | 10 |

Figure 30A represents the GLC profile for the reaction product prior to distillation containing a mixture of compounds having the structure:

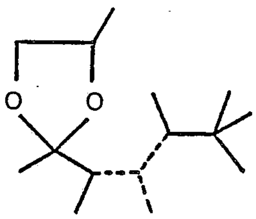

Figure 30B is the GLC profile for Fraction 9 of the distillation product of the reaction product of this example containing a mixture of compounds having the generic structure:

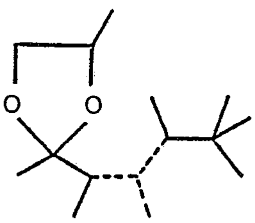

wherein in the mixture, one of the dashed lines in each compound represents a carbon-carbon double bond and the other dashed lines represent carbon-carbon single bonds.

## Example XII

*Preparation of ethylene ketal of acetyl diisoamylene*

Reaction:

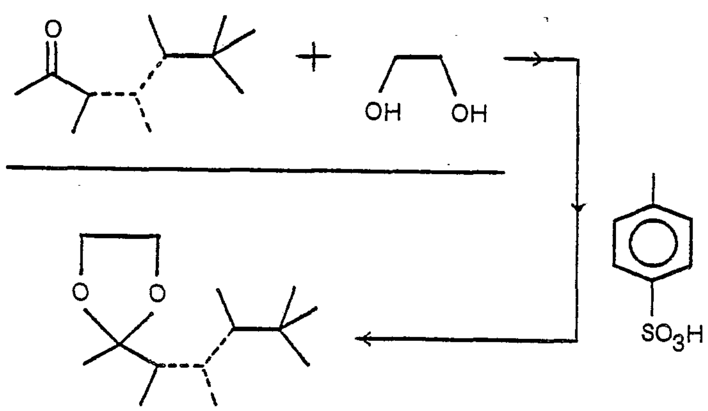

Into a 3 liter reaction flask equipped with heating mentle, stirrer, thermometer, reflux condenser and apparatus for provision of nitrogen blanket is added 1000 ml (4.7 moles) of acetyl diisoamylene produced according to Example III, 310 grams (5.0 moles) of ethylene glycol; 1000 ml of anhydrous toluene and 0.4 grams of para-toluene sulfonic acid. The resulting mixture is heated to reflux (138°C) while trapping out water of reaction using a Bidwell trap. The refluxing is continued for a period of 10.5 hours yielding a total of 65 ml water.

The reaction mass is cooled to room temperature and 200 ml of 10% aqueous sodium hydroxide is added to the reaction mass. The reaction mass is stirred and transferred to a separatory funnel. The aqueous layer is separated from the upper organic phase and the upper organic phase is then washed with three 500 ml portions of saturated aqueous sodium chloride solution.

The solvent (toluene) is stripped off of the reaction mass using a rotary evaporator and the crude reaction product is distilled on a 2″ (50 mm) splash column yielding the following fractions:

| Fraction Number | Vapor Temp. (°C) | Liquid Temp. (°C) | Pressure mm/Hg | (N/m$^2$ $\times 10^{-2}$) | Weight of Fraction (g.) |
|---|---|---|---|---|---|
| 1 | 55/67 | 73/73 | 3/3 | ( 4 ) | 83 |
| 2 | 67 | 73 | 3 | ( „ ) | 70 |
| 3 | 67 | 73 | 3 | ( „ ) | 89 |
| 4 | 69 | 75 | 3 | ( „ ) | 99 |
| 5 | 69 | 75 | 3 | ( „ ) | 95 |
| 6 | 75 | 80 | 3 | ( „ ) | 66 |

GLC, NMR, IR and mass spectral analyses yield the information that the resulting product is a mixture of compounds defined according to the generic structure:

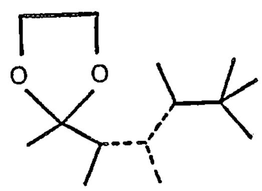

wherein in each of the molecules of the mixture, one of the dashed lines is a carbon-carbon double bond and the other dashed lines are carbon-carbon single bonds. Each of the constituents of this reaction mass may be trapped out using preparative GLC to yield the following compounds:

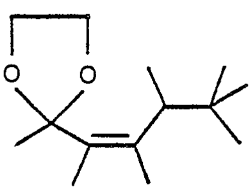 ; 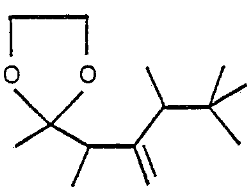 and 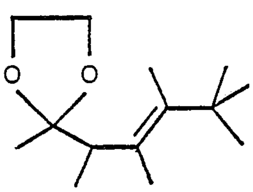

Figure 31 is the GLC profile for the reaction product of this example.

Example XIII

*Hydrolysis product of reaction product of methyl lithium and acetyl diisoamylene*

Reaction:

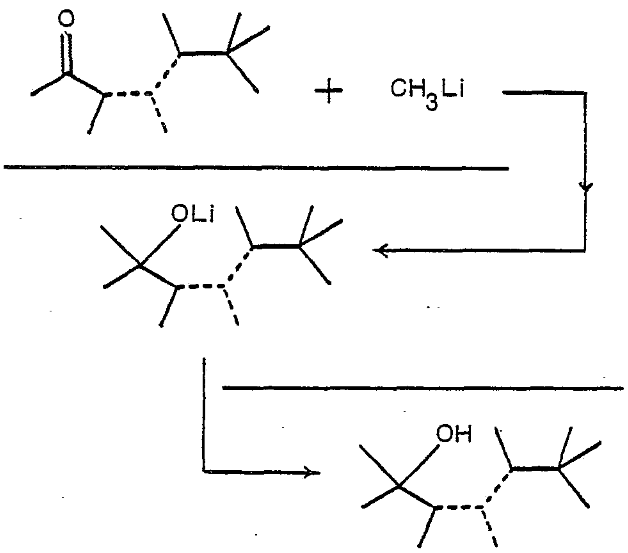

(wherein the reactant and product are mixtures and the mixtures contain molecules wherein one of the dashed lines represents a carbon-carbon double bond and the other dashed lines represent carbon-carbon single bonds).

Into a 2 liter reaction flask equipped with reflux condenser, addition funnel, thermometer, cooling bath and nitrogen blanket provision apparatus, is placed 1 mole of methyl lithium and 1000 cc of anhydrous diethylether. Over a period of 30 minutes while maintaining the reaction mass at 18—29°C, 142 grams (0.8 moles) of acetyl diisoamylene produced according to Example III is added dropwise. The reaction mass is then maintained at 29—41°C over a period of 8 hours.

To the reaction mass 300 ml water is added with exotherming. The resulting aqueous solution is poured into a 2 liter separatory funnel and the lower aqueous phase is removed. The organic phase is washed with 2 portions of 500 ml of aqueous sodium chloride. The solvent (diethylether) is then evaporated from the reaction mass and the reaction mass is distilled on a microdistillation apparatus yielding the following fractions:

| Fraction Number | Vapor Temp. (°C) | Liquid Temp. (°C) | Pressure mm/Hg | ($N/m^2$ $\times 10^{-2}$) | Reflux Ratio | Weight of Fraction (gm.) |
|---|---|---|---|---|---|---|
| 1 | 71/72 | 75/74 | 3/3 | ( 4 ) | RO | 19 |
| 2 | 71 | 75 | 3 | ( „ ) | 1:1 | 17 |
| 3 | 74 | 78 | 3 | ( „ ) | 1:1 | 24 |
| 4 | 74 | 78 | 3 | ( „ ) | 1:1 | 7 |
| 5 | 75 | 78 | 3 | ( „ ) | 1.1 | 16 |
| 6 | 74 | 78 | 3 | ( „ ) | 1:1 | 21 |
| 7 | 75 | 78 | 3 | ( „ ) | 1:1 | 19 |
| 8 | 83 | 89 | 2.8 | (3.7) | 1:1 | 25 |
| 9 | 155 | 195 | 3 | ( 4 ) | 1:1 | 9 |

Figure 32 represents the GLC profile for the reaction product of this example containing a mixture of compounds defined according to the structure:

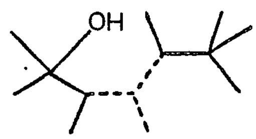

wherein in each of the molecules one of the dashed lines represents a carbon-carbon double bond and the other dashed lines represent carbon-carbon single bonds.

Figure 33 is the NMR spectrum for the reaction product of this example containing a mixture of compounds having the structures defined according to the following structure:

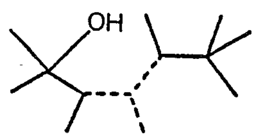

wherein in each of the molecules of the mixture one of the dashed lines represents a carbon-carbon double bond and the other dashed lines represent carbon-carbon single bonds.

Figure 34 is the infra-red spectrum for the reaction product of this example containing the compounds defined according to the structure:

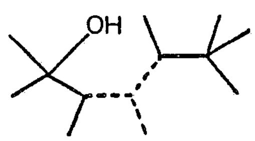

wherein in each of the molecules in the resulting mixture one of the dashed lines represents a carbon-carbon double bond and the other dashed lines represent carbon-carbon single bonds.

Example XIV

*Hydrolysis product of reaction product of propionyl diisoamylene and methyl lithium*

Reaction:

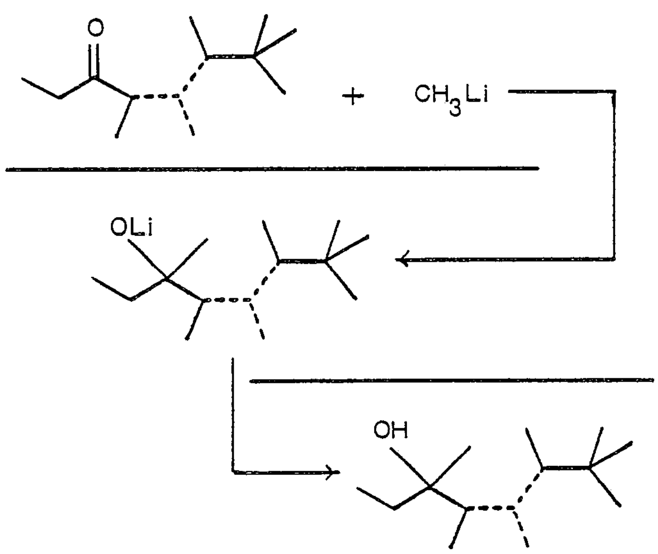

(wherein the reactant and product are mixtures and the mixtures contain molecules wherein one of the ashed lines represents a carbon-carbon double bond and the other dashed lines represent carbon-carbon single bonds).

Into a two liter reaction flask equipped with reflux condenser, addition funnel, thermometer, cooling bath and apparatus for provision of a nitrogen atmosphere is placed 1 mole of methyl lithium in 1000 ml anhydrous diethylether. Over a period of 45 minutes while maintaining the reaction mass at 20—25°C, 0.8 moles (156 grams) of the propionyl diisoamylene material produced according to Example IV is added to the methyl lithium solution. After the addition of the propionyl diisoamylene, the reaction mass is

maintained at 20—23°C for a period of 4 hours. The reaction mass is then admixed with 300 ml water during cooling. The resulting organic phase is then separated from the aqueous phase and the organic phase is stripped of ether and distilled on a microdistillation column yielding the following fractions:

| Fraction Number | Vapor Temp. (°C) | Liquid Temp. (°C) | Pressure mm/Hg | ($N/m^2$ $\times 10^{-2}$) | Weight of Fraction (gm.) |
|---|---|---|---|---|---|
| 1 | 70/70 | 84/84 | 3/3 | (4) | 22 |
| 2 | 68 | 79 | 3 | (〃) | 18 |
| 3 | 69 | 82 | 3 | (〃) | 48 |
| 4 | 68 | 82 | 3 | (〃) | 48 |
| 5 | 72 | 84 | 3 | (〃) | 21 |
| 6 | 78 | 105 | 3 | (〃) | 11 |
| 7 | | 163 | 3 | (〃) | 1 |

Figure 35 is the GLC profile of the crude reaction product of this example containing the compounds defined according to the structure:

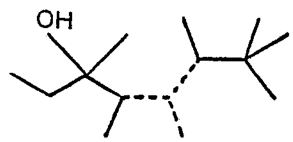

wherein in each of the molecules, one of the dashed lines represents a carbon-carbon single bond and the other dashed lines represent carbon-carbon double bonds.

Figure 36 is the GLC profile for fraction 4 of the distillation product of the reaction product of this example containing the compounds having the structure:

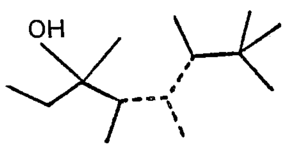

wherein in each of the molecules one of the dashed lines represents a carbon-carbon double bond and the other dashed lines represent carbon-carbon single bonds.

Figure 37 is the infra-red spectrum for fraction 4 of the distillation product of the reaction product of this example containing a mixture of compounds defined according to the structure:

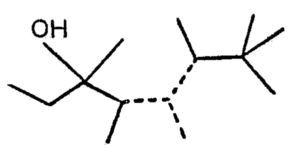

wherein in each of the molecules one of the dashed lines represents a carbon-carbon double bond and the other dashed lines represent carbon-carbon single bonds.

Example XV

*Hydrolysis product of the reaction product of methyl lithium and isobutyryl diisoamylene*

Reaction:

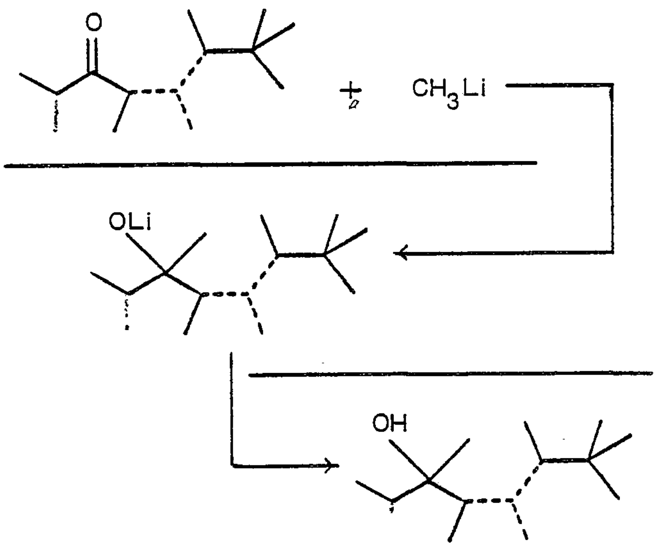


(wherein the reactant and product are mixtures and the mixtures contain molecules wherein one of the dashed lines represents a carbon-carbon double bond and the other dashed lines represent carbon-carbon single bonds).

Into a 2 liter reaction flask equipped with reflux condenser, addition funnel, thermometer, cooling bath and apparatus for provision of a nitrogen atmosphere is placed 1 mole of methyl lithium dissolved in 1000 ml diethylether. Over a period of 45 minutes while maintaining the reaction temperature at 19—25°C, 167 grams (0.8 moles) of isobutyryl diisoamylene prepared according to Example VI is added. At the end of the addition of the isobutyryl diisoamylene, the reaction mass is admixed with 300 ml water and the temperature of the reaction mass is cooled to 10°C. The reaction mass is then added to a 2 liter separatory funnel and the aqueous layer is separated from the organic layer. The organic layer is then stripped of solvent on a rotary evaporator and the crude product is then distilled on a microdistillation column yielding the following fractions:

| Fraction Number | Vapor Temp. (°C) | Liquid Temp. (°C) | Pressure mm/Hg | ($N/m^2$ $\times 10^{-2}$) | Weight of Fraction (gm.) |
|---|---|---|---|---|---|
| 1 | 75/72 | 81/81 | 3/3 | (4) | 25 |
| 2 | 72 | 81 | 3 | („) | 15 |
| 3 | 74 | 83 | 3 | („) | 44 |
| 4 | 74 | 83 | 3 | („) | 47 |
| 5 | 84 | 88 | 3 | („) | 14 |
| 6 | 86 | 95 | 3 | („) | 20 |
| 7 | 95 | 112 | 3 | („) | 10 |
| 8 | 160 | 210 | 3 | („) | 6 |

Figure 38 is the GLC profile of the crude reaction product of this example containing a mixture of compounds defined according to the structure:

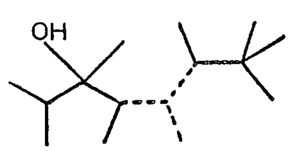

wherein in each of the molecules one of the dashed lines represents a carbon-carbon double bond and the other dashed lines represent carbon-carbon single bonds.

Figure 39 is the GLC profile for fraction 7 of the distillation product of the reaction product of this example containing a mixture of compounds defined according to the structure:

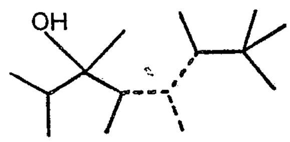

wherein in each of the molecules one of the dashed lines represents a carbon-carbon double bond and the other dashed lines represent carbon-carbon single bonds.

Figure 40 is the infra-red spectrum for fraction 7 of the distillation product of the reaction product of this example containing a mixture of compounds defined according to the structure:

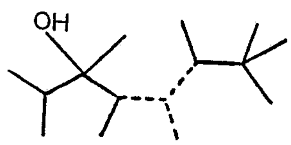

wherein in each of the molecules one of the dashed lines is a carbon-carbon double bond and the other dashed lines represent carbon-carbon single bonds.

Figure 41 is the NMR spectrum for fraction 7 of the distillation product of the reaction product of this example containing a mixture of compounds defined according to the structure:

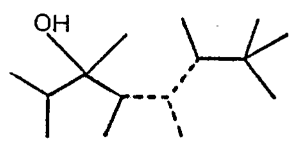

wherein in each of the molecules one of the dashed lines represents a carbon-carbon double bond and the other dashed lines represent carbon-carbon single bonds.

Example XVI

*Hydrolysis product of the reaction product of n-butyl lithium and acetyl diisoamylene*

Reaction:

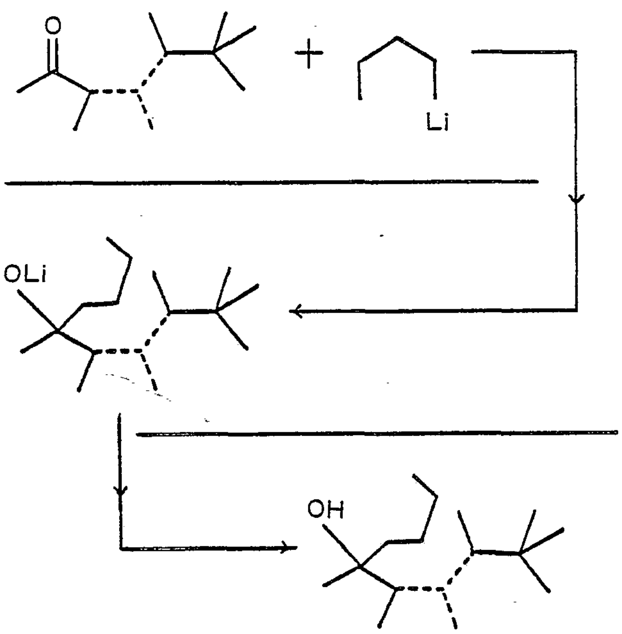

(wherein the reactant and product are mixtures and the mixtures contain molecules wherein one of the dashed lines represents a carbon-carbon double bond and the other dashed lines represent carbon-carbon single bonds).

Into a 2 liter reaction flask equipped with reflux condenser, addition funnel, thermometer, cooling bath and apparatus for provision of the nitrogen atmosphere is placed 1 mole of n-butyl lithium dissolved in 1000 cc anhydrous diethylether. To the n-butyl lithium solution is added 147 grams (0.8 moles) of acetyl diisoamylene prepared according to Example III over a period of 20 minutes while maintaining the reaction mass at 27—28°C. The reaction mass is then refluxed at 68—69°C for about 5 minutes and then cooled to 27°C. Saturated aqueous ammonium chloride in water is then added to the reaction mass in order to hydrolize the resulting lithium salt. The reaction mass now exists in two phases and is added to a 2 liter separatory funnel. The layers are separated and the organic layer is stripped of solvent and distilled on a microdistillation column yielding the following fractions:

| Fraction Number | Vapor Temp. (°C) | Liquid Temp. (°C) | Pressure mm/Hg | ($N/m^2$ $\times 10^{-2}$) | Weight of Fraction (gm.) |
|---|---|---|---|---|---|
| 1 | 76/83 | 88/90 | 3/3 | (4) | 19 |
| 2 | 87 | 94 | 3 | („) | 13 |
| 3 | 94 | 102 | 3 | („) | 15 |
| 4 | 105 | 112 | 3 | („) | 15 |
| 5 | 108 | 110 | 3 | („) | 38 |
| 6 | 110 | 120 | 3 | („) | 32 |
| 7 | 112 | 125 | 3 | („) | 9 |
| 8 | 116 | 135 | 3 | („) | 9 |
| 9 | 160 | 220 | 3 | („) | 7 |

Figure 42 is the GLC profile for the crude reaction product produced according to the procedure of this example containing a mixture of compounds defined according to the structure:

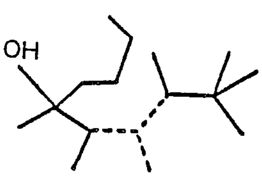

wherein in each of the molecules one of the dashed lines represents a carbon-carbon double bond and the other dashed lines represent carbon-carbon single bonds.

Figure 43 is the GLC profile for fractin 8 of the distillation product of the reaction product of this example containing a mixture of compounds defined according to the structure:

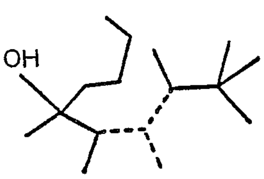

wherein in each of the molecules one of the dashed lines represents a carbon-carbon double bond and the other dashed lines represent carbon-carbon single bonds.

Figure 44 is the infra-red spectrum for fraction 8 of the distillation product of the reaction product of this example containing a mixture of compounds defined according to the structure:

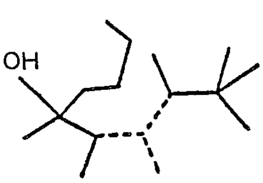

wherein one of the dashed lines is a carbon-carbon double bond and the other dashed lines represent carbon-carbon single bonds.

Figure 45 is the NMR spectrum for fraction 8 of the distillation product of the reaction product of this example containing the compounds defined according to the structure:

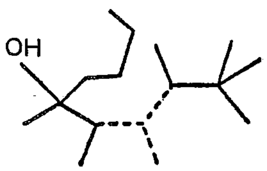

Example XVII

*Preparationof mixture of diisoundecanals and diisoundecanols via oxo reactions*

Reaction:

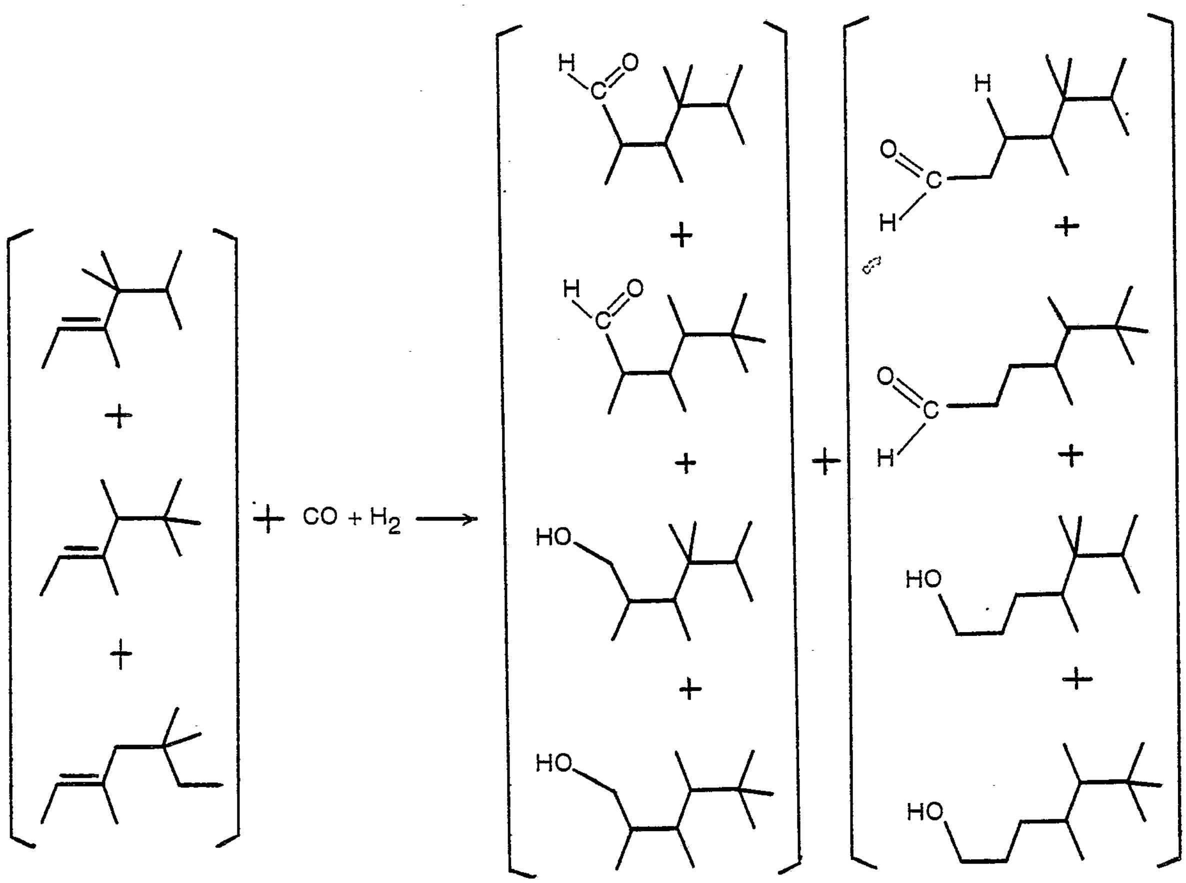


2500 ml (1835.5 grams) of the diisoamylene prepared according to Example I is intimately admixed with 200 ml (182 grams) of cobalt octoate in 200 cc of toluene. The reaction mass is placed in a 3 liter autoclave and pressurized to 2500 psig ($1.7 \times 10^7$ N/m$^2$) while heating the contents of the autoclave to a temperature in the range of 180—204°C. The partial pressure of the hydrogen gas is 1000 pounds per square inch ($6.9 \times 10^5$ N/m$^2$) and the partial pressure of the carbon monoxide is 1500 pounds per square inch gauge ($10^7$ N/m$^2$). The contents of the autoclave are maintained at 180—204°C for 4 hours whereupon the autoclave is depressurized and the contents are filtered. The toluene solvent is evaporated and the reaction product is then distilled on a 2″ (50 mm) splash column yielding the following fractions:

| Fraction Number | Vapor Temp. (°C) | Liquid Temp. (°C) | Pressure mm/Hg | (N/m$^2$ ×10$^{-2}$) | Weight of Fraction (gm.) |
|---|---|---|---|---|---|
| 1 | 28/81 | 52/52 | 40/50 | (53/67) | 190 |
| 2 | 64 | 78 | 25 | (33) | 190 |
| 3 | 50 | 75 | 5 | ( 7 ) | 150 |
| 4 | 50 | 85 | 5 | ( „ ) | 128 |
| 5 | 87 | 103 | 1 | ( 1 ) | 192 |
| 6 | 91 | 130 | 1 | „ ) | 205 |
| 7 | 150 | 220 | 1 | ( „ ) | 42 |

The reaction mass is then redistilled on a 1 foot (305 mm) Goodloe silver mirror column to yield the following fractions:

| Fraction Number | Vapor Temp. (°C) | Liquid Temp. (°C) | Pressure mm/Hg | (N/m$^2$ ×10$^{-2}$) | Reflux Ratio | Weight of Fraction (gm.) |
|---|---|---|---|---|---|---|
| 1 | 25/20 | 50/52 | 1/6 | (1/8) | 4:1 | 41 |
| 2 | 24 | 78 | 5 | ( 7 ) | 4:1 | 47 |
| 3 | 53 | 83 | 4 | ( 5 ) | 4:1 | 20 |
| 4 | 50 | 88 | 2 | ( 3 ) | 4:1 | 76 |
| 5 | 59 | 90 | 2 | ( „ ) | 4:1 | |
| 6 | 73 | 160 | 2 | ( „ ) | 1:3 | 87 |
| 7 | 63 | 163 | 2 | ( „ ) | 1:3 | 40 |
| 8 | | 220 | 2 | ( „ ) | 1:3 | 4 |

GLC, NMR and IR analyses yield the information that the reaction product contains compounds having the structures:

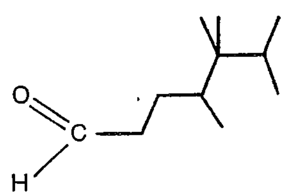

;

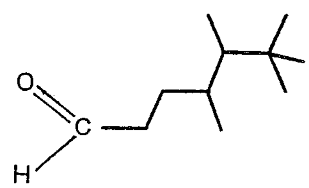

;

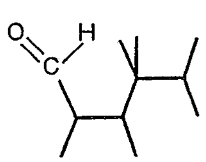

;

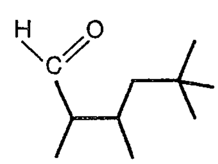

;

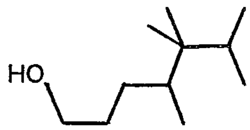 ; 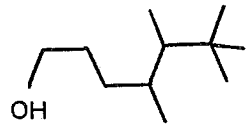 ;

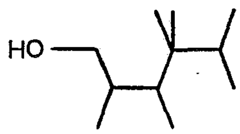 and 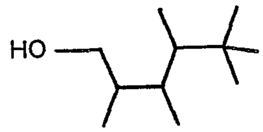

GLC, NMR and IR analyses yield the information that fractions 3 and 4 and 5 represent diisoundecanal isomers having the structures:

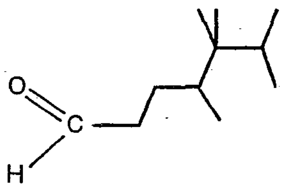 ; 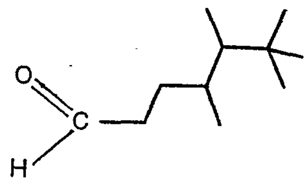 ;

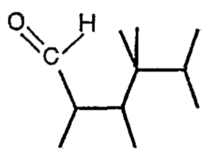 and 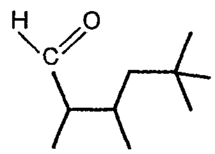 ;

whereas fractions 6, 7 and 8 of the foregoing distillation contain diisoundecanol isomers having the structures:

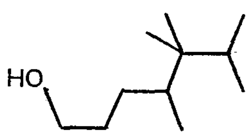 ; 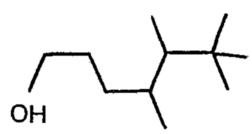 ;

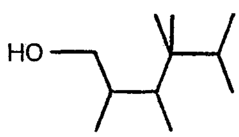 and 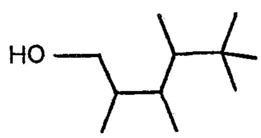

Figure 46 is the GLC profile for the crude reaction product produced according to this example (conditions: SF-96 column, 6′ × 1/4″ (1.83 m × 6 mm), programmed at 180°C, isothermal) containing the compounds having the structures:

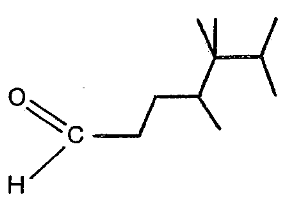 ; 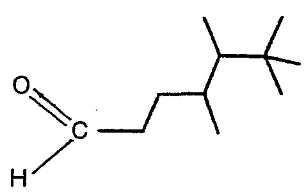 ;

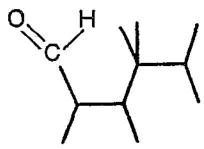 ; 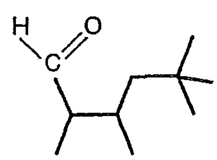 ;

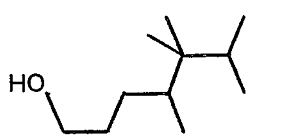 ; 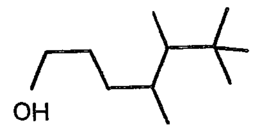 ;

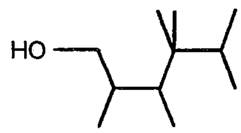 and 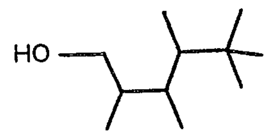

Figure 47 is the GLC profile for the diisoundecanal compounds produced according to this example having the structures:

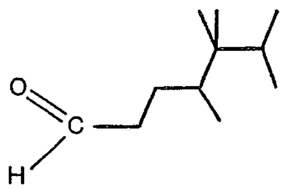 ; 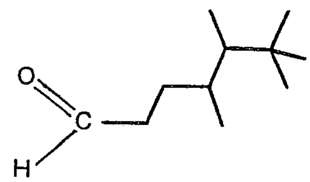 ;

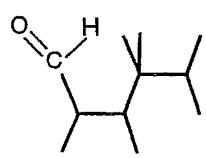 and 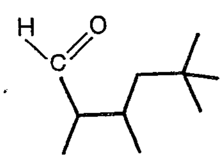 ;

Figure 48 is the NMR spectrum for the diisoundecanal compounds produced according to this example having the structures:

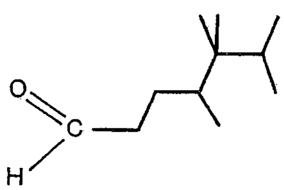 ; 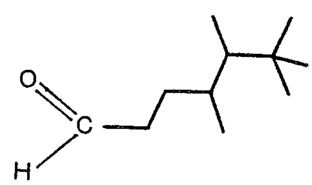 ;

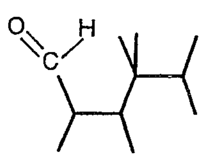 and 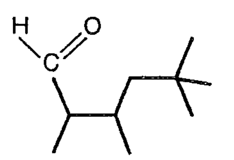 ;

Figure 49 is the infra-red spectrum for the diisoundecanal compounds produced according to this example having the structures:

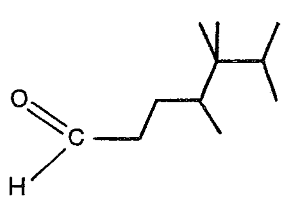 ; 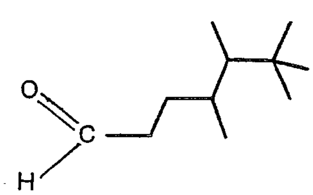 ;

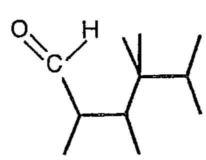 and 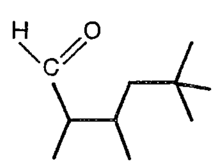 ;

Figure 50 is the GLC profile conditions: SF-96, 6′ × 1/4″ (1.83 m × 6 mm) 180°C isothermal column) for the reaction product produced according to this example containing the compounds having the structures:

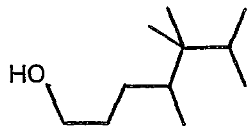 ; 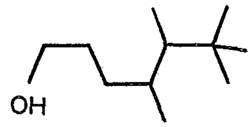 ;

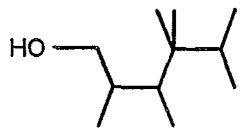 and 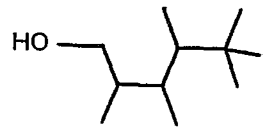

Figure 51 is the NMR spectrum for the diisoundecanol compounds produced according to this example having the structures:

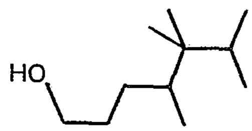 ; 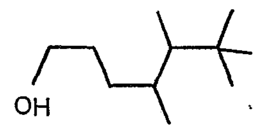 ;

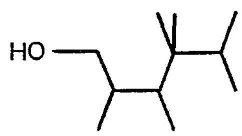 and 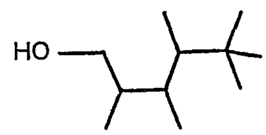

Figure 52 is the infra-red spectrum for the diisoundecanol compounds produced according to this example having the structures:

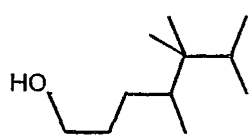 ; 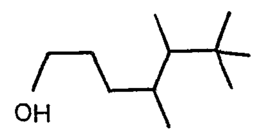 ;

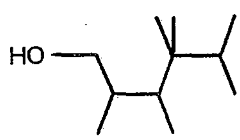 and 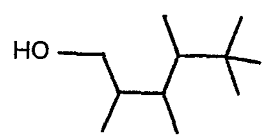

Example XVIII

*Part (i)*

Aromox® DMMC-W in various quantities is mixed with 0.1 gram of one of the diisoamylene compositions prepared according to Example I or one of the diisoamylene epoxide compositions prepared according to Example II. The resulting premises are then added to 200 grams of an aqueous hydrochlorite solution. Sufficient 12.5 M aqueous NaOH is added to bring the pH of the mixture up to 13.

The following results are obtained:

| Percentage Aromox® DMMC-W | Clarity of hypochlorite solution after addition of premix |
|---|---|
| 0.23 | Clear after three days |
| 0.15 | Clear after three days |
| 0.08 | Initially slightly turbid; two phases exist after three days |

When used as laundry bleaches, the resulting bleached laundries on dry-out in an atmosphere of 50% relative humidity in each of the three cases above retain a "clean fresh", "woody", "herbaceous", "piney" aroma (when using the diisoamylenes of Example I) whereas without the use of one of the diisoamylene products prepared according to Example I, the bleached laundry has a faint characteristic disagreeable hypochlorite aroma.

When used as laundry bleaches, the resulting bleached laundry batches, on dry-out in an atmosphere of 50% relative humidity in each of the three cases above retain a "woody", "eucalyptol-like", "minty" aroma (when using one of the diisoamylene epoxide compositions prepared according to Example II) whereas without the use of at least one of the diisoamylene epoxide derivatives prepared according to Example II, the bleached laundry has a faint characteristic disagreeable hypochlorite aroma.

*Part (ii)*

Dowfax® 2A1 (see Note 1, infra) in various quantities, as set forth below, is mixed with 0.1 gram of one of the diisoamylene compositions prepared according to Example I or one of the diisoamylene epoxide compositions prepared according to Example II. The resulting premixes are then added to 200 grams of an aqueous 7% sodium hypochlorite solution. Sufficient 12.5 M aqueous NaOH is added to bring the pH of the mixture up to 13.5 .The following results are obtained:

| Percentage of DOWFAX® 2A1 | Clarity of hypochlorite solution after addition of premix |
|---|---|
| 0.23 | Clear after seven days |
| 0.15 | Clear after five days |
| 0.08 | Clear after three days |
| 0.01 | Initially slightly turbid; two phases exist after three days. |

When used as laundry bleaches, the resulting bleached laundry batches, on dry-out in an atmosphere of 50% relative humidity in each of the four cases above, retain a "clean fresh", "woody", "herbaceous", "piney" aroma (when using one of the diisoamylene compositions prepared according to Example I) whereas without the use of one of the diisoamylene products prepared according to Example I, the bleached laundry batches have faint characteristic, disagreeable hypochlorite aromas.

When used as laundry bleaches, the resulting bleached laundry batches, on dry-out in an atmosphere of 50% relative humidity in each of the four cases above, retain a "woody", "eucalyptol-like", "minty" aroma (when using one of the diisoamylene epoxide compositions prepared according to Exmaple II) whereas without the use of at least one of the diisoamylene epoxide derivatives prepared according to Example II, the bleached laundry has a faint characteristic disagreeable hypochlorite aroma.

Figure 53A represents a graph of *percent residual chlorine* versus *time in hours* for hypochlorite solutions containing DOWFAX® 2A1 (a registered trademark of the Dow Chemical Company of Midland, Michigan) identifying the compound having the structure:

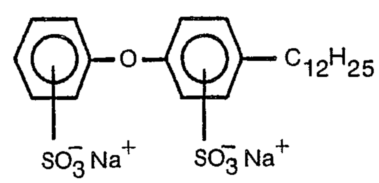

wherein the $C_{12}H_{25}$ moiety is branched chain and the $SO_3^-Na^+$ moieties are at various positions on each of the benzene rings, or AROMOX® DMMC-W, a 30% aqueous solution of dimethylcocoamine oxide having the structure:

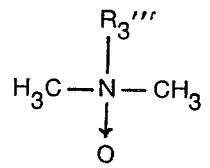

a trademark of Akzo Corporation of Chicago, Illinois (product produced by Armac, Division of Akzo Corporation of Chicago, Illinois) with the ratio of AROMOX® DMMC-W: base being 0.8:99 and the ratio of DOWFAX® 2A1: base being 0.8:99.

Figure 53B is a graph of *percent residual chlorine* versus *time in hours* for hypochlorite solutions of (1) DOWFAX® 2A1 and AROMOX® DMMC-W in the absence of fragrance or essential oils with the ratios of AROMOX® DMMC—W:base being 1.8:99 and 3.8:96 and the ratios of DOWFAX® 2Al: base being 1.8:99 and 3.8:99.

Figure 54A is a graph of *percent residual chlorine* versus *time in hours* comparing the performance of hypochlorite solutions containing (i) DOWFAX® 2A1 versus (ii) AROMOX® DMMC-W using a diisoamylene product produced according to Example I or not using any fragrance or essential oils with the ratio of AROMOX® DMMC-W:diisoamylene:base being 3.8:0.2:96 and the ratio of DOWFAX® 2A1:diisoamylene:base being 3.8:0.2:96.

Figure 54B is a graph of *percent residual chlorine* versus *time in hours* comparing hypochlorite solutions of DOWFAX® 2A1 versus AROMOX® DMMC-W with the perfuming material being one of the diisoamylene products produced according to Example I, wherein the ratio of AROMOX® DMMC-W:diisoamylene:base is ether 0.8:0.2:99 or 1.8:0.2:98 and the ratio of DOWFAX® 2A1:diisoamylene:base is 0.8:0.2:99 or 1.8:0.2:98.

Figure 55A is a graph of *percent residual chlorine* versus *time in hours* comparing hypochlorite solutions of DOWFAX® 2A1 versus AROMOX® DMMC-W with the perfuming material being one of the diisoamylene epoxide products produced according to Example II wherein the weight ratio of AROMOX® DMMC-W:diisoamylene epoxide:base is either 0.8:0.2:9 or 1.8:0.2:9 and the weight ratio of DOWFAX® 2A1:diisoamylene epoxide:base is 0.8:0.2:9 or 1.8:0.2:9.

Figure 55B is a graph of *percent residual chlorine* versus *time in hours* comparing the performance of hypochlorite solutions containing (i) DOWFAX® 2A1 versus (ii) AROMOX® DMMC-W using a diisoamylene epoxide product produced according to Example II or not using any fragrance or essential oils with the weight ratio of AROMOX® DMMC-W:diisoamylene epoxide:base being 3.8:0.2:9 and the ratio of DOWFAX® 2A1:diisoamylene epoxide:base being 3.8:0.2:9.

*Note 1*: DOWFAX® 2A1 is a material consisting essentially of a mixture of compounds defined according to the structure:

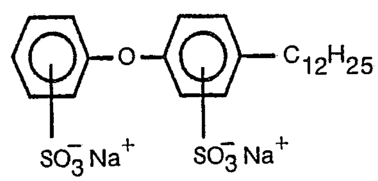

wherein the $C_{12}H_{25}$ moiety is branched chain and the $SO_3^-Na^+$ moieties are at various positions on each of the benzene rings.

Example XIX

*Preparation of mixture of diisoamylene methyl carbinols*

Reaction:

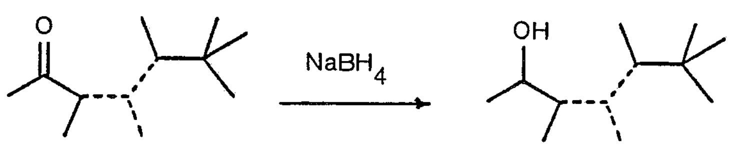


Into a 2 liter reaction flask equipped with reflux condenser, additional funnel, thermometer, heating mantle, and nitrogen blend is placed 1 liter of isopropyl alcohol followed by 38 grams of sodium borohydride. The resulting mixture is heated to reflux and over a period of 40 minutes while maintaining the reflux temperature at 48°C dropwise addition of 368 grams of acetyl diisoamylene prepared according to Example III (bulked fractions 2—12 of the distillation) is carried out.

At the end of the addition of the acetyl diisoamylene, the reaction mass is stirred at a temperature of 73°C for a period of 3 hours. The reaction mass is then transferred to a separatory flask containing 1 liter of water. 200 ml 5% hydrochloric acid is added to the separatory funnel and the organic layer is separated from the inorganic layer.

The organic layer is washed with 1 liter of sodium carbonate and is then distilled on a 1" (25 mm) packed stone column yielding the following fractions:

| Fraction Number | Vapor Temp. (°C) | Liquid Temp. (°C) | Pressure mm/Hg. | ($N/m^2 \times 10^{-2}$ |
|---|---|---|---|---|
| 1 | 25/20 | 18/20 | 10 | (13) |
| 2 | 80 | 90 | 0.2 | (0.3) |
| 3 | 81 | 92 | 0.2 | ( „ ) |
| 4 | 83 | 96 | 0.2 | ( „ ) |
| 5 | 81 | 130 | 0.2 | ( „ ) |
| 6 | 80 | 200 | 0.2 | ( „ ) |

The resulting product (bulked fractions 2—4) is analyzed by GLC, NMR and IR analysis to contain a mixture of compounds defined according to the structure:

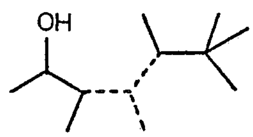

wherein in each of the compounds one of the dashed lines represents a carbon-carbon double bond and the other dashed lines represent carbon-carbon single bonds.

Figure 56 is the GLC profile for the above reaction product (conditions: 6' × 1/2" (1.83 m × 13 mm) SF-96 column programmed at 100-220°C at 8°C per minute).

Figure 57 represents the infra-red spectrum for bulked fractions 2—4.

The resulting product has a woody, amber, slightly fruity aroma with a strong vetiver-like aroma on dry-out which is very long-lasting and very intense. In smoking tobacco, the resulting material at a level of 1 ppm gives rise to a pleasant woody, oriental-like, minty aroma both prior to and on smoking in the main stream and in the side stream.

Example XX

*Preparation of diisoamylene isopropyl carbonil*

Reaction:

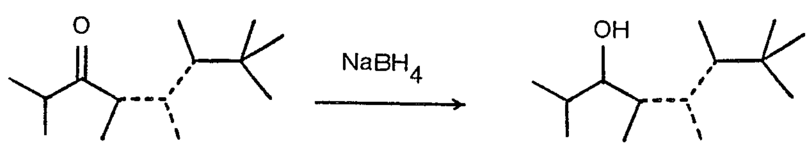


Into a 500 ml reaction flask equipped with reflux condenser, addition funnel, heating mantle, thermometer and nitrogen blanket provision is placed 210 grams of isobutyryl diisoamylene prepared according to Example VI (rebulked fractions 2—7). 18.7 grams of sodium borohydride is then dissolved in 100 ml ethanol. The resulting sodium borohydride solution is then added to the isobutyryl diisoamylene via the addition funnel while refluxing the reaction mass at 78—80°C over a 10 minute period. At the end of the 10 minute period, the addition is completed and the temperature of the reaction mass is 80°C. The reaction mass is then heated at 65—78°C for an additional 12 hours after which period of time the reaction mass is transferred to a separatory funnel containing 1 liter of water. The organic phase is separated from the inorganic phase is washed with two additional 1 liter portions of water. The resulting product is then distilled on a microvigreux column yielding the following fractions:

| Fraction Number | Liquid Temp. (°C) | Vapor Temp. (°C) | Pressure mm/Hg. | (N/m² × 10⁻² | Weight of Fraction (gm.) |
|---|---|---|---|---|---|
| 1 | 93/90 | 87/89 | 3 | (4) | 18 |
| 2 | 91 | 89 | 3 | ( „ ) | 19 |
| 3 | 88 | 79 | 2.5 | (3.3) | 47 |
| 4 | 83 | 84 | 2 | (3) | 47 |
| 5 | 91 | 85 | 2 | ( „ ) | 47 |
| 6 | 115 | 97 | 2 | ( „ ) | 20 |
| 7 | 200 | 165 | 2 | ( „ ) | 3 |

The resulting product has an intense, woody perfume aroma and a pleasant oriental/woody aroma in smoking tobacco both prior to and on smoking in the main stream and in the side stream.

Figure 58 is the GLC profile for fraction 4 of this distillation product of the reaction product of this example.

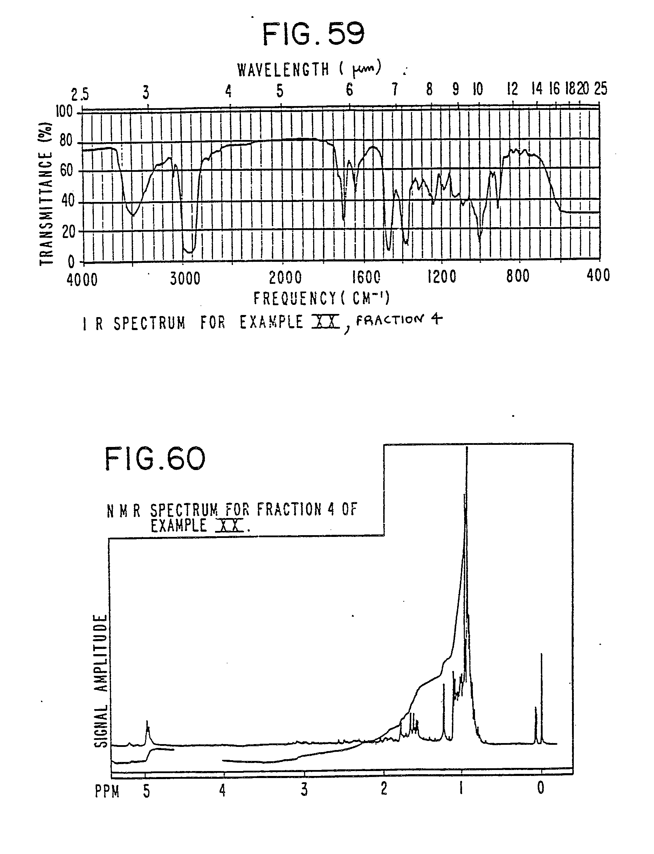

Figure 59 is the infra-red spectrum for the aforementioned reaction product subsequent to distillation; fraction 4 of the distillation product.

Figure 60 is the NMR spectrum for fraction 4 of the distillation product of the reaction product of this example.

Example XXI

The diisoamylene produced according to Exmaple I has a woody, piney, herbaceous note; and the diisoamylene epoxide product produced according to Example II has a woody, eucalyptol, minty note which may be utilized to a great extent in inexpensive functional products. The following pine fragrance demonstrates the use of these materials in perfume compositions. In this case, the dissoamylene derivatives are each used at concentrations of 47.9%.

| Ingredients | Parts by Weight Example XXI (A) | Example XXI (B) |
|---|---|---|
| Diisoamylene product of Example I | 479 | 0 |
| Diisoamylene epoxide product of Example II | 0 | 479 |
| Isobornyl acetate | 100 | 100 |
| Camphor | 10 | 10 |
| Teripineol | 25 | 25 |
| Fir balsam absolute (50% diethyl phthalate) | 20 | 20 |
| Coumarin | 4 | 4 |
| Linalool | 30 | 30 |
| Anethol | 2 | 2 |
| Fenchyl alcohol | 10 | 10 |
| Lemon terpenes washed | 50 | 50 |
| Borneol | 5 | 5 |

| Ingredients | Parts by Weight<br>Example XXI (A) | <br>Example XXI (B) |
|---|---|---|
| Galbanum oil | 5 | 5 |
| Turpentine Russian | 150 | 150 |
| Pinus pumilionus | 50 | 50 |
| Eucalyptol | 50 | 50 |
| 2,2,6-trimethyl-1-cyclohexene-1-carboxaldehyde | 5 | 5 |
| Maltol 1% in diethyl phthalate | 5 | 5 |

(A) The presence of the diisoamylene supports the pine notes and produces a considerable savings in the cost of the formulation.

(B) The presence of the diisoamylene epoxide supports the pine notes and produces a considerable savings in the cost of the formulation. It also lends a pleasant and strong minty, herbaceous and woody character to this pine fragrance. The pine fragrance is extremely stable, particularly in hypochlorite bleaches as will be seen infra.

### Example XXII

*Preparation of a cosmetic powder composition*

A cosmetic powder is prepared by mixing in a ball mill, 100 grams of talcum powder with 0.25 grams of the perfume composition prepared according to Example XXI(A) or XXI(B). When using the perfume composition of Example XXI(A), it has an excellent piney aroma with woody and herbaceous nuances. When using the perfume composition of Example XXI(B), it has an excellent piney aroma with woody, eucalyptol, and minty nuances.

### Example XXIII

*Perfumed liquid detergent*

Concentrated liquid detergents (Lysine salt of n-dodecylbenzene sulfonic acid as more specifically described in U.S. Patent No. 3,948,818, issued on April 6, 1976) with either (a) herbaceous, woody and piney aroma nuances are prepared containing 0.10%, 0.15%, 0.20%, 0.25%, 0.30% and 0.35% of the fragrance prepared according to Example XXI(A) or (b) eucalyptol-like, woody and minty aroma nuances are prepared containing 0.10%, 0.15%, 0.20%, 0.25%, 0.30% and 0.35% of the fragrance prepared according to Example XXI(B). They are prepared by adding and homogeneously mixing the appropriate quantity of a fragrance formulation prepared according to Example XXI in the liquid detergent. The detergents all possess either (a) ecellent piney aromas with woody and herbaceous undertones, the intensity increasing with greater concentrations of perfume composition of Example XXI(A) or (b) excellent piney aromas with woody, eucalyptol-like and minty undertones, the intensity increasing with greater concentrations of perfume composition of Example XXI(B).

### Example XXIV

*Preparation of a cologne and handkerchief perfume*

The composition prepared according to Example XXI(A) is incorporated into a cologne at concentrations of 2.0%, 2.5%, 3.0%, 3.5%, 4.0%, 4.5% and 5.0% in 85% aqueous food grade ethanol, and into a handkerchief perfume at concentrations of 15%, 20%, 25% and 30% (in 95% aqueous food grade ethanol). A distinctive and definite herbaceous, woody, piney aroma is imparted to the cologne and to the handkerchief perfume at all levels indicated above.

The composition prepared according to Example XXI(B) incorporated into a cologne at concentrations of 2.0%, 2.5%, 3.0%, 3.5%, 4.0%, 4.5% and 5.0% in 75%, 80%, 85% and 90% aqueous food grade ethanol; and into a handkerchief perfume at concentrations of 15%, 20%, 25%, and 30% (in 90% and 95% aqueous food grade ethanol). A distinctive and definite eucalyptol, woody, minty and piney aroma is imparted to the cologne and to the handkerchief perfume at all levels indicated above.

### Example XXV

*Preparation of soap composition*

One hundred grams of soap chips (IVORY®, produced by the Procter & Gamble Company, Cincinnati,

Ohio) are mixed with one gram of the formulations of Example XXI(A) and XXI(B) until homogeneous compositions are obtained. In each of the cases, the homogeneous compositions are heated under three atmospheres ($3 \times 10^5$ N/$m^2$) pressure at 180°C for a period of three hours and the resulting liquids are placed into soap molds. (a) When using the formula of Example XXI(A), the resulting soap cakes, on cooling, manifest excellent herbaceous, woody and piney aromas with an emphasis on the piney aspects of the aroma. (b) When using the formulation of Example XXI(B), the resulting soap cakes, on cooling, manifest excellent eucalyptol-like, woody and minty aromas with an emphasis on the piney aspects of the aroma.

Example XXVI

*Preparation of a solid detergent composition*

A detergent is prepared from the following ingredients according to Example 1 of Canadian Patent No. 1,007,948:

| Ingredient | Parts by Weight |
|---|---|
| "Neodol® 45—11" (a $C_{14}$—$C_{15}$ alcohol ethoxylated with 11 moles of etheylene oxide) | 12 |
| Sodium carbonate | 55 |
| Sodium citrate | 20 |
| Sodium sulfate, water brighteners | q.s. |

This detergent is a "phosphate-free" detergent. (a) A totol of 100 grams of this detergent is admixed with 0.10, 0.15, 0.20 and 0.25 grams of the pine perfume of Example XXI(A). The detergent sample has an excellent herbaceous woody and piney aroma. (b) A total of 100 grams of said detergent is admixed with 0.10, 0.15, 0.20 and 0.25 grams of the pine perfume of Example XXI(B). The detergent sample has an excellent woody, eucalyptol-like, minty and piney aroma.

Example XXVII

*Preparation of a cosmetic powder composition*

(a) A cosmetic powder is prepared by admixing in a ball mill, 100 grams of talcum powder with 0.25 grams of one of the diisoamylene compounds prepared according to Example I. The resulting cosmetic powder has an excellent herbaceous, piney and woody aroma. (b) A cosmetic powder is prepared by admixing in a ball mill, 100 grams of talcum powder with 0.25 grams of one of the diisoamylene epoxide compound mixtures prepared according to Example II. The resulting cosmetic powder has an excellent eucalyptol-like, woody and minty aroma.

Example XXVIII

*Perfumed liquid detergent*

Concentrated liquid detergents (Lysine salt of n-dodecylbenzene sulfonic acid as more specifically described in U.S. Patent No. 3,948,818, issued on April 6, 1976) with herbaceous, woody and piney aroma notes are prepared containing 0.10%, 0.15%, 0.20% and 0.25% of one or more of the diisoamylene prepared according to Example I or one or more of the diisoamylene epoxides prepared according to Exmaple II. They are prepared by adding and homogeneously mixing the appropriate quantity of diisoamylene composition or diisoamylene epoxide composition in the liquid detergent. (a) With respect to the diisoamylene composition of Example I, the detergents all possess piney, woody and herbaceous nuances, the intensity of each characteristic increasing with greater concentrations of diisoamylene composition of Example I. (b) With respect to the diisoamylene epoxide composition of Example II, the detergents all possess eucalyptol-like, woody and minty aroma nuances, the intensity of each characteristic increasing with greater concentrations of diisoamylene epoxide compositions of Example II.

Example XXIX

*Preparation of colognes and handkerchief perfumes*

(a) The diisoamylene derivatives prepared according to Example I are incorporated into colognes at concentrations of 2.0%, 2.5%, 3.0%, 3.5%, 4.0% and 4.5% in 85% aqueous food grade ethanol; and into handkerchief perfumes at concentrations of 15%, 20%, 25% and 30% (in 95% aqueous ethanol). Distinctive herbaceous, woody and piney nuances are imparted to the colognes and to the handkerchief perfumes at various levels indicated above.

(b) The diisoamylene epoxide derivatives prepared according to Example II are incorporated into colognes at concentrations of 2.0%, 2.5%, 3.0%, 3.5%, 4.0% and 4.5% in 85% aqueous food grade ethanol;

and into handkerchief perfumes at concentrations of 15%, 20%, 25% and 30% (in 80%, 85%, 90% and 95% aqueous ethanol solutions). Distinctive eucalyptol-like, woody and minty aroma nuances are imparted to the colognes and to the handkerchief perfumes at various levels indicated above.

Example XXX

Utilizing the procedure of Example I of column 15 of U.S. Patent No. 3,632,396, a nonwoven cloth substrate useful as a dryer-added fabric softening article of manufacture prepared wherein the substrate, the substrate coating and the outer coating and the perfuming material are as follows:

1. a water "dissolvable" paper ("Dissolvo Paper")
2. Adogen 448® (m.p. about 140°F (60°C)) as the substrate coating and
3. an outer coating having the following formulation (m.p. about 150°F (66°C)):
   - 57% $C_{20-22}$ HAPS
   - 22% isopropyl alcohol
   - 20% antistatic agent
   - 1% of one or more of the diisoamylene derivatives of Example I or one of the diisoamylene epoxide derivative of Example II.

Fabric softening compositions having aroma characteristics in accordance with Table I, infra, essentially consist of a substrate having a weight of about 3 grams per 100 square inches (0.06 $m^2$) of substrate coating, and a weight of outer coating of about 1.4 grams per 100 square inches (0.06 $m^2$) of substrate coating thereby providing a total aromatized substrate and an outer coating weight ratio of about 1:1 by weight of the substrate. The aroma is indicated in Table I, infra, and is imparted in a pleasant manner to the head space in the dryer on operation thereof using said dryer-added fabric softening nonwoven fabric:

TABLE I

| Composition | Aroma Characteristics |
|---|---|
| Diisoamylene composition of Example I | A woody, piney and herbaceous aroma characteristic |
| Diisoamylene epoxide composition of Example II | A eucalyptol-like, woody and minty aroma characteristic |
| Perfume composition of Example XXI(A) | A piney aroma characteristic with woody and herbaceous nuances |
| Perfume composition of Example XXI(B) | A piney aroma with eucalyptol-like, woody and minty nuances |

In the following examples, AROMOX® DMC-W and AROMOX® DMMC-W are 30% aqueous solutions of dimethyl cocoamine oxide; and AROMOX® NCMDW is a 40% aqueous solution of N-cocomorpholine oxide produced by Armac Division of AKZO of Chicago, Illinois.

Example XXXI

Four drops of one of (a) the diisoamylene compositions prepared according to Example I or (b) the diisoamylene epoxide compositions prepared according to Example II, is added to two grams of AROMOX® DMC-W to produce a clear premix. The clear premix is added to 200 grams of CLOROX® with stirring resulting in a clear staple single phase solution. Sufficient 1 M aqueous NaOH is added to bring the pH of the mixture up to 12.8. The solution remains substantially stable at 120°F (49°C) for a period of seven days. When the 5% aqueous sodium hydrochlorite solution is used as a laundry bleach, the resulting laundry on dry-out in an atmosphere of 65% relative humidity yields substantially no characteristic "hypochlorite" odor but does have a faint pleasant aroma as indicated in Table I in Example XXX. Furthermore, no such characteristic "hypochlorite" aroma is retained on the hands of the individual handling such laundry in both the wet and the dry states.

Example XXXII

AROMOX® DMMC-W in various quantities is mixed with 0.1 grams of either (a) one of the diisoamylene compositions prepared according to Example I or (b) one or a mixture of the diisoamylene epoxide compositions prepared according to Example 11. The resulting premixes are then added to 200 grams of an aqueous 5% sodium hypochlorite solution. Sufficient 12.5 M aqueous NaOH is added to bring the pH of the mixture up to 13. The following results are obtained:

| Percentage Aromox® DMMC-W | Clarity of hypochlorite solution after addition of premix |
|---|---|
| 0.23% | Clear after three days |
| 0.15% | Clear after three days |
| 0.08% | Initially slightly turbid; two phases exist after three days |

When the 5% aqueous sodium hypochlorite solutions are used as laundry bleaches, the resulting laundry batches on dry-out in an atmosphere of 65% relative humidity yields substantially no characteristic "hydrochlorite" odor, but do have faint, pleasant aromas as indicated in Table I of Example XXX. Furthermore, no such characteristic "hypochlorite" aroma is retained on the hands of the individual handling such laundry batches in both the wet and the dry states.

### Example XXXIII

Two grams of AROMOX® DMMC-W is admixed with eight drops of either (a) one of the diisoamylene compositions of Example I or (b) one or a mixture of the diisoamylene epoxide compositions prepared according to Example II. The premix is then added with stirring to 200 grams of a 7% aqueous solution of lithium hypochlorite. Sufficient 3 M aqueous LiOH is added to bring the pH of the solution to 13.4. The mixture is then heated to 120°F (49°C) and maintained at that temperature with stirring for a period of 1 week. The resulting solution remains clear in a single phase. When used as a laundry bleach, the resulting bleached laundry on dry-out in an atmosphere of 50% relative humidity retains an aroma as shown in Table I in Example XXX; whereas without the use of one or more of the diisoamylene derivatives prepared according to Examples I or II, the bleached laundry has a faint characteristic disagreeable "hypochlorite" aroma.

### Example XXXIV

Two grams of AROMOX® DMMC-W is admixed with eight drops of either (a) one of the diisoamylene compositions of Example I or (b) one or a mixture of the diisoamylene epoxide compositions produced according to Example II. This premix is then added, stirring, to 200 grams of a mixture containing 4.5% aqueous sodium hypochlorite and 4.5% aqueous lithium hypochlorite. Sufficient 4 M aqueous LiOH is added to bring the pH of the solution of 13.4. The mixture is then heated to 120°F (49°C) and maintained at that temperature for a period of one week. The resulting solutions remain clear in a single phase. When used as a laundry bleach, the resulting bleached laundry on dry-out in an atmosphere of 50% relative humidity retains an aroma as set forth in Table I of Example XXX whereas without the use of the diisoamylene derivatives prepared according to Examples I or II, the bleached laundry has a faint characteristic disagreeable "hypochlorite" aroma.

### Example XXXV

Two grams of AROMOX® DMMC-W is admixed with eight drops of either (a) one of the diisoamylene products produced according to Example I or (b) one of the diisoamylene epoxide compositions produced according to Example II. This premix is then added, with stirring, to 200 grams of a mixture containing 4.5% aqueous sodium hypochlorite and 4.5% aqueous lithium hypochlorite. Sufficient 2 M aqueous NaOH is added to bring the pH of the solution to 13.4. The mixture is then heated to 110°F (43°C) and maintained at that temperature with stirring for a period of 2 weeks. The resulting solution remains clear as a single phase when used as a laundry bleach. The resulting bleached laundry, on dry-out in an atmosphere of 50% relative humidity, retains an aroma as indicated in Table I in Example XXX whereas without the use of the diisoamylene derivative compositions of Examples I or II, the bleached laundry has a faint characteristic disagreeable "hypochlorite" aroma.

### Example XXXVI

Four drops of either (a) one of the diisoamylene mixtures produced according to Example I or (b) one of the diisoamylene epoxide compositions produced according to Example II, is added to 1.5 grams of AROMOX® NCMDW to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 120°F (49°C) for a period of 7 days. When the 5% aqueous sodium hypochlorite solution is used as a laundry bleach, the resulting laundry on dryout in an atmosphere of 65% relative humidity yields substantially no characteristic "hypochlorite' odor but does have a faint pleasant aroma as indicated in Table I of Example XXX. Furthermore, no such characteristic "hypochlorite" aroma is retained on the hands of the individual handling such laundry in both the wet and the dry states.

Example XXXVII

Four drops of either (a) one of the diisoamylene mixtures produced according to Example I or (b) one of the diisoamylene epoxide compositions produced according to Example II is added to 1 gram n-undecyl dimethyl amine oxide to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 120°F (49°C) for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant aroma as described in Table I of Example XXX. Furthermore, no such characteristic "hypochlorite" aroma is retained on the hands of the individual handling such laundry in both the wet and the dry states.

Example XXXVIII

Four drops of either (a) one of the diisoamylene mixtures produced according to Exmaple I or (b) one of the diisoamylene epoxide compositions produced according to Example II is added to 1 gram of n-dodecyl dimethyl amine oxide to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 120°F (49°C) for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant aroma as described in Table I of Example XXX. Furthermore, no such characteristic "hypochlorite" aroma is retained on the hands of the individual handling such laundry in both the wet and the dry states.

Example XXXIX

One gram of n-tridecyl dimethyl amine oxide is admixed with eight drops of either (a) one of the diisoamylene compositions of Example I or (b) one of the diisoamylene epoxide compositions produced according to Example II. This premix is then added with stirring to 200 grams of a 7% aqueous solution of lithium hypochlorite. Sufficient 3 M aqueous LiOH is added to bring the pH of the solution to 13.4. The mixture is then heated to 120°F (49°C) and maintained at that temperature with stirring for a period of one week. The resulting solution remains clear in a single phase. When used as a laundry bleach, the resulting bleached laundry on dry-out in an atmosphere of 50% relative humidity retains a faint pleasant aroma as described in Table I of Example XXX; whereas without the use of one of the diisoamylene derivative compositions of Examples I or II, the bleached laundry has a faint characteristic disagreeable "hypochlorite" aroma.

Example XL

DOWFAX® 3B2 (see Note 2, infra) in various quantities, as set forth below, is mixed with 0.1 grams of either (a) one of the diisoamylene compositions prepared according to Example I or (b) one of the diisoamylene epoxide compositions prepared according to Example II. The resulting premixes are then added to 200 grams of an aqueous 7% sodium hypochlorite solution. Sufficient 12.5 M aqueous NaOH is added to bring the pH of the mixture up to 13.5. The following results are obtained:

| Percentage of DOWFAX® 3B2 | Clarity of hypochlorite solution after addition of premix |
|---|---|
| 0.23% | Clear after seven days |
| 0.15% | Clear after five days |
| 0.08% | Clear after three days |
| 0.01% | Initially slightly turbid; two phases exist after three days |

When used as laundry bleaches, the resulting bleached laundries on dry-out in an atmosphere of 50% relative humidity in each of the four cases above retain a pleasant faint aroma as set forth in Table I of Example XXX whereas without the use of the diisoamylene materials prepared according to Examples I or II, the bleached laundry has a faint characteristic disagreeable hypochlorite aroma.

*Note 2:* DOWFAX® 3B2 is a mixture of compounds essentially defined according to the structure:

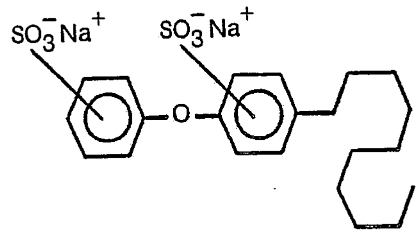

wherein the $SO_3^-Na^+$ moieties are at various positions on the phenyl moieties. DOWFAX® 3B2 is a registered trade mark of the Dow Chemical Company of Midland, Michigan.

In the following examples AROMOX® DMC-W and AROMOX® DMMC-W are 30% aqueous solutions of dimethyl cocoamine oxide; and AROMOX® NCMDW is a 40% aqueous solution of N-cocomorpholine oxide produced by Armac Division of AKZO of Chicago, Illinois.

Example XLI

Four drops of either (a) one of the diisoamylene compositions prepared according to Example I or (b) one of the diisoamylene epoxide compositions prepared according to Example II is added to two grams of DOWFAX® 3B2 and 0.5 grams of AROMOX® DMC-W to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 120°F (49°C) for a period of seven days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant aroma as set forth in Table I of Example XXX. Furthermore, no such characteristic "hypochlorite" aroma is retained on the hands of the individual handling such laundry in both the wet and the dry states.

Example XLII

One gram of DOWFAX® 3B2; one gram of DOWFAX® 2A1 and 0.25 grams of AROMOX® DMMC-W is admixed with eight drops of either (a) one of the diisoamylene compositions of Example I or (b) one of the diisoamylene epoxide compositions prepared according to Example II. This premix is then added with stirring to 200 grams of a mixture containing 4.5% aqueous sodium hypochlorite and 4.5% aqueous lithium hypochlorite. Sufficient 4 M aqueous LiOH is added to bring the pH of the solution to 13.4. The mixture is then heated to 120°F (49°C) and maintained at that temperature for a period of one week. The resulting solution remains clear in a single phase. When used as a laundry bleach, the resulting bleached laundry on dry-out in an atmosphere of 50% relative humidity retains an aroma as set forth in Table I of Example XXX whereas without the use of one of the diisoamylene derivatives prepared according to either Examples I or II, the bleached laundry has a faint characteristic disagreeable "hypochlorite" aroma.

Example XLIII

One gram of DOWFAX® 2A1 and one gram of DOWFAX® 3B2 is admixed with eight drops of either (a) one of the diisoamylene compositions of Example I or (b) one of the diisoamylene epoxide compositions prepared according to Example II. This premix is then added with stirring at 200 grams of a mixture containing 4.5% aqueous sodium hypochlorite. Sufficient 4M aqueous LiOH is added to bring the pH of the solution to 13.4. The mixture is then heated to 120°F (49°C) and maintained at that temperature for a period of one week. The resulting solution remains clear in a single phase. When used as a laundry bleach, the resulting bleached laundry on dry-out in an atmosphere of 50% relative humidity retains an aroma as set forth in Table I of Example XXX, whereas without the use of one of the diisoamylene derivatives prepared according to Example I or II, the bleached laundry has a faint characteristic disagreeable "hypochlorite" aroma.

Example XLIV

1.5 grams of DOWFAX® 2A1 is admixed with eight drops of either (a) one of the diisoamylene products produced according to Example I or (b) one of the diisoamylene epoxide compositions prepared according to Example II. This premix is then added with stirring to 200 grams of a mixture containing 4.5% aqueous sodium hypochlorite and 4.5% aqueous lithium hypochlorite. Sufficient 2 M aqueous NaOH is added to bring the pH of the solution to 13.4. The mixture is then heated to 110°F (43°C) and maintained at that temperature with stirring for a period of 2 weeks. The resulting solution remains clear as a single phase when used as a laundry bleach. The resulting bleached laundry on dry-out in an atmosphere of 50% relative humidity, retains an aroma as set forth in Table I of Example XXX whereas without the use of one of the diisoamylene derivatives of Example I or II, the bleached laundry has a faint characteristic disagreeable "hypochlorite" aroma.

Example XLV

Four drops of either (a) one of the diisoamylene mixtures produced according to Example I or (b) one of the diisoamylene epoxide compositions prepared according to Example II is added to 1.0 grams of DOWFAX® 3B2 and 0.25 grams of AROMOX® NCMDW to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 120°F (49°C) for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant aroma as set forth in Table I of Example XXX. Furthermore, no such characteristic "hypochlorite" aroma is retained on the hands of the individual handling such laundry in both the wet and the dry states.

Example XLVI

Four drops of either (a) one of the diisoamylene mixtures produced according to Example I or (b) one of the diisoamylene epoxide compositions prepared according to Example II is added to 0.1 gram n-undecyl dimethyl amine oxide and 0.9 grams of DOWFAX® 3B2 to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 120°F (49°C) for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant aroma as set forth in Table I of Example XXX. Furthermore, no such characteristic "hypochlorite" aroma is retained on the hands of the individual handling such laundry in both the wet and the dry states.

Example XLVII

Four drops of either (a) one of the diisoamylene mixtures produced according to Example I or (b) one of the diisoamylene epoxide compositions prepared according to Example II is added to 0.1 gram of n-dodecyl dimethyl amine oxide and 0.9 grams of DOWFAX® 2A1 to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 120°F (49°C) for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant aroma as set forth in Table I of Example XXX. Furthermore, no such characteristic "hypochlorite" aroma is retained on the hands of the individual handling such laundry in both the wet and the dry states.

Example XLVIII

0.2 grams of n-tridecyl dimethyl amine oxide and 0.7 grams of DOWFAX® 3B2 are admixed with eight drops of either (a) one of the diisoamylene compositions of Example I or (b) one of the diisoamylene epoxide compositions prepared according to Example II. This premix is then added with stirring to 200 grams of a 7% aqueous solution of lithium hypochlorite. Sufficient 3 M aqueous LiOH is added to bring the pH of the solution to 13.4. The mixture is then heated to 120°F (49°C) and maintained at that temperature with stirring for a period of one week. The resulting solution remains clear in a single phase. When used as a laundry bleach, the resulting bleached laundry on dry-out in an atmosphere of 50% relative humidity retains an aroma as set forth in Table I of Example XXX; whereas without the use of the diisoamylene derivatives of Examples I or II, the bleached laundry has a faint characteristic disagreeable "hypochlorite" aroma.

Example XLIX

A mixture is prepared consisting of 39 grams DOWFAX® 2A1 (60.75%); 4.5 grams sodium palmitate (7.00%): and 20.7 grams of water (32.25%). The mixture is heated while stirring followed by ultrasonic dispersion thereby yielding a homogeneous gel. 64.2 grams of this material is used as follows: 4 drops of one of the diisoamylene derivative mixtures produced according to either of Examples I or II is added to 2.0 grams of the foregoing gel to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 120°F (49°C) for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant aroma as set forth in Table I of Example XXX. Furthermore, no such characteristic "hypochlorite" aroma is retained on the hands of the individual handling such laundry in both the wet and the dry states.

Example L

A mixture is prepared consisting of 39 grams DOWFAX® 2A1 (60.75%); 4.5 grams sodium laurate (7.00%); and 20.7 grams of water (32.25%). The mixture is heated while stirring followed by ultrasonic

dispersion thereby yielding a homogeneous gel. 64.2 grams of this material is used as follows: 4 drops of either (a) one of the diisoamylene mixtures produced according to Example I or (b) one of the diisoamylene epoxide compositions prepared according to Example II is added to 2.0 grams of the foregoing gel to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 120°F (49°C) for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant aroma as set forth in Table I of Example XXX. Furthermore, no such characteristic "hypochlorite" aroma is retained on the hands of the individual handling such laundry in both the wet and the dry states.

Example LI

A mixture is prepared consisting of 20.1 grams of DOWFAX® 2A1 (60.75%); 2.0 grams sodium palmitate (7.00%); and 20.0 grams of water (32.25%). The mixture is heated while stirring followed by ultrasonic dispersion thereby yielding a homogeneous gel. 64.2 grams of this material is used as follows: 4 drops of either (a) one of the diisoamylene mixtures produced according to Example I or (b) one of the diisoamylene epoxide compositions prepared according to Example II is added to 2.0 grams of the foregoing gel to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 120°F (49°C) for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant aroma as set forth in Table I of Example XXX. Furthermore, no such characteristic "hypochlorite" aroma is retained on the hands of the individual handling such laundry in both the wet and the dry states.

Example LII

A mixture is prepared consisting of 20.1 grams DOWFAX® 2A1 (60.75%); 2.0 grams sodium laurate (7.00%); and 20.0 grams of water (32.25%). The mixture is heated while stirring followed by ultrasonic dispersion thereby yielding a homogeneous gel. 64.2 grams of this material is used as follows: 4 drops of either (a) one of the diisoamylene mixtures produced according to Example I or (b) one of the diisoamylene epoxide compositions prepared according to Example II is added to 2.0 grams of the foregoing gel to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 120°F (49°C) for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint pleasant aroma as set forth in Table I of Example XXX. Furthermore, no such characteristic "hypochlorite" aroma is retained on the hands of the individual handling such laundry in both the wet and the dry states.

Example LIII

A mixture is prepared consisting of 10 grams DOWFAX® 2A1 and 10 grams DOWFAX® 3B2 (60.75%); and 2.0 grams sodium laurate (7.00%); and 20.0 grams of water (32.25%). The mixture is heated while stirring followed by ultrasonic dispersion thereby yielding a homogeneous gel. 64.2 grams of this material is used as follows: 4 drops of either (a) one of the diisoamylene mixtures produced according to Example I or (b) one of the diisoamylene epoxide compositions prepared according to Example II is added to 2.0 grams of the foregoing gel to produce a clear premix. The clear premix is added to 200 grams of CLOROX® with stirring resulting in a clear stable single phase solution. Sufficient 1 M aqueous NaOH is added to bring the pH of the mixture up to 12.8. The solution remains substantially stable at 120°F (49°C) for a period of 7 days. When the 5% aqueous sodium hypochlorite solution is used as a laundry bleach, the resulting laundry on dry-out in an atmosphere of 65% relative humidity yield substantially no characteristic "hypochlorite" odor but does have a faint pleasant aroma as set forth in Table I of Example XXX. Furthermore, no such characteristic "hypochlorite" aroma is retained on the hands of the individual handling such laundry in both the wet and the dry states.

## Example LIV

*Dill spice flavor*

The following formulation is produced:

| Ingredients | Parts by Weight |
|---|---|
| Oil of dillweed | 84.5 |
| Oil of garlic | 1.75 |
| Oil of capsicum | 3.75 |
| Oil of cassia | 2.50 |
| Oil of cloves | 2.50 |
| Oil of pimenta berries | 2.50 |
| Oil of mustard | 1.25 |
| Oil of bay leaves | 1.25 |
| L-carvone | 4.60 |
| D-carvone | 3.80 |
| Cumin aldehyde | 2.51 |

The formulation is split into two parts. To one part is added the acetyl diisobutylene mixture produced according to Example X. To the second part nothing is added. Both formulations are prepared at levels of 5 ppm, 10 ppm and 20 ppm in water and in tomato ketchup. The formulation containing the acetyl diisobutylene produced according to Example X causes the flavor to be much more full-bodied and more natural-like as the flavor has caraway and minty nuances in addition to "natural" dill-like nuances. The "natural" aspect of this flavor together with the caraway-like and minty nuances are missing in the flavor that does not contain the acetyl diisobutylene composition of matter produced according to Example X.

## Example LV

*Spearmint flavor*

The following spearmint flavor is produced.

| Ingredients | Parts by Weight |
|---|---|
| Spearmint oil | 42.0 |
| L-carvone | 12.0 |
| D-carvone | 8.0 |

This formulation is split into two portions. To the first portion, 4.0 parts by weight of the acetyl diisobutylene produced according to Example X is added. To the second portion nothing is added. The flavor is tested at levels of 5 ppm, 10 ppm and 20 ppm in water and in chewing gum which heretofore was unflavored. The chewing gum containing the flavor formulation with the acetyl diisobutylene material produced according to Example X has a more natural-like, minty aroma and taste with excellent caraway-like and dill-like nuances present. The formulation has a great amount of "natural mintyness" which is not only "natural-like" but which is also "long lasting" and remains of high strength throughout the chewing procedure of the chewing gum.

Example LVI

A toothpaste is prepared according to the following formula:

| Ingredients | Parts by Weight |
|---|---|
| Sorbitol (70% solution) | 20.00 |
| Sodium saccharin | 0.21 |
| Veegum® (colloidal magnesium aluminum silicate) | 0.40 |
| Precipitated urea/ formaldehyde condensate (abrasive) | 30.00 |
| Acetyl diisobutylene prepared according to Example X, fraction 9 of distillate | 1.00 |
| Sodium carboxymethylcellulose | 1.30 |
| Glycerine | 10.00 |
| 1,2-bis($N^5$-p-chlorophenyl-$N^1$-biguanido) ethane digluconate | 0.70 |
| Polyoxyethylene sorbitan monoisostearate | 1.50 |
| Distilled water | balance to 100 |

This toothpaste when used in the normal manner, is not only effective in retarding the formation of dental plaque and produces an appreciably lower level of stain on the teeth than does chlorhexidine; but, in addition, has an excellent sweet, spicy, minty, herbaceous, breadlike, caraway-like and dill-like taste profile.

Example LVII

A mouthwash in accordance with the present invention is formulated as follows:

| Ingredients | Parts by Weight |
|---|---|
| Ethyl alcohol (95%) in water) | 12.00 |
| Cetyl pyridinum chloride | 0.10 |
| Polyoxyethylene (20) sorbitan monooleate | 0.12 |
| Sodium hydroxide (10% in water) | 0.02 |
| Sodium saccharin | 0.055 |
| Acetyl diisobutylene product produced according to Example X; fraction 2 | 0.16 |
| 1,2-bis($N^5$-p-chlorophenyl-$N^1$-biguanido) ethane dihydrochloride | 0.20 |
| Color | 0.50 |
| Sorbitol (70% in water) | 12.00 |
| Distilled water | balance to 100 |

When used in the normal manner to rinse the mouth, this product is effective not only in retarding the formation of dental plaque and produces an appreciably lower level of stain on the teeth than does chlorohexidine; but it also has an excellent sweet, spicy, minty, herbaceous, bread-like, caraway-like, and dill-like taste profile.

Example LVIII

A chewing gum in accordance with the present invention is formulated as follows:

| Ingredients | Parts by Weight |
|---|---|
| Gum base | 21.30 |
| Ester gum | 6.40 |
| Coumarone resin | 9.60 |
| Dry latex rubber | 3.20 |
| Paraffin was (m.p. 180°C) | 2.10 |
| Sugar | 58.45 |
| Corn syrup (Baume 45) | 18.20 |
| Acetyl diisobutylene product produced according to Example X; bulked fractions 2—9 | 1.05 |
| 1,2-bis($N^5$-p-chlorophenyl-$N^1$-biguanido) ethane diacetate | 1.00 |

Chewing this gum in the normal manner not only retards the formation of dental plaque and produces appreciably less staining on the teeth than does chlorhexidine but it also gives rise to a pleasant, long-lasting and intense sweet, spicy, minty, herbaceous, bread-like, caraway-like and dill-like taste profile.

Example LIX

The dioxolanes produced according to Example XI(A), XI(B) and XII have very long-lasting cedar, amber, woody, sweet and patchouli-like aroma nuances which may be utilized to a great extent in inexpensive, functional products. The following pine fragrance demonstrates the use of these materials in perfume compositions. In each of the cases, the ketals of Examples XI(A), XI(B) and XII are used in in an amount of 47.9%.

| | Parts by Weight | |
|---|---|---|
| Ingredients | Example LIX (A) | Example LIX (B) |
| Isobornyl acetate | 100 | 100 |
| Camphor | 10 | 10 |
| Terpineol | 25 | 25 |
| Fir balsam absolute (50% in diethyl phthalate) | 20 | 20 |
| Coumarin | 4 | 4 |
| Linalool | 30 | 30 |
| Anethol | 2 | 2 |
| Fenchyl alcohol | 10 | 10 |

| Ingredients | Parts by Weight | |
|---|---|---|
| | Example LIX (A) | Example LIX (B) |
| Lemon terpenes washed | 50 | 50 |
| Borneol | 5 | 5 |
| Galbanum oil | 5 | 5 |
| Turpentine Russian | 150 | 150 |
| Pinus Pumilionus | 50 | 50 |
| Eucalyptol | 50 | 50 |
| 2,2,6-trimethyl-1-cyclohexene-1-carboxaldehyde | 5 | 5 |
| Maltol 1% in diethyl phthalate | 5 | 5 |
| Product produced according to either Example XI (A) or XI (B), a mixture of products defined by the structure: 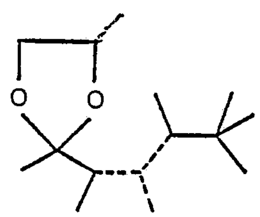 wherein in each of the molecules in the mixture, one of the dashed lines represents a carbon-carbon double bond and the other dashed lines represent carbon-carbon single bonds. | 479 | 0 |

| Ingredients | Parts by Weight | |
|---|---|---|
| | Example LIX (A) | Example LIX (B) |
| Product produced according to either Example XI(A) or XI(B), a mixture of products defined by the structure: 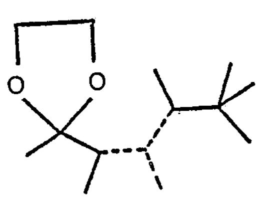 wherein in each of the molecules in the mixture, one of the dashed lines represents a carbon-carbon double bond and the other dashed lines represent carbon-carbon single bonds. | 0 | 479 |

Example LX

*Preparation of a cosmetic powder preparation*

A cosmetic powder is prepared by mixing in a ball mill, 100 grams of the 2,4-dimethyl-2(1,2,3,4,4-pentamethyl)-pentenyl-1,3-dioxolane isomer mix prepared according to either Example XI(A) or XI(B). The resulting cosmetic powder has a pleasant cedar, amber, woody, sweet, patchouli aroma.

Example LXI

*Perfumed liquid detergent*

Concentrated liquid detergents (lysine salt of n-dodecylbenzene sulfonic acid as more specifically described in U.S. Patent No. 3,948,818 issued on April 6, 1976) with pleasant cedar, amber, woody, sweet and patchouli aroma profiles are prepared containing 0.10%, 0.15%, 0.20%, 0.25%, 0.30% and 0.35% of 2,4-dimethyl-2-(1,2,3,4,4-pentamethyl)-pentenyl-1,3-dioxolane isomer mix prepared according to either Example XI(A) or XI(B). They are prepared by adding and homogenously mixing the appropriate quantity of the mixture prepared according to either Example XI(A) or XI(B) in the liquid detergent. The detergents all possess excellent intense pleasant, cedar, amber, woody, sweet and patchouli aroma profiles, the intensity increasing with greater concentrations of the dioxolane isomer mixture prepared according to either Example XI(A) or XI(B).

Example LXII

*Preparation of a cologne and handkerchief perfume*

The 2,4-dimethyl-2-(1,2,3,4,4-pentamethyl)-pentenyl-1,3-dioxolane isomer mixture prepared according to either Example XI(A) or XI(B) is incorporated into colognes at concentrations of 2.0%, 2.5%, 3.0%, 3.5%, 4.0%, 4.5% and 5.0% in 75%, 80%, 85% and 90% aqueous food grade ethanol; and into handkerchief perfumes at concentrations of 15%, 20%, 25% and 30% in 80%, 85%, 90% and 95% aqueous food grade ethanol solutions. Distinctive and definitive long-lasting cedar, amber, woody, sweet and patchouli aroma profiles are imparted to the cologne and to the handkerchief perfume compositions at all levels indicated.

Example LXIII

*Preparation of soap composition*

One hundred grams of soap chips (IVORY®, produced by the Procter & Gamble Company, Cincinnati, Ohio) are admixed with one gram of the 2,4-dimethyl-2-(1,2,3,4,4-pentamethyl)-pentenyl-1,3-dioxolane mixture produced according to Examples XI(A) or XI(B) until a homogeneous composition is obtained. The homogeneous composition is then heated under 3 atmospheres ($3 \times 10^5$ N/$m^2$) pressure at 180°C for a period of 3 hours and the resulting liquid is placed in a soap mold. The resulting soap cake, on cooling, manifests an excellent long-lasting pleasant cedar, amber, woody, sweet and patchouli aroma.

Example LXIV

*Preparation of a solid detergent composition*

Detergents are prepared from the following ingredients according to Example I of Canadian Patent No. 1,007,948:

| Ingredient | Parts by Weight |
|---|---|
| Neodol® 45—11 (a $C_{14}$—$C_{15}$ alcohol ethoxylated with 11 moles of ethylene oxide) | 12 |
| Sodium carbonate | 55 |
| Sodium citrate | 20 |
| Sodium sulfate, water brighteners | q.s. |

This detergent is a phosphate-free detergent. A total of 100 grams of said detergent is admixed with 0.10, 0.15, 0.20 and 0.25 grams of the 2,4-dimethyl-2-(1,2,3,4,4-pentamethyl)-pentenyl-1,3-dioxolane isomer mix prepared according to either Example XI(A) or XI(B). Each of the detergent samples has an excellent pleasant, cedar, amber, woody, sweet and patchouli aroma profile.

Example LXV

*Dryer-added fabric softener article*

Utilizing the procedure of Example I at column 15 of U.S. Patent 3,632,396, a non-woven cloth substrate useful as a dryer-added fabric softening article of manufacture prepared wherein the substrate, the substrate coating and the outer coating and the perfuming material are as follows:

1. a water "dissolvable" paper ("Dissolvo Paper")
2. Adogen 448® (m.p. about 140°F (60°C)) as the substrate coating; and
3. an outer coating having the following formulation (m.p. about 150°F (66°C)):

   57% $C_{20-22}$ HAPS

   22% isopropyl alcohol

   20% antistatic agent

   1% of the 2,4-dimethyl-2-(1,2,3,4,4-pentamethyl)-pentenyl-1,3-dioxolane isomer mix prepared according to either Example XI(A) or XI(B).

Fabric softening compositions containing the dioxolane mixture prepared according to either Example XI(A) or XI(B) consist essentially of a substrate having a weight of about 3 grams per 100 square inches (0.06 $m^2$), a substrate coating of about 1.85 grams per 100 square inches (0.06 $m^2$) of substrate, and an outer coating of about 1.4 grams per 100 square inches (0.06 $m^2$) of substrate, thereby providing a totally aromatized substrate and outer coating weight ratio of about 1:1 by weight of the substrate. A pleasant cedar, amber, woody, sweet and patchouli faint aroma profile is imparted to the head space in the dryer on operation thereof using the said dryer-added fabric softening non-woven fabric.

In the following examples, AROMOX® DMC—W and AROMOX® DMMC—W are 30% aqueous solutions of dimethyl cocoamine oxide; and AROMOX® NCMDW is a 40% aqueous solution of N-cocomorpholine oxide produced by Armac Division AKZO of Chicago, Illinois.

Example LXVI

Four drops of the 2,4-dimethyl-2-(1,2,3,4,4-pentamethyl)-pentenyl-1,3-dioxolane isomer mix prepared according to either Example XI(A) or XI(B) is added to 2 grams of AROMOX® DMC—W to produce a clear premix. The clear premix is added to 200 grams of CLOROX® with stirring resulting in a clear stable single phase solution.

Sufficient 1M aqueous NaOH is added to bring the pH of the mixture up to 12.8. The solution remains substantially stable at 120°F (49°C) for a period of seven days. When the 5% aqueous sodium hypochlorite solution is used as a laundry bleach, the resulting laundry on dry-out in an atmosphere of 65% relative humidity yields substantially no characteristic "hypochlorite" odor but does have a faint, pleasant, cedar, amber, woody, sweet and patchouli aroma. Furthermore, no such characteristic "hypochlorite" aroma is retained on the hands of the individual handling such laundry in both the wet and the dry states.

Example LXVII

AROMOX® DMMC—W in various quantities is mixed with 0.1 gram of the 2,4-dimethyl-2-(1,2,3,4,4-pentamethyl-1-pentenyl)-1,3-dioxolane isomer mix prepared according to either Example XI(A) or XI(B). The resulting premixes are then added to 200 grams of an aqueous 5% sodium hypochlorite solution. Sufficient 12.5 M aqueous NaOH is added to bring the pH of the mixture up to 13. The following results are obtained:

| Percentage of Aromox® DMMC—W | Clarity of hypochlorite solution after addition of premix |
|---|---|
| 0.23% | Clear after three days |
| 0.15% | Clear after three days |
| 0.08% | Initially slightly turbid; two phases exist after three days |

When the 5% sodium hypochlorite solutions are used as laundry bleaches, the resulting laundry batches on dry-out in an atmosphere of 65% relative humidity yield substantially no characteristic "hypochlorite" odor but do have faint, pleasant, cedary, amber, woody, sweet, and patchouli aroma profiles. Furthermore, no such characteristic "hypochlorite" aromas are retained on the hands of the individuals handling the laundry batches in both the wet and the dry states.

Example LXVIII

Two grams of AROMOX® DMMC—W is admixed with eight drops of the 2,4-dimethyl-2-(1,2,3,4,4-pentamethyl-1-pentenyl)-1,3-dioxolane isomer mix produced according to either Example XI(A) or XI(B). The premix is then added with stirring to 200 grams of a 7% aqueous solution of lithium hypochlorite. Sufficient 3M aqueous LiOH is added to bring the pH of the solution to 13.4. The mixture is then heated to 120°F (49°C) and maintained at that temperature with stirring for a period of 1 week. The resulting solution remains clear in a single phase. When used as a laundry bleach, the resulting bleached laundry on dry-out in an atmosphere of 50% relative humidity retains a "clean fresh" pleasant, cedar, amber, woody, sweet, patchouli aroma profile; whereas without the use of the 1,3-dioxolane derivative prepared according to Example XI, the bleached laundry has a faint, characteristic disagreeable "hypochlorite" aroma.

Example LXIX

Two grams of AROMOX® DMMC—W is admixed with eight drops of the 2,4-dimethyl-2-(1,2,3,4,4-pentamethyl-1-pententyl)-1,3-dioxolane isomer mix prepared according to either Example XI(A) or XI(B). This premix is then added, with stirring, to 200 grams of a mixture containing 4.5% aqueous sodium hypochlorite and 4.5% aqueous lithium hypochlorite. Sufficient 4M aqueous LiOH is added to bring the pH of the solution to 13.4. The mixture is then heated to 120°F (49°C) and maintained at that temperature for a period of one week. The resulting solution remains clear in a single phase. When used as a laundry bleach,

the resulting bleached laundry on dry-out in an atmosphere of 50% relative humidity retains a "clean fresh", pleasant, cedar, amber, woody, sweet and patchouli aroma profile whereas without the use of the 1,3-dioxolane derivative prepared according to Example XI, the bleached laundry has a faint characteristic disagreeable "hypochlorite" aroma.

Example LXX

Two grams of AROMOX® DMMC—W is admixed with eight drops of 2,4-dimethyl-2-(1,2,3,4,4-pentamethyl-1-pentenyl)-1,3-dioxolane isomer mix prepared according to either Example XI(A) or XI(B). This premix is then added with stirring to 200 grams of a mixture containing 4.5% aqueous sodium hypochlorite and 4.5% aqueous lithium hypochlorite. Sufficient 2M aqueous NaOH is added to bring the pH of the solution to 13.4. The mixture is then heated to 110°F (43°C) and maintained at that temperature with stirring for a period of 2 weeks. The resulting solution remains clear as a single phase when used as a laundry bleach. The resulting bleached laundry, on dry-out in an atmosphere of 50% relative humidity, retains a pleasant, cedar, amber, woody, sweet and patchouli aroma profile whereas without the use of the 1,3-dioxolane composition prepared according to either Example XI(A) or XI(B), the bleached laundry has a faint characteristic disagreeable "hypochlorite" aroma.

Example LXXI

Four drops of a 50:50 mixture containing 50 weight percent of 2,4-dimethyl-2-(1,2,3,4,4-pentamethyl-1-pentenyl)-1,3-dioxolane isomer mixture prepared according to either Example XI(A) or XI(B) and 50% of acetyl diisoamylene prepared according to Example III is added to 1.5 grams of AROMOX® NCMDW to produce a clear premix. The clear premix is added to 200 grams of CLOROX® with stirring resulting in a clear, stable, single phase solution. Sufficient 1M aqueous NaOH is added to bring the pH of the mixture up to 12.8. The solution remains substantially stable at 120°F (49°C) for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a faint, pleasant, cedar, amber, woody, sweet, patchouli aroma profile. Furthermore, no such characteristic "hypochlorite" aroma is retained on the hands of the individual handling such laundry in both the wet and the dry states.

Example LXXII

Four drops of the dioxolane prepared according to Example XII is added to 1 gram of n-undecyl dimethyl amine oxide to produce a clear premix. The clear premix is added to 200 grams of CLOROX® with stirring resulting in a clear stable single phase solution. Sufficient 1M aqueous NaOH is added to bring the pH of the mixture up to 12.8. The solution remains substantially stable at 120°F (49°C) for a period of 7 days. When the 5% aqeuous sodium hypochlorite solution is used as a laundry bleach, the resulting laundry on dry-out in an atmosphere of 65% relative humidity yields substantially no characteristic "hypochlorite" aroma but does have a faint, pleasant, cedar-like, amber, patchouli-like aroma. Furthermore, no such characteristic "hypochlorite" aroma is retained on the hands of the individual handling such laundry in both the wet and the dry states.

Example LXXIII

Four drops of a 50:50 mixutre of the dioxolane of either Example XI(A) or XI(B) and the dioxolane of Example XII is added to 1 gram of n-dodecyl dimethyl amine oxide to produce a clear premix. The clear premix is added to 200 grams of CLOROX® with stirring resulting in a clear, stable, single phase solution. Sufficient 1M aqueous NaOH is added to bring the pH of the mixture up to 12.8. The solution remains substantially stable at 120°F (49°C) for a period of 7 days. When the 5% aqueous sodium hypochlorite solution is used as a laundry bleach, the resulting laundry on dry-out in an atmosphere of 65% relative humidity yields substantially no characteristic "hypochlorite" odor but does have a very warm, pleasant, cedar, amber, woody, vetiver-like, sweet and patchouli aroma. Furthermore, no such characteristic "hypochlorite" aroma is retained on the hands of the individual handling such laundry in both the wet and the dry states.

Example LXXIV

One gram of the dioxolane of Example XII is intimately admixed with 1 gram of n-tridecyl dimethylamine oxide. This premix is then added with stirring to 200 grams of a 7% aqueous solution of lithium hypochlorite. Sufficient 3M aqueous LiOH is added to bring the pH of the solution to 13.4. The mixture is then heated to 120°F (49°C) and maintained at that temperature with stirring for a period of one week. The resulting solution remains clear in a single phase. When used as a laundry bleach, the resulting bleached laundry on dry-out in an atmosphere of 50% relative humidity retains a clean, fresh, cedar, amber, woody, sweet, patchouli-like aroma whereas without the use of the dioxolane composition mixture of Examples XII and either of XI(A) or XI(B), the bleached laundry has a faint characteristic disagreeable "hypochlorite" aroma.

Example LXXV

*Flavor composition*

The following basic walnut flavor formulation is prepared:

| Ingredients | Parts by Weight |
|---|---|
| Ethyl-2-methyl butyrate | 10 |
| Vanillin | 40 |
| Butyl valerate | 40 |
| 2,3-diethyl pyrazine | 5 |
| Methyl cyclopentenolone | 80 |
| Benzaldehyde | 60 |
| Valerian oil Indian (1% in 95% aqueous ethanol alcohol). | 0.5 |
| Propylene glycol | 764.5 |

The 1,3-dioxolane derivative isomer mixture prepared according to either Example XI(A) or XI(B) is added to the above formulation at the rate of 1.5%. This formulation is compared to a formulation which does not have such 1,3-dioxolane derivative mixture added to it at the rate of 20 ppm in water. The formulation containing the 1,3-dioxolane derivative mixture has a "woody, balsamic", fresh, walnut kernel and walnut skin-like taste and, in addition, has a fuller mouth feel and longer lasting taste. The flavor that has added to it the 1,3-dioxolane isomer mixture prepared according to either Example XI(A) or XI(B) is preferred by a group of flavor panelists and they consider it to be a substantially improved walnut flavor.

Example LXXVI

*Beverage*

The addition of the 1,3-dioxolane derivative isomer mixture prepared according to the process of Example XII is added at the rate of 0.3 ppm to a commercial cola beverage giving the beverage a fuller "woody, balsamic" long-lasting taste. The dioxolane derivative mixture adds to the pleasant top-notes of the beverage. When comparing the cola beverage containing the 1,3-dioxolane isomer mixture to one having the same formula but not containing the dioxolane derivative isomer mixture, a five member bench panel prefers the beverage containing the 1,3-dioxolane isomer mixture.

Example LXXVII

*Tobacco flavor formulation*

Cigarettes are produced using the following tobacco formuation:

| Ingredients | Parts by weight |
|---|---|
| Bright | 40.1 |
| Burley | 24.9 |
| Maryland | 1.1 |
| Turkish | 11.6 |
| Stem (flue-cured) | 14.2 |
| Glycerine | 2.8 |
| $H_2O$ | 5.3 |

At the rate of 0.2%, the following tobacco flavor formulation is applied to all of the cigarettes produced with the above tobacco formulation:

| Ingredients | Parts by weight |
|---|---|
| Ethyl butyrate | 0.05 |
| Ethyl balerate | 0.05 |
| Maltol | 2.00 |
| Cocoa extract | 26.00 |
| Coffee extract | 10.00 |
| Ethyl alcohol (95%) | 20.00 |
| $H_2O$ | 41.90 |

To 50% of the cigarettes, 10 and 20 ppm of the 1,3-dioxolane derivative isomer mixture prepared according to either Example XI(A) or XI(B) are added. These cigarettes are hereinafter called "experimental" cigarettes and the cigarettes without the 1,3-dioxolane derivative isomer mixture are hereinafter called "control" cigarettes. The control and experimental cigarettes are then evaluated by paired comparison and the results are as follows:

a. In aroma, the experimental cigarettes are found to be more aromatic.

b. In smoke flavor, the experimental cigareetes are found to be more aromatic, more sweet, more bitter, more green, richer and slightly less harsh in the mouth and more cigarettes tobacco-like than the control cigarettes.

The experimental cigarettes containing 20 ppm of the 1,3-dioxolane derivative isomer mixture prepared according to either Example XI(A) or XI(B) are found to be woody, slightly chemical and mouthcoating in the smoke flavor. All cigarettes, both control and experimental, are evaluated for smoke flavor with a 20 mm cellulose acetate filter. The dioxolane derivative prepared according to either Example XI(A) or XI(B) enhances the tobacco-like taste of the blended cigarette. When the dioxolane derivative of either Example XI(A) or XI(B) is replaced by the dioxolane derivative prepared according to Example XII, closely similar results are obtained.

Example LXXVIII

*Perfume formulation*

The branched chain unsaturated tertiary alcohols produced according to Examples XIII—XVI inclusive, have intense amber, woody, fruity, floral, orris-like and camphoraceous and patchouli-like notes which may be utilized to a great extent in inexpensive functional products. The following pine fragrance demonstrates the use of these materials in perfume compositions. In this case, it is used in concentrations of 47.9%.

| | Parts by Weight | | | |
|---|---|---|---|---|
| Ingredients | Example C/A) | Example C(B) | Example C(C) | Example C(D) |
| Isobornyl acetate | 100 | 100 | 100 | 100 |
| Camphor | 10 | 10 | 10 | 10 |
| Terpineol | 25 | 25 | 25 | 25 |
| Fir balsam absolute (50% in diethyl phthalate) | 20 | 20 | 20 | 20 |
| Coumarin | 4 | 4 | 4 | 4 |
| Linalool | 30 | 30 | 30 | 30 |
| Anethol | 2 | 2 | 2 | 2 |
| Fenchyl alcohol | 10 | 10 | 10 | 10 |

| Ingredients | Parts by Weight<br>Example C(A) | Example C(B) | Example C(C) | Example C(D) |
|---|---|---|---|---|
| Lemon terpenes washed | 50 | 50 | 50 | 50 |
| Borneol | 5 | 5 | 5 | 5 |
| Galbanum | 5 | 5 | 5 | 5 |
| Turpentine Russian | 150 | 150 | 150 | 150 |
| Pinus Pumilionus | 50 | 50 | 50 | 50 |
| Eucalyptol | 50 | 50 | 50 | 50 |
| 2,2,6-trimethyl-1-cyclohexene-1-carboxaldehyde | 5 | 5 | 5 | 5 |
| Maltol 1% in diethyl phthalate | 5 | 5 | 5 | 5 |
| Product of Example XIII | 479 | 0 | 0 | 0 |
| Product of Example XIV | 0 | 479 | 0 | 0 |
| Product of Example XV | 0 | 0 | 479 | 0 |
| Product of Example XVI | 0 | 0 | 0 | 479 |

The presence of the various unsaturated branched chain tertiary alcohols support the pine notes and produce considerable savings in the cost of the formulation. However, each of the materials of each of the examples gives rise to separate nuances as follows:

TABLE II

| Structure | Organoleptic Properties (in perfumery and in perfumed articles) |
|---|---|
| 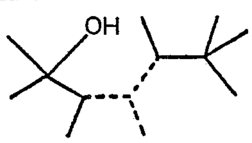 Mixture prepared according to Example XIII supra wherein in each of the molecules of the mixture one of the dashed lines represents a carbon-carbon double bond and the other dashed lines represent carbon-carbon single bonds | An amber, woody, camphoraceous aroma with patchouli top-notes. |

TABLE II (continued)

| Structure | Organoleptic Properties (in perfumery and in perfumed articles) |
| --- | --- |
| 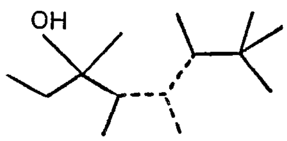 Prepared according to Example XIV, a mixture wherein in each of the molecules of the mixture one of the dashed lines represents a carbon-carbon double bond and the other dashed lines represent carbon-carbon single bonds. | An amber, woody, fruity aroma, |
| 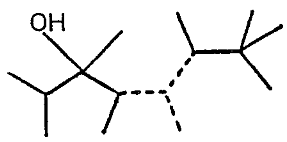 A mixture produced according to Example XV wherein in each of the molecules of the mixture, one of the dashed lines is a carbon-carbon double bond and the other dashed lines represent carbon-carbon single bonds. | An orris-like aroma. |
| 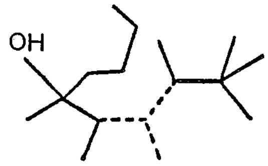 A mixture produced according to Example XVI wherein in each of the molecules of the mixture one of the dashed lines is a carbon-carbon double bond and the other dashed lines represent carbon-carbon single bonds. | A floral, woody aroma. |

Example LXXIX

*Preparation of a cosmetic powder composition*

Cosmetic powders are prepared by mixing in a ball mill 100 grams of talcum powder with 0.25 grams of each of the substances set forth in Table II of Example LXXVIII. Each of the substances have aromas as set forth in Table II of Example LXXVIII.

Example LXXX

*Perfumed liquid detergents*

Concentrated liquid detergents (Lysine salt of n-dodecylbenzene sulfonic acid as more specifically described in U.S. Patent No. 3,948,818, issued on April 6, 1976) with aroma nuances as indicated in Table II of Example LXXVIII are prepared containing 0.10%, 0.15%, 0.20%, 0.25%, 0.30% and 0.35% of the substances as set forth in Table II of Example LXXVIII. They are prepared by adding and homogeneously mixing the appropriate quantity of such substances in the liquid detergent. The detergents all possess aromas as set forth in Table II of Example LXXVIII. The intensities of each of the aromas increases with greater concentrations of substance.

Example LXXXI

*Preparation of colognes and handkerchief perfumes*

Compositions as set forth in Table II of Example LXXVIII are incorporated into colognes at concentrations of 2.0%, 2.5%, 3.0%, 3.5%, 4.0%, 4.5% and 5.0% in 75%, 80%, 85% and 90% aqueous food grade ethanol solutions; and into handkerchief perfumes at concentrations of 15%, 20%, 25% and 30% (in 90%, 95% and 96% aqueous food grade ethanol solutions). Distinctive long-lasting pleasant aromas are imparted as set forth in Table II of Example LXXVIII to the colognes and to the handkerchief perfumes at all levels indicated above.

Example LXXXII

*Preparation of soap composition*

100 grams of soap chips (IVORY® produced by the Procter and Gamble Company of Cincinnati, Ohio) are mixed with 1 gram of each of the substances of Table II of Example LXXVIII until homogeneous compositions are obtained. In each of the cases, the homogeneous compositions are heated under 3 atmospheres ($3 \times 10^5 N/m^2$) pressure at 180°C for a period of 3 hours and the resulting liquids are then placed into soap molds. The resulting soap cakes, on cooling, manifest aromas as set forth in Table II of Example LXXVIII.

Example LXXXIII

*Preparation of solid detergent compositions*

Detergents are prepared from the following ingredients according to Example I of Canadian Patent No. 1,007,948:

| Ingredient | Percent by Weight |
|---|---|
| Neodol® 45—11 (a $C_{14}$—$C_{15}$ alcohol ethoxylated with 11 moles of ethylene oxide) | 12 |
| Sodium carbonate | 55 |
| Sodium citrate | 20 |
| Sodium sulfate, water brighteners | q.s. |

This detergent is a phosphate-free detergent. A total of 100 grams of said detergent is admixed with 0.10, 0.15, 0.20 and 0.25 grams of each of the substances set forth in Table II of Example LXXVII. Each of the detergent samples has pleasant and long-lasting aromas as set forth in Table II of Example LXXVIII.

Example LXXXIV

*Dryer-added fabric softener article*

Utilizing the procedure of Example I at column 15 of U.S. Patent No. 3,632,296, a nonwoven cloth substrate useful as a dryer-added fabric softening article of manufacture is prepared wherein the substrate, the substrate coating and the outer coating as well as the perfuming material are as follows:

1. a water "dissolvable" paper ("Dissolvo Paper")
2. Adogen 448® (m.p. about 140°F (60°C)) as the substrate coating; and

3. an outer coating having the following formulation (m.p. about 150°F (66°C)):
57% $C_{20-22}$ HAPS
22% isopropyl alcohol
20% antistatic agent
1% of one of the substances set forth in Table II of Example LXXVIII

Fabric softening compositions prepared according to Example I of U.S. Patent No. 3,632,396 having aroma characteristics as set forth in Table II of Example LXXVIII essentially consist of a substrate having a weight of about 3 grams per 100 square inches (0.06 $m^2$); a substrate coating having a weight of about 1.85 grams per 100 square inches (0.06 $m^2$); and an outer coating having a weight of about 1.4 grams per 100 square inches (0.06 $m^2$) of substrate, thereby providing a total aromatized substrate and outer coating weight ratio of about 1:1 by weight of the substrate. The aroma characteristics are as set forth in Table II of Example LXXVIII and are imparted in a pleasant manner to the head space in the dryer on operation thereof, using said dryer-added fabric softening non-woven fabric.

## Example LXXXV

*Flavor composition*

The following basic walnut flavor formulation is prepared:

| Ingredients | Parts by Weight |
|---|---|
| Ethyl-2-methyl butyrate | 10 |
| Vanillin | 40 |
| Butyl valerate | 40 |
| 2,3-diethyl pyrazine | 5 |
| Methyl cyclopentenolone | 80 |
| Benzaldehyde | 60 |
| Valerian oil Indian (1% in 95% aqueous ethanol alcohol) | 0.5 |
| Propylene glycol | 764.5 |

The branched chain unsaturated tertiary alcohol prepared according to Example XIII is added to the above formulation at the rate of 1.5%. The formulation is compared to the formulation which does not have such branched chain unsaturated tertiary alcohol added thereto at the rate of 20 ppm in water. The formulation containing the branched chain unsaturated tertiary alcohol prepared according to Example XIII has a "woody balsamic" fresh walnut kernel and walnut skin-like taste and, in addition, has a fuller mouth feel and longer lasting taste. The flavor that has added to it the branched chain unsaturated tertiary alcohol is preferred by a group of flavor panelists and they consider it to be a substantially improved walnut flavor.

## Example LXXXVI

*Beverage*

The addition of the branched chain unsaturated tertiary alcohol prepared according to the process of Example XIV at the rate of 0.3 ppm is added to a commercial cola beverage and gives the beverage a fuller "woody balsamic" long-lasting taste and adds to the pleasant top-notes of the beverage. When comparing the cola beverage containing the branched chain unsaturated tertiary alcohol prepared according to Example XIV, to one having the same formula but not containing such tertiary alcohol, a five member bench panel prefers the beverage containing the branched chain unsaturated alcohol.

Example LXXXVII

*Tobacco Flavor formulation*

Cigarettes are produced using the following tobacco formulation:

| Ingredients | Parts by Weight |
|---|---|
| Bright | 40.1 |
| Burley | 24.9 |
| Maryland | 1.1 |
| Turkish | 11.6 |
| Stem (flue-cured) | 14.2 |
| Glycerine | 2.8 |
| $H_2O$ | 5.3 |

At the rate of 0.2%, the following tobacco flavor formulation is applied to all the cigarettes produced with the above tobacco formulation

| Ingredients | Parts by Weight |
|---|---|
| Ethyl butyrate | 0.05 |
| Ethyl valerate | 0.05 |
| Maltol | 2.00 |
| Cocoa extract | 26.00 |
| Coffee extract | 10.00 |
| Ethyl alcohol (95%) | 20.00 |
| $H_2O$ | 41.90 |

To 50% of the cigarettes, 10 and 20 ppm of the branched chain unsaturated tertiary alcohol prepared according to Example XV are added. These cigarettes are hereinafter called "experimental" cigarettes and the cigarettes without the tertiary alcohol are hereinafter called "control" cigarettes. The control and experimental cigarettes are then evaluated by paired comparison and the results are as follows:

a. In aroma, the experimental cigarettes are found to be more aromatic and more black tobacco-like.

b. In smoke flavor, the experimental cigarettes are found to be more aromatic, more sweet, more bitter, richer, "cigar box-like" and slightly less harsh in the mouth and more cigarette tobacco-like than the control cigarettes . . . and they all have "black tobacco" nuances.

The experimental cigarettes containing 20 ppm of the said tertiary alcohol produced according to Example XV are found to be woody, slightly chemical and mouth coating in the smoke flavor.

All cigarettes, both experimental and control, are evaluated for smoke flavor with 20 mm cellulose acetate filters. The tertiary alcohol prepared according to Example XV is very similar to the tertiary alcohol prepared according to Example XVI and Example XIV and each of these materials enhance the tobacco-like taste of the blended cigarette on smoking in both the main stream and the side stream.

Example LXXXVIII

*Citrus cologne*

The following citrus cologne formulation is prepared:

| Ingredients | Parts by Weight |
|---|---|
| Mixture of diisoundecanal compounds having the structures: 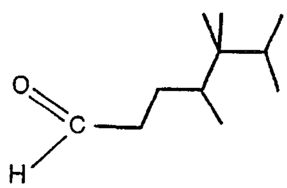 ; 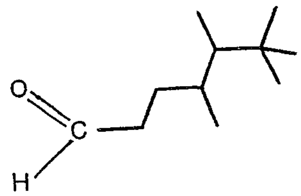 ; 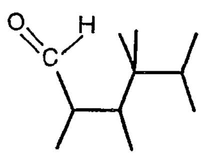 and 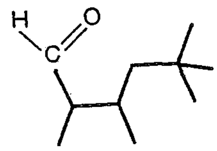 ; (fractions 3, 4 and 5 of the distillation product of the reaction product of Example XVII) | 600 |
| Bergamot oil | 100 |
| Phenyl ethyl alcohol | 100 |
| Hydroxy citronellal | 100 |
| Benzyl salicylate | 100 |

The diisoundecanals produced according to Example XVII impart to this citrus cologne formulation an intense, long-lasting citrus, green, melony aroma with excellent dry, woody nuances.

Example LXXXIX

The diisoundecanols produced according to Example XVII (fractions 6, 7 and 8) have peanut oil-like aromas becoming strongly vetiver on dry-out. These peanut oil and vetiver aroma nuances may be utilized to a great extent in inexpensive functional products. The following pine fragrance demonstrates the use of this material in perfume compositions. In this case, it is used at a rate of 47.9%.

| Ingredients | Parts by Weight |
|---|---|
| Diisoundecanols produced according to Example XVII having the structures: 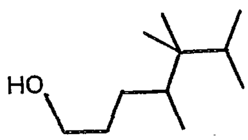 ; 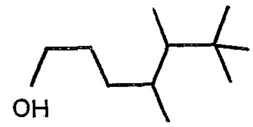 ; 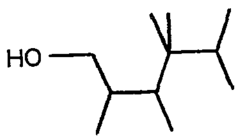 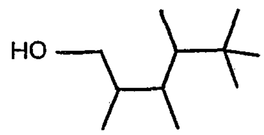 (fractions 6, 7 and 8 produced according to Example XVII) | 479 |
| Isobornyl acetate | 100 |
| Camphor | 10 |
| Terpineol | 25 |
| Fir balsam absolute (50% in diethyl phthalate) | 20 |
| Coumarin | 4 |
| Linalool | 30 |
| Anethol | 2 |
| Fenchyl alcohol | 10 |
| Lemon terpenes washed | 50 |
| Borneol | 5 |
| Galbanum oil | 5 |
| Turpentine Russian | 150 |
| Pinus pumilionus | 50 |
| Eucalyptol | 50 |
| 2,2,6-trimethyl-1-cyclohexene-1-carboxaldehyde | 5 |
| Maltol 1% in diethyl phthalate | 5 |

The presence of the diisoundecanols prepared according to Example XVII supports the pine notes and imparts a strong, vetiver character, particularly on dry-out, thus producing a considerable savings in the cost of the formulation. In addition, this piney formualation is enhanced with the vetiver and peanut oil aroma nuances.

Example XC

*Preparation of cosmetic powder compositions*

Cosmetic powder compositions are prepared by mixing in a ball mill, 100 grams of talcum powder (per composition) with 0.25 grams of the substance set forth in Table III below (per composition). Each of the cosmetic powder compositions has an excellent aroma as described in Table III below:

TABLE III

| Substance | Aroma Description |
|---|---|
| Mixture prepared according to Example XVII prior to second distillation but subsequent to splash column distillation | A citrus, green, melony, dry woody, vetiver aroma becoming more vetiver on dry-out. |
| Mixture of diisoundecanals prepared according to Example XVII, distillation fractions 3, 4 and 5 having the structures: 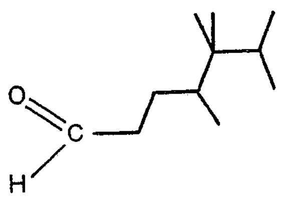 ; 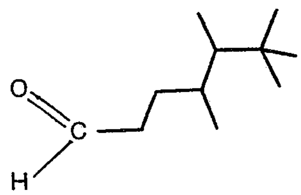 ; 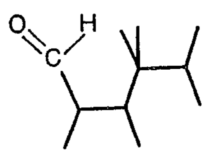 and 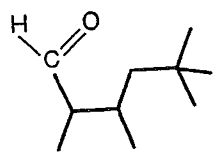 ; | A citrus, green, melony aroma with dry woody nuances. |
| Mixture of diisoundecanols produced according to Example XVII, fractions 6, 7 and 8 having the structures: 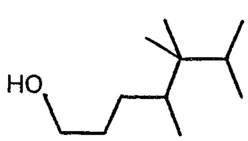 ; 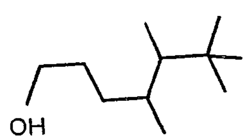 ; 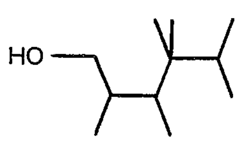 and 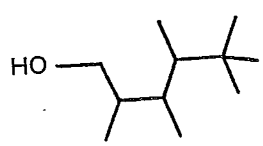 . | A peanut oil-like aroma becoming strongly vetiver-like on dry-out. |

TABLE III (continued)

| Substance | Aroma Description |
|---|---|
| Perfume composition of Example LXXXVIII | A strong, citrus aroma with green, melony and dry woody nuances. |
| Perfume composition prepared according to Example LXXXIX | A piney aroma having vetiver nuances and becoming piney/vetiver-like on dry-out. |

### Example XCI

*Perfumed liquid detergents*

Concentrated liquid detergents (Lysine salt of n-dodecylbenzene sulfonic acid as more specifically described in U.S. Patent No. 3,948,818, issued on April 6, 1976) with aroma nuances as set forth in Table III of Example XC are prepared containing 0.10%, 0.15%, 0.20%, 0.25%, 0.30% and 0.35% of the substance set forth in Table III of Example XC. They are prepared by adding and homogeneously mixing the appropriate quantity of substance set forth in Table III of Example XC in the liquid detergent. The detergents all possess excellent aromas as set forth in Table III of Example XC, the intensity increasing with greater concentrations of substance as set forth in Table III of Example XC.

### Example XCII

*Preparation of colognes and handkerchief perfumes*

Compositions as set forth in Table III of Example XC are incorporated into colognes at concentrations of 2.0%, 2.5%, 3.0%, 3.5%, 4.0%, 4.5% and 5.0% in 85%, 90% and 95% aqueous food grade ethanol solutions; and into handkerchief perfumes at concentrations of 15%, 20%, 25% and 30% (in 85%, 90% and 95% aqueous food grade ethanol solutions). Distinctive and definitive fragrances as set forth in Table III of Example XC are imparted to the colognes and to the handkerchief perfumes at all levels indicated above.

### Example XCII

*Preparation of soap compositions*

One hundred grams of soap chips per sample (IVORY®, produced by the Procter & Gamble Company of Cincinnati, Ohio) are each mixed with one gram samples of substances as set forth in Table III of Example XC until homogeneous compositions are obtained. In each of the cases, the homogeneous compositions are heated under three atmospheres ($3 \times 10^5 N/m^2$) pressure at 180°C for a period of three hours and the resulting liquids are placed into soap molds. The resulting soap cakes, on cooling, manifest aromas as set forth in Table III of Example XC.

### Example XCIV

*Preparation of solid detergent compositions*

Detergents are prepared using the following ingredients according to Example I of Canadian Patent No. 1,007,948:

| Ingredient | Percent by Weight |
|---|---|
| Neodol® 45—11 (a $C_{14}$—$C_{15}$ alcohol ethoxylated with 11 moles of ethylene oxide) | 12 |
| Sodium carbonate | 55 |
| Sodium citrate | 20 |
| Sodium sulfate, water brighteners | q.s. |

This detergent is a phosphate-free detergent. Samples of 100 grams each of this detergent are admixed with 0.10, 0.15, 0.20 and 0.25 grams of each of the substances as set forth in Table III of Example XC. Each of the detergent samples has excellent aromas as indicated in Table III of Example XC.

Example XCV

Utilizing the procedure of Example I at column 15 of U.S. Patent No. 3,632,396, non-woven cloth substrates useful as dryer-added fabric softening articles of manufacture are prepared wherein the substrate, the substrate coating and the outer coating and the perfuming material are as follows:

1. A water "dissolvable" paper ("Dissolvo Paper")
2. Adogen 448® (m.p. about 140°F (60°C)) as the substrate coating; and
3. An outer coating having the following formulation (m.p. about 150°F (66°C)):

57% $C_{20-22}$ HAPS
22% isopropyl alcohol
20% antistatic agent
1% of one of the substances as set forth in Table III of Example XC.

Fabric softening compositions prepared according to Example I at column 15 of U.S. Patent No. 3,632,396 having aroma characteristics as set forth in Table III of Example XC, consist of a substrate coating having a weight of about 3 grams per 100 square inches (0.06 $m^2$) of substrate; a first coating consisting of about 1.85 grams per 100 square inches of substrate (0.06 $m^2$); and an outer coating consisting of about 1.4 grams per 100 square inches (0.06 $m^2$) of substrate. The substrate of Table III of Example XC is admixed with the outer coating mixture thereby providing a total aromatized outer coating weight ratio to substrate of about 1:1 by weight of the substrate. The aroma characteristics are imparted in a pleasant manner to the head space in the dryer on operation thereof using the said dryer-added fabric softening non-woven fabrics.

Example XCVI

*Hair spray formulation*

The following hair spray formulation is prepared by first dissolving PVP/VA E-735 copolymer manufactured by the GAF Corporation of 140 West 51st Street, New York, N.Y., in 91.62 grams of 95% food grade ethanol. 8.0 grams of the polymer is dissolved in the alcohol. The following ingredients are added to the PVP/VA alcoholic solution:

| Ingredient | Percent by Weight |
|---|---|
| Dioctyl sebacate | 0.05 |
| Benzyl alcohol | 0.10 |
| Dow Corning 473® fluid (prepared by the Dow Corning Corporation) | 0.10 |
| Tween 20® surfactant (prepared by ICI Americas Corporation) | 0.03 |
| Perfuming substance as set forth in Table III of Example XC | 0.10 |

The perfuming substance as set forth in Table III of Example XC adds an aroma characteristic as set forth in Table III of Example XC which is rather intense and aesthetically pleasing to the user of the soft-feel, good hold pump hair spray.

Example XCVII

*Conditioning shampoos*

Monamid CMA (prepared by the Mona Industries Company) (3.0 weight percent) is melted with 2.0 weight percent coconut fatty acid (prepared by Procter & Gamble Company of Cincinnati, Ohio); 1.0 weight percent ethylene glycol distearate (prepared by the Armak Corporation) and triethanolamine (a product of Union Carbide Corporation) (1.4 weight percent). The resulting melt is admixed with Stepanol WAT produced by the Stepan Chemical Company (35.0 weight percent). The resulting mixture is heated to 60°C and mixed until a clear solution is obtained (at 60°C).

GAFQUAT® 755 N polymer (manufactured by GAF Corporation of 140 West 51st Street, New York N.Y.) (5.0 weight percent) is admixed with 0.1 weight percent sodium sulfite and 1.4 weight percent polyethylene glycol 6000 distearate produced by Armak Corporation.

The resulting material is then mixed and cooled to 45°C and 0.3 weight percent of perfuming substance as set forth in Table III of Example XC is added to the mixture. The resulting mixture is cooled to 40°C and blending is carried out for an additional one hour. At the end of this blending period, the resulting material has a pleasant fragrance as indicated in Table III of Example XC.

Example XCVIII

*Perfume formulation*

The following vetiver perfume formuation is prepared:

| Ingredients | Parts by Weight |
|---|---|
| Vetivone | 25 |
| Mixture of compounds defined according to the structure: 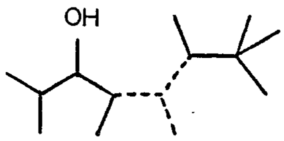 prepared according to Example XX, fraction 4. | 12 |
| Mixture of compounds defined according to the structure: 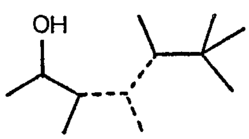 prepared according to Example XIX, bulked fractions 2—4 | 12 |
| Vetiverol | 5 |
| Musk ketone | 8 |
| Styrax essence | 12.4 |

The addition of the mixture of secondary alcohols prepared according to Examples XIX and XX impart to this vetiver formulation intense woody, long-lasting aromas with fruity and powerful amber nuances.

Example XCIX

*Perfumed liquid detergent*

Concentrated liquid detergents with aromas as described in Table IV below (which detergents are produced from the lysine salt of n-dodecyl benzene sulfonic acid as more specifically described in U.S. Patent No. 3,948,818 issued on April 6, 1976) are prepared containing one of the substances set forth in Table IV below. They are prepared by adding and homogeneously mixing the appropriate quantity of substance as indicated in Table IV below. The detergents all possess aroma profiles as set forth in Table IV below, the intensity increasing with greater concentrations of the composition of matter as set forth in Table IV below:

TABLE IV

| Aroma Ingredient | Aroma Profile |
|---|---|
| Secondary alcohol mixture defined according to the structure: 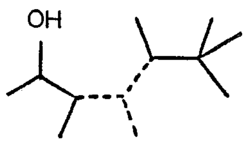 wherein one of the dashed lines represents a carbon-carbon double bond and the other dashed lines represent carbon carbon single bonds, prepared according to Example XIX, fractions 2—4. | A fruity, amber, woody aroma with vetiver notes and having an intense vetiver aroma on dry-out. |
| **Aroma Ingredient** | **Aroma Profile** |
| Mixture of compounds defined according to the structure: 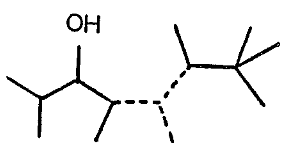 produced according to Example XX, fraction 4, wherein in each of the molecules of the mixture, one of the dashed lines represents a carbon-carbon double bond and the other dashed lines represent carbon-carbon single bonds. | An intense woody, long-lasting aroma with strong, long-lasting vetiver nuances. |
| Perfume composition of Example XCVIII. | An intense vetiver aroma which is long-lasting containing woody, fruity and unusually powerful amber nuances. |

## Example C

*Preparation of a cologne and handkerchief perfume*

Aroma containing and augmenting ingredients as defined according to Table IV in Example XCIX are incorporated into colognes at concentrations of 1.5%, 2.0%, 2.5%, 3.0%, 4.0% and 5.0% in 75%, 80%, 85%, 90% and 95% solutions of aqueous ethanol; and into handkerchief perfumes at concentrations of 15%, 20%, 25% and 30% (in 80%, 85% and 95% aqueous ethanol solutions). The use of the compositions of matter as set forth in Table IV of Example XCIX affords distinct and definitive aroma profiles as set forth in Table IV and Example XCIX to the handkerchief perfumes and to the colognes.

Example I

*Preparation of a soap composition*

One hundred grams of soap chips (IVORY® manufactured by the Procter & Gample Company of Cincinnati, Ohio) are melted and intimately admixed with one of the aroma materials as set forth in Table IV of Example XCIX supra, the amount of composition of matter of Table IV of Example XCIX being one gram of each composition of matter. The conditions of mixing are: 180°C, 3 hours, 12 atmospheres pressure ($12 \times 10^5$ N/m$^2$). At the end of the mixing cycle, while the soap is still under 12 atmospheres ($12 \times 10^5$ N/m$^2$) pressure, the mixture of soap and perfume ingredients is cooled to room temperature. At this temperature, the resulting mixture is in a solid state. The resulting soap block is then cut up into soap cakes. Each of the soap cakes manifests an excellent aroma as set forth in Table IV of Example XCIX. None of the soap samples show any discoloration even after two weeks in the oven at 90°F (32°C).

Example CII

*Preparation of a detergent composition*

A total of 100 grams of a detergent powder (nonionic detergent powder containing a proteolytic enzyme prepared according to Example I of Canadian Patent No. 985.190 issued on March 9, 1976) is mixed with 0.15 grams of one of the compositions of matter as set forth in Table IV of Example XCIX until a substantially homogeneous composition is obtained. Each of the compositions has excellent aroma profiles as set forth in Table IV of Example XCIX.

Example CIII

*Perfumed liquid detergents*

Concentrated liquid detergents with rich, pleasant aromas as set forth in Table IV of Example XCIX are prepared containing 0.10%, 0.15%, and 0.20% of each of the compositions of matter set forth in Table IV of Example XCIX. They are prepared by adding and homogeneously admixing the appropriate quantity of composition of matter of Table IV of Example XCIX in the liquid detergent. The liquid detergents are all produced using anionic detergents containing a 50:50 mixture of sodium lauroyl sarcosinate and potassium N-methyl lauroyl tauride. The detergents all possess pleasant aromas as defined in Table IV of Example XCIX, the intensity increasing with greater concentrations of composition of matter of Table IV of Example XCIX.

Example CIV

*Tobacco formulation*

A tobacco mixture is prepared by admixing the following ingredients:

| Ingredients | Parts by Weight |
|---|---|
| Bright | 40.1 |
| Burley | 24.9 |
| Maryland | 1.1 |
| Turkish | 11.6 |
| Stem (flue-cured) | 14.2 |
| Glycerine | 2.8 |
| Water | 5.3 |

Cigarettes are prepared from this tobacco.

The following flavor formulation is prepared:

| Ingredients | Parts by Weight |
|---|---|
| Ethyl butyrate | 0.05 |
| Ethyl valerate | 0.05 |
| Maltol | 2.00 |
| Cocoa extract | 26.00 |
| Coffee extract | 10.00 |
| Ethyl alcohol | 20.00 |
| Water | 41.90 |

The above stated tobacco flavor formulation is applied at the rate of 1.0% to all of the cigarettes produced using the above tobacco formulation. One-third of the cigarettes are then treated with 500 or 1000 ppm of the secondary alcohol mixture defined according to the structure:

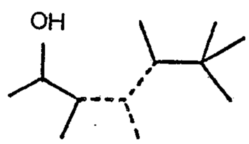

wherein in each of the molecules of the mixture, one of the dashed lines represents a carbon-carbon double bond and the other dashed lines represent carbon-carbon single bonds. Another third of the cigarettes are treated with 500 or 1000 ppm of the mixture produced according to Example XX defined according to the structure:

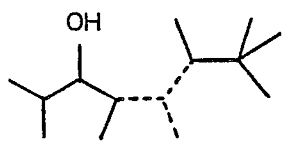

wherein in each of the molecules of the mixture, one of the dashed lines represents a carbon-carbon double bond and the other dashed lines represent carbon-carbon single bonds. The last third of the cigarettes are "control cigarettes" and do not contain any of the unsaturated alcohols of either Example XIX or Example XX but only contain untreated flavor formulation as set forth above. The control cigarettes and the treated experimental cigarettes are then evaluated by paired comparison and the results are as follows:

The experimental cigarettes are found to have more body and to be, on smoking, more Turkish tobacco-like, more aromatic and to have sweet, spicy, woody/oriental, minty and fruity aroma nuances in both the main stream and the side stream with additional clove-like nuances with respect to the composition of matter produced according to Example XIX. These aroma nuances are missing from the control cigarettes. The experimental cigarettes containing the composition of matter prepared according to Example XIX in the filter have a woody, tobacco character with heavy air-cured and cigar-like aroma nuances. The cigarettes containing the composition of matter of Example XX in the filter have sweet, minty aroma nuances both prior to and on smoking. The description can also be set forth as "menthol-like".

*Brief description of the drawings*

Figure 1A represents GLC profile for the reaction product of Example I using a 70% sulfuric acid catalyst at 35°C.

Figure 1B represents the GLC profile for the reaction product of Example I using an Amberlyst® 15 acidic ion exchange resin catalyst at a temperature of 150°C.

Figure 1C represents the GLC profile for the reaction product of Example I, using an Amberlyst® 15 catalyst at 100°C.

Figure 1D represents the GLC profile for the reaction product of Example I, using a sulfuric acid catalyst and an alpha-methylstyrene diluent at 35°C according to the conditions of United Kingdom Patent Specification 796,130 (crude reaction product).

Figure 1E represents the GLC profile for the reaction product of Example I, using a sulfuric acid catalyst,

at 35°C and an alpha-methylstyrene diluent according to the conditions of United Kingdom Patent Specification 796,130 (distilled reaction product).

Figure 2A represents the NMR spectrum for Peak 1 of the GLC profile of Figure 1E.

Figure 2B represents the infra-red spectrum for Peak 1 of the GLC profile of Figure 1E.

Figure 3A represents the NMR spectrum for Peak 2 of the GLC profile of Figure 1E.

Figure 3B represents the infra-red spectrum for Peak 2 of the GLC profile of Figure 1E.

Figure 4 represents the NMR spectrum for Peak 2 of the GLC profile of Figure 1B.

Figure 5 is the GLC profile of the reaction product of Example II containing the compounds having the structures:

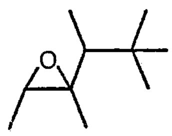 , 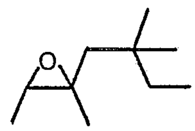 and 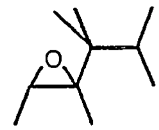

Figure 6 is the NMR spectrum for the reaction product of Example II containing the compounds having the structures:

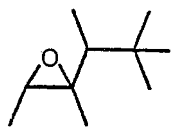 , 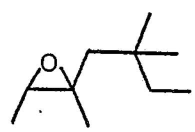 and 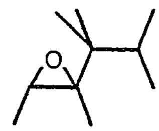

Figure 7 is the infra-red spectrum for the reaction product of Example II containing the compounds having the structures:

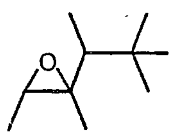 , 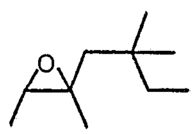 and 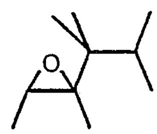

Figure 8 sets forth the GLC profile for the reaction product of Example III, containing compounds defined according to the structure:

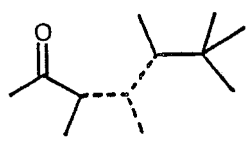

wherein in each molecule of the mixture, one of the dashed lines is a carbon-carbon double bond and the other dashed lines are carbon-carbon single bonds.

Figure 9A represents the infra-red spectrum of Peak 3 of the GLC profile of Figure 8.

Figure 9B represents the infra-red spectrum of Peak 4 of the GLC profile of Figure 8.

Figure 9C represents the infra-red spectrum of Peak 5 of the GLC profile of Figure 8.

Figure 9D represents the infra-red spectrum of Peak 6 of the GLC profile of Figure 8.

Figure 9E represents the infra-red spectrum of Peak 7 of the GLC profile of Figure 8.

Figure 9F represents the infra-red spectrum of Peak 8 of the GLC profile of Figure 8.

Figure 9G represents the infra-red spectrum of Peak 9 of the GLC profile of Figure 8.

Figure 9H represents the infra-red spectrum of Peak 10 of the GLC profile of Figure 8.

Figure 9J represents the NMR spectrum for a mixture of compounds having the structures:

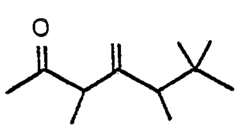 and 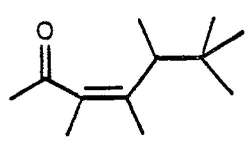

produced according to Example III.

Figure 9K represents the NMR spectrum for the compound having the structure:

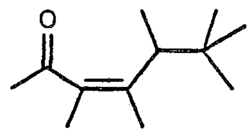

produced according to Example III.

Figure 9L represents the NMR spectrum for the compound containing the structure:

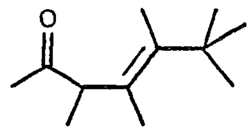

produced to Example III.

Figure 10 represents the GLC profile for the reaction product of Example IV containing a mixture of compounds, each of which is defined according to the generic structure:

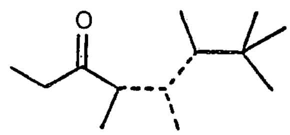

wherein in each molecule one of the dashed lines is a carbon-carbon double bond and the other dashed lines are carbon-carbon single bonds.

Figure 11 represents the infra-red spectrum for the product produced according to Example IV containing the compounds having the structures:

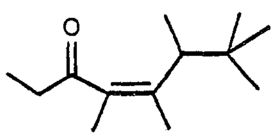 ; 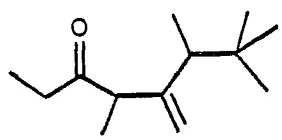 and 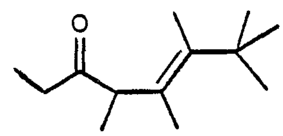

Figure 12 represents the mass spectrum for the reaction product of Example IV containing the compounds having the structures:

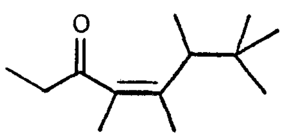 ; 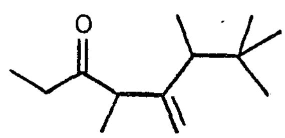 and 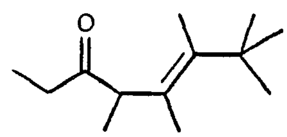

Figure 13 represents the GLC profile for the reaction product of Example V containing the compounds defined according to the generic structure:

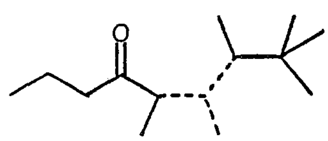

wherein in each of the molecules of the mixture, one of the dashed lines represents a carbon-carbon double bond and the other dashed lines represent carbon-carbon single bonds.

Figure 14 represents the infra-red spectrum for the reaction product of Example V containing the compounds having the structures:

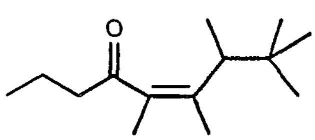 , 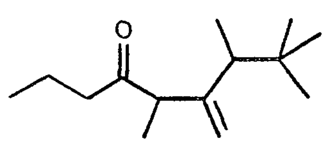 and 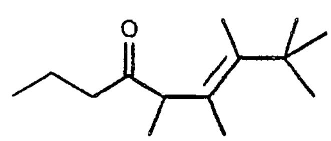

Figure 15 represents the mass spectrum for the reaction product of Example V containing the compounds having the structures:

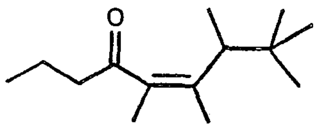 , 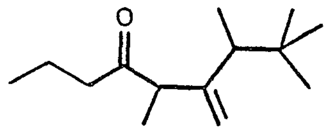 and 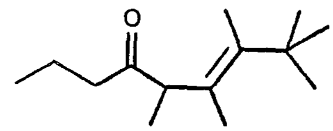

Figure 16 represents the GLC profile for the reaction product of Example VI, containing a mixture of compounds, each of which is defined according to the generic structure:

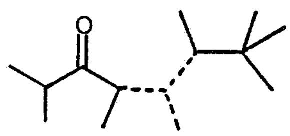

wherein in each of the molecules, one of the dashed lines represents a carbon-carbon double bond and the other dashed lines represent carbon-carbon single bonds.

Figure 17 represents the infra-red spectrum for the reaction product of Example VI containing the compounds having the structures:

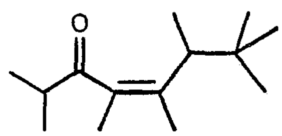 , 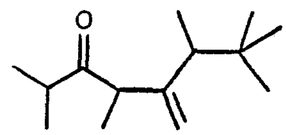 and 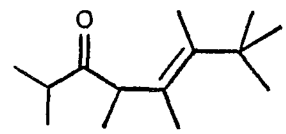

Figure 18 represents the mass spectrum for the reaction product of Example VI containing the compounds having the structures:

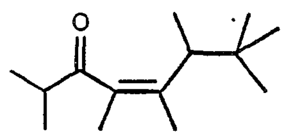 , 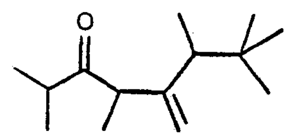 and 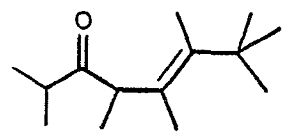

Figure 19 represents the GLC profile for the reaction product of Example VII(A) containing compounds defined according to the genus having the structure:

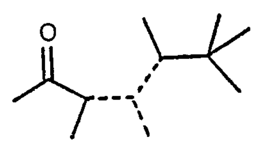

wherein in each of the molecules of the mixture, one of the dashed lines represents a carbon-carbon double bond and the other dashed lines represent carbon-carbon single bonds.

Figure 20 represents the GLC profile for the reaction product of Example VII(B) containing a mixture of compounds defined according to the structure:

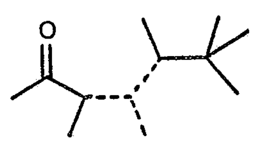

wherein in each of the molecules of the mixture, one of the dashed lines represents a carbon-carbon double bond and the other dashed lines represent carbon-carbon single bonds.

Figure 21 represents the GLC profile for the reaction product of Example IX(A) containing a mixture of compounds defined according to the genus having the structure:

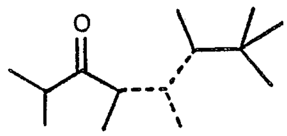

wherein in each of the molecules of the mixture, one of the dashed lines represents a carbon-carbon double bond and the other dashed lines represent carbon-carbon single bonds.

Figure 22 is the GLC profile for distillation fraction 2 of the reaction product of Example X containing the compounds having the structures:

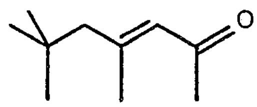 and 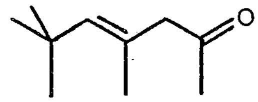

Figure 23 is the GLC profile for distillation fraction 9 of the reaction product of Example X containing the compounds having the structures:

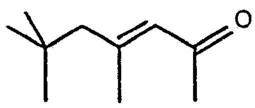 and 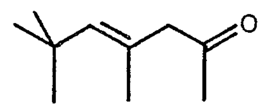

Figure 24 is the NMR spectrum for peak 1 of the GLC profile of Figure 23 containing the compounds having the structures:

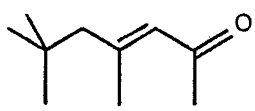 and 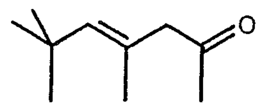

Figure 25 is the NMR spectrum for peak 2 of the GLC profile of Figure 23 containing the compounds having the structures:

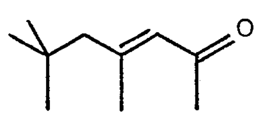 and 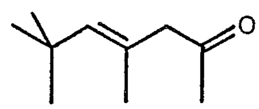

Figure 26 is the infra-red spectrum for peak 1 of the GLC profile of Figure 23 containing the compounds having the structures:

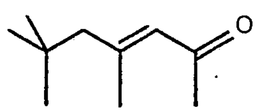 and 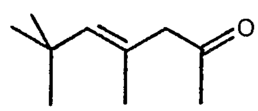

Figure 27 is the infra-red spectrum for peak 2 of the GLC profile of Figure 23 containing the compounds having the structures:

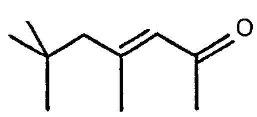 and 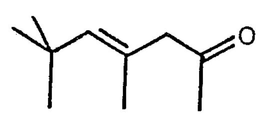

Figure 28 sets forth the GLC profile for the reaction product produced according to Example XI(A) containing the compounds defined according to the generic structure:

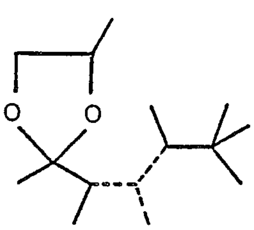

wherein one of the dashed lines in each of the molecules represents a carbon-carbon double bond and the other dashed lines in each of the molecules represent carbon-carbon single bonds.

Figure 29 is the NMR spectrum for the mixture of compounds produced according to Example XI(A) containing the compounds defined according to the generic structure:

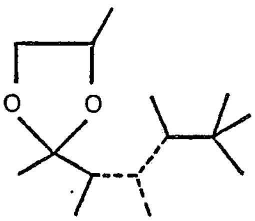

Figure 30 is the infra-red spectrum for the product produced according to Example XI(A) containing the compounds defined according to the generic structure:

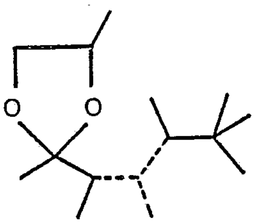

Figure 30A is the GLC profile for the reaction product produced according to Example XI(B) containing the compounds defined according to the generic structure:

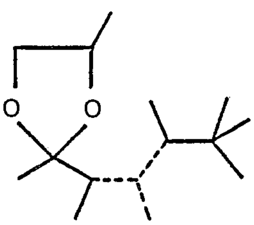

Figure 30B is the GLC profile for the distillation product (fraction 9) of the reaction product of Example XI(B) containing the compounds defined according to the generic structure:

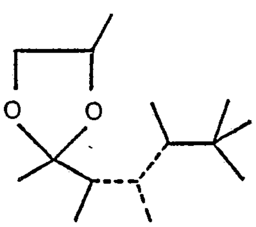

Figure 31 is the GLC profile for the reaction product of Example XII.

Figure 32 represents the GLC profile for the reaction product of Example XIII cotnaining a mixture of compounds defined according to the structure:

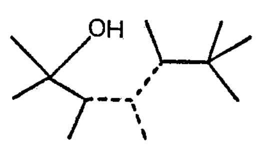

wherein in each of the molecules one of the dashed lines represents a carbon-carbon double bond and the other dashed lines represent carbon-carbon single bonds.

Figure 33 is the NMR spectrum for the reaction product of Example XIII containing a mixture of compounds having the structures defined according to the following structure:

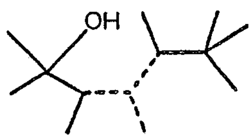

wherein in each of the molecules of the mixture one of the dashed lines represents a carbon-carbon double bond and the other dashed lines represent carbon-carbon single bonds.

Figure 34 is the infra-red spectrum for the reaction product of Example XIII containing the compounds defined according to the structure:

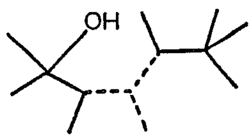

wherein in each of the molecules in the resulting mixture one of the dashed lines represents a carbon-carbon double bond and the other dashed lines represent carbon-carbon single bonds.

Figure 35 is the GLC profile of the crude reaction product of Example XIV containing the compounds defined according to the structure:

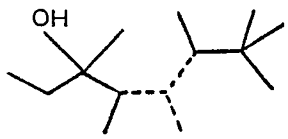

wherein in each of the molecules, one of the dashed lines represents a carbon-carbon single bond and the other dashed lines represent carbon-carbon double bonds.

Figure 36 is the GLC profile for fraction 4 of the distillation product of the reaction product of Example XIV containing the compounds having the structure:

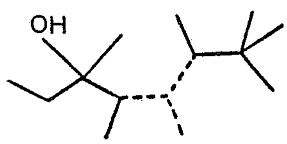

wherein in each of the molecules one of the dashed lines represents a carbon-carbon double bond and the other dashed lines represent carbon-carbon single bonds.

Figure 37 is the infra red spectrum for fraction 4 of the distillation product of the reaction product of Example XIV containing a mixture of compounds defined according to the structure:

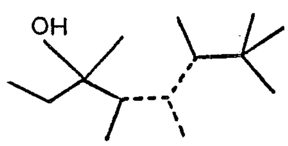

wherein in each of the molecules, one of the dashed lines represents a carbon-carbon double bond and the other dashed lines represent carbon-carbon single bonds.

Figure 38 is the GLC profile of the crude reaction product of Example XV containing a mixture of compounds defined according to the structure:

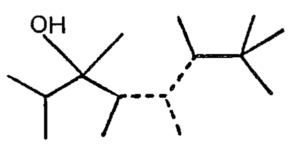

wherein in each of the molecules, one of the dashed lines represents a carbon-carbon double bond and the other dashed lines represent carbon-carbon single bonds.

Figure 39 is the GLC profile for fraction 7 of the distillation product of the reaction product of Example XV containing a mixture of compounds defined according to the structure:

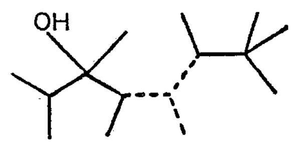

wherein in each of the molecules, one of the dashed lines represents a carbon-carbon double bond and the other dashed lines represent carbon-carbon single bonds.

Figure 40 is the infra-red spectrum for fraction 7 of the distillation product of the reaction product of Example XV containing a mixture of compounds defined according to the structure:

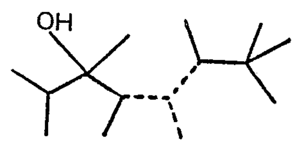

wherein in each of the molecules, one of the dashed lines is a carbon-carbon double bond and the other dashed lines represent carbon-carbon single bonds.

Figure 41 is the NMR spectrum for fraction 7 of the distillation product of the reaction product of Example XV containing a mixture of compounds defined according to the structure:

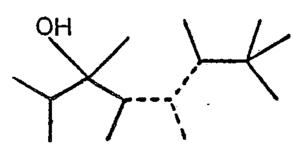

wherein in each of the molecules, one of the dashed lines represents a carbon-carbon double bond and the other dashed lines represent carbon-carbon single bonds.

Figure 42 is the GLC profile for the crude reaction product produced according to the procedure of Example XVI containing a mixture of compounds defined according to the structure:

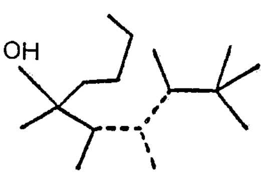

wherein in each of the molecules one of the dashed lines represents a carbon-carbon double bond and the other dashed lines represent carbon-carbon single bonds.

Figure 43 is the GLC profile for fraction 8 of the distillation product of the reaction product of Example XVI containing a mixture of compounds defined according to the structure:

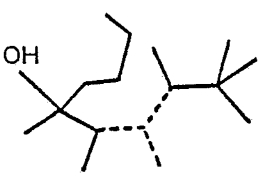

wherein in each of the molecules one of the dashed lines represents a carbon-carbon double bond and the other dashed lines represent carbon-carbon single bonds.

Figure 44 is the infra-red spectrum for fraction 8 of the distillation product of the reaction product of Example XVI containing a mixture of compounds defined according to the structure:

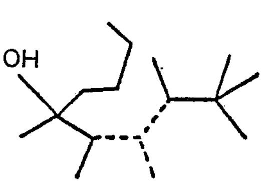

wherein one of the dashed lines is a carbon-carbon double bond and the other dashed lines represent carbon-carbon single bonds.

Figure 45 is the NMR spectrum for fraction 8 of the distillation product of the reaction product of Example XVI containing the compounds defined according to the structure:

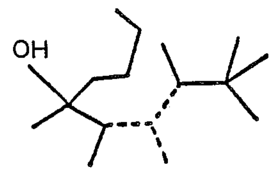

wherein in each of the molecules one of the dashed lines represents a carbon-carbon double bond and the other dashed lines represent carbon-carbon single bonds.

Figure 46 is the GLC profile for the crude reaction product produced according to Example XVII (conditions: SF—96 column, 6′ × 1/4″ (1.83 m × 6 mm), programmed at 180°C, isothermal) containing the compounds having the structures:

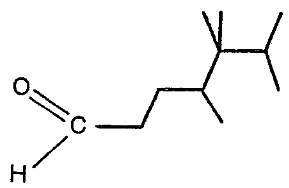
;
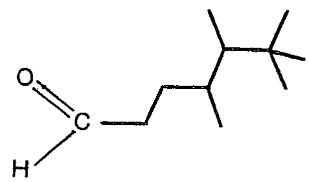
;

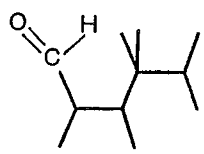
;
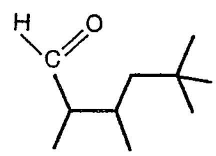
;

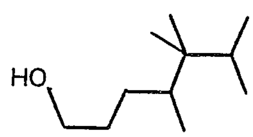
;
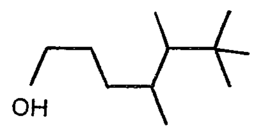
;

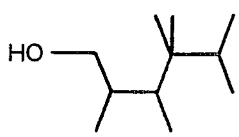
and
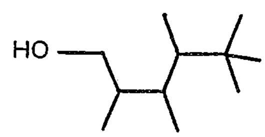

Figure 47 is the GLC profile for the diisoundecanal compounds produced according to Example XVII having the structures:

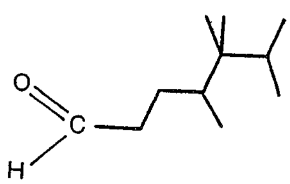
;
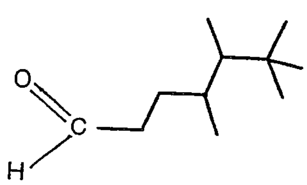
;

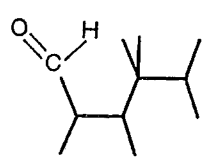
and
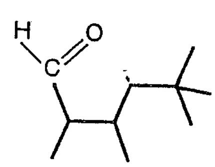
;

Figure 48 is the NMR spectrum for the diisoundecanal compounds produced according to Example XVII having the structures:

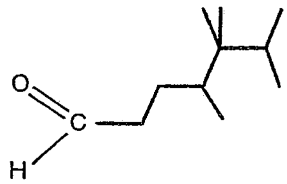 ; 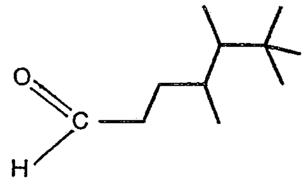 ;

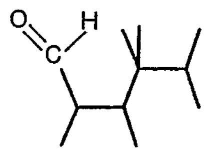 and 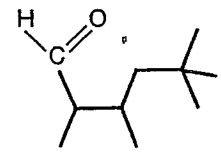 ;

Figure 49 is the infra-red spectrum for the diisoundecanal compounds produced according to Example XVII having the structures:

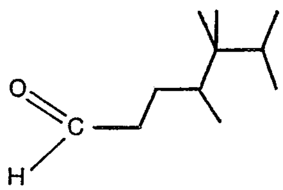 ; 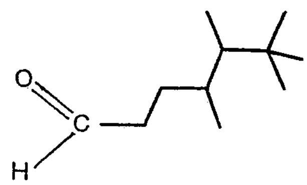 ;

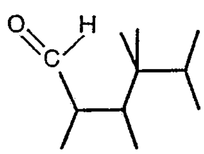 ; 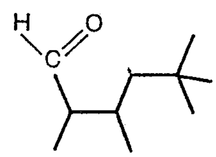 ;

Figure 50 is the GLC profile (conditions: SF—96, 6′ × 1/4″ (1.83 m × 6 mm) 180°C isothermal column) for the reaction product produced according to Example XVII containing the compounds having the structures:

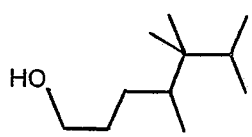 ; 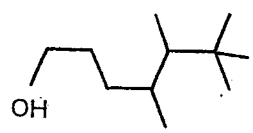

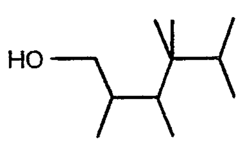 and 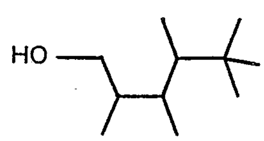

Figure 51 is the NMR spectrum for the diisoundecanol compounds produced according to Example XVII having the structures:

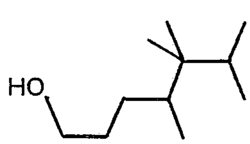 ; 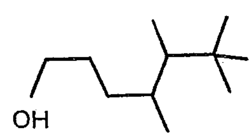 ;

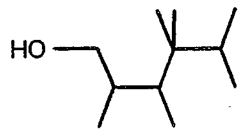 and 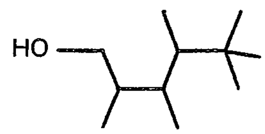

Figure 52 is the infra-red spectrum for the diisoundecanol compounds produced according to Example XVII having the structures:

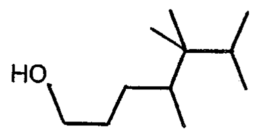 ; 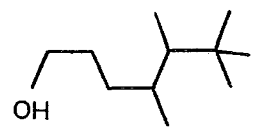 ;

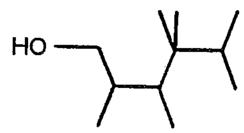 and 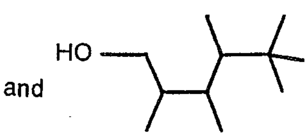

Figure 53A represents a graph of *percent residual chlorine* versus *time in hours* for hypochlorite solutions containing either (i) DOWFAX® 2Al (a registered trademark of the Dow Chemical Company of Midland, Michigan) identifying a mixture of compounds defined according to the structure:

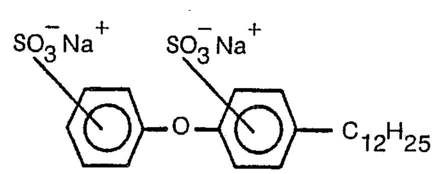

wherein the $SO_3^-Na^+$ moieties are at various positions on the phenyl moieties and the $C_{12}H_{25}$ moiety represents various branched chain alkyl moieties having 12 carbon atoms each, or (ii) AROMOX® DMMC—W, a 30% aqueous solution of dimethylcocoamine oxide having the structure:

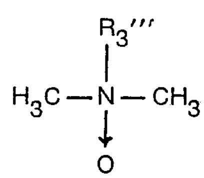

a trademark of the Akzo Corporation of Chicago, Illinois (product produced by Armac, Division of Akzo Corporation of Chicago Illinois) with the weight ratio of AROMOX® DMMC—W:base being 0.8:99 and the ratio of DOWFAX® 2Al:base being 0.8:99, as described in Example XVIII, supra.

Figure 53B is a graph of *percent residual chlorine* versus *time in hours* for hypochlorite solutions of either (i) DOWFAX® 2Al or (ii) AROMOX® DMMC—W, in the absence of fragrance or essential oils with the weight ratio of AROMOX® DMMC—W:base being 1.8:99 and 3.8:96 and the ratios of DOWFAX® 2Al:base being 1.8:99 and 3.8:99, as described in Example XVIII, supra.

Figure 54A is a graph of *percent residual chlorine* versus *time in hours* comparing the performance of hypochlorite solutions of (i) DOWFAX® 2Al versus (ii) AROMOX® DMMC—W using one of the diisoamylene products produced according to Example I, supra, or not using any fragrance or essential oils, with the weight ratio of AROMOX® DMMC—W:diisoamylene:base being 3.8:0.2:96 and the ratio of DOWFAX® 2Al: diisoamylene:base being 3.8:0.2:2.96, as described in Example XVIII, supra.

Figure 54B is a graph of *percent residual chlorine* versus *time in hours* comparing hypochlorite solutions of (i) DOWFAX® 2Al versus (ii) AROMOX® DMMC—W, with the perfuming material being one of the diisoamylene products produced according to Example I, supra, wherein the ratio of AROMOX® DMMC—W:diisoamylene:base is either 0.8:0.2:99 or 1.8:0.2:98 and the weight ratio of DOWFAX® 2Al:diisoamylene:base is 0.8:0.2:99 or 1.8:0.2:98 as described in Example XVIII, supra.

Figure 55A is a graph of *percent residual chlorine* versus *time in hours* comparing the hypochlorite solutions of DOWFAX® 2Al versus (ii) AROMOX® DMMC—W, with the perfuming material being one of the diisoamylene epoxide products produced according to Example II, supra, wherein the weight ratio of

AROMOX® DMMC—W:diisoamylene epoxide:base is either 0.8:0.2:9 or 1.8:0.2:9 and the weight ratio of DOWFAX® 2AI:diisoamylene epoxide:base is 0.8:0.2:9 or 1.8:0.2:9, as described in Example XVIII, supra.

Figure 55B is a graph of *percent residual chlorine* versus *time in hours* comparing the performance of hypochlorite solutions of (i) DOWFAX® 2AI versus (ii) AROMOX® DMMC—W using one of the diisoamylene epoxide products produced according to Example II, supra, or not using any fragrance or essential oils, with the weight-ratio of AROMOX® DMMC—W:diisoamylene epoxide:base being 3.8:0.2:9 and the weight ratio of DOWFAX® 2AI:diisoamylene epoxide:base being 3.8:0.2:9 as described in Example XVIII, supra.

Figure 56 is the GLC profile for the reaction product of Example XIX containing a mixture of compounds defined according to the structure:

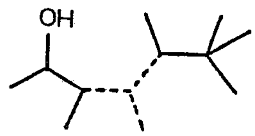

Figure 57 is the infra-red spectrum for the reaction product of Example XIX containing the mixture of compounds defined according to the structure:

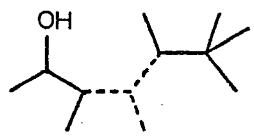

wherein in each compound of said mixture, one of the dashed lines represents a carbon-carbon double bond and each of the other of the dashed lines represents a carbon-carbon single bond.

Figure 58 is the GLC profile for fraction 4 of the distillation product of the reaction product of Example XX containing a mixture of compounds defined according to the structure:

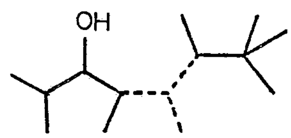

wherein in each compound of said mixture, one of the dashed lines represents a carbon-carbon double bond and the other dashed lines represent carbon-carbon single bonds.

Figure 59 represents the infra-red spectrum fraction 4 of the distillation product of the reaction product of Example XX containing the mixture of compounds defined according to the structure:

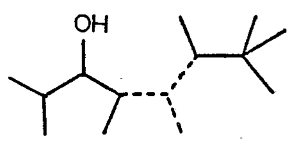

wherein in each compound of said mixture, one of the dashed lines represents a carbon-carbon double bond and the other dashed lines represent carbon-carbon single bonds.

Figure 60 represents the NMR spectrum for fraction 4 of the distillation product of the reaction product of Example XX containing a mixture of compounds defined according to the structure:

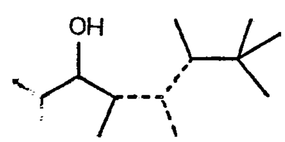

wherein in each compound of the said mixture, one of the dashed lines represents a carbon-carbon double bond and the other dashed lines represent carbon-carbon single bonds.

**Claims**

1. A process for augmenting or enhancing the aroma or taste of a consumable material which is in the alternative, a perfume composition, a perfumed article, a cologne, a foodstuff, a chewing gum, a

toothpaste, a medicinal product, a chewing tobacco composition or a smoking tobacco article, comprising the step of adding to either of a perfume composition, a perfumed article base, a cologne base, a foodstuff, a chewing gum, a toothpaste, a chewing tobacco base, a medicinal product base, a smoking tobacco composition or a smoking tobacco article component, one or more compounds in an organoleptic property modifying augmenting or enhancing quantity, characterized in that the said compounds are in the alternative:

a. one or more diisoamylenes;

b. one or more diisoamylene epoxides;

c. one or more acylated diisoamylenes, excluding the Ketones defined according to the structure

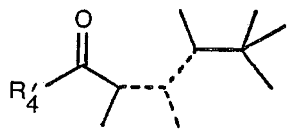

wherein R4′ represents C1—C3 lower alkyl and wherein one of the dashed lines represents a carbon-carbon double bond and the other dashed lines represent carbon-carbon single bonds;

d. one or more alcohol moiety-containing reduction products of one or more acylated diisoamylenes;

e. one of more ketal or thioketal derivatives of one or more acylated diisoamylenes;

f. one or more oxo reaction products of one or more diisoamylenes;

g. one or more alkyl metallo derivatives of one or more acylated diisoamylenes;

h. one or more hydrolysis products of one or more alkyl metallo derivatives of one or more acylated derivatives of one or more diisoamylenes; or

i. compounds defined according to the structure

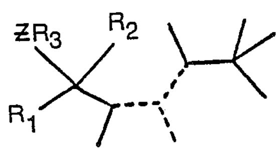

wherein Z represents oxygen or sulfur; R1 and R2 represent C1—C4 alkyl; R3 represents hydrogen, C1—C3 lower alkyl or C1—C3 lower acyl; with the proviso that when Z is oxygen, R3 is not hydrogen; wherein one of the dashed lines represents a carbon-carbon double bond and the other dashed lines represent carbon-carbon single bonds.

2. The process of Claim 1 further characterized in that the said compounds of diisoamylene isomers defined according to the structure:

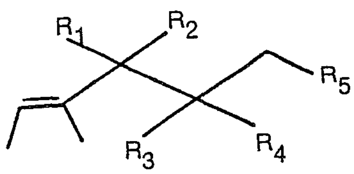

wherein $R_1$, $R_2$, $R_3$, $R_4$, and $R_5$ and represent hydrogen or methyl with the provisos that (i) at least one of $R_3$ and $R_4$ represents methyl, (ii) the sum of the carbon atoms in $R_1$, $R_2$, $R_3$, $R_4$ and $R_5$ is three and (iii) $R_1$ and $R_2$ represent hydrogen when $R_5$ is methyl; and the consumable material is, in the alternative, a perfume composition, a perfumed article or a cologne and the said diisoamylene compounds are produced by the step of reacting two moles of isoamylene in the presence of an acid catalyst.

3. The process of Claim 1 further characterized in that the diisoamylene compound or diisoamylene derivative is a diisoamylene epoxide defined according to the structure:

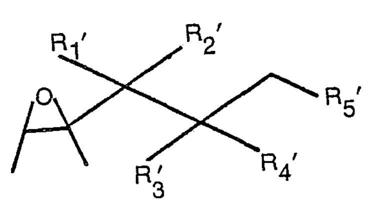

wherein $R_1'$, $R_2'$, $R_3'$, $R_4'$ and $R_5'$ are the same or different and each represents hydrogen or methyl with the proviso that (i) the sum total of carbon atoms in $R_1'$, $R_2'$, $R_3'$, $R_4'$ and $R_5'$ is three, (ii) $R_1'$ and $R_2'$ each represent hydrogen when $R_5'$ represents methyl, (iii) when either $R_1'$ or $R_2'$ is methyl, $R_5'$ is hydrogen and

(iv) at least one of $R_3'$ and $R_4'$ represents methyl and the consumable material is in the alternative, a perfume composition, a perfumed article or a cologne.

4. The process of Claim 1 further characterized in that the diisoamylene derivative or diisoamylene compound is defined according to the structure:

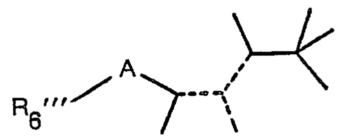

wherein A represents, in the alternative, one of the moieties:

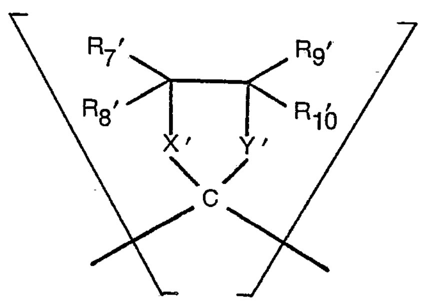

or

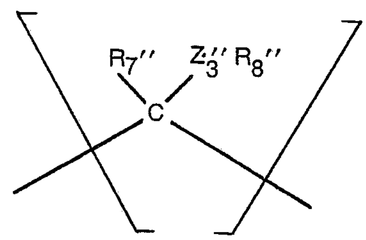

and wherein $R_6'''$ represents $C_1$—$C_3$ lower alkyl; and wherein $R_7'$, $R_8'$, $R_9'$ and $R_{10}'$ each represents hydrogen or $C_1$—$C_3$ lower alkyl; and wherein X' and Y' are the same or different and each represents oxygen or sulfur and wherein one of the dashed lines represents a carbon-carbon double bond and each of the other of the dashed lines represents a carbon-carbon single bond; and wherein $Z_3''$ represents oxygen or sulfur; $R_7''$ represents hydrogen or $C_1$—$C_4$ alkyl; and $R_8''$ represents hydrogen, MgX, Li, $C_1$—$C_3$ lower alkyl or $C_1$—$C_3$ lower acyl.

5. The process of Claim 1 further characterized in that the diisoamylene compound or diisoamylene derivative is defined according to the structure:

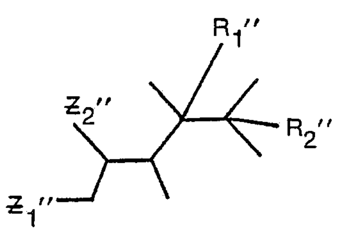

wherein one of $R_1''$ or $R_2''$ is hydrogen and the other of $R_1''$ or $R_2''$ is methyl; and wherein one of $Z_1''$ or $Z_2''$ is hydrogen and the other of $Z_1''$ or $Z_2''$ is hydroxymethyl having the structure:

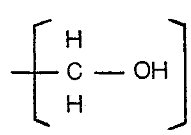

or carboxaldehyde having the structure:

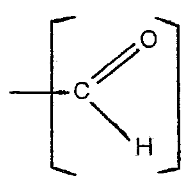

6. A diisoamylene derivative created as a result of one or more reactions of a given reagent with a diisoamylene compound prepared by reacting two moles of isoamylene in the presence of an acid catalyst, which diisoamylene is defined according to the structure:

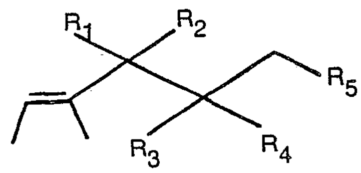

wherein $R_1$, $R_2$, $R_3$, $R_4$, and $R_5$ represent hydrogen or methyl with the provisos that (i) at least one of $R_3$ and $R_4$ represents methyl; (ii) the sum of the carbon atoms in $R_1$, $R_2$, $R_3$, $R_4$ and $R_5$ is three and (iii) $R_1$ and $R_2$ represent hydrogen when $R_5$ is methyl, said diisoamylene derivative being characterized in that it is in the alternative:

a. one or more diisoamylene epoxides;

b. one or more acylated diisoamylenes, excluding the Ketones defined according to the structure

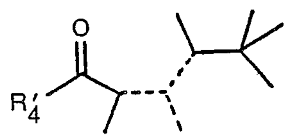

wherein R4′ represents C1—C3 lower alkyl and wherein one of the dashed lines represents a carbon-carbon double bond and the other dashed lines represent carbon-carbon single bonds;

c. one or more ketal or thioketal derivatives of one or more acylated diisoamylenes;

d. one or more alcohol moiety-containing reduction products of one or more acylated diisoamylenes;

e. one or more oxo reaction derivatives of one or more diisoamylenes;

f. one or more alkyl metallo derivatives of one or more acylated diisoamylenes;

g. one or more hydrolysis products of one or more alkyl metallo derivatives of one or more acylated diisoamylenes; or

h. compounds defined according to the structure

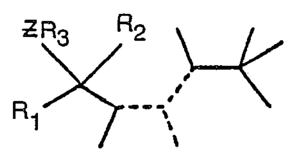

wherein Z represents oxygen or sulfur; R1 and R2 represent C1—C4 alkyl; R3 represents hydrogen, C1—C3 lower alkyl or C1—C3 lower acyl; with the proviso that when Z is oxygen, R3 is not hydrogen; wherein one of the dashed lines represents a carbon-carbon double bond and the other dashed lines represent carbon-carbon single bonds.

7. A diisoamylene derivative of Claim 6 further characterized in that it is a diisoamylene epoxide defined according to the structure:

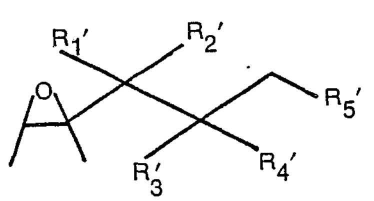

wherein $R_1'$, $R_2'$, $R_3'$, $R_4'$ and $R_5$ are the same or different and each represents hydrogen or methyl with the proviso that (i) the sum total of the carbon atoms in $R_1'$, $R_2'$, $R_3'$, $R_4'$ and $R_5'$ is three, (ii) $R_1'$ and $R_2'$ each represent hydrogen when $R_5'$ represents methyl, and (iii) when either $R_1'$ or $R_2'$ is methyl, $R_5'$ is hydrogen.

8. The diisoamylene derivative of Claim 6 further characterized in that it is either an alcohol moiety-containing reduction product of one or more acylated diisoamylenes, a ketal or thioketal derivative of one or more acylated diisoamylenes; an alkyl metallo derivative of one or more acylated diisoamylenes or a hydrolysis product of one or more alkyl metallo derivatives of one or more acylated diisoamylenes defined according to the structure:

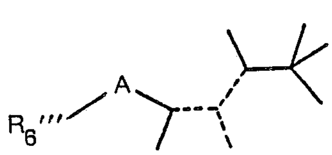

wherein A is the alternative one of the moieties:

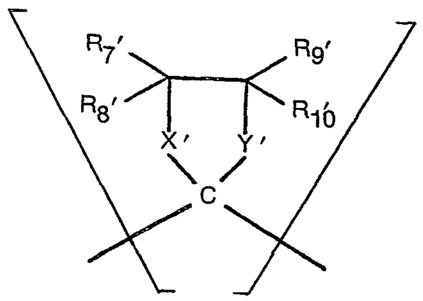

or

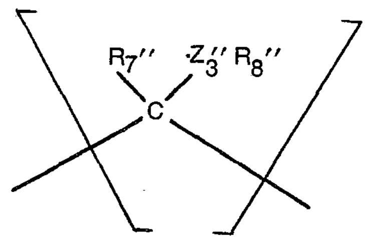

and wherein $R_6'''$ represents $C_1$—$C_3$ lower alkyl; one of the dashed lines represents a carbon-carbon double bond and the other dashed lines represent carbon-carbon single bonds; $R_7'$, $R_8'$, $R_9'$ and $R_{10}'$ each represent hydrogen or $C_1$—$C_3$ lower alkyl; X' and Y' are the same or different and each represents oxygen or sulfur; $Z_3''$ represents oxygen or sulfur; $R_7''$ represents hydrogen or $C_1$—$C_4$ alkyl; and $R_8''$ represents hydrogen, MgX, Li, $C_1$—$C_3$ lower alkyl or $C_1$—$C_3$ lower acyl.

9. The diisoamylene derivative of Claim 6 further characterized in that it has the structure:

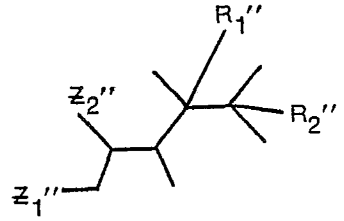

wherein one of $R_1''$ or $R_2''$ is hydrogen and the other of $R_1''$ or $R_2''$ is methyl; and wherein one of $Z_1''$ or $Z_2''$ is hydrogen and the other of $Z_1''$ or $Z_2''$ is hydroxymethyl having the structure:

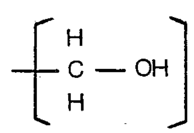

or carboxaldehyde having the structure:

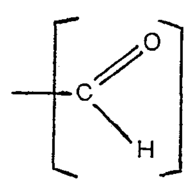

10. A single phase aqueous alkali metal hypochlorite solution having a pH in the range of 11 to 14.0, comprising as a sole detergent from 0.10% up to 2.0%, based on the total weight of solution, of a composition of matter selected from the group consisting of (1) at least one substance defined according to the structure:

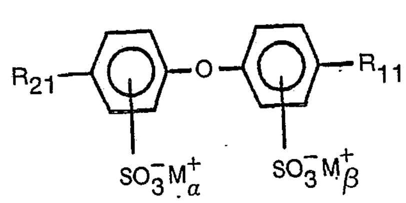

wherein at least one of $R_{11}$ and $R_{21}$ is $C_{10}$—$C_{12}$ straight chain or branched chain alkyl; when only one of $R_{11}$ or $R_{21}$ is $C_{10}$—$C_{12}$ straight chain or branched chain alkyl, the other of $R_{11}$ or $R_{21}$ is hydrogen; wherein $M_\alpha$ and $M_\beta$ are the same or different and each represents alkali metal selected from the group consisting of sodium, potassium and lithium and (2) a mixture comprising a material having the structure:

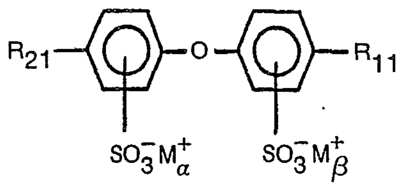

and intimately admixed therewith an amine oxide composition consisting essentially of one or more substances having the structure:

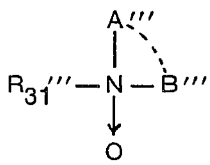

wherein $R_{31}'''$ is straight chain alkyl; wherein more than 55% of the $R_{31}'''$ moieties of said amine oxides consist of straight chain alkyl having from 11 up to 13 carbon atoms and wherein A''' and B''' are each separately methyl or, taken together, complete a morpholine ring; characterized in that the said solution further comprises from 0.02% up to 0.2% based on the total weight of solution of one or more diisoamylene derivatives, wherein the diisoamylene derivative is in the alternative:

a. one or more diisoamylenes, or
b. one or more epoxidized diisoamylenes;

and further characterized in that the diisoamylene derivative is defined according to the structure:

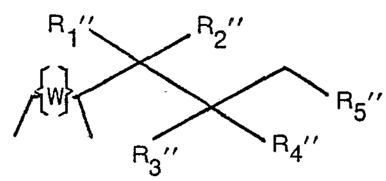

wherein W represents a carbon-carbon double bond or a vicinal epoxy moiety and wherein $R_1''$, $R_2''$, $R_3''$, $R_4''$ and $R_5''$ are the same or different and each represents hydrogen or methyl with the provisos that (i) the sum total of the carbon atoms in $R_1''$, $R_2''$, $R_3''$, $R_4''$ and $R_5''$ is three; (ii) $R_1''$ and $R_2''$ represent hydrogen when $R_5''$ represents methyl and (iii) when either $R_1''$ or $R_2''$ is methyl, $R_5''$ is hydrogen.

11. The aqueous alkali metal hypochlorite solution of Claim 10 further characterized in that the diisoamylene derivative is produced by reacting two moles of diisoamylene in the presence of an acid catalyst.

12. The composition of Claim 10 further characterized in that in addition to the ingredients of the composition being present, a thickening or gel-forming agent is present in the mixture.

13. A process for augmenting or enhancing the aroma or taste of a consumable material selected from the group consisting of foodstuffs, chewing gums, toothpastes and medicinal products comprising the steps of adding to said consumable material from 0.02 ppm up to about 50 ppm based on the total weight of said consumable material of an acetyl diisobutylene having a structure selected from the group consisting of:

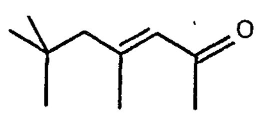

and

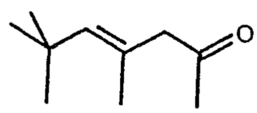

**Patentansprüche**

1. Verfahren zum Verbessern oder Verstärken des Aromas oder Geschmacks eines Verbrauchsmaterials, welches alternativ eine Parfümzusammensetzung, ein parfümierter Artikel, ein Eau de Cologne, ein Nahrungsmittel, ein Kaugummi, eine Zahnpasta, ein medizinisches Produkt, ein Kautabak, eine Rauch-Tabak-Zusammensetzung oder ein Rauchtabakartikel ist, welches den Schritt der Zugabe zu einer Parfümzusammensetzung, einem Grundstoff für einen parfümierten Artikel, einem Grundstoff von Eau de Cologne, einem Nahrungsmittel, einen Kaugummi, einer Zahnpasta, einem Kautabakgrundstoff, einem medizinischen Produktgrundstoff, einer Rauchtabakzusammensetzung oder einer Rauchtabakartikelzusammensetzung von einer oder mehreren Verbindung(en) ineiner die organoleptischen Eigenschaften modifizierenden, verstärkenden oder verbessernden Menge aufweist, dadurch gekennzeichnet, daß diese Verbindungen alternativ sind:

a. ein oder mehrere Diisoamylene;
b. ein oder mehrere Diisoamylen-Epoxid(e);

c. ein oder mehrere acylierte Diisoamylene, ausgeschlossen die mit der Struktur:

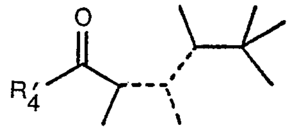

wobei R4' eine C1—C3 niedere Alkylgruppe repräsentiert und wobei eine der unterbrochenen Linien eine Kohlenstoff-Kohlenstoffdoppelbindung und die übrigen unterbrochenen Linien Kohlenstoff-Kohlenstoffeinfachbindungen repräsentieren;

d. ein oder mehrere Alkoholreste enthaltende Reduktionsprodukte von ein oder mehreren acylierten Diisoamylenen;

e. ein oder mehr Ketal- oder Thioketal-Abkömmlinge von einem oder mehreren acylierten Diisoamylenen;

f. ein oder mehrere Oxo-Reaktionsprodukte von einem oder mehreren Diisoamylenen;

g. ein oder mehrere Alkylmetall-Reste von einem oder mehreren acylierten Diisoamylenen;

h. ein oder mehr Hydrolyseprodukte von einem oder mehreren Alkylmetall-Derivaten von einem oder mehreren acylierten Derivaten eines oder mehrerer Diisoamylen(en); oder

i. Verbindungen, definiert durch die Struktur

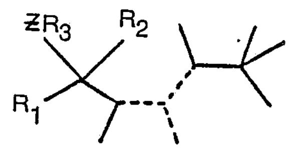

wobei Z Sauerstoff oder Schwefel repräsentiert; R1 und R2 C1—C4 Alkyl repräsentieren; R3 Wasserstoff repräsentiert; C1—C3 eine niedere Alkylgruppe oder C1—C3 niedere Acylgruppe; unter der Voraussetzung, daß wenn Z Sauerstoff ist, R3 nicht Wasserstoff ist; wobei eine der unterbrochenen Linien eine Kohlenstoff-Kohlenstoffdoppelbindung repräsentiert und die anderen unterbrochenen Linien Kohlenstoff-Kohlenstoffeinfachbindungen repräsentieren.

2. Verfahren nach Anspruch 1, ferner dadurch gekennzeichnet, daß die Verbindungen die Diisoamylen-Isomere, definiert durch die Struktur sind

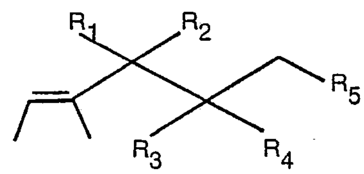

wobei $R_1$, $R_2$, $R_3$, $R_4$ und $R_5$ jeweils Wasserstoff oder Methyl repräsentieren, unter der Voraussetzung, daß (i) mindestens eines von $R_3$ und $R_4$ für Methyl steht, (ii) die Summe der Kohlenstoffatome in $R_1$, $R_2$, $R_3$, $R_4$ und $R_5$ drei beträgt und (iii) $R_1$ und $R_2$ Wasserstoff repräsentieren, wenn $R_5$ Methyl ist, und das verzehrbare Material alternativ eine Parfümzusammensetzung, ein parfümierter Artikel oder ein Eau de Cologne ist und die Diisoamylen-Verbindungen durch den Verfahrensschritt der Umsetzung von zwei Mol Isoamylen in Gegenwart eines sauren Katalysators hergestellt werden.

3. Verfahren nach Anspruch 1, ferner dadurch gekennzeichnet, daß die Diisoamylen-Verbindung oder das Diisoamylenderivat ein Diisoamylen-Epoxid, definiert durch die Struktur ist:

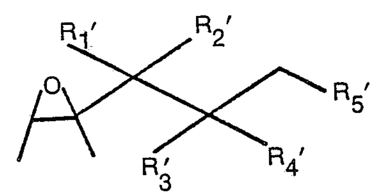

wobei $R_1'$, $R_2'$, $R_3'$, $R_4'$ und $R_5'$ gleich oder unterschiedlich sind und jedes Wasserstoff oder Methyl repräsentiert, unter der Voraussetzung, daß (i) die Gesamtsumme der Kohlenstoffatome in $R_1'$, $R_2'$, $R_3'$, $R_4'$ und $R_5'$ drei beträgt (ii) $R_1'$ und $R_2'$ jeweils Wasserstoff repräsentieren, wenn $R_5'$ Methyl repräsentiert, (iii) wenn entweder $R_1'$ oder $R_2'$ Methyl ist, $R_5'$ Wasserstoff ist und (iv) mindestens eines von $R_3'$ und $R_4'$ Methyl repräsentiert und das verzehrbare Material alternativ eine Parfümzusammensetzung, ein parfümierter Artikel oder ein Eau de Cologne ist.

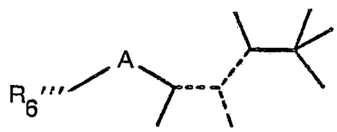

wobei A alternativ eine der Gruppen repräsentiert:

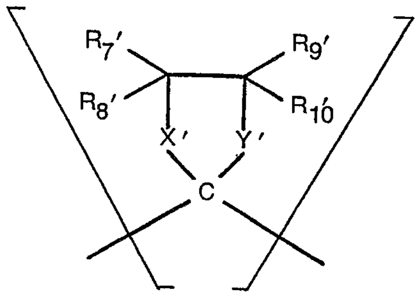

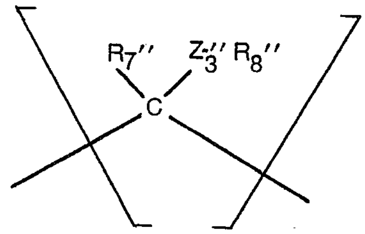

und wobei $R_6'''$ eine niedere $C_1$—$C_3$ Alkylgruppe; und $R_7'$, $R_8'$, $R_9'$ und $R_{10}'$ jeweils Wasserstoff oder einem niederen $C_1$—$C_3$ Alkylrest repräsentieren, und wobei X' und Y' gleich oder unterschiedlich sind und jedes Sauerstoff oder Schwefel repräsentiert, und wobei eine der unterbrochenen Linien eine Kohlenstoff-Kohlenstoffdoppelbindung und jede der anderen unterbrochenen Linien eine Kohlenstoff-Kohlenstoff-einfachbindung reprädsentiert; und wobei $Z_3''$ Sauerstoff oder Schwefel repräsentiert; $R_7''$ Wasserstoff oder eine $C_1$—$C_4$ Alkylgruppe repräsentiert; und $R_8''$ Wasserstoff, MgX, Li, eine niedere $C_1$—$C_3$ Alkylgruppe oder eine niedere $C_1$—$C_3$ Acylgruppe repräsentiert.

5. Verfahren nach Anspruch 1, ferner dadurch gekennzeichnet, daß die diisoamylenverbindung oder das Diisoamylenderivat durch die Stuktur definiert ist:

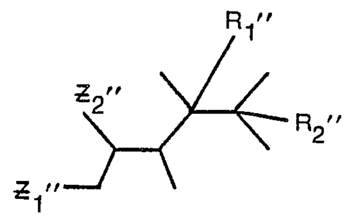

wobei eines von $R_1''$ oder $R_2''$ Wasserstoff und das andere von $R_1''$ und $R_2''$ Methyl ist; und wobei eines von $Z_1''$ oder $Z_2''$ Wasserstoff und das andere von $Z_1''$ oder $Z_2''$ Hydroxymethyl folgender Struktur ist:

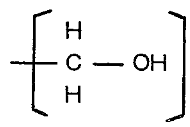

oder Carboxaldehyd der Struktur:

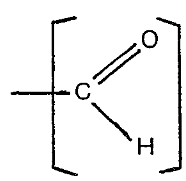

6. Ein Diisoamylenderivat, welches als Resultat von ein oder mehreren Reaktionen eines vorgegebenen Reagens mit einer Diisoamylenverbindung hergestellt ist, welches durch Umsetzen von 2 Mol Isoamylen in Gegenwart eines sauren Katalysators präpariert worden ist, wobei Diisoamylen durch die folgende Struktur definiert ist:

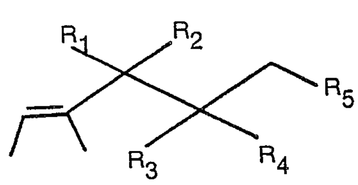

wobei $R_1$, $R_2$, $R_3$, $R_4$ und $R_5$ Wasserstoff oder Methyl unter den Voraussetzungen repräsentiert, daß (i) mindestens eines von $R^3$ oder $R_4$ Methyl repräsentiert; (ii) die Summe der Kohlenstoffatome in $R_1$, $R_2$, $R_3$, $R_4$ und $R_5$ drei beträgt und (iii) $R_1$ und $R_2$ Wasserstoff repräsentieren, wenn $R_5$ Methyl ist, *wobei das Diisoamylenderivat dadurch gekennzeichnet ist, daß es alternativ ist:*

a. ein oder mehrere Diisoamylen-Epoxid(e);

b. ein oder mehrere acyliert(s) Diisoamylen(e), ausgeschlossen die Ketone, definiert durch die Struktur:

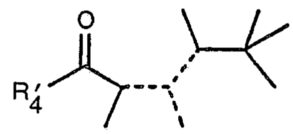

wobei R4' eine niedere C1—C3 Alkylgruppe repräsentiert und wobei eine der unterbrochenen Linien eine Kohlenstoff-Kohlenstoffdoppelbindung und die anderen unterbrochenen Linien Kohlenstoff-Kohlenstoffeinfachbindungen repräsentieren;

c. ein oder mehrere Ketal- oder Thioketalderivate von einem oder mehreren acylierten Diisoamylenen;

d. ein oder mehrere einen Alkoholrest enthaltenden Reduktionsprodukt(e) von einem oder mehreren acylierten Diisoamylen(en);

e. ein oder mehrere Oxo-Reaktionsderivate von einem oder mehreren Diisoamylen(en);

f. ein oder mehrere Alkylmetallderivate von ein oder mehreren acylierten Diisoamylenen;

g. ein oder mehrere Hydrolyseprodukte von einem oder mehreren Alkylmetallderivaten von einem oder mehreren acylierten Diisoamylen(en); oder

h. Verbindungen, definiert durch die Struktur:

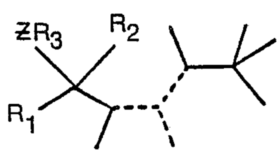

wobei Z Sauerstoff oder Schwefel repräsentiert; R1 und R2 einen C1—C4 Alkylrest repräsentieren; R3 Wasserstoff repräsentiert, eine niedere C1—C3 Alkyl- oder eine niedere C1—C3 Acylgruppe; vorausgesetzt, daß wenn Z Sauerstoff ist, R3 nicht Wasserstoff ist; wobei eine der unterbrochenen Linien eine Kohlenstoff-Kohlenstoffdoppelbindung und die andere der unterbrochene Linien Kohlenstoff-Kohlenstoffeinfachbindungen repräsentieren.

7. Ein Diisoamylen-Derivat nach Anspruch 6, ferner dadurch gekennzeichnet, daß es ein Diisoamylen-Epoxid, definiert nach der Struktur ist:

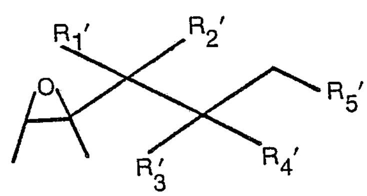

wobei $R_1'$, $R_2'$, $R_3'$, $R_4'$ und $R_5'$ gleich oder unterschiedlich sind und jedes Wasserstoff oder Methyl unter der Voraussetzung repräsentiert, daß (i) die Gesamtsumme der Kohlenstoffatome in $R_1'$, $R_2'$, $R_3'$, $R_4'$ und $R_5'$ drei beträgt; (ii) $R_1'$ und $R_2'$ jeweils Wasserstoff repräsentieren, wenn $R_5'$ Methyl repräsentiert; und (iii) wenn entweder $R_1'$ oder $R_2'$ Methyl ist, $R_5'$ Wasserstoff ist.

8. Das Diisoamylen-Derivat nach Anspruch 6, ferner dadurch gekennzeichnet, daß es entweder ein Alkoholrest enthaltendes Reaktionsprodukt von einem oder mehreren acylierten Diisoamylen(en), ein Ketal oder Thioketal-Derivat von einem oder mehreren acylierten Diisoamylen(en); ein Alkylmetall-Derivat von ein oder mehreren acylierten Diisoamylenen oder ein Hydrolyseprodukt von einem mehreren Alkylmetall-Derivat(en) von einem oder mehreren acylierten Diisoamylen(en) definiert durch die Struktur:

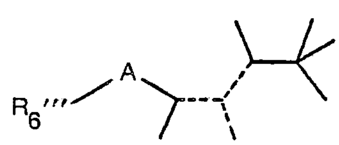

wobei A alternativ einer der Reste ist;

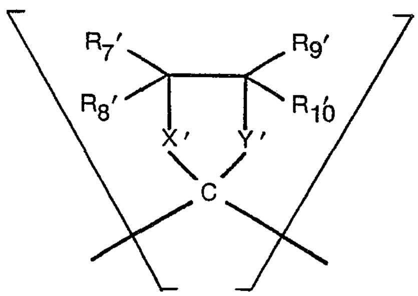

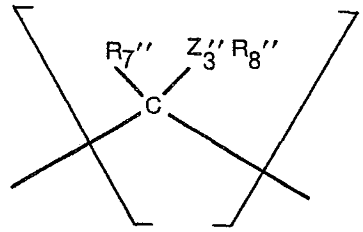

und wobei $R_6'''$ eine niedere $C_1$—$C_3$ Alkylgruppe repräsentiert; wobei eine der unterbrochenen Linien eine Kohlenstoff-Kohlenstoffdoppelbindung und die anderen unterbrochenen Linien Kohlenstoff-Kohlenstoff-einfachbindungen repräsentieren; $R_7'$, $R_8'$, $R_9'$ und $R_{10}'$ jeweils Wasserstoff oder eine niedere $C_1$—$C_3$ Alkylgruppe repräsentieren; X' und Y' gleich oder unterschiedlich sind und jedes Sauerstoff oder Schwefel repräsentiert; $Z_3''$ Sauerstoff oder Schwefel repräsentiert; $R_7''$ Wasserstoff oder eine $C_1$—$C_4$ Alkylgruppe repräsentiert; und $R_8''$ Wasserstoff, MgX, Li, eine niedere $C_1$—$C_3$ Alkylgruppe oder eine niedere $C_1$—$C_3$ Acylgruppe repräsentiert.

9. Das Diisoamylen-Derivat nach Anspruch 6, ferner dadurch gekennzeichnet, daß es die Struktur besitzt:

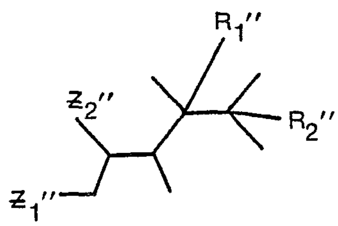

wobei eines von $R_1''$ oder $R_2''$ Wasserstoff und das andere von $R_1''$ oder $R_2''$ Methyl ist; und wobei eines von $Z_1''$ oder $Z_2''$ Wasserstoff und das andere von $Z_1''$ oder $Z_2''$ Hydroximethyl der Struktur ist:

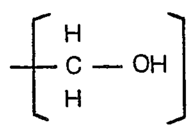

oder Carboxaldehyd der Struktur:

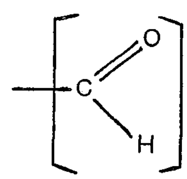

10. Eine einphasige wäßrige Alkalimetall-Hypochloritlösung mit einem pH im Bereich von 11 bis 14,0, welche als einziges Detergens zwischen 0,10% bis zu 2.0%, berechnet auf Basis des Gesamptgewichtes der Lösung, eine Materialzusammensetzung ausgewählt aus der Gruppe bestehend aus (1) mindestens einer Substanz, definiert durch die Struktur:

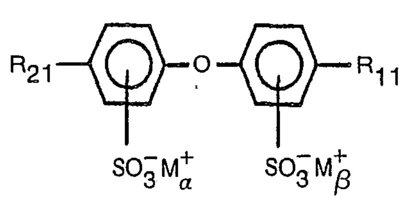

aufweist, wobei mindestens von $R_{11}$ und $R_{21}$ ein geradkettiger oder verzweigter Alkylrest ist; während, wenn lediglich einer von $R_{11}$ oder $R_{21}$ ein geradkettiger oder verzweigtkettiger $C_{10}$—$C_{12}$ Alkylrest, der andere von $R_{11}$ oder $R_{21}$ Wasserstoff ist; wobei $M_\alpha$ und $M_\beta$ gleich oder unterschiedlich sind und jedes ein Alkylimetall ausgewählt aus der Gruppe bestehend aus Natrium, Kalium und Lithium repräsentiert und (2) eine Mischung mit einem Gehalt an einem Material der Struktur:

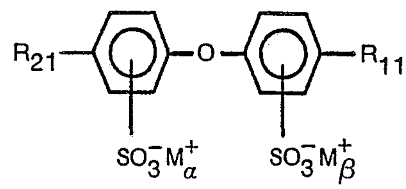

und innig damit eine Aminoxid-Verbindung, die im wesentlichen aus ein oder mehreren Substanzen der Struktur besteht

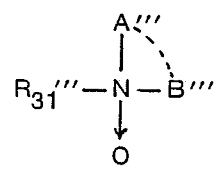

vermischt ist; wobei $R_{31}'''$ ein geradkettiger Alkylrest ist; wobei mehr als 55% der $R_{31}'''$ Reste der Aminoxide aus geradkettigen Alkylresten mit zwischen 11 bis 13 Kohlenstoffatomen bestehen und wobei A''' und B''' jeweils einzelne Methylgruppen sind oder gemeinsam einen Morpholinring vervollständigen, dadurch gekennzeichnet, daß diese Lösung ferner zwischen 0,02% bis zu 0,2%, berechnet auf dem Gesamtgewicht der Lösung, von einem oder mehreren Diisoamylen-Derivaten aufweist, wobei das Diisoamylen-Derivat alternativ ist:

a. ein oder mehrere Diisoamylene; oder

b. ein oder mehrere epoxidierte Diisoamylene;

ferner dadurch gekennzeichnet, daß das Diisoamylen-Derivat durch die Struktur definiert ist:

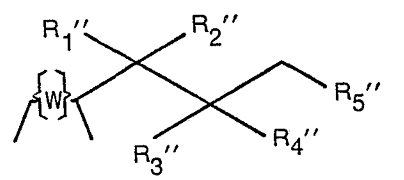

wobei W eine Kohlenstoff-Kohlenstoffdoppelbindung oder einen vicinalen Epoxyrest repräsentiert und wobei $R_1''$, $R_2''$, $R_3''$, $R_4''$ und $R_5''$ gleich oder unterschiedlich sind und jeweils Wasserstoff oder Methyl repräsentieren, unter den Voraussetzungen, daß (i) die Gesamtsumme der Kohlenstoffatome in $R_1''$, $R_2''$, $R_3''$, $R_4''$ und $R_5''$ drei beträgt; (ii) $R_1''$ und $R_2''$ Wasserstoff repräsentieren, wenn $R_5''$ Methyl repräsentiert; und (iii) wenn entweder $R_1''$ oder $R_2''$ Methyl ist, $R_5''$ Wasserstoff ist.

11. Die wäßrige Alkalimetallhypochlorit-Lösung nach Anspruch 10, ferner dadurch gekennzeichnet, daß das Diisoamylen-Derivat durch Umsetzen von zwei Mol Diisoamylen in Gegenwart eines sauren Katalysators hergestellt wird.

12. Die Zusammensetzung nach Anspruch 10, ferner dadurch gekennzeichnet, daß zusätzlich zu den anwesenden Stoffen der Zusammensetzung ein Andickungsmittel oder ein Gelbildungsagens in der Mischung anwesend ist.

13. Ein Verfahren zum Verstärken oder Verbessern des Aromas oder Geschmacks eines verzehrbaren Materials, ausgewählt aus der Gruppe bestehend aus Nahrungsmitteln, Kaugummis, Zahnpasten und medizinischen Produkten, welches die Schritte des Zugebens zum verzehrbaren Material zwischen 0,02 ppm bis zu etwa 50 ppm, berechnet auf dem Gesamtgewicht des verzehrbaren Materials, an einem Acetyl-Diisobutylen aufweist, welches eine Struktur ausgewählt aus der Gruppe bestehend aus:

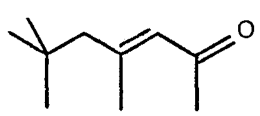

und

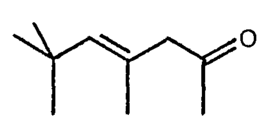

besitzt.

**Revendications**

1. Procédé pour augmenter ou améliorer l'arôme ou le goût d'une matière consommable, qui est au choix une composition de parfum, un article parfumé, une eau de Cologne, un aliment, une gomme à mâcher, une pâte dentifrice, un produit médicinal, un tabac à chiquer, une composition de tabac à fumer ou un article de tabac à fumer, comprenant l'étape consistant à ajouter soit à une composition de parfum, soit à une base d'article parfumé, soit à une base d'eau de Cologne, soit à un aliment, soit à une gomme à mâcher, soit à une pâte dentifrice, soit à une base de tabac à chiquer, soit à une base de produit médicinal,

soit à une composition de tabac à fumer, soit à un constituant d'article de tabac à fumer, un ou plusieurs composés dans une quantité modifiant, augmentant ou améliorant les propriétés organoleptiques, caractérisé en ce que ces composés sont au choix:

a. un ou plusieurs diisoamylènes;

b. un ou plusieurs époxydes de diisoamylène;

c. un ou plusieurs diisoamylènes acylés, à l'exclusion des cétones définis conformément à la structure

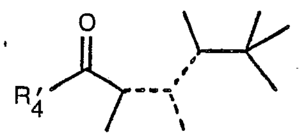

dans laquelle R4' représente un alkyle inférieur en $C_1$—$C_3$ et dans laquelle une des lignes en traits interrompus représente une double liaison carbone-carbone et l'autre ligne en traits interrompus représente des simples liaisons carbone-carbone;

d. un ou plusieurs produits de réduction contenant une partie alcool d'un ou plusieurs diisoamylènes acylés;

e. un ou plusieurs dérivés de cétal ou de thiocétal d'un ou plusieurs diisoamylènes acylés;

f. un ou plusieurs produits de réaction oxo d'un ou plusieurs diisoamylènes;

g. un ou plusieurs dérivés alkyles métalliques d'un ou plusieurs diisoamylènes acylés;

h. un ou plusieurs produits d'hydrolyse d'un ou plusieurs dérivés alkyles métalliques d'un ou plusieurs dérivés acylés d'un ou plusieurs diisoamylènes; ou

i. des composés définis conformément à la structure

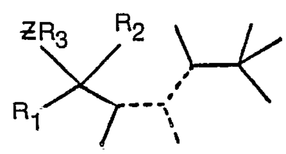

dans laquelle Z représente un oxygène ou un soufre; $R_1$ et $R_2$ représentent un alkyle en $C_1$—$C_4$; $R_3$ représente l'hydrogène, un alkyle inférieur en $C_1$—$C_3$ ou un acyle inférieur en $C_1$—$C_3$; sous réserve que lorsque Z est l'oxygène, $R_3$ n'est pas l'hydrogène; dans laquelle une des lignes en traits interrompus représente und double liaison carbone-carbone et l'autre ligne en traits interrompus représente une simple liaison carbone-carbone.

2. Procédé selon la revendication 1, caractérisé en outre en ce que ces composés sont des isomères du diisoamylène définis conformément à la structure:

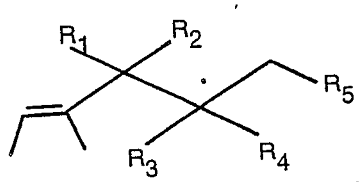

dans laquelle $R_1$, $R_2$, $R_3$, $R_4$ et $R_5$ représentent chacun l'hydrogène ou un méthyle sous réserve que (i) au moins un des $R_3$ et $R_4$ représente un méthyle, (ii) la somme des atomes de carbone dans $R_1$, $R_2$, $R_3$, $R_4$ et $R_5$ est trois et (iii) $R_1$ et $R_2$ représentent l'hydrogène lorsque $R_5$ est un méthyle; et la matière consommable est, au choix, une composition de parfum, un article parfumé, ou une eau de Cologne et ces composés du diisoamylène sont produits par l'opération consistant à faire réagir deux moles d'isoamylène en présence d'un catalyseur acide.

3. Procédé selon la revendication 1, caractérisé en outre en ce que le composé du diisoamylène ou le dérivé du diisoamylène est un époxyde de diisoamylène défini conformément à la structure:

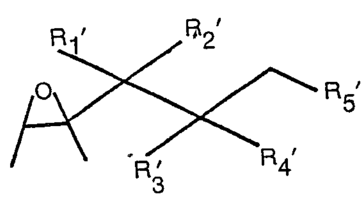

dans laquelle $R_1'$, $R_2'$, $R_3'$, $R_4'$ et $R_5'$ sont identiques ou différents et représentent chacun l'hydrogène ou un méthyle sous réserve que (i) la somme totale des atomes de carbone dans $R_1'$, $R_2'$, $R_3'$, $R_4'$ et $R_5'$ est trois, (ii) $R_1'$ et $R_2'$ représentent chacun l'hydrogène lorsque $R_5'$ représente un méthyle, (iii) lorsque soit $R_1'$, soit

$R_2'$ est un méthyle, $R_5'$ est l'hydrogène et (iv) au moins un des $R_3'$ et $R_4'$ représente un méthyle et la matière consommable est au choix une composition de parfum, un article parfumé ou une eau de cologne.

4. Procédé selon la revendication 1, caractérisé en outre en ce que le dérivé du diisoamylène ou le composé du diisoamylène est défini conformément à la structure

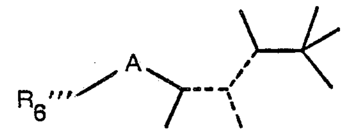

dans laquelle A représente, au choix, une des parties

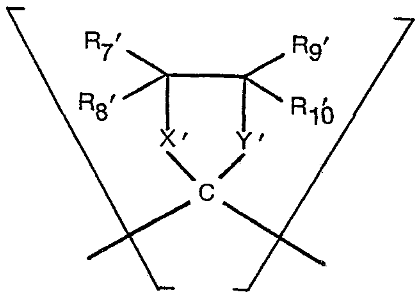

ou

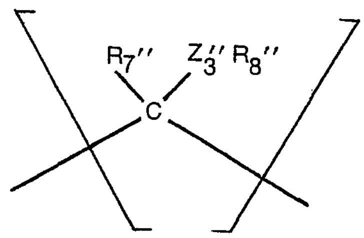

et dans laquelle $R_6'''$ représente un alkyle inférieur en $C_1$—$C_3$; et dans laquelle $R_7'$, $R_8'$, $R_9'$ et $R_{10}'$ représentent chacun l'hydrogène ou un alkyle inférieur en $C_1$—$C_3$; et dans laquelle X' et Y' sont identiques ou différents et représentent chacun un oxygène ou un soufre et dans laquelle une des lignes en traits interrompus représente une double liaison carbone-carbone, et chacune des autres lignes en traits interrompus représente une simple liaison carbone-carbone; et dans laquelle $Z_3''$ représente l'oxygène ou le soufre; $R_7''$ représente l'hydrogène ou un alkyle en $C_1$—$C_4$; et $R_8''$ représente l'hydrogène, MgX, Li, un alkyle inférieur en $C_1$—$C_3$ ou un acyle inférieur en $C_1$—$C_3$.

5. Procédé selon la revendication 1, caractérisé en outre en ce que le composé du diisoamylène ou le dérivé du diisoamylène est défini conformément à la structure:

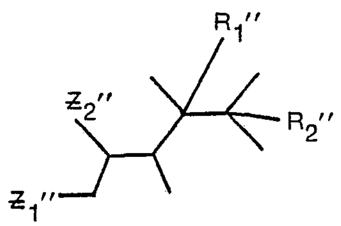

dans laquelle un des $R_1''$ ou $R_2''$ est l'hydrogène et l'autre des $R_1''$ ou $R_2''$ est un méthyle; et dans laquelle un des $Z_1''$ ou $Z_2''$ est l'hydrogène et l'autre des $Z_1''$ ou $Z_2''$ est un hydroxyméthyle ayant la structure:

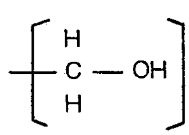

ou un carboxyaldéhyde ayant la structure:

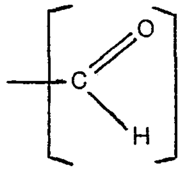

6. Dérivé du diisoamylène obtenu comme résultat d'une ou plusieurs réactions d'un réactif donné avec un composé du diisoamylène préparé en faisant réagir deux moles de d'isoamylène en présence d'un catalyseur acide, lequel diisoamylène est défini conformément à la structure:

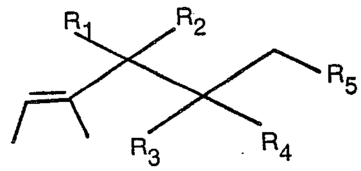

dans laquelle $R_1$, $R_2$, $R_3$, $R_4$ et $R_5$ représentent l'hydrogène ou un méthyle, sous les réserves que (i) au moins un des $R_3$ et $R_4$ représente un méthyle; (ii) la somme des atomes de carbone dans $R_1$, $R_2$, $R_3$, $R_4$ et $R_5$ est trois et (iii) $R_1$ et $R_2$ représentent l'hydrogène lorsque $R_5$ est un méthyle.

7. Dérivé du diisoamylène selon la revendication 6, caractérisé en outre en ce qu'il est un époxyde du diisoamylène défini conformément à la structure:

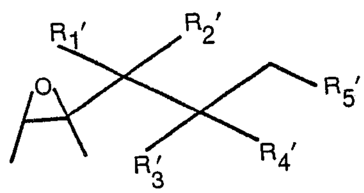

dans laquelle $R_1'$, $R_2'$, $R_3'$, $R_4'$ et $R_5'$ sont identiques ou différents et représentent chacun l'hydrogène ou un méthyle, sous réserve que (i) le total des atomes de carbone dans $R_1'$, $R_2'$, $R_3'$, $R_4'$ et $R_5'$ est trois, (ii) $R_1'$ et $R_2'$ représentent chacun l'hydrogène lorsque $R_5'$ représente un méthyle, et (iii) lorsque soit $R_1'$ soit $R_2'$ est un méthyle, $R_5'$ est l'hydrogène.

8. Dérivé du diisoamylène selon la revendication 6, caractérisé en outre en ce qu'il est soit un produit de réduction contenant une partie alcool d'un ou plusieurs diisoamylènes acylés, un dérivé de cétal ou de thiocétal d'un ou plusieurs diisoamylènes acylés; un dérivé alkyle métallique d'un ou plusieurs diisoamylènes acylés ou un produit d'hydrolyse d'un ou plusieurs dérivés alkyles métalliques d'un ou plusieurs diisoamylènes acylés définis conformément à la structure:

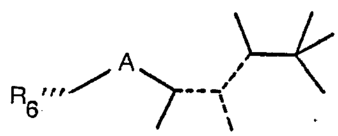

dans laquelle A est au choix une des parties:

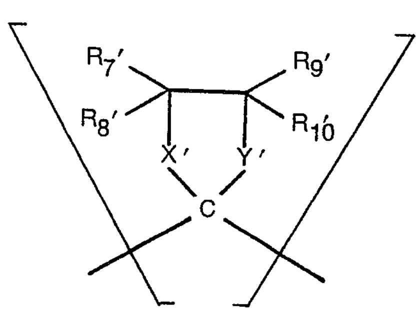

ou

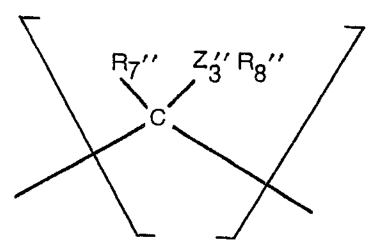

dans laquelle $R_6'''$ représente un alkyle inférieur en $C_1$—$C_3$; une des lignes en traits interrompus représente une double liaison carbone-carbone et l'autre ligne en traits interrompus représente une simple liaison carbone-carbone; $R_7'$, $R_8'$, $R_9'$ et $R_{10}'$ représentent chacun l'hydrogène ou un alkyle inférieur en $C_1$—$C_3$; X' et Y' sont identiques ou différents et représentent chacun l'hydrogène ou le soufre; $Z_3''$ représente l'oxygène ou le soufre; $R_7''$ représente l'hydrogène ou un alkyle en $C_1$—$C_4$; et $R_8''$ représente l'hydrogène, MgX, Li, un alkyle inférieur en $C_1$—$C_3$ ou un acyle inférieur en $C_1$—$C_3$.

9. Dérivé du diisoamylène selon la revendication 6, caractérisé en outre en ce qu'il a la structure:

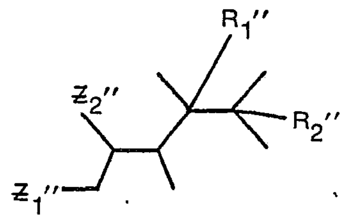

dans laquelle un des $R_1''$ ou $R_2''$ est l'hydrogène et l'autre des $R_1''$ ou $R_2''$ est un méthyle; et dans laquelle un des $Z_1''$ ou $Z_2''$ est l'hydrogène et l'autre des $Z_1''$ ou $Z_2''$ est un hydroxyméthyle ayant la structure:

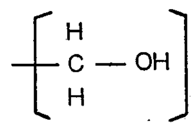

ou un carboxaldéhyde ayant la structure:

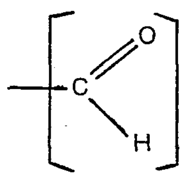

10. Solution aqueuse d'hypochlorite de métal alcalin en phase unique ayant un pH dans l'intervalle de 11 à 14,0, comprenant comme détergent unique de 0,10% à 2,0% par rapport au poids total de solution, d'une composition choisie dans le groupe constitué de (1) au moins une substance définie conformément à la structure:

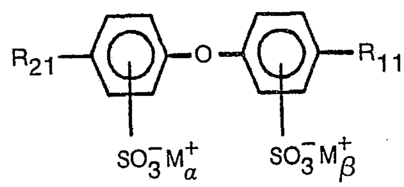

dans laquelle au moins l'un des $R_{11}$ et $R_{21}$ est un alkyle à chaîne droite ou ramifiée en $C_{10}$—$C_{12}$; lorsque l'un seulement de $R_{11}$ ou $R_{21}$ est un alkyle à chaîne droite ou ramifiée en $C_{10}$—$C_{12}$, l'autre des $R_{11}$ ou $R_{21}$ est l'hydrogène; dans laquelle $M_\alpha$ et $M_\beta$ sont identiques ou différents et représentent chacun un métal alcalin choisi dans le groupe constitué du sodium, du potassium et du lithium et (2) un mélange comprenant une matière ayant la structure;

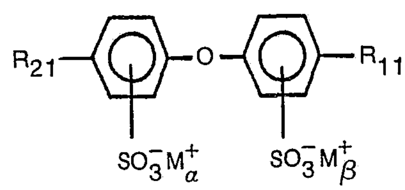

et intimement mélangée à celli-ci, une composition d'oxyde d'amine essentiellement constituée d'une ou plusieurs substances ayant la structure:

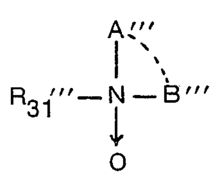

dans laquelle $R_{31}'''$ est un alkyle à chaîne droite; dans laquelle plus de 55% des parties $R_{31}'''$ de ces oxydes d'amine se composent d'un alkyle à chaîne droite ayant de 11 à 13 atomes de carbone et dans laquelle A'''

et B''' sont chacun séparément un méthyle ou, pris ensemble, complètent un cycle morpholine; caractérisée en ce que cette solution comprend en outre de 0,02% jusqu'à 0,2% par rapport au poids total de solution, d'un ou plusieurs dérivés du diisoamylène, dans lequel le dérivé du diisoamylène est au choix:

a. un ou plusieurs diisoamylènes, ou

b. un ou plusieurs diisoamylènes époxydés;

et caractérisée en outre en ce que le dérivé du diisoamylène est défini conformément à la structure:

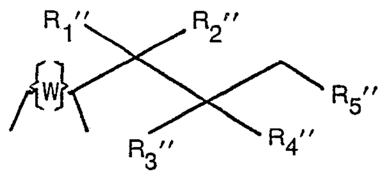

dans laquelle W représente une double liaison carbone-carbone ou une partie époxy vicinale et dans laquelle $R_1''$, $R_2''$, $R_3''$, $R_4''$ et $R_5''$ sont identiques ou différents et représentent chacun l'hydrogène ou un méthyle, sous réserve que (i) la somme des atomes de carbone dans $R_1''$, $R_2''$, $R_3''$, $R_4''$ et $R_5''$ est trois; (ii) $R_1''$ et $R_2''$ représentent l'hydrogène lorsque $R_5''$ représente un méthyle et (iii) lorsque soit $R_1''$ soit $R_2''$ est un méthyle, $R_5''$ est l'hydrogène.

11. Solution aqueuse d'hypochlorite de métal alcalin selon la revendication 10, caractérisée en outre en ce que le dérivé du diisoamylène est produit en faisant réagir deux moles de diisoamylène en présence d'un catalyseur acide.

12. Composition selon la revendication 10, caractérisée en outre en ce que en plus des ingrédients de la composition présents un agent épaississant ou gélifiant est présent dans le mélange.

13. Procédé pour augmenter ou améliorer l'arôme ou le goût d'une matière consommable choisie dans le groupe constitué des aliments, des gommes à mâcher, des pâtes dentifrice et des produits médicinaux, comprenant les étapes consistant à ajouter à cette matière consommable de 0,02 ppm jusqu'à environ 50 ppm, par rapport au poids total de cette matière consommable d'un acétyl diisobutylène ayant une structure choisie dans le groupe constitué de:

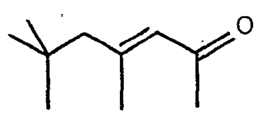

et

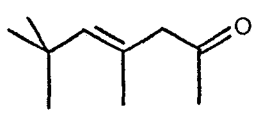

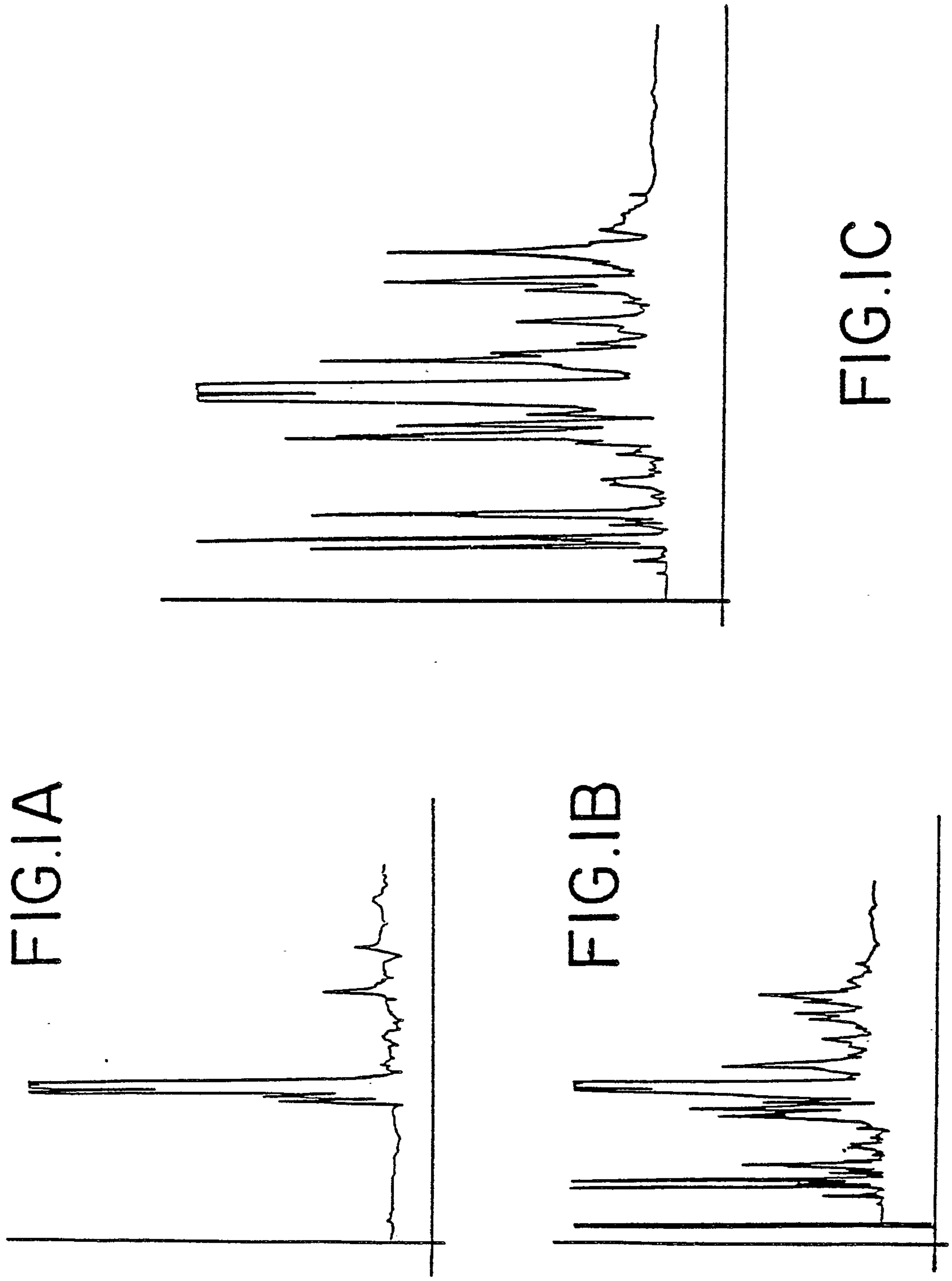

FIG.1A

FIG.1B

FIG.1C

FIG.ID

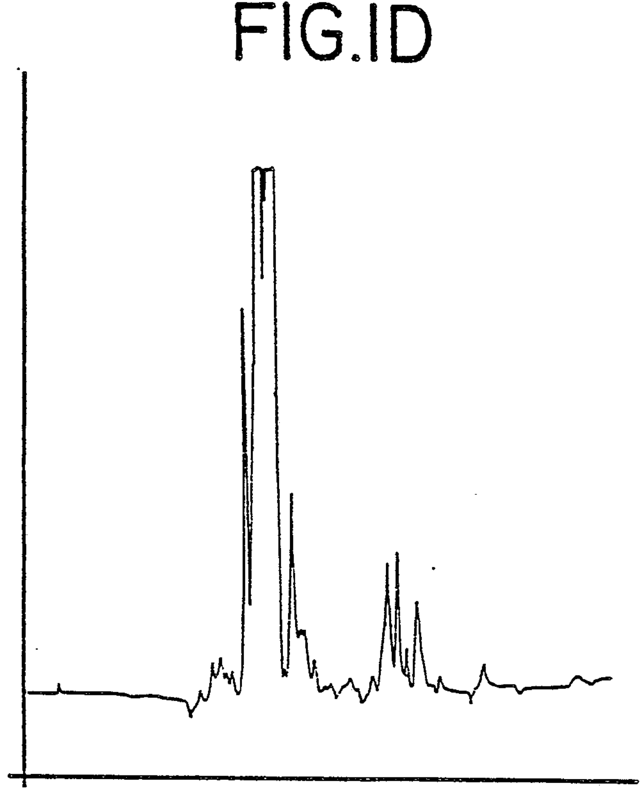

FIG.IE

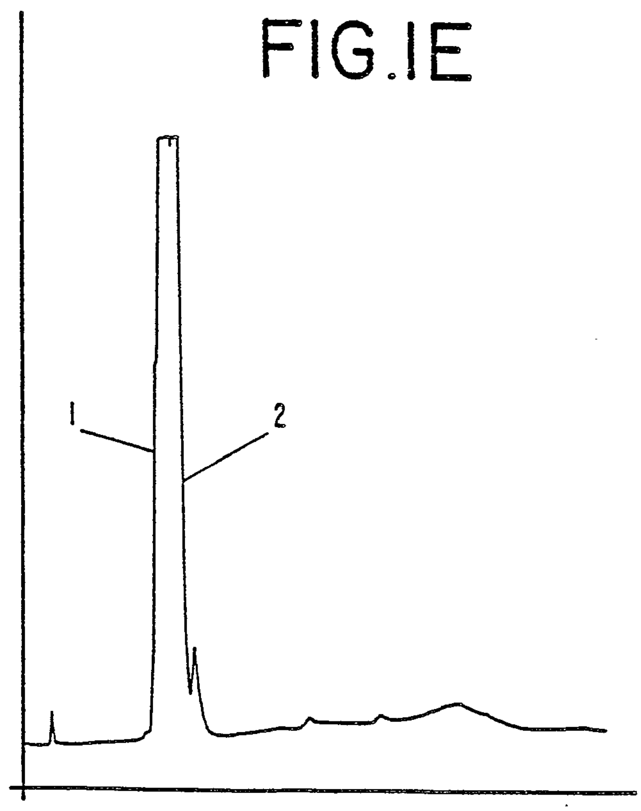

FIG.2A

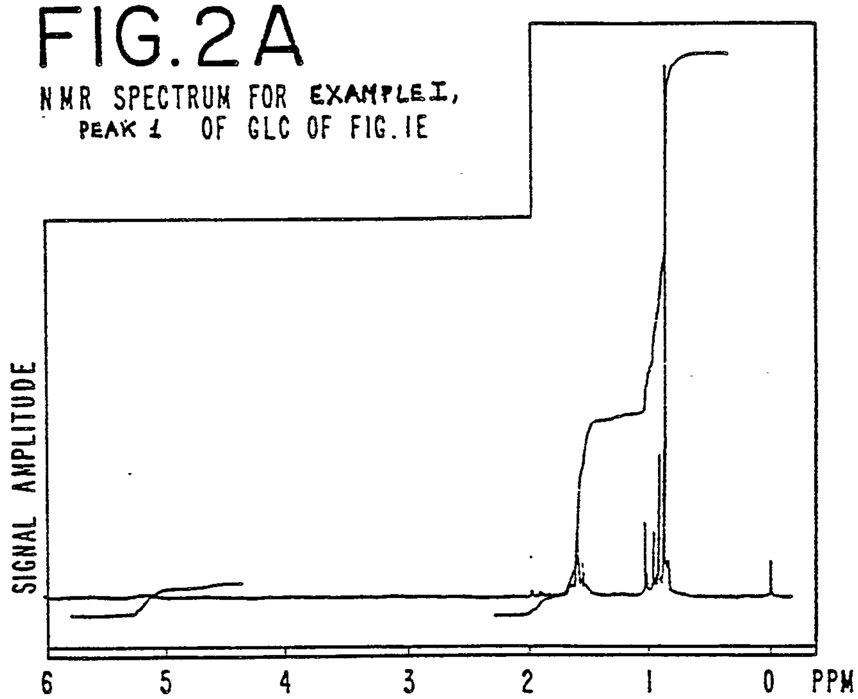
NMR SPECTRUM FOR EXAMPLE I,
PEAK 1 OF GLC OF FIG. IE
SIGNAL AMPLITUDE
6
5
4
3
2
1
0
PPM

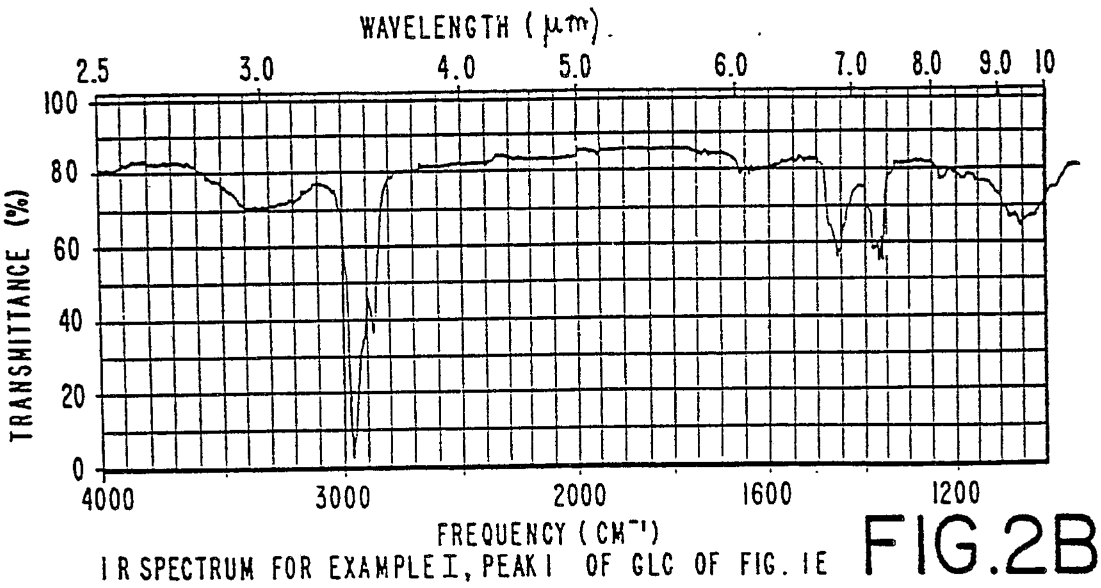
WAVELENGTH (μm)
2.5
3.0
4.0
5.0
6.0
7.0
8.0
9.0
10
100
80
60
40
20
0
TRANSMITTANCE (%)
4000
3000
2000
1600
1200
FREQUENCY (CM⁻¹)
IR SPECTRUM FOR EXAMPLE I, PEAK I OF GLC OF FIG. IE

FIG.2B

# FIG.3A

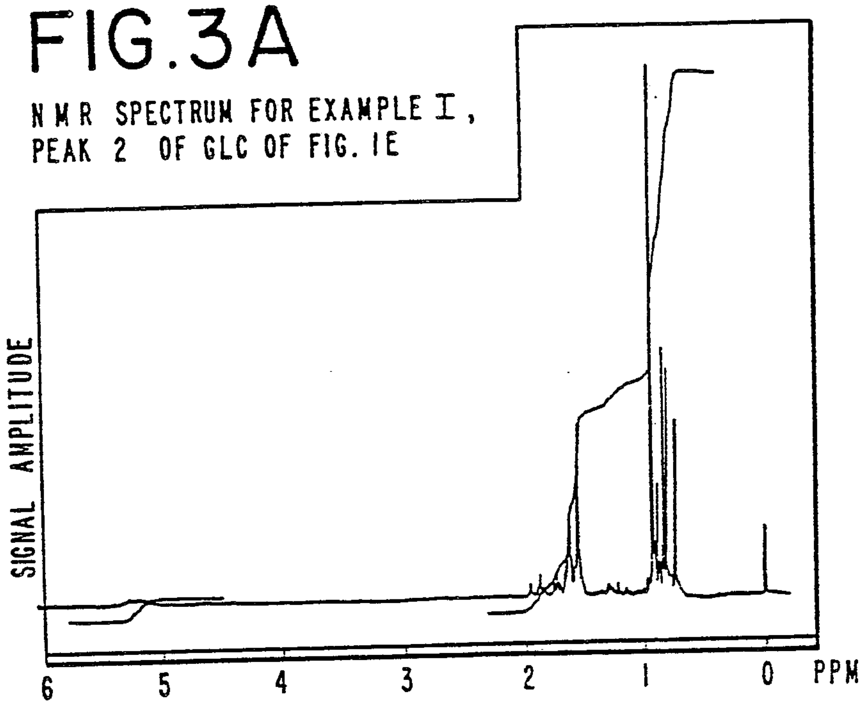


NMR SPECTRUM FOR EXAMPLE I, PEAK 2 OF GLC OF FIG. IE

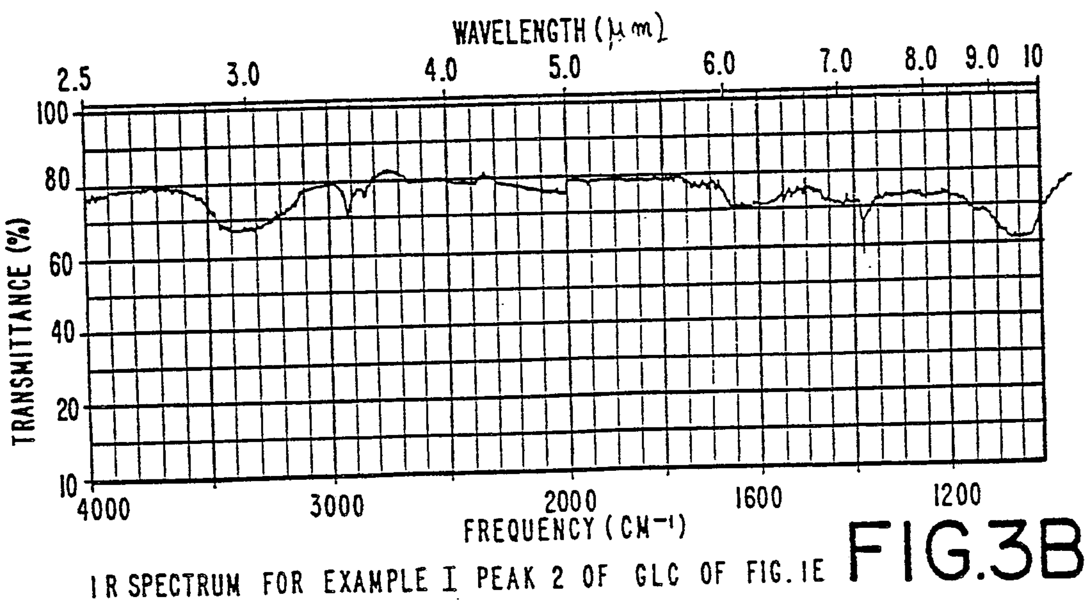


FREQUENCY (CM⁻¹)

IR SPECTRUM FOR EXAMPLE I PEAK 2 OF GLC OF FIG. IE

# FIG.3B

FIG. 4

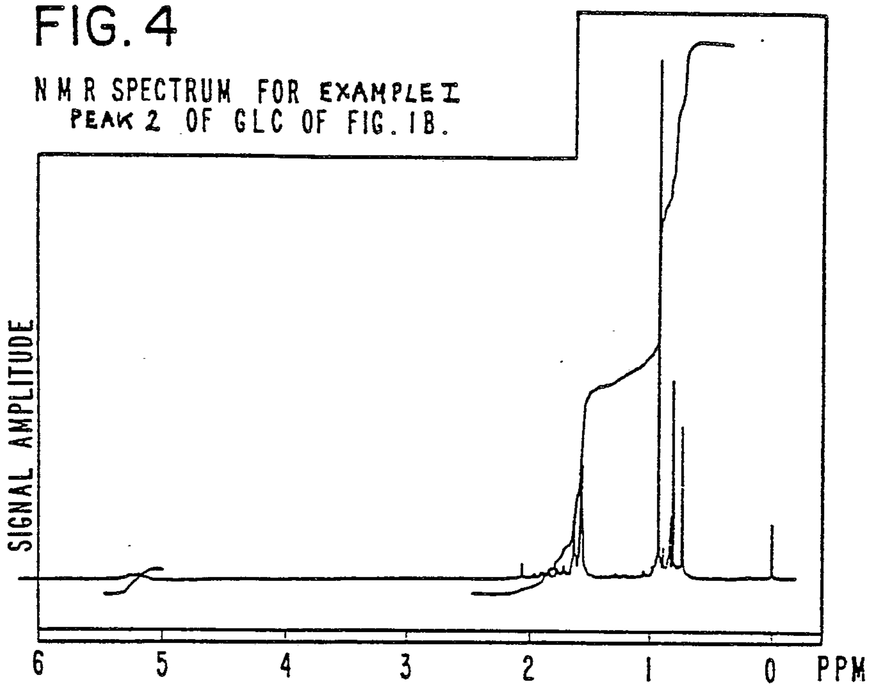
N M R SPECTRUM FOR EXAMPLE I
PEAK 2 OF GLC OF FIG. 1B.
SIGNAL AMPLITUDE
6
5
4
3
2
1
0
PPM

FIG. 5

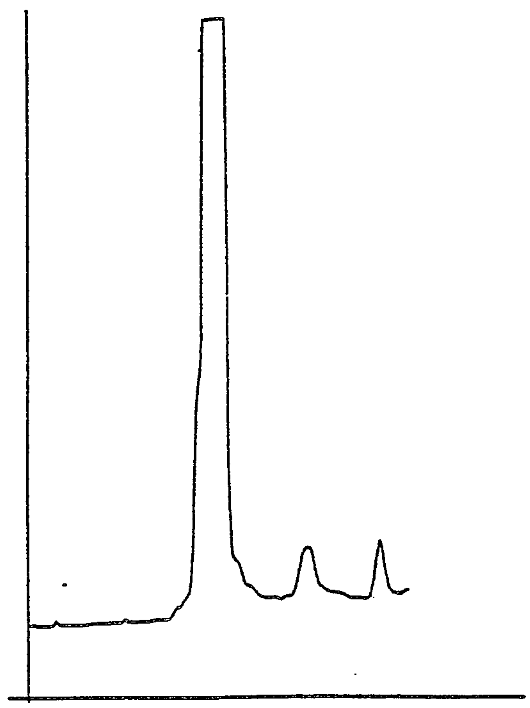

FIG.6

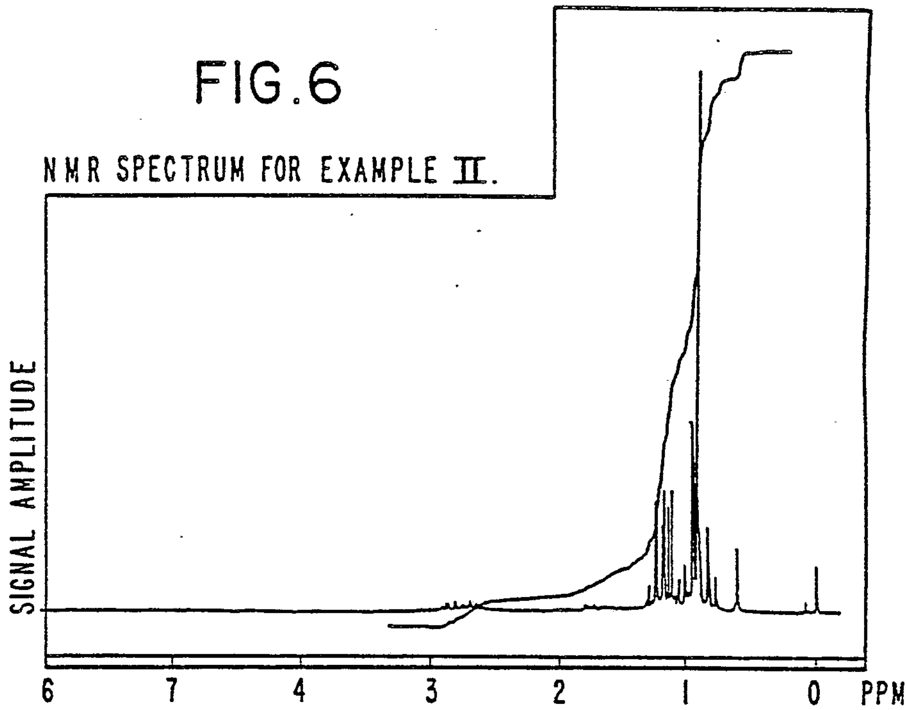
NMR SPECTRUM FOR EXAMPLE II.
SIGNAL AMPLITUDE
6
7
4
3
2
1
0
PPM

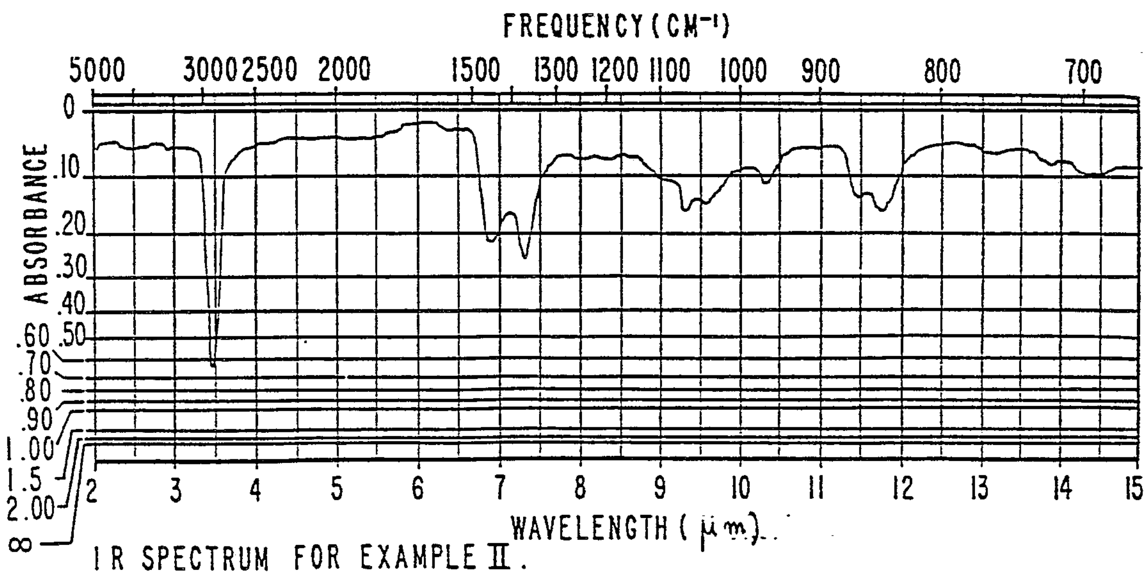
FREQUENCY (CM⁻¹)
5000
3000
2500
2000
1500
1300
1200
1100
1000
900
800
700
ABSORBANCE
0
.10
.20
.30
.40
.50
.60
.70
.80
.90
1.00
1.5
2.00
∞
2
3
4
5
6
7
8
9
10
11
12
13
14
15
WAVELENGTH (μm)
IR SPECTRUM FOR EXAMPLE II.

FIG.7

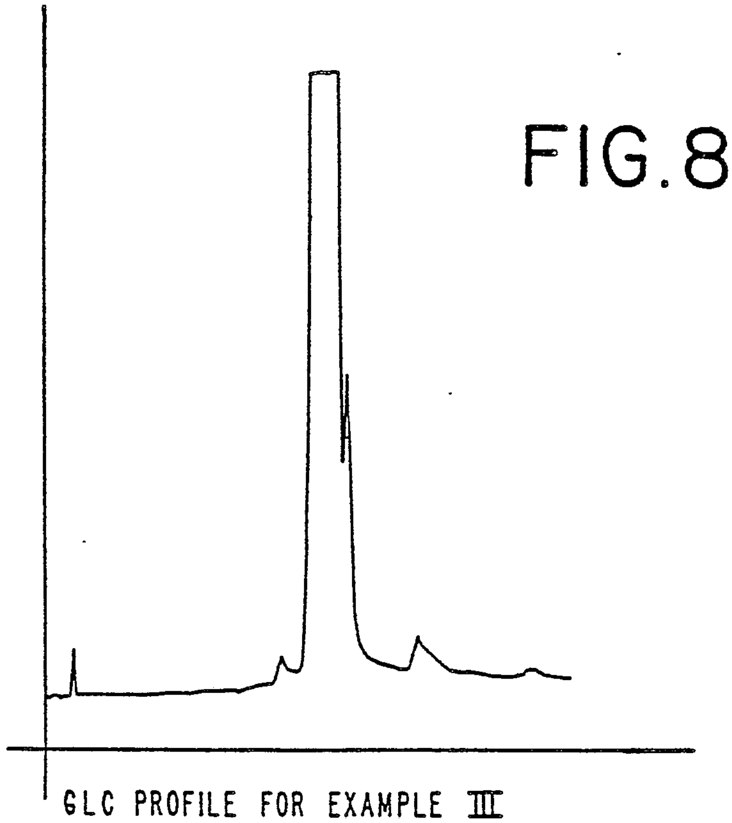

FIG.8

GLC PROFILE FOR EXAMPLE III

FIG.9A

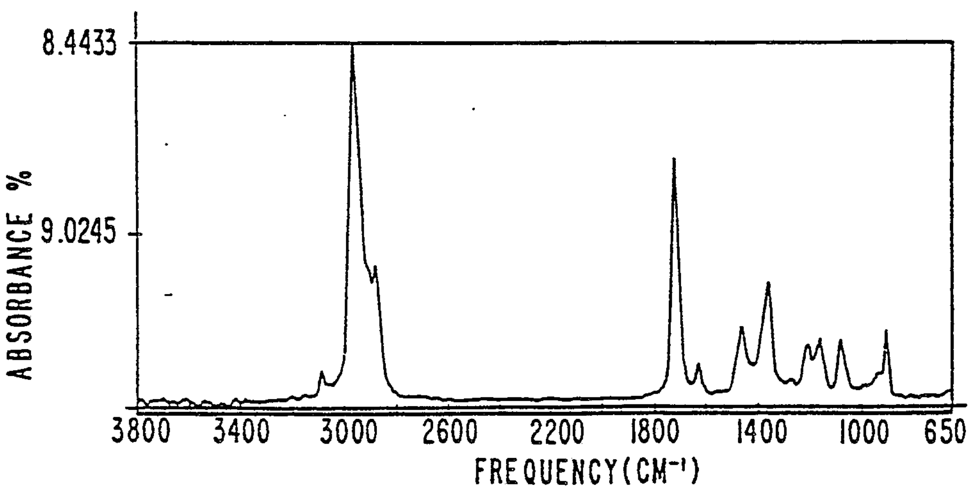


IR SPECTRUM FOR EXAMPLE III, PEAK 3 OF GLC OF FIG. 8

FIG.9B

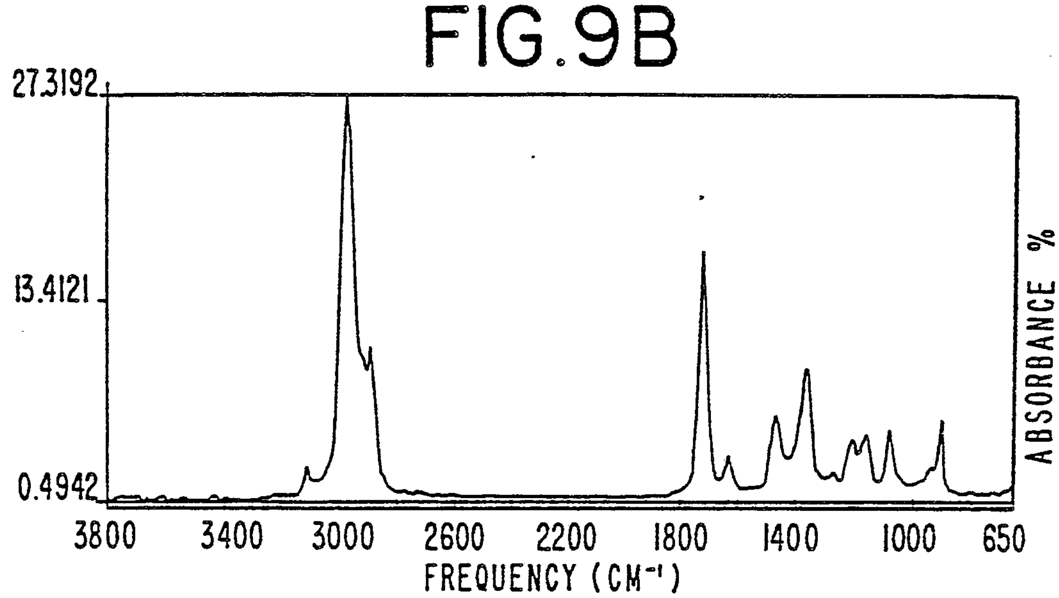


I R SPECTRUM FOR EXAMPLE III, PEAK 4 OF GLC OF FIG.8

FIG.9C

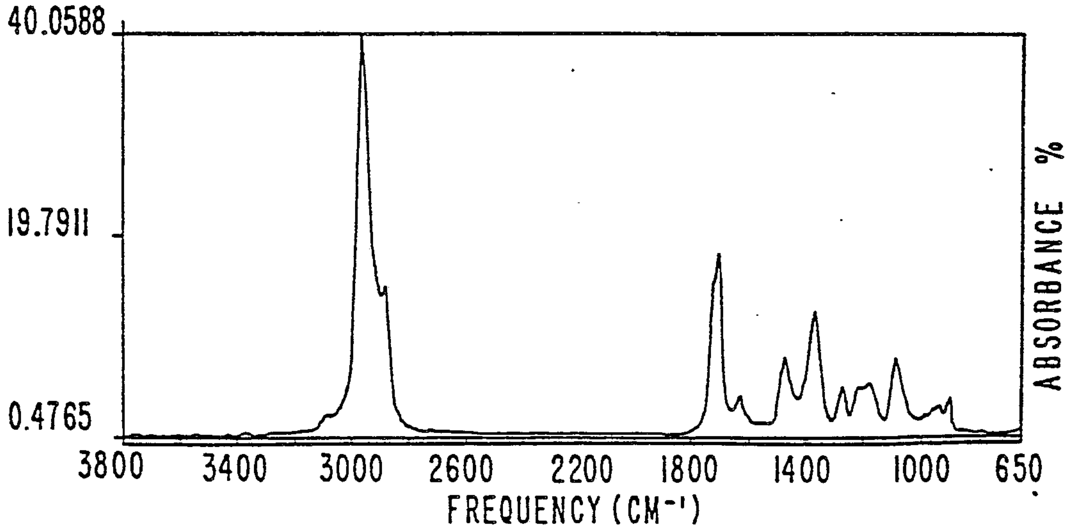


I R SPECTRUM FOR EXAMPLE III, PEAK 5 OF GLC OF FIG.8

FIG. 9D

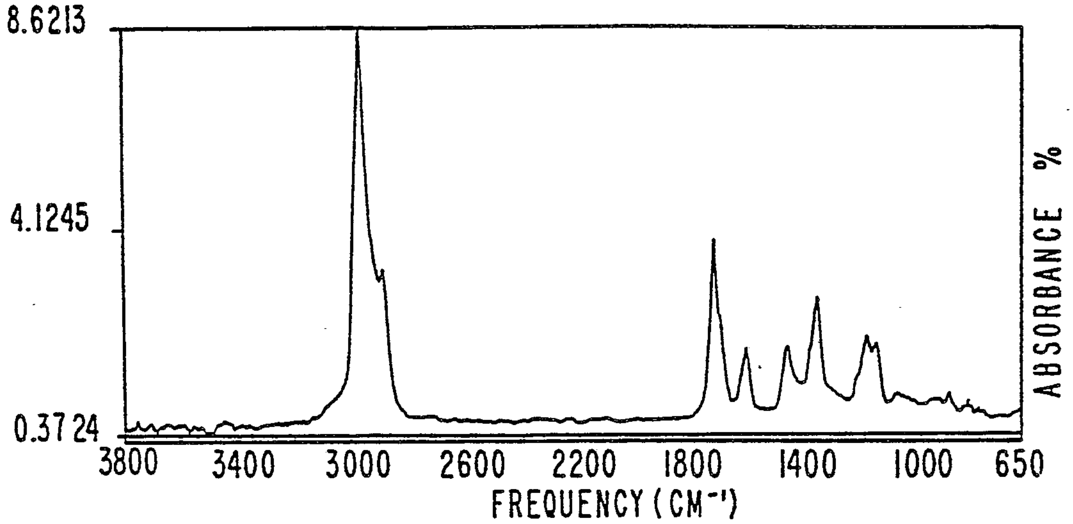


IR SPECTRUM FOR EXAMPLE III, PEAK 6 OF GLC OF FIG. 8

FIG. 9E

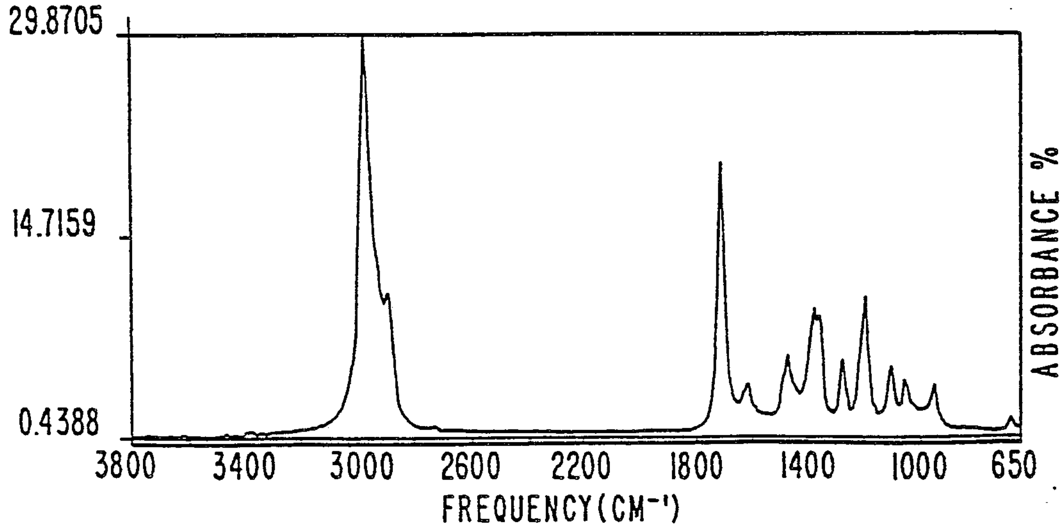


IR SPECTRUM FOR EXAMPLE III, PEAK 7 OF GLC OF FIG. 8

FIG.9F

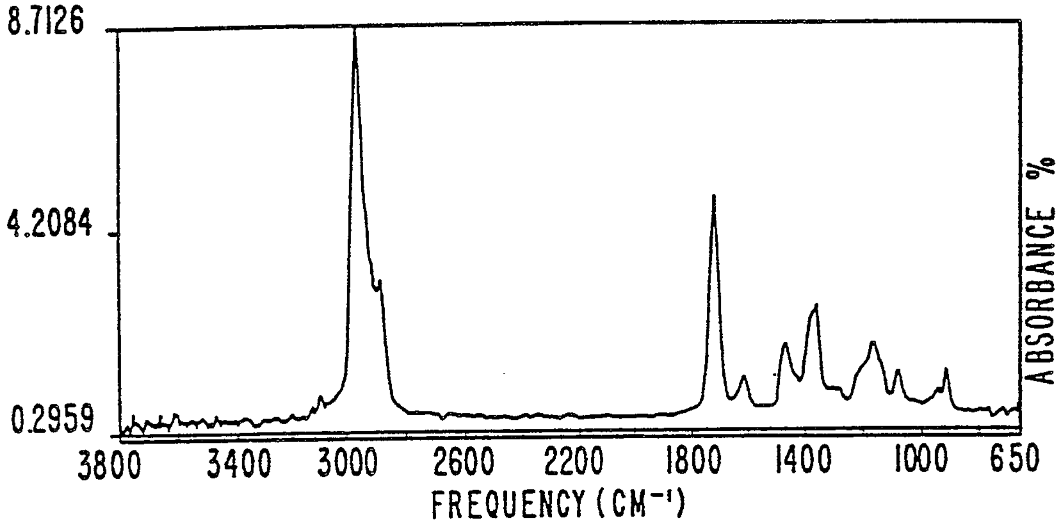


I R SPECTRUM FOR EXAMPLE III, PEAK 8 OF GLC OF FIG.8

FIG.9G

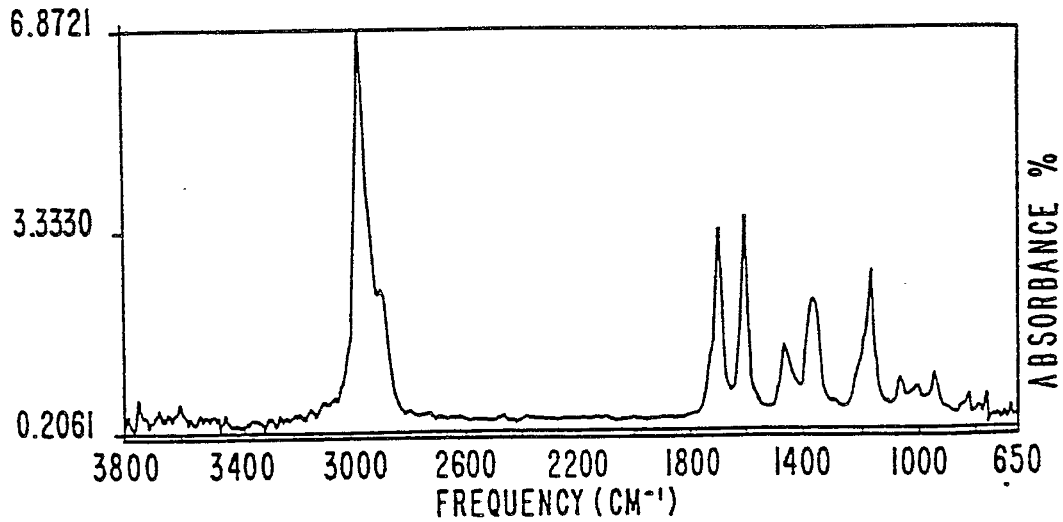


I R SPECTRUM FOR EXAMPLE III, PEAK 9 OF GLC OF FIG.8

FIG.9H

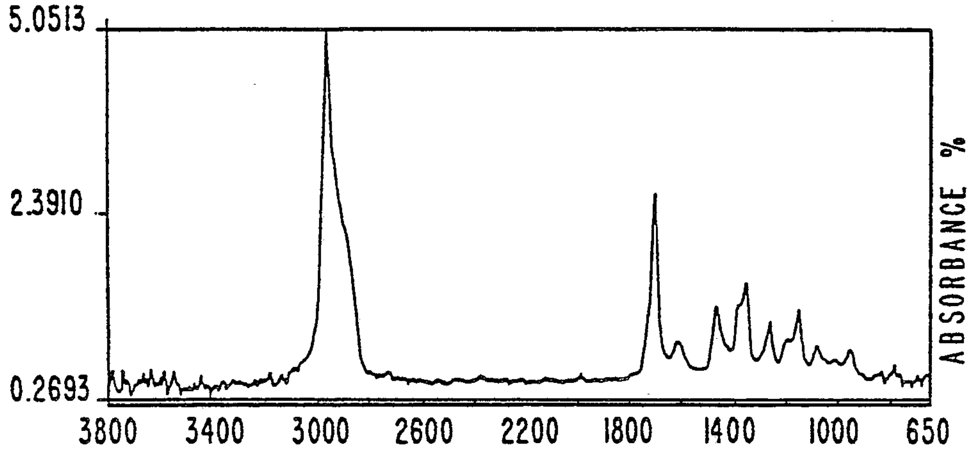


I R SPECTRUM FOR EXAMPLE III, PEAK 10 OF GLC OF FIG.8

FIG.9J

NMR SPECTRUM FOR EXAMPLE III

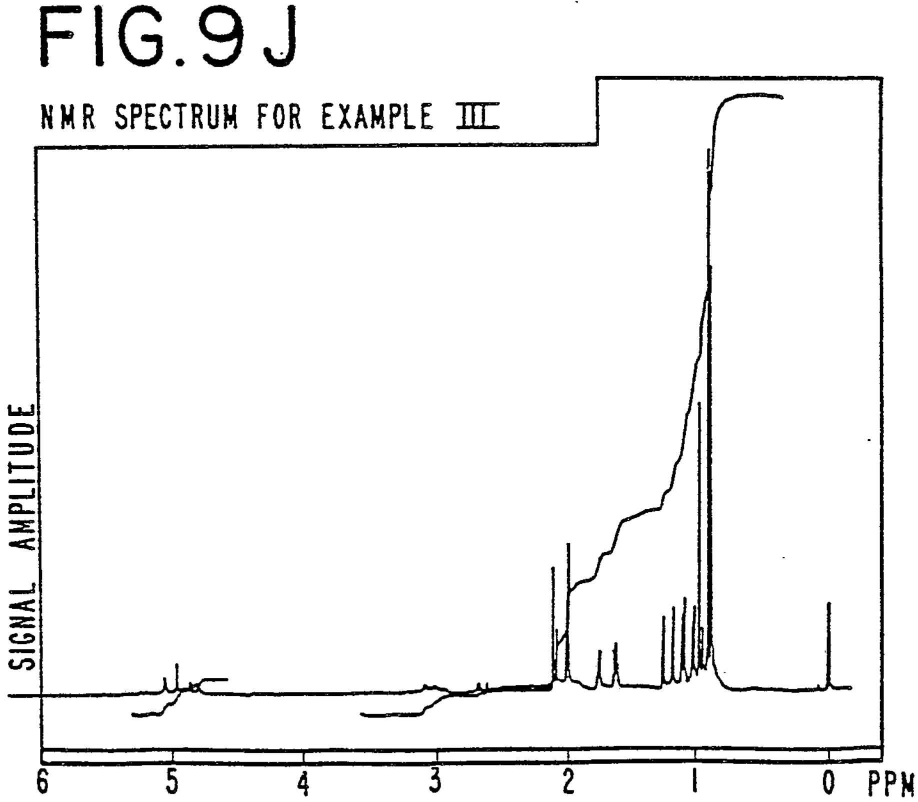

FIG.9K

N M R SPECTRUM FOR EXAMPLE III

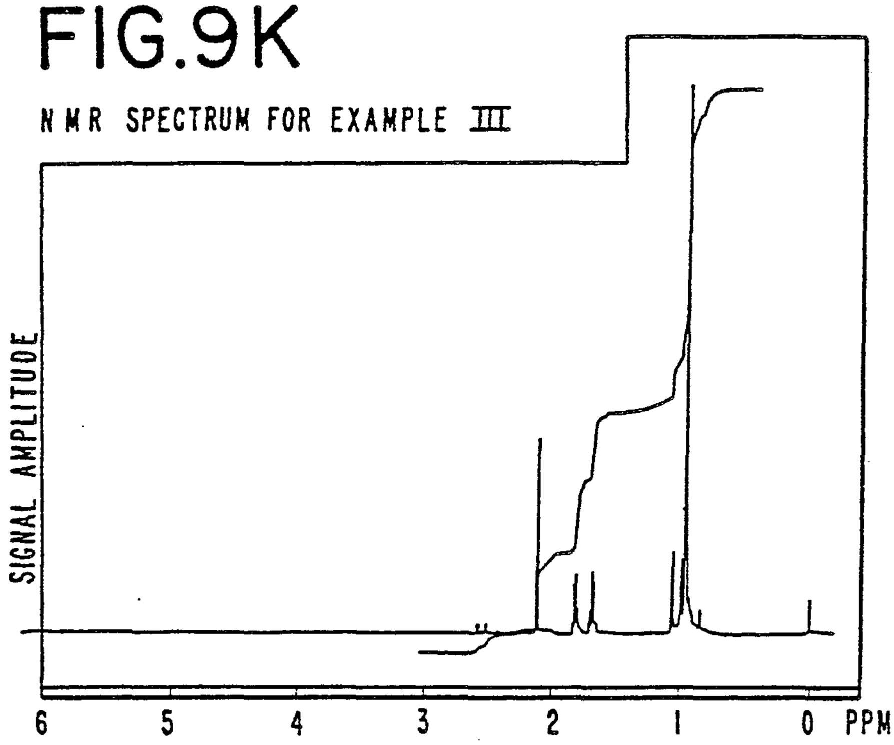


FIG.9L

N M R SPECTRUM FOR EXAMPLE III

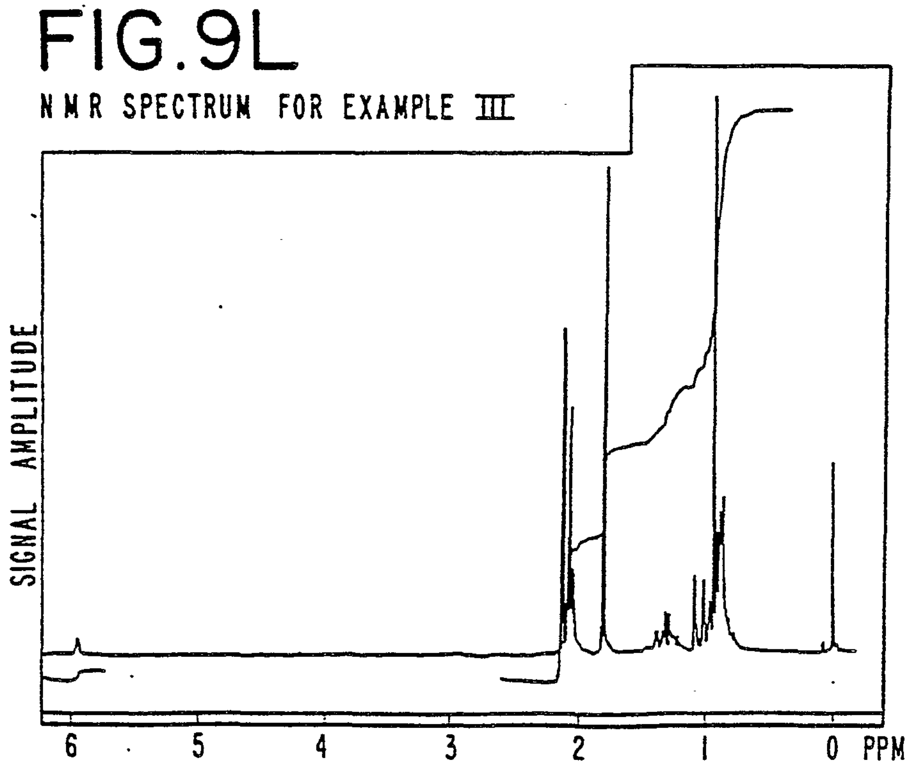

FIG.10

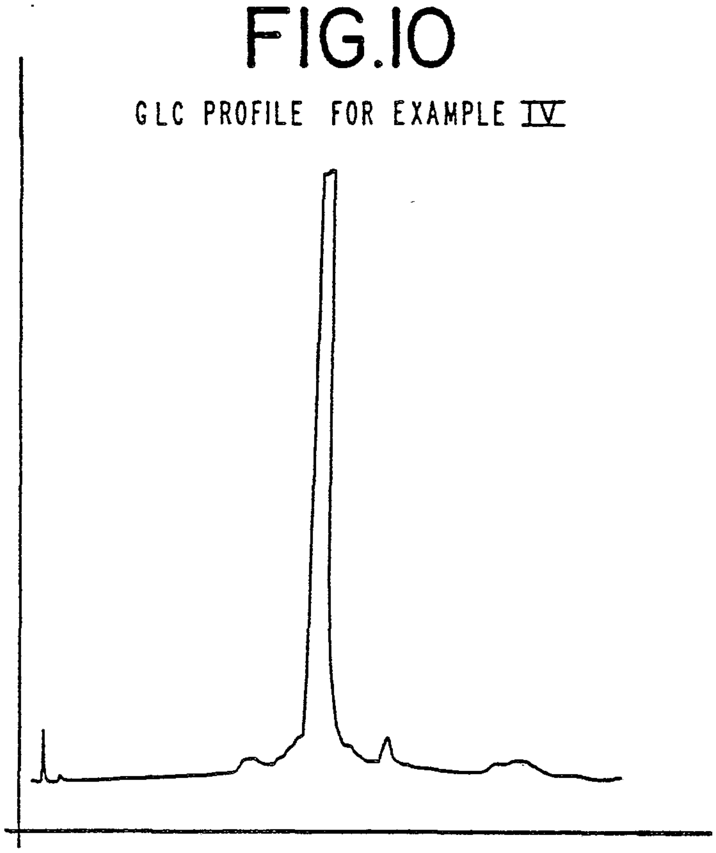


FIG.11

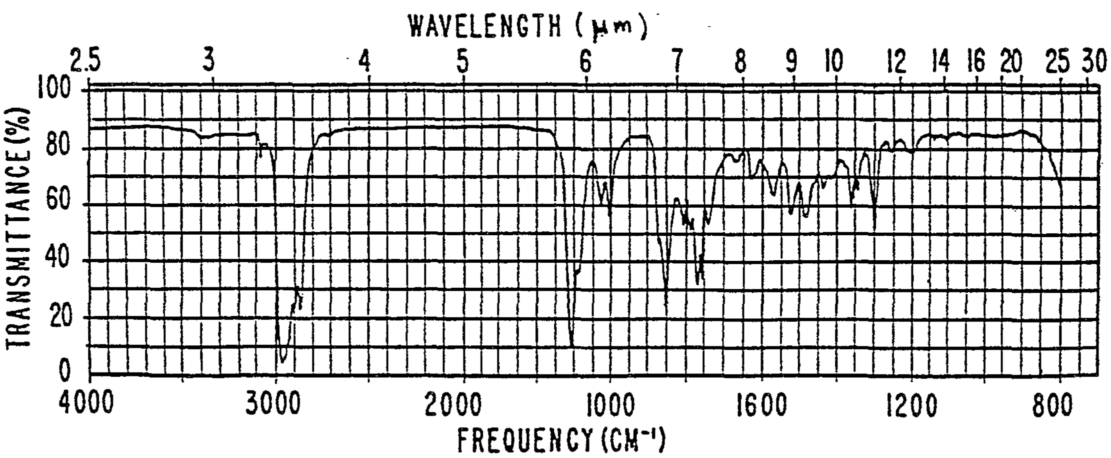


I R SPECTRUM FOR EXAMPLE IV.

FIG.12

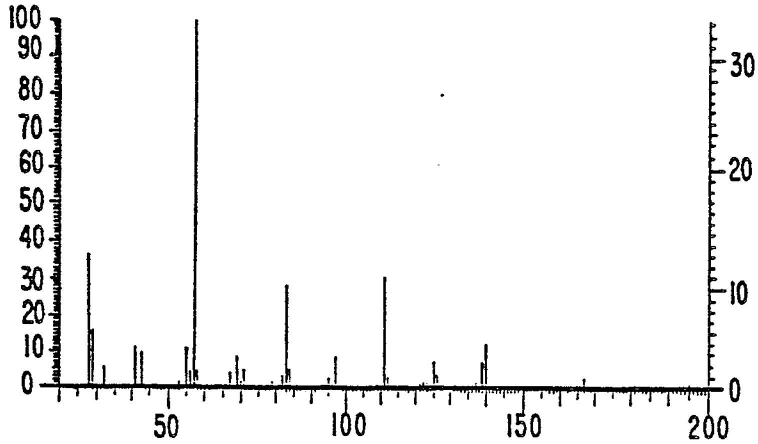


FIG.13

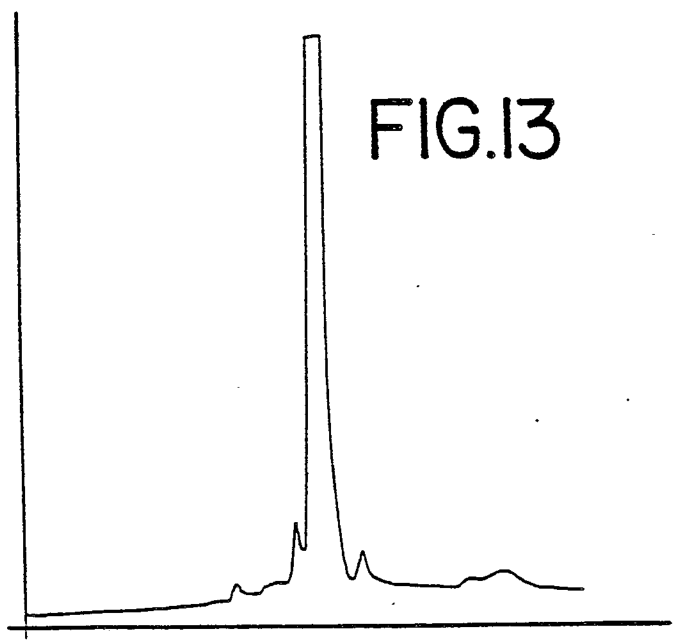

FIG.14

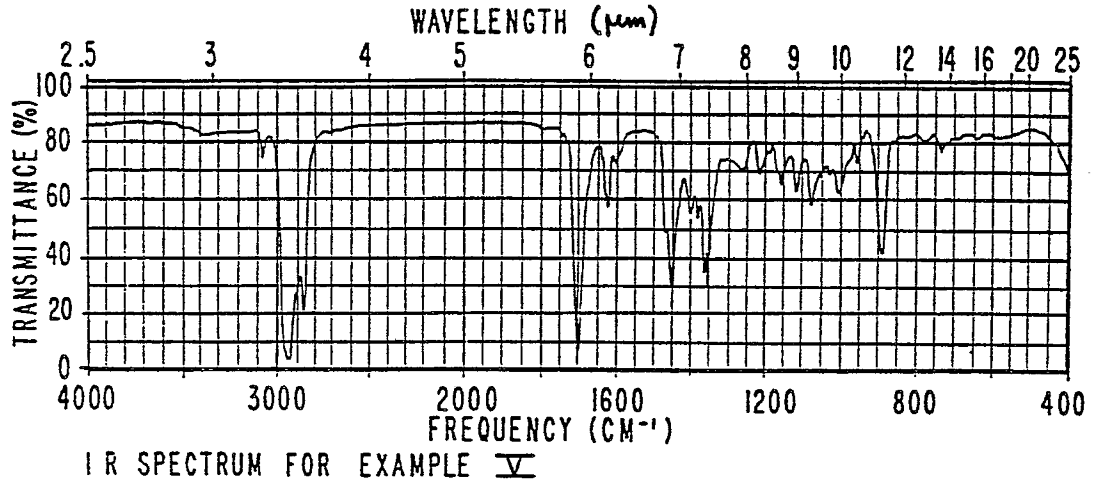


I R SPECTRUM FOR EXAMPLE V

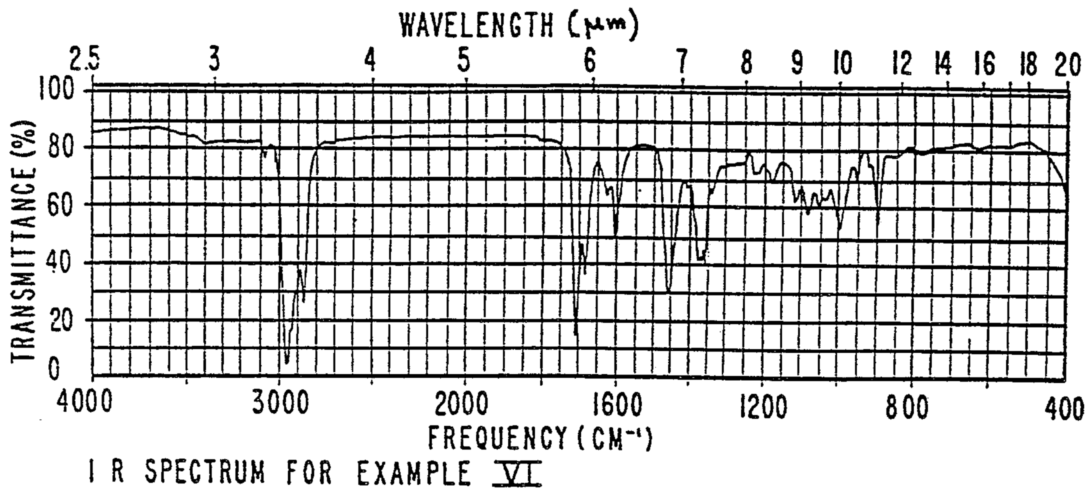


I R SPECTRUM FOR EXAMPLE VI

FIG.17

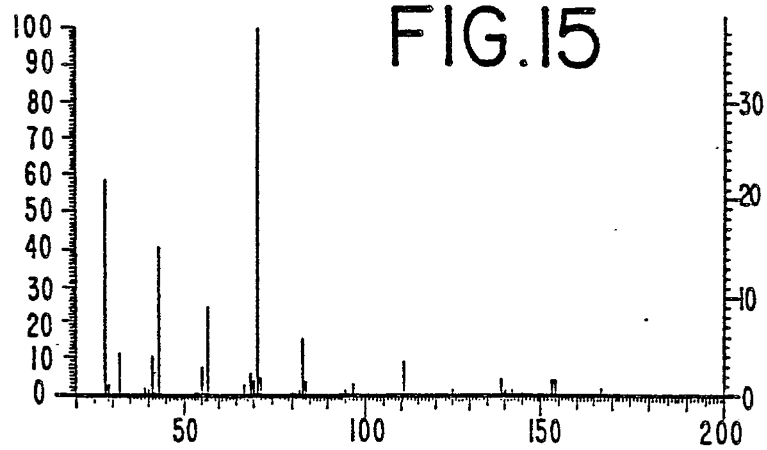


FIG.15

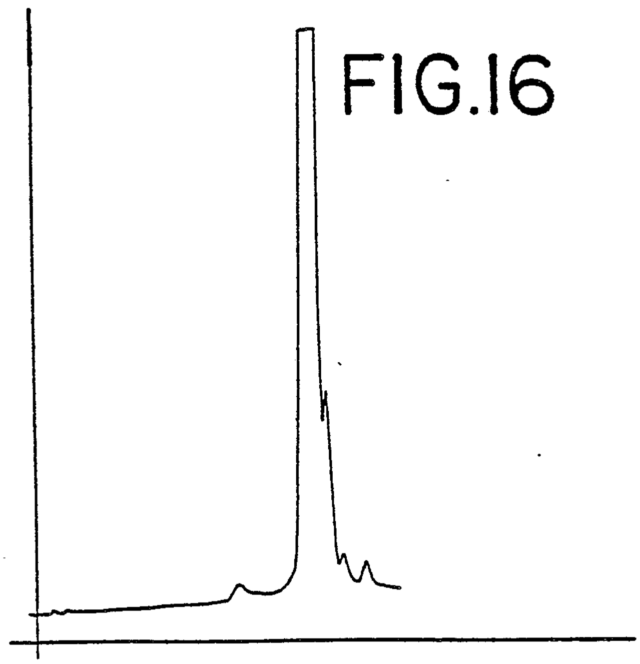

FIG.16

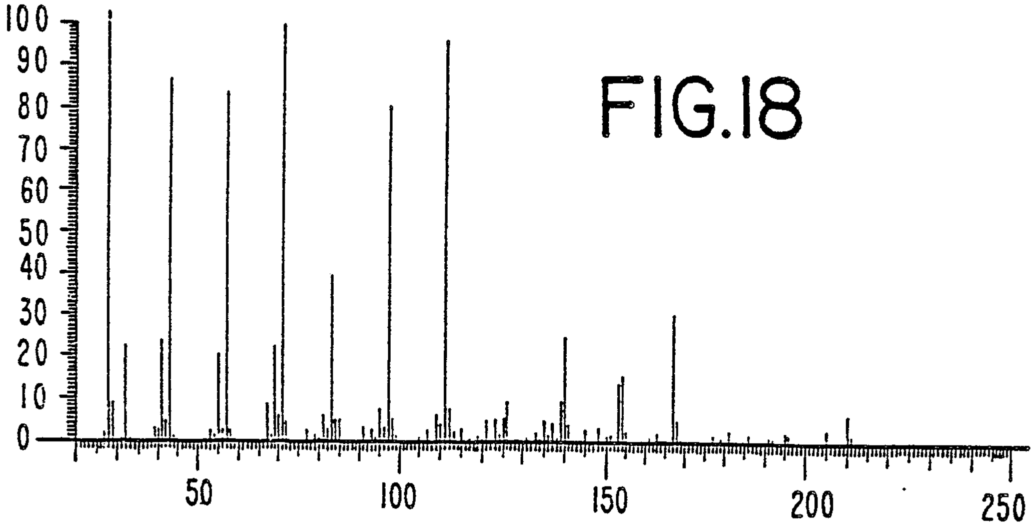


FIG.18

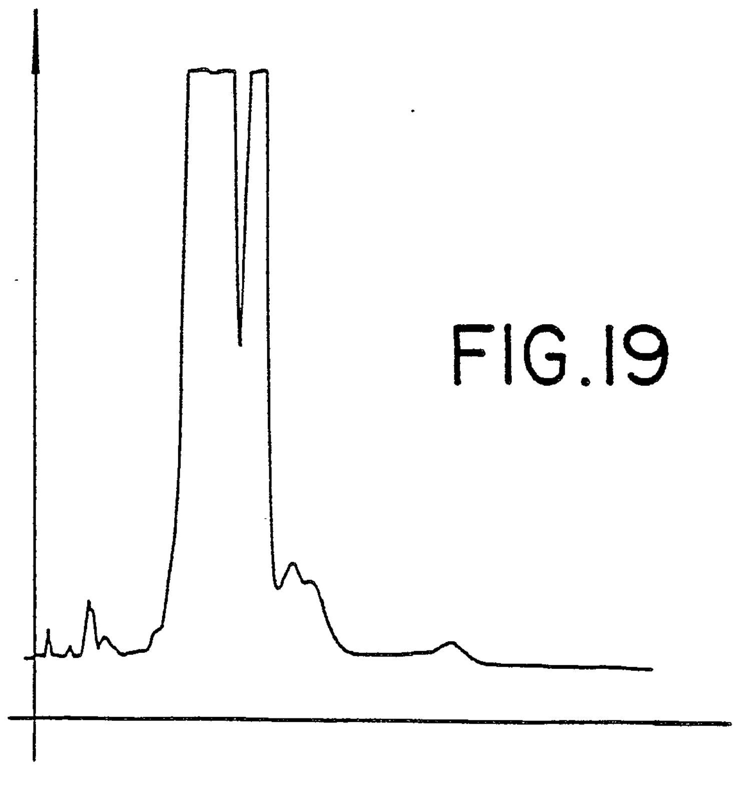

FIG.19

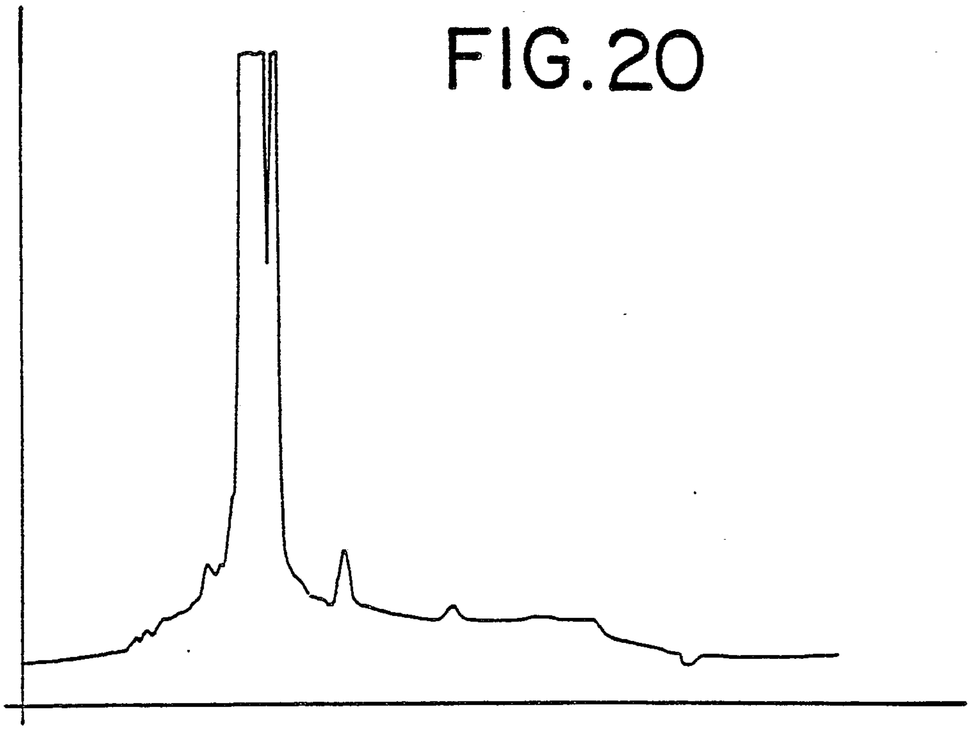

FIG. 20

FIG. 21

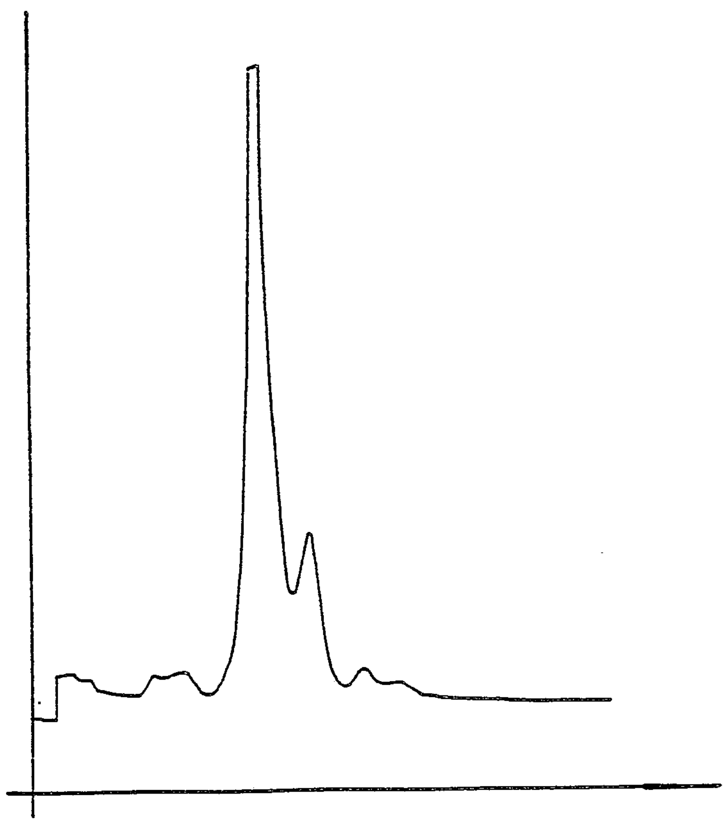

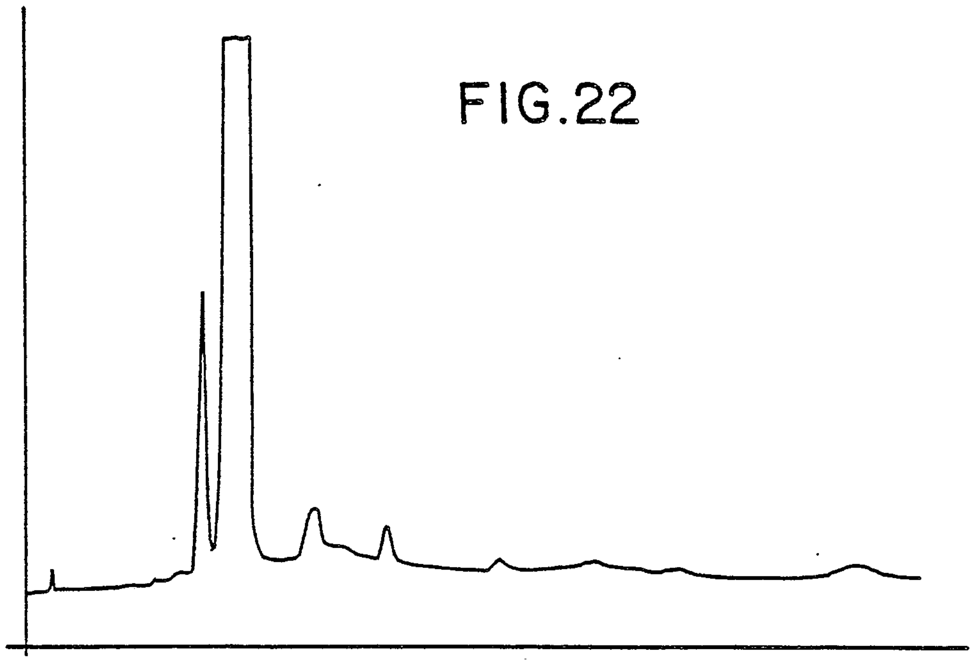

FIG.22

FIG.23

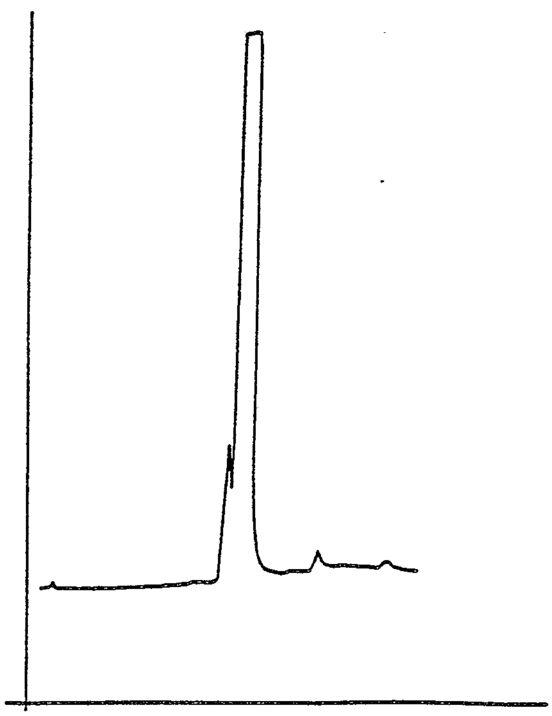

FIG. 24

NMR SPECTRUM FOR EXAMPLE X, PEAK 1 OF GLC PROFILE OF FIG. 23

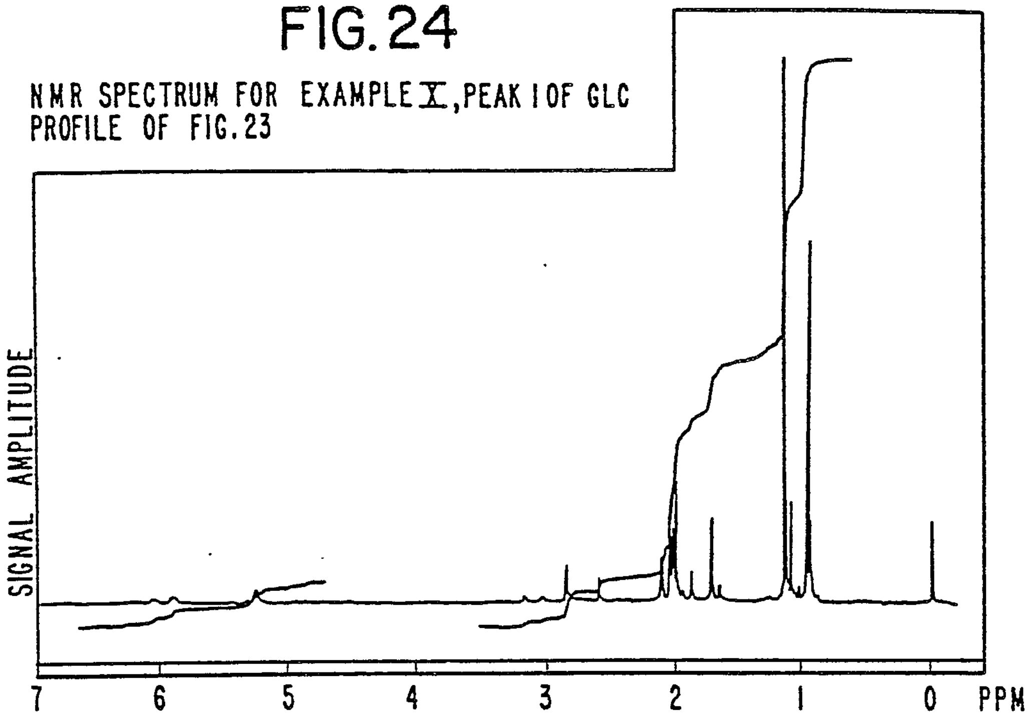


FIG. 25

NMR SPECTRUM FOR EXAMPLE X, PEAK 2 OF GLC PROFILE OF FIG. 23

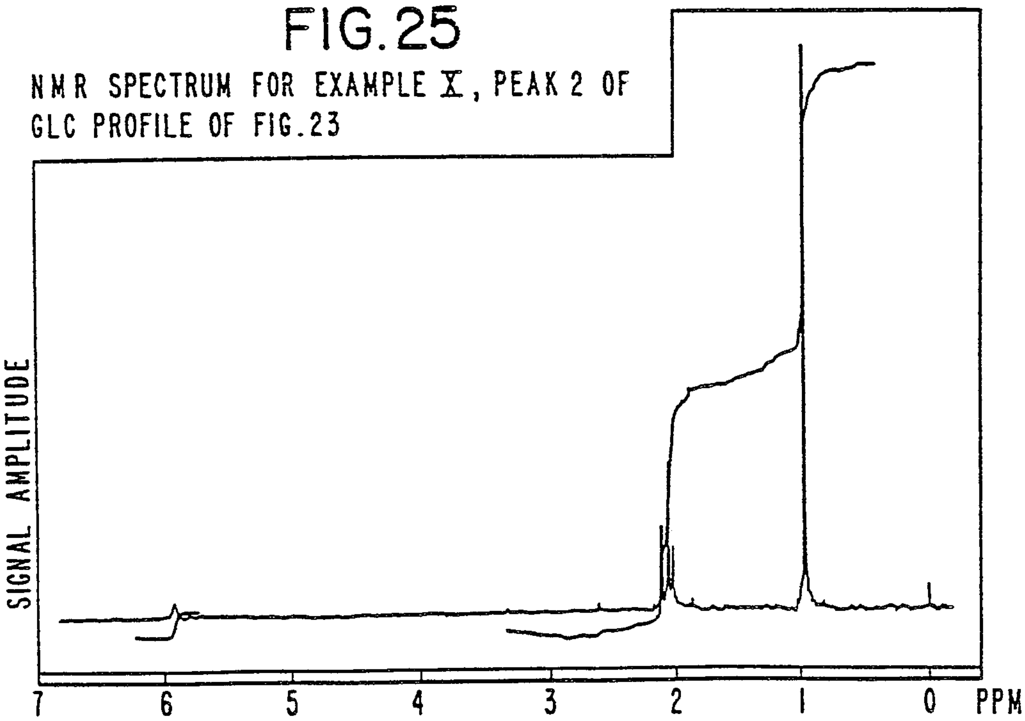

FIG.26

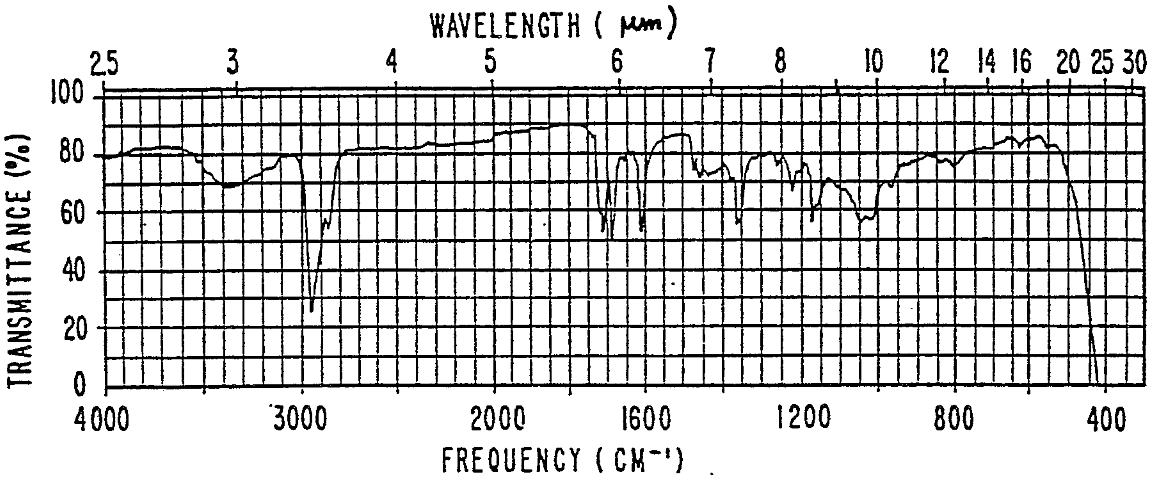


I R SPECTRUM FOR EXAMPLE X ,PEAK I OF GLC PROFILE OF FIG. 23

FIG. 27

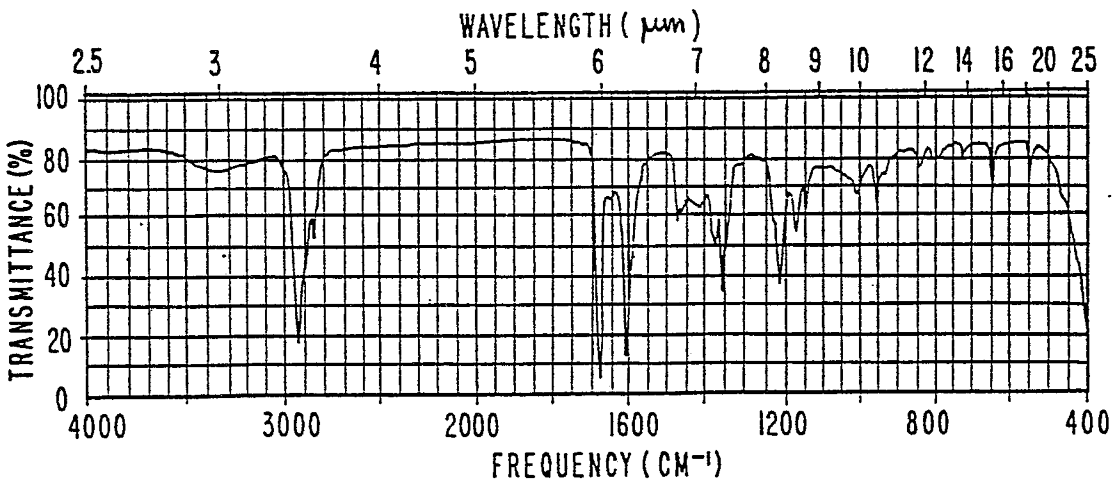


I R SPECTRUM FOR EXAMPLE X, PEAK 2 OF GLC PROFILE OF FIG.23.

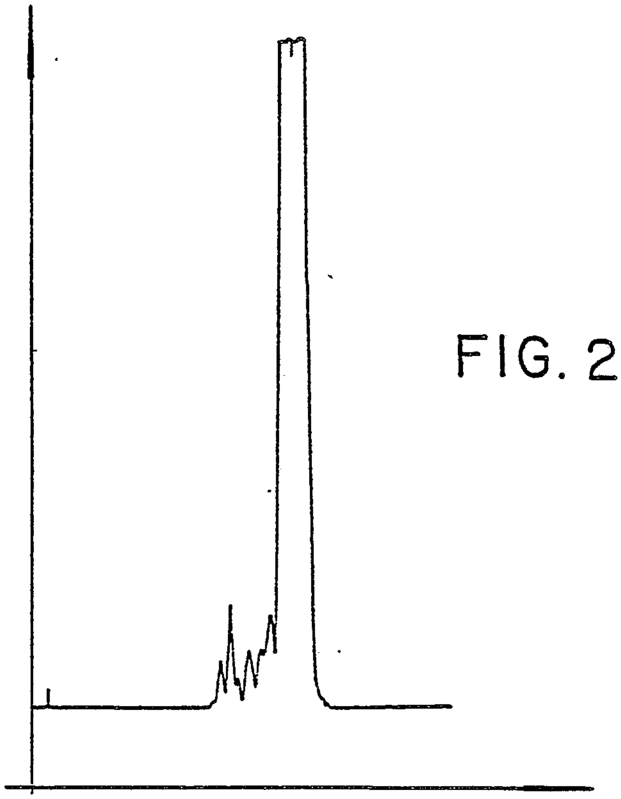

FIG. 28

FIG.29

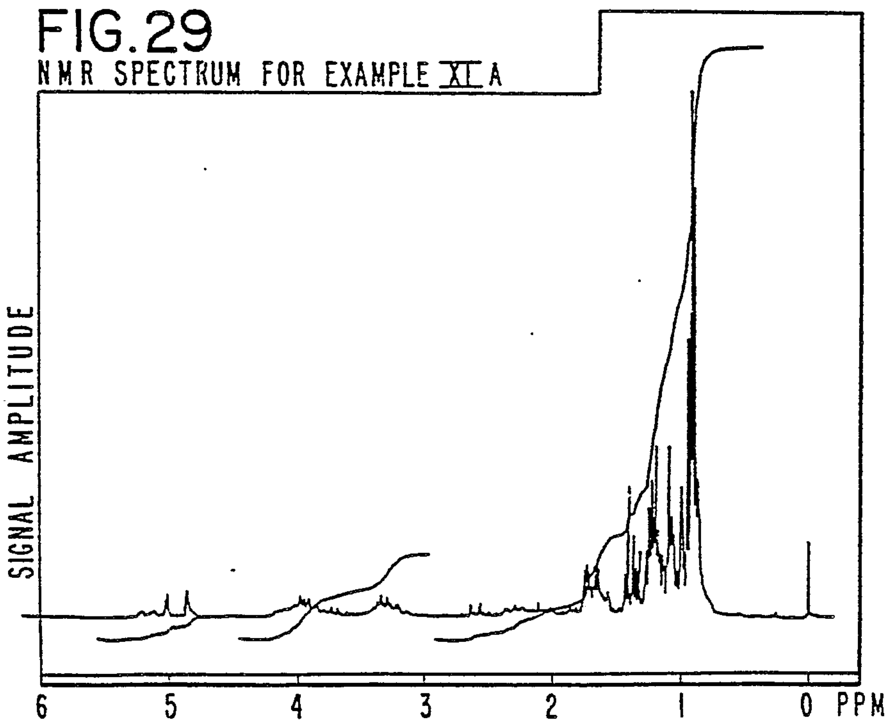

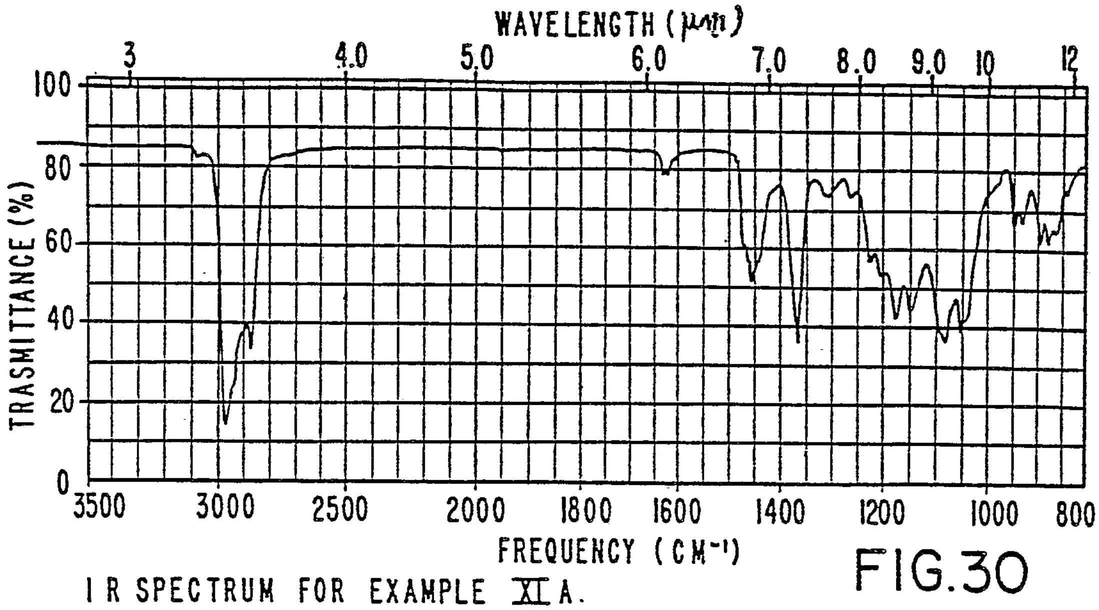


I R SPECTRUM FOR EXAMPLE XI A.

FIG.30

FIG.30A

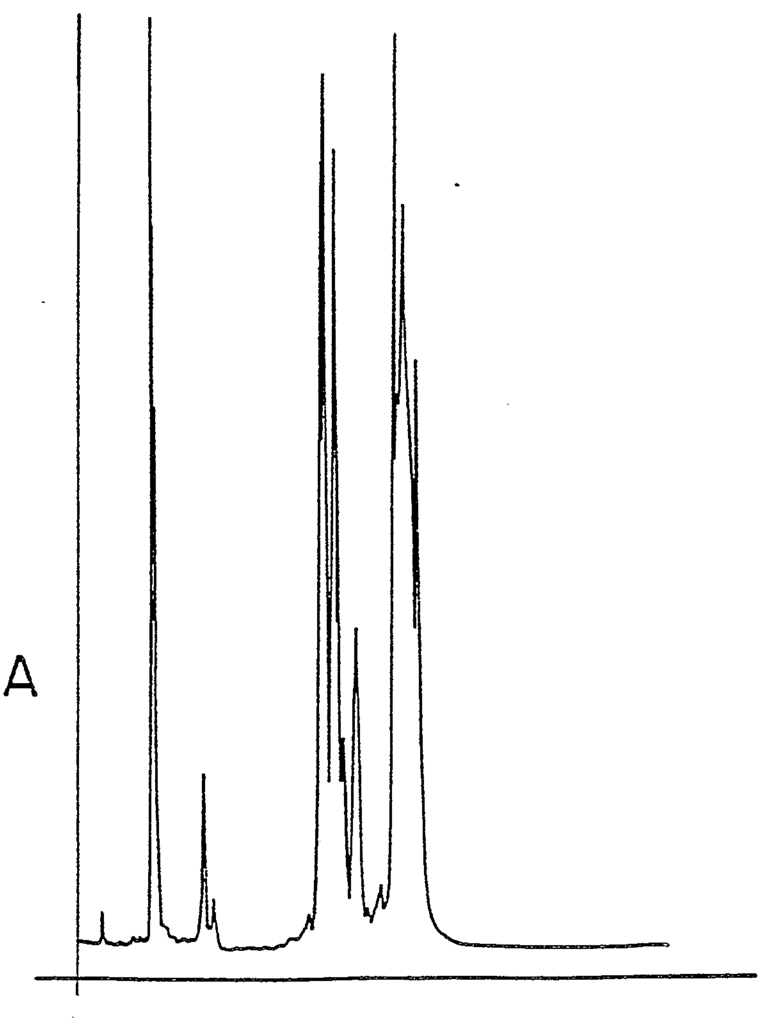

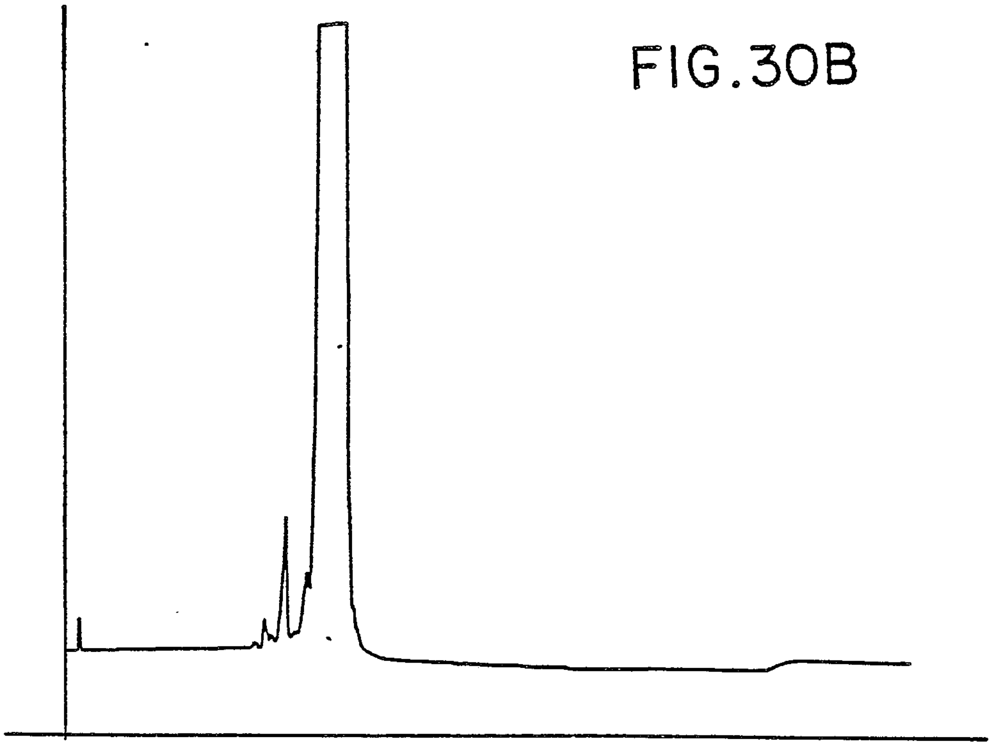

FIG. 30B

FIG. 31

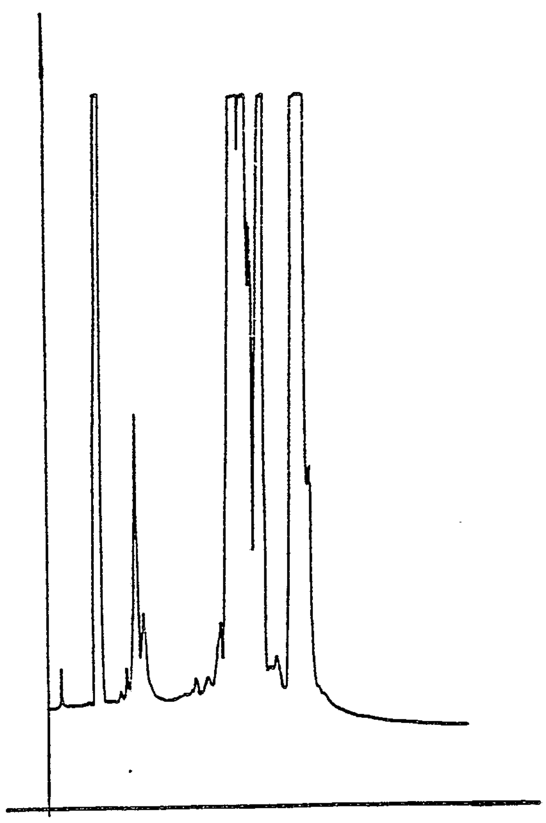

FIG. 32

GLC PROFILE FOR EXAMPLE XIII.

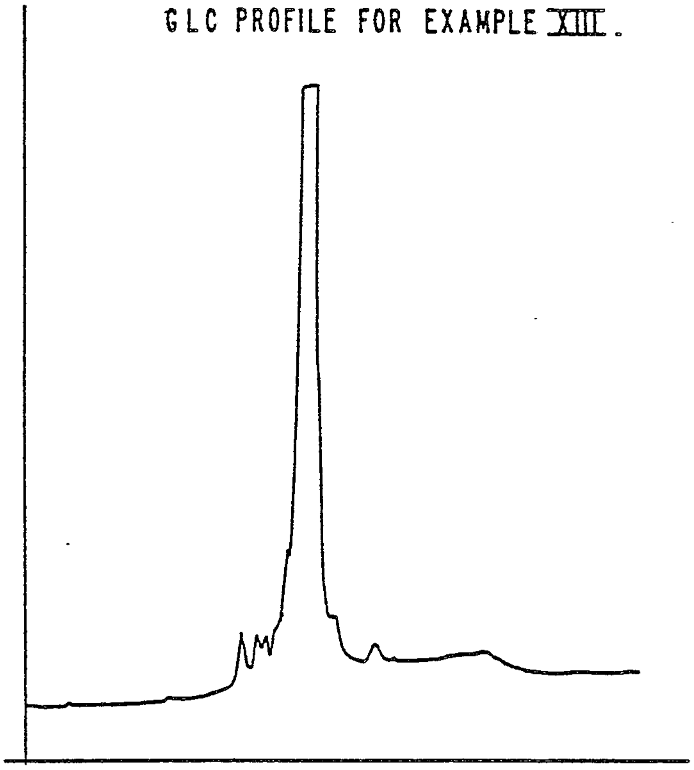

FIG. 33

N M R SPECTRUM FOR EXAMPLE XIII

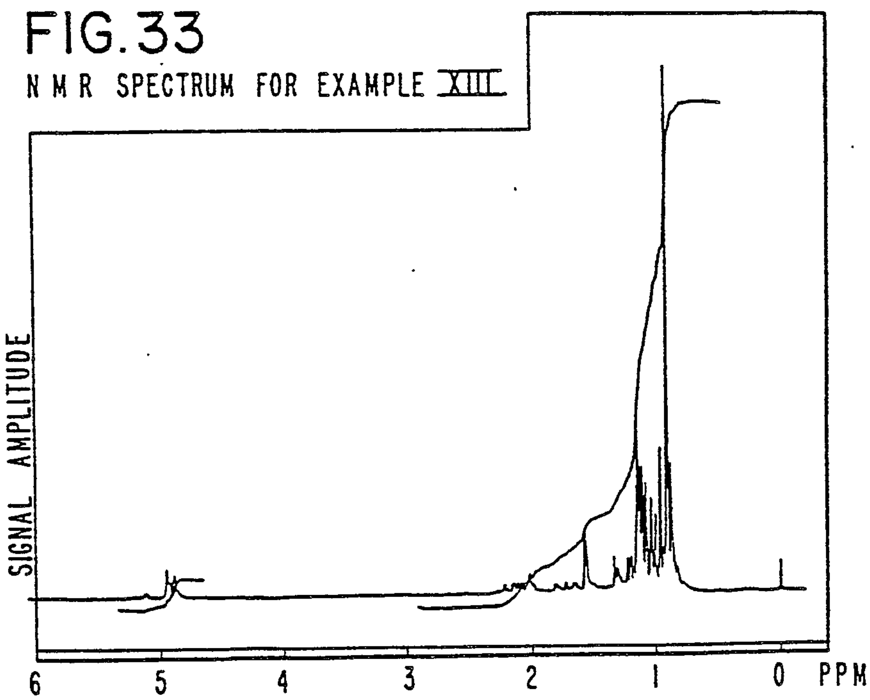

FIG.34

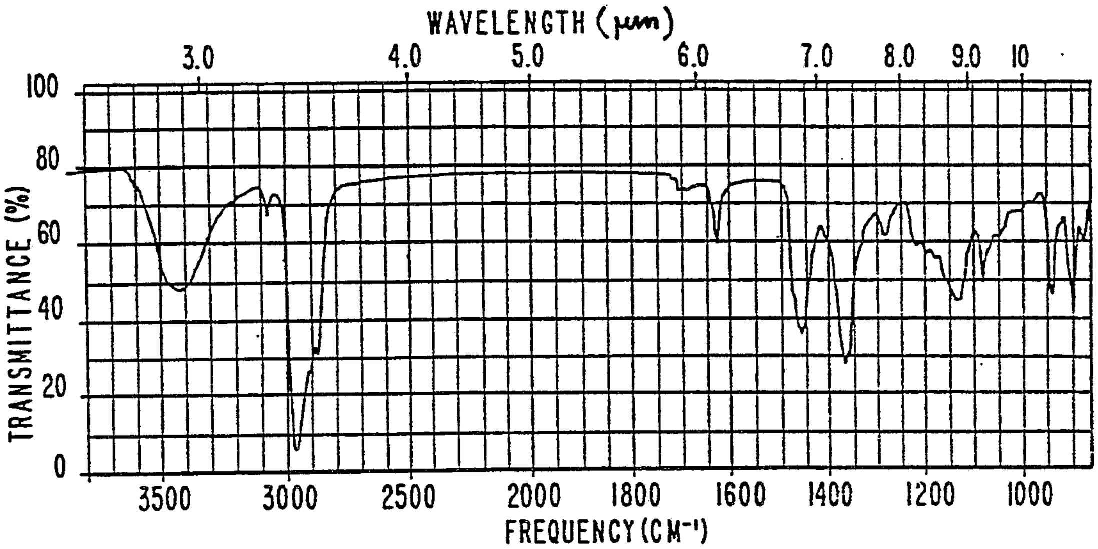


IR SPECTRUM FOR EXAMPLE XIII.

FIG.35

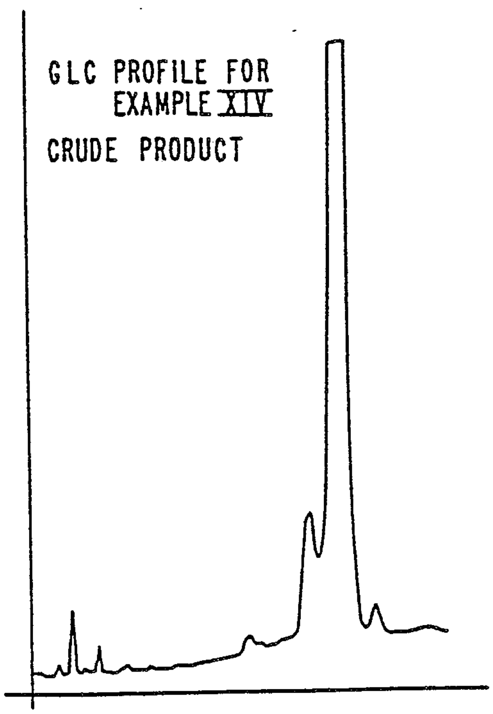


FIG.36

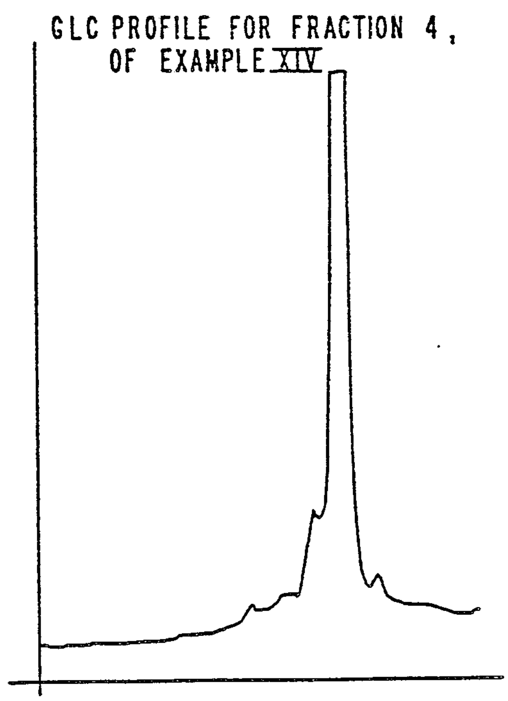

FIG.37

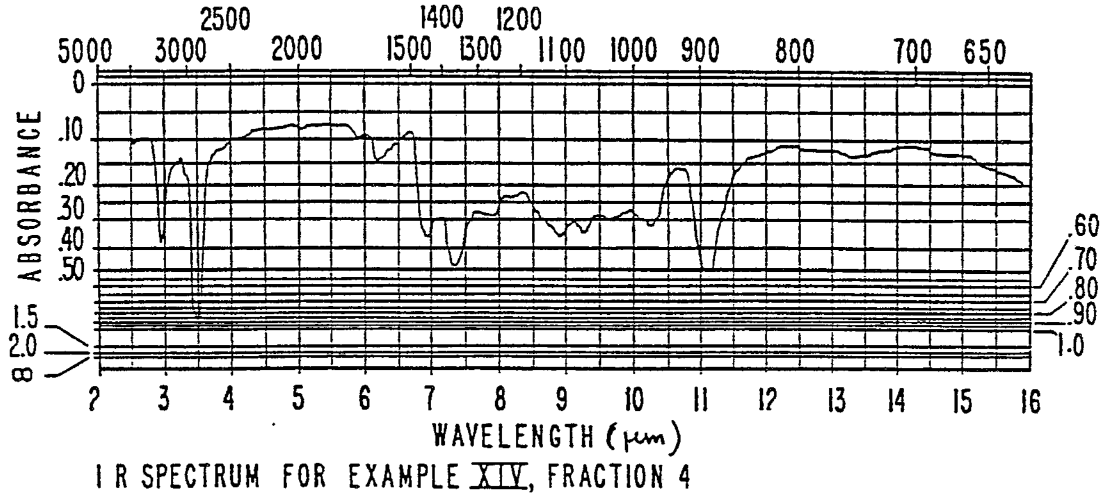


I R SPECTRUM FOR EXAMPLE XIV, FRACTION 4

FIG.38

GLC PROFILE FOR EXAMPLE XV. CRUDE PRODUCT.

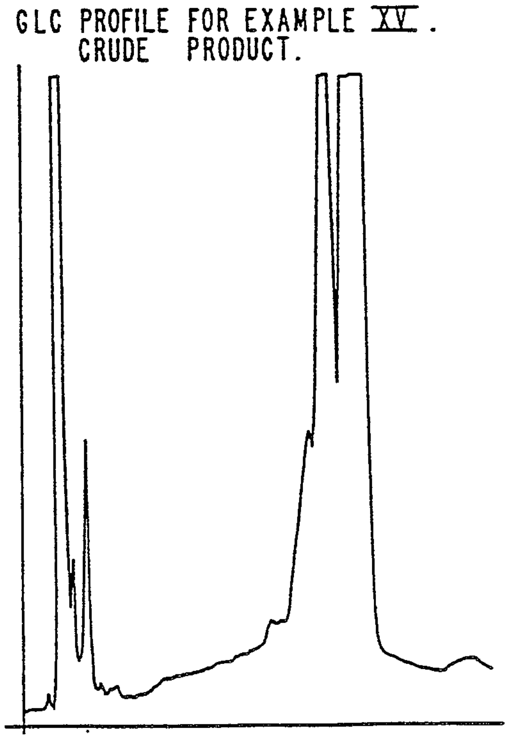

FIG.39

GLC PROFILE FOR FRACTION 7, OF EXAMPLE XV.

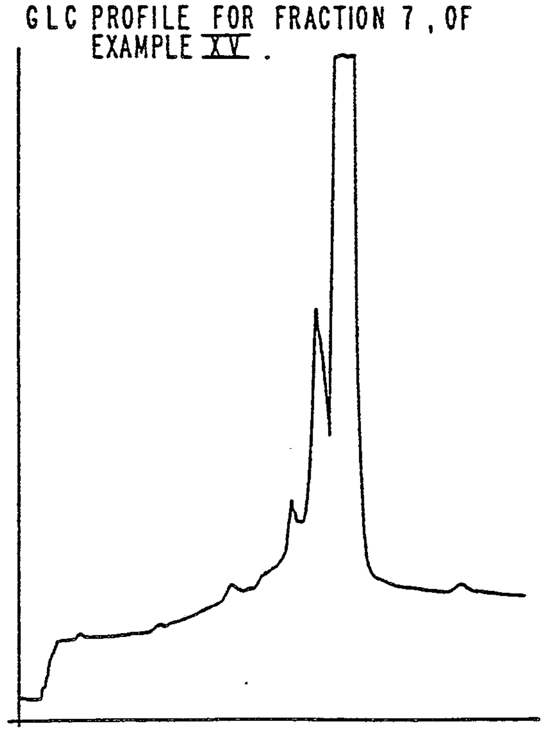

FIG.40

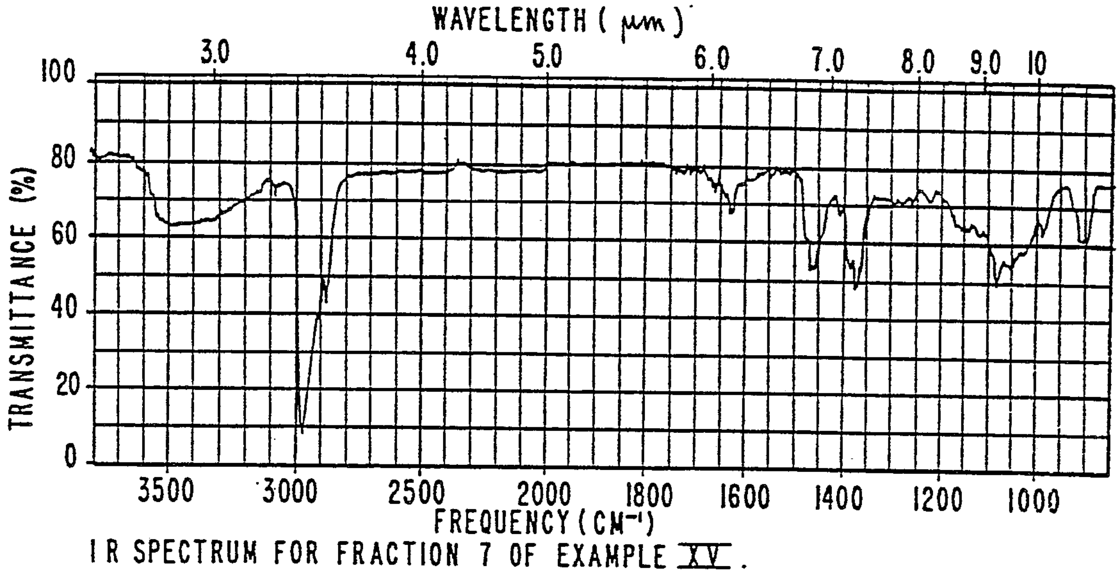


IR SPECTRUM FOR FRACTION 7 OF EXAMPLE XV.

FIG.41

NMR SPECTRUM FOR EXAMPLE XV
FRACTION 7.

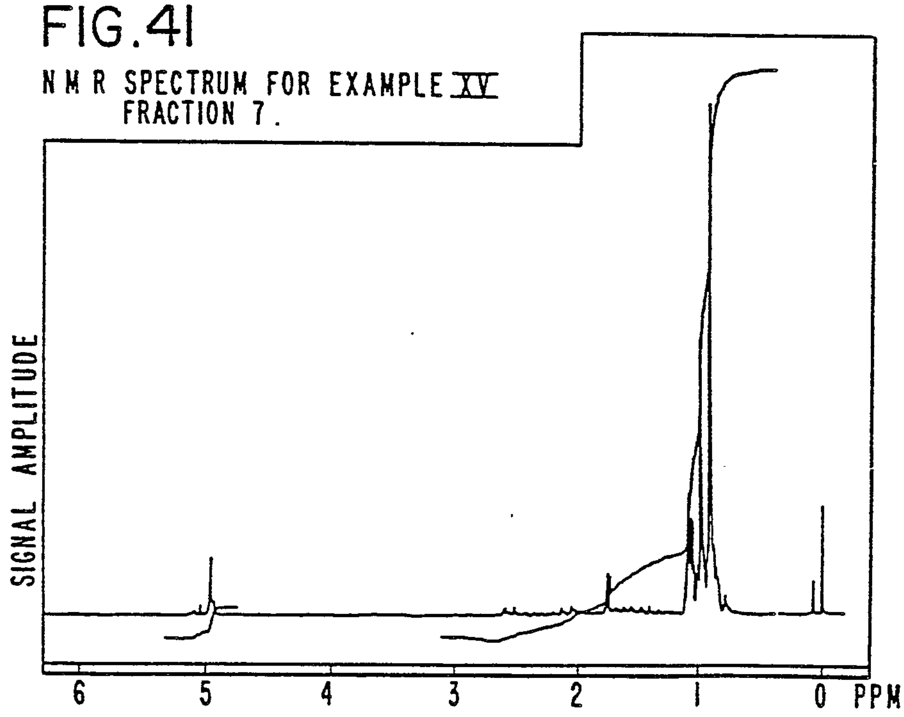

FIG.43

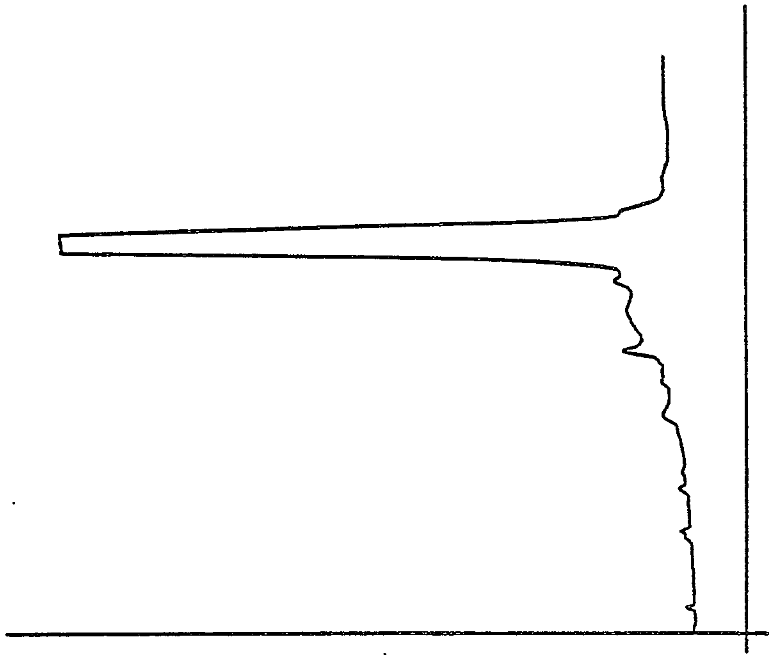

FIG.42

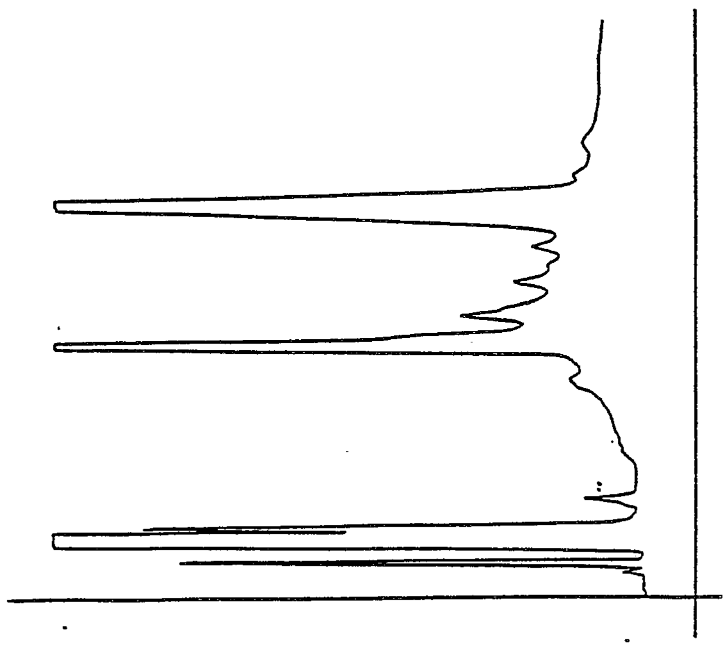

FIG.44

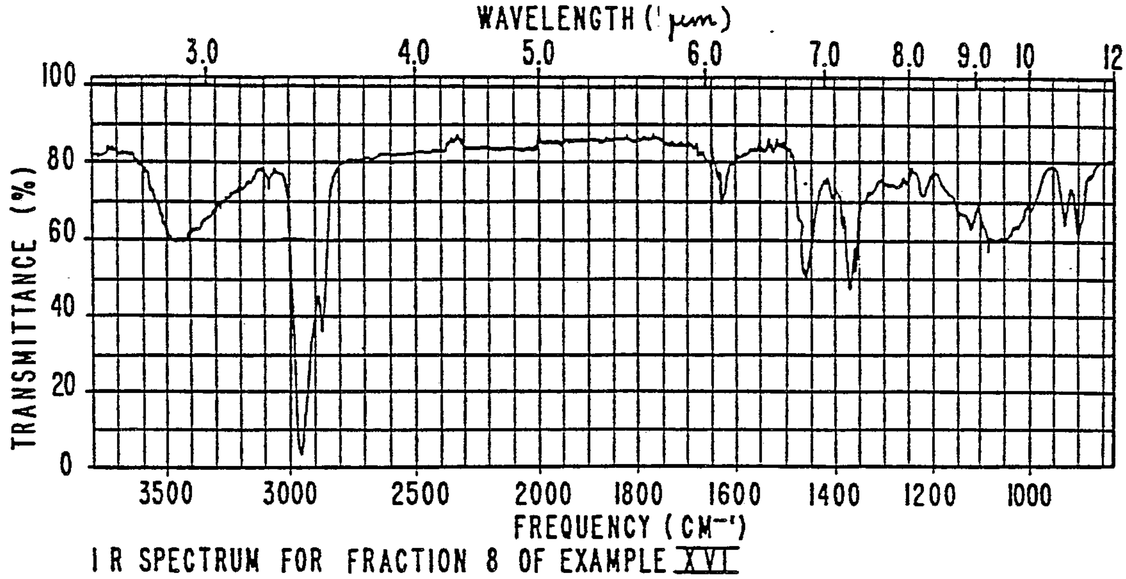


IR SPECTRUM FOR FRACTION 8 OF EXAMPLE XVI

FIG.45

NMR SPECTRUM FOR FRACTION 8
OF EXAMPLE XVI

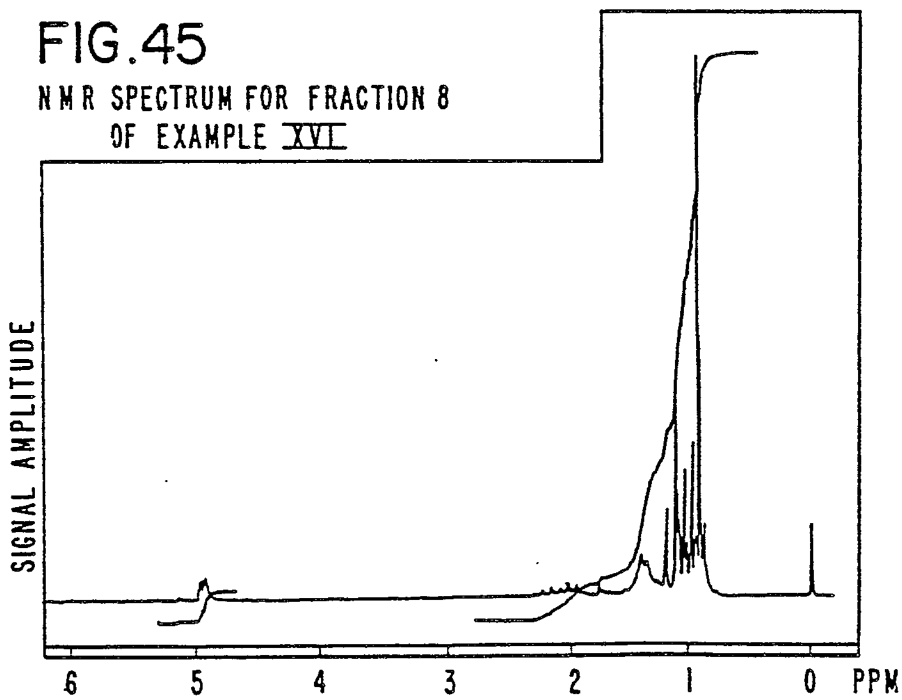

FIG.46

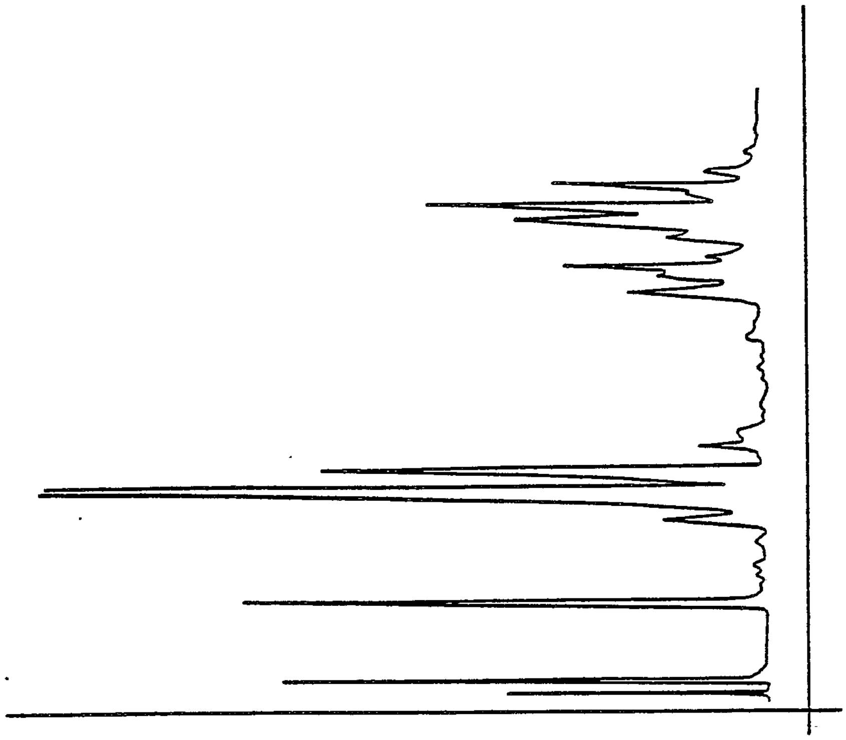

FIG.47

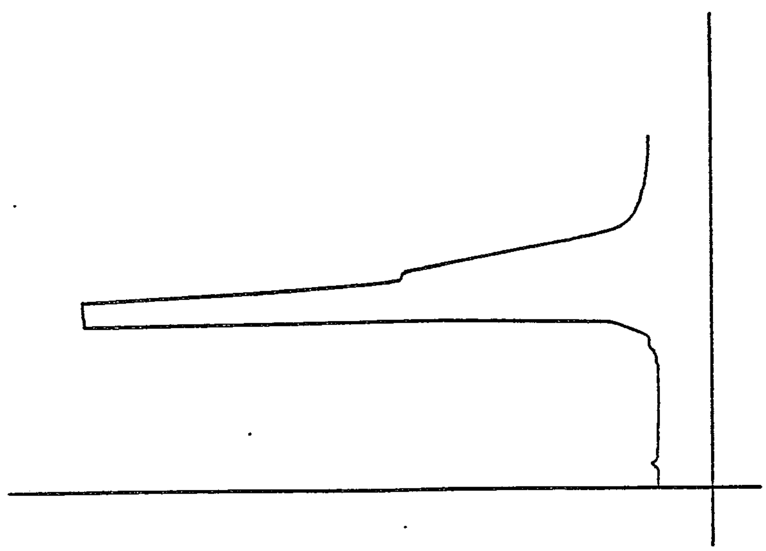

FIG. 48

N M R SPECTRUM FOR EXAMPLE XVII, DIISOUNDECANAL.

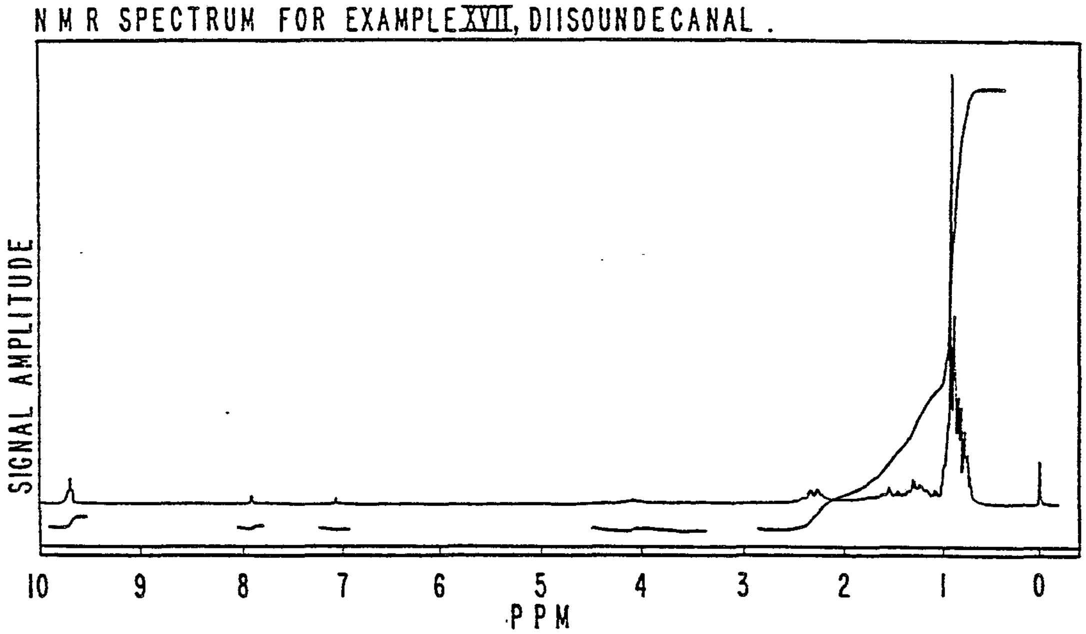


FIG. 49

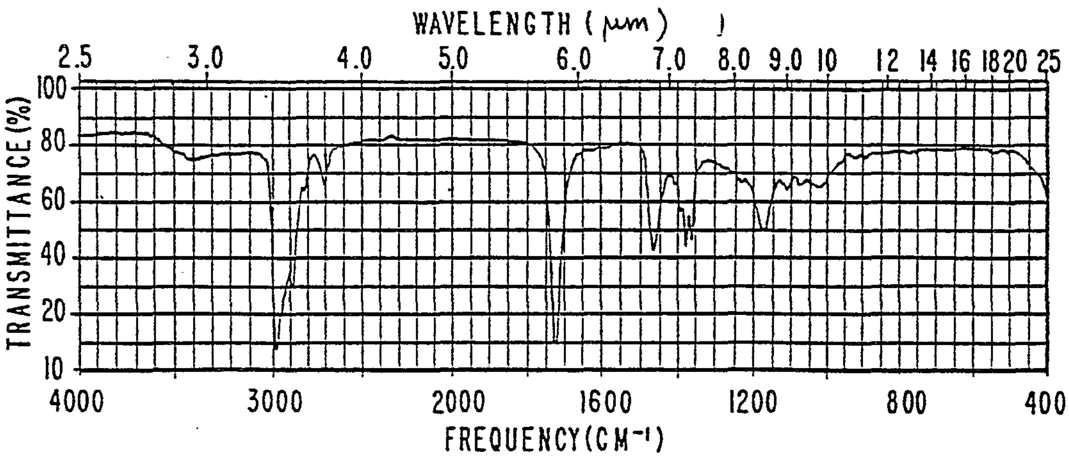


I R SPECTRUM FOR EXAMPLE XVII, DIISOUNDECANAL.

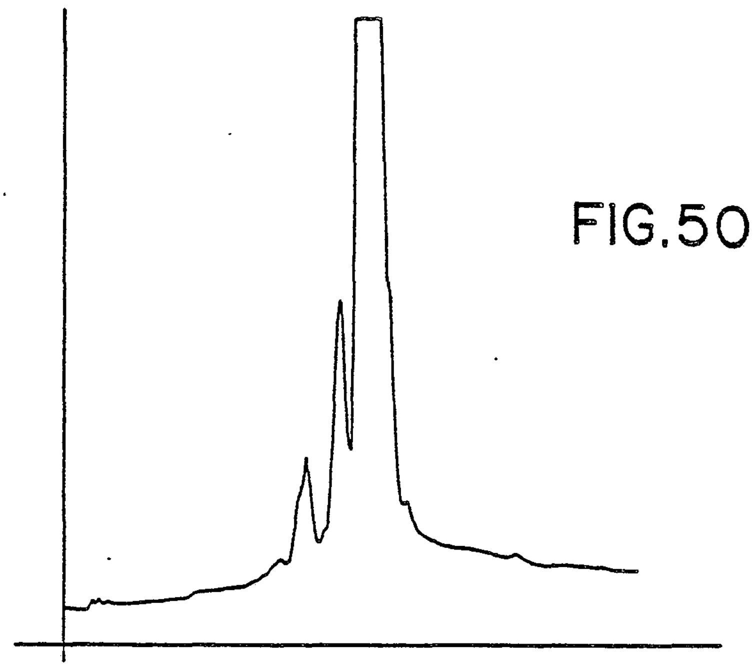

FIG.50

N M R SPECTRUM FOR EXAMPLE XVII, DIISOUNDECANOL

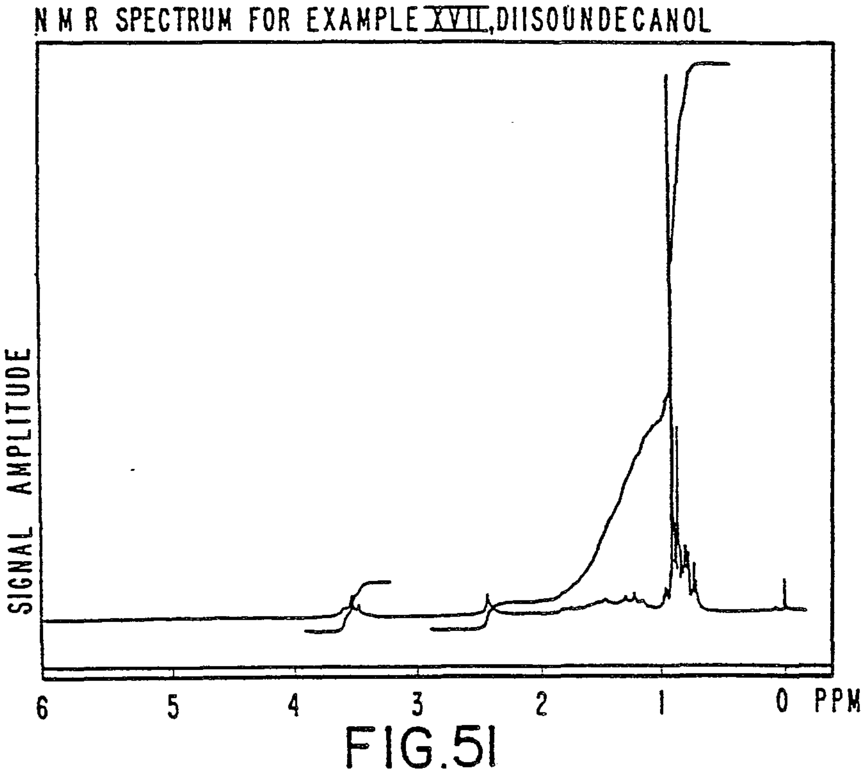


FIG.51

FIG. 52

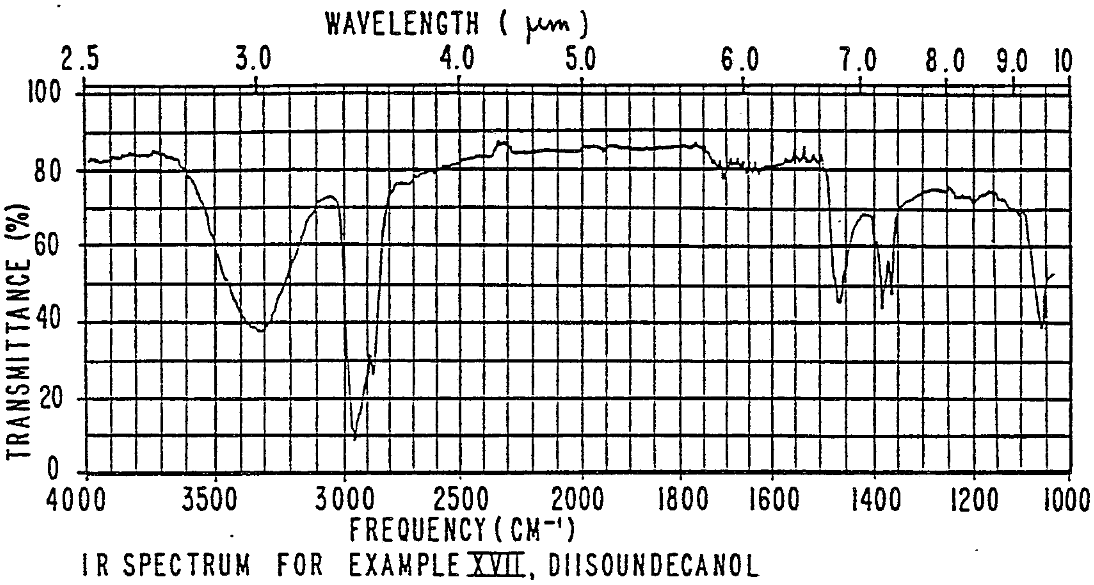


IR SPECTRUM FOR EXAMPLE XVII, DIISOUNDECANOL

FIG. 53A

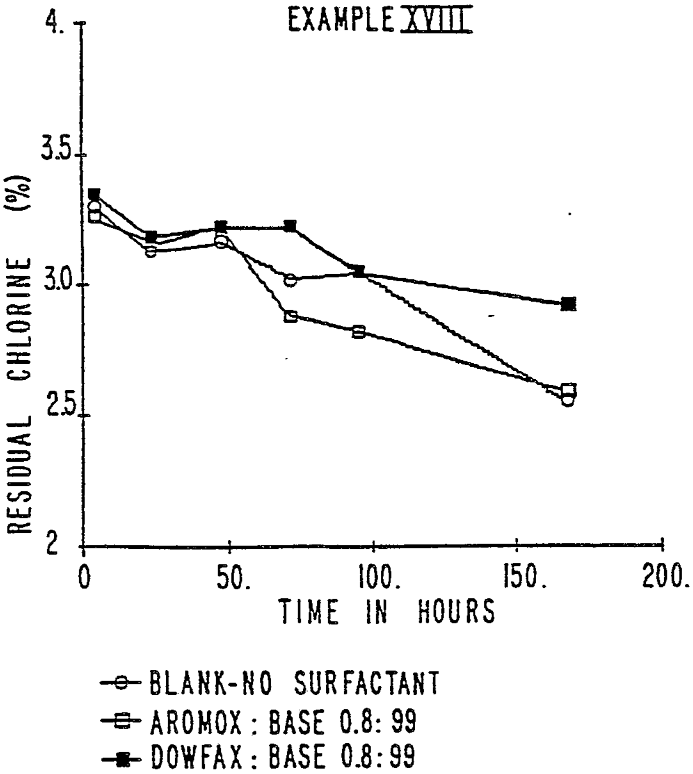

FIG.53B
EXAMPLE XVIII

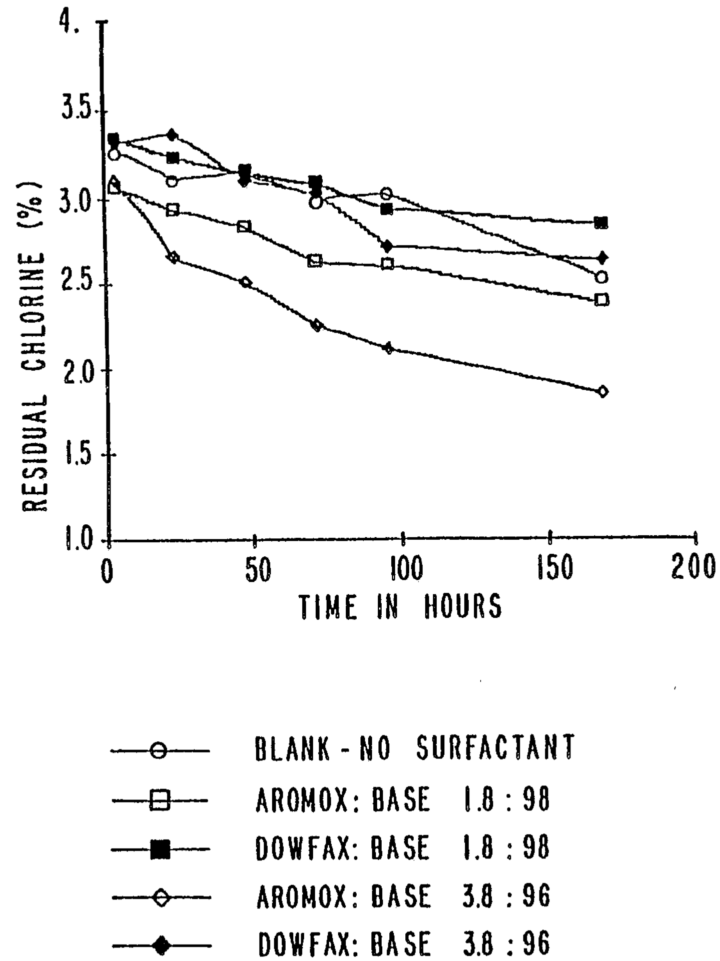


FIG.54A
EXAMPLE XVIII.

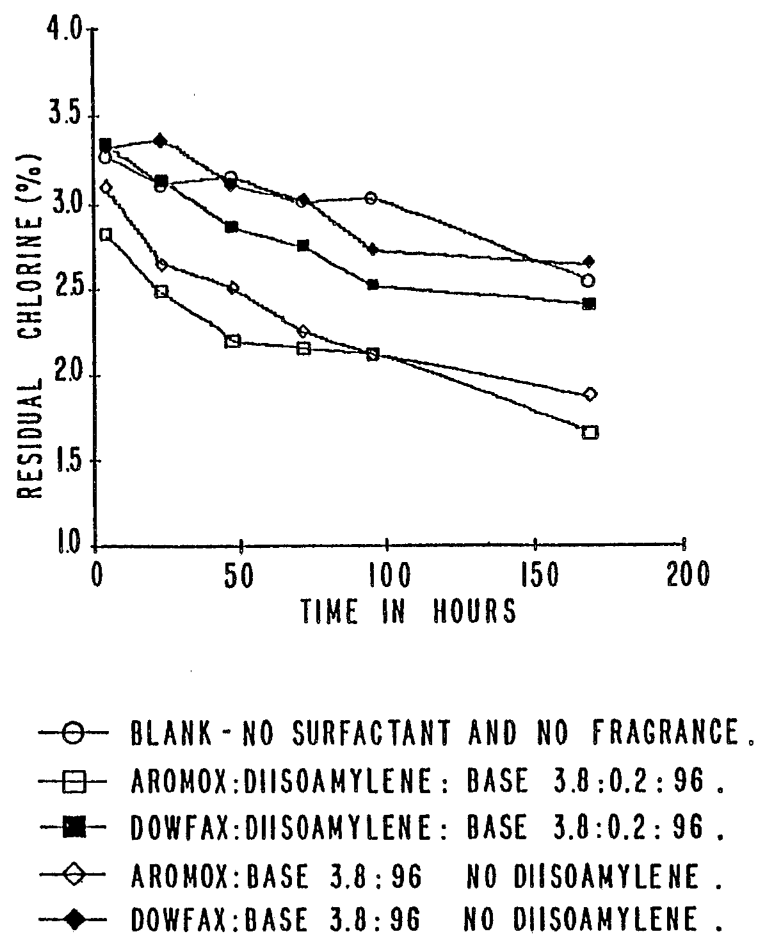

FIG.54B

EXAMPLE XVIII

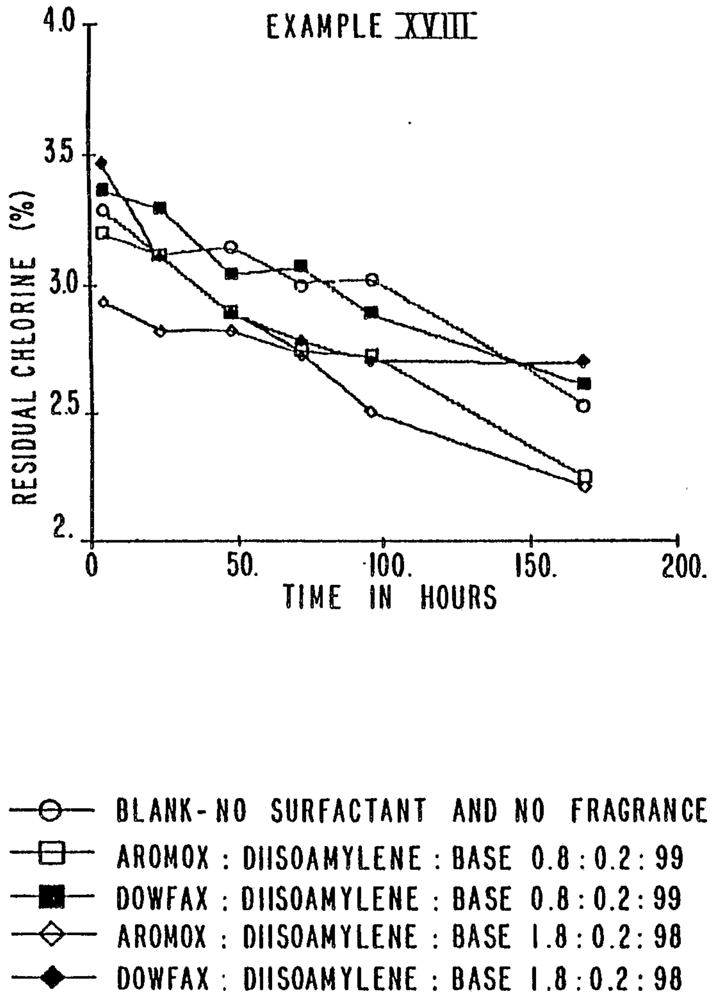


FIG.55A

EXAMPLE XVIII

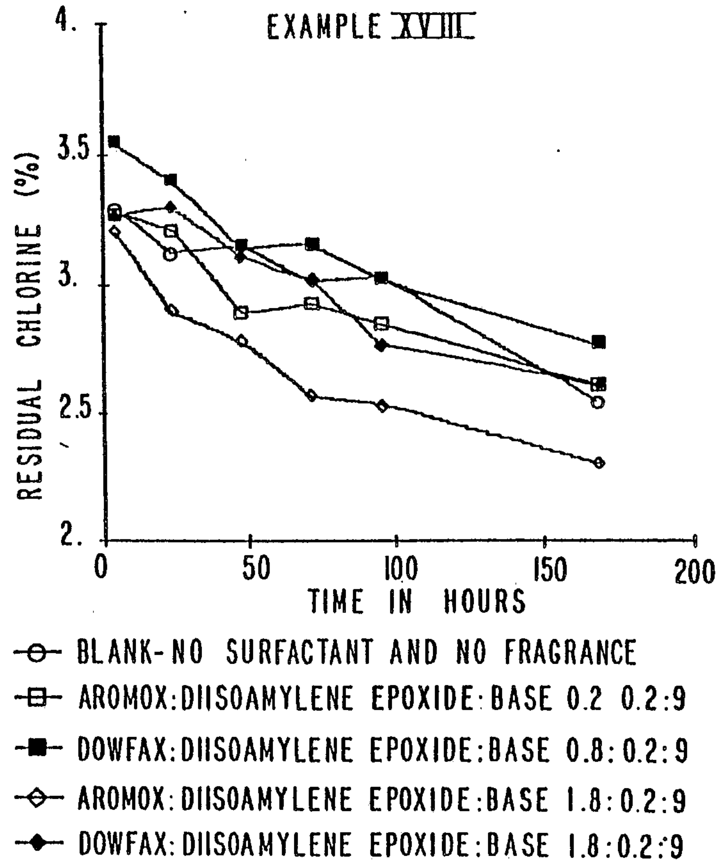

FIG.55 B
EXAMPLE XVIII.

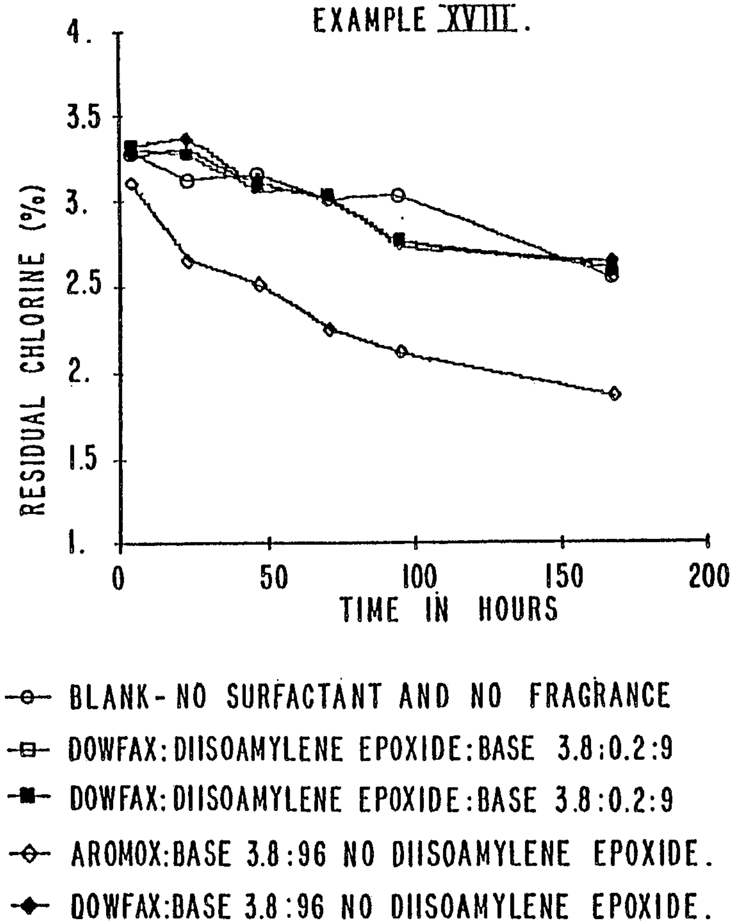


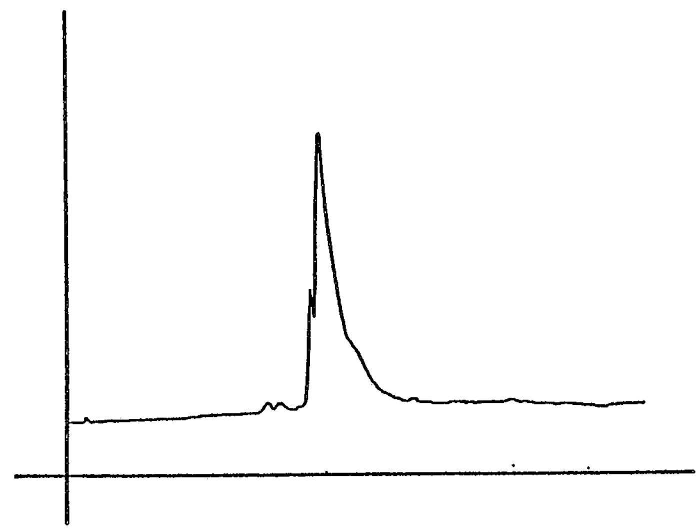

FIG.56

GLC PROFILE FOR EXAMPLE XIX

FIG.57

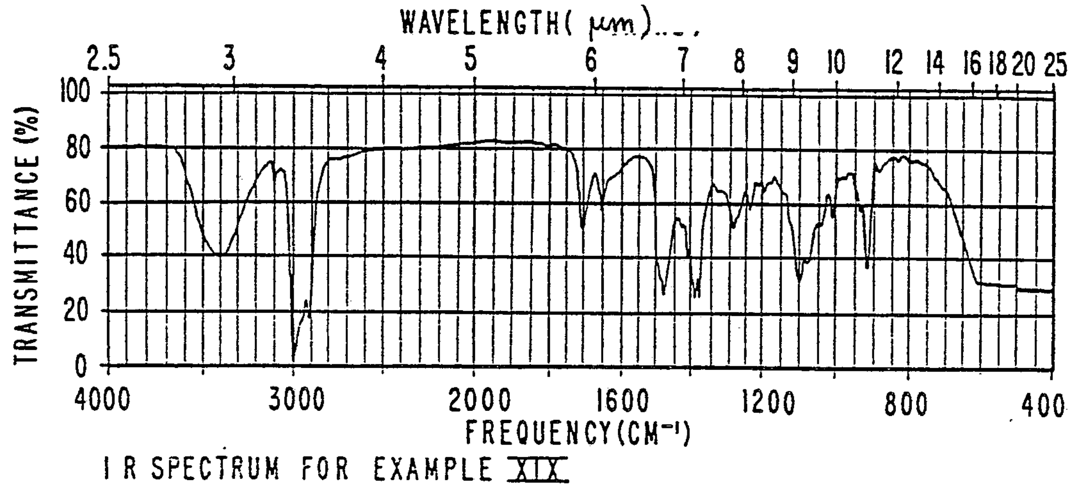


I R SPECTRUM FOR EXAMPLE XIX

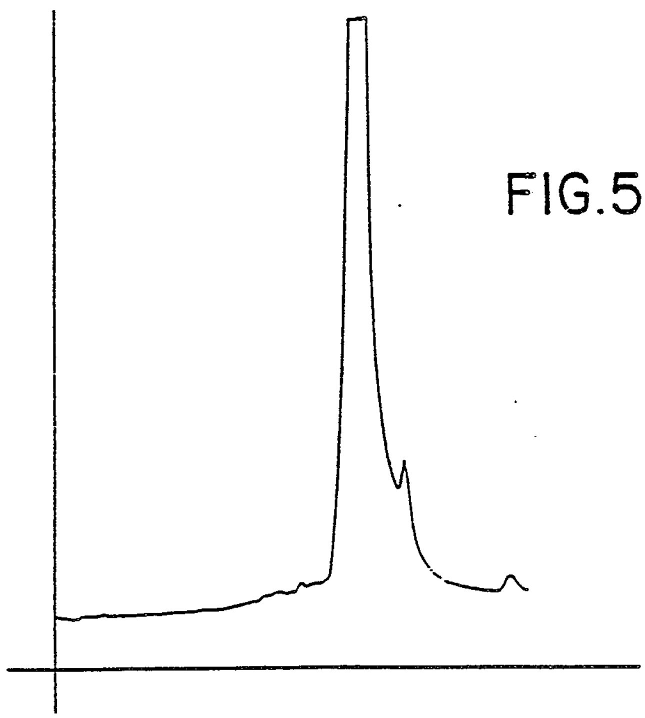

FIG.58

GLC PROFILE FOR FRACTION 4 OF EXAMPLE XX.

FIG. 59

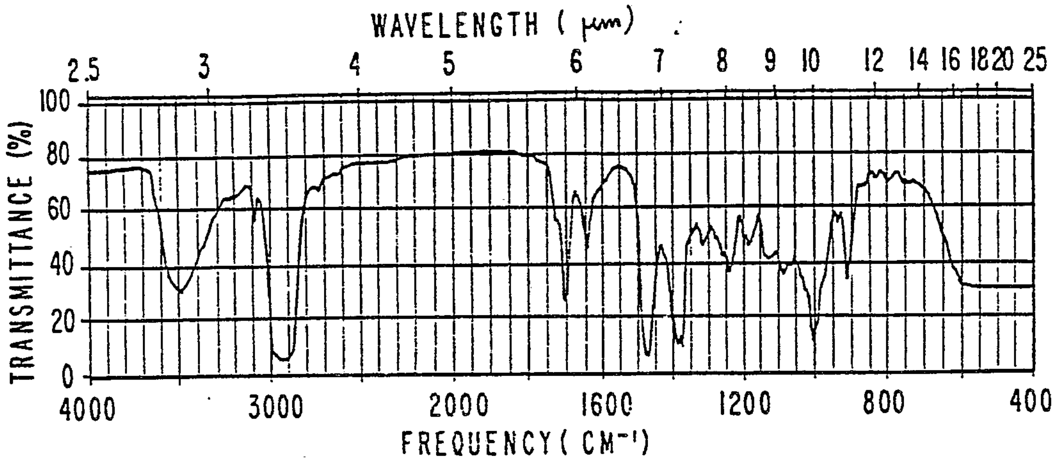


I R SPECTRUM FOR EXAMPLE XX, FRACTION 4

FIG.60

N M R SPECTRUM FOR FRACTION 4 OF EXAMPLE XX.